(19) Europäisches Patentamt
European Patent Office
Office européen des brevets

(11) **EP 2 155 788 B1**

(12) **EUROPEAN PATENT SPECIFICATION**

(45) Date of publication and mention
of the grant of the patent:
**27.06.2012 Bulletin 2012/26**

(51) Int Cl.:
*C07K 16/28* (2006.01)  *C07K 16/46* (2006.01)
*C07K 16/30* (2006.01)  *C07K 16/32* (2006.01)
*C07K 16/42* (2006.01)  *C07K 14/705* (2006.01)

(21) Application number: **08735000.5**

(22) Date of filing: **03.04.2008**

(86) International application number:
**PCT/EP2008/002663**

(87) International publication number:
**WO 2008/119566 (09.10.2008 Gazette 2008/41)**

(54) **CROSS-SPECIES-SPECIFIC BISPECIFIC BINDERS**

KREUZSPEZIESSPEZIFISCHE BISPEZIFISCHE BINDUNGSGLIEDER

ÉLÉMENTS DE LIAISON BISPÉCIFIQUES SPÉCIFIQUES D'ESPÈCES CROISÉES

(84) Designated Contracting States:
AT BE BG CH CY CZ DE DK EE ES FI FR GB GR
HR HU IE IS IT LI LT LU LV MC MT NL NO PL PT
RO SE SI SK TR
Designated Extension States:
AL BA MK RS

(30) Priority: 03.04.2007 EP 07006990
03.04.2007 EP 07006988
24.04.2007 US 913668 P
22.10.2007 EP 07020641
22.10.2007 EP 07020646
22.10.2007 EP 07020640
13.03.2008 EP 08004741

(43) Date of publication of application:
**24.02.2010 Bulletin 2010/08**

(73) Proprietor: **Micromet AG**
**81477 München (DE)**

(72) Inventors:
• **KLINGER, Matthias**
**82205 Gilching (DE)**
• **RAUM, Tobias**
**81025 München (DE)**
• **RAU, Doris**
**82008 Unterhaching (DE)**
• **MANGOLD, Susanne**
**81476 München (DE)**
• **KISCHEL, Roman**
**85757 Karlsfeld (DE)**

• **LUTTERBÜSE, Ralf**
**82061 Neuried (DE)**
• **HOFFMANN, Patrick**
**83670 Bad Heilbrunn (DE)**
• **KUFER, Peter**
**85368 Moosburg (DE)**

(74) Representative: **Schiweck, Weinzierl & Koch**
**European Patent Attorneys**
**Landsberger Straße 98**
**80339 München (DE)**

(56) References cited:
WO-A-99/01556      WO-A-03/087131
WO-A-2005/056606   WO-A-2005/061547
WO-A-2005/118635   WO-A-2007/033230
WO-A-2007/042261   US-A- 6 037 453
US-A1- 2002 173 629

• HAYASHI HIROKI ET AL: "A highly effective and stable bispecific diabody for cancer immunotherapy: cure of xenografted tumors by bispecific diabody and T-LAK cells" CANCER IMMUNOLOGY, IMMUNOTHERAPY : CII JUN 2004,, vol. 53, no. 6, 1 June 2004 (2004-06-01), pages 497-509, XP002499717
• KORNACKER M ET AL: "THE APOPTOTIC AND PROLIFERATIVE FATE OF CYTOKINE-INDUCED KILLER CELLS AFTER REDIRECTION TO TUMOR CELLS WITH BISPECIFIC AB" CYTOTHERAPY, ISIS MEDICAL MEDIA, OXFORD, vol. 8, no. 1, 1 February 2006 (2006-02-01), pages 13-23, XP008077923 ISSN: 1465-3249

**Description**

The present invention relates to a polypeptide comprising a first human binding domain which is an antibody capable of binding to an epitope of human and non-chimpanzee primate CD3 (epsilon) and a second binding domain capable of binding to EGFR, Her2/neu or IgE of a human and/or a non-chimpanzee primate as well as to a process for the production of the mentioned polypeptide. The invention further relates to nucleic acids encoding for the polypeptide, to vectors comprising the same and to host cells comprising the vector. In another aspect, the invention provides for a pharmaceutical composition comprising the mentioned polypeptide and medical uses of the polypeptide.

T cell recognition is mediated by clonotypically distributed alpha beta and gamma delta T cell receptors (TcR) that interact with the peptide-loaded molecules of the peptide MHC (pMHC) (Davis & Bjorkman, Nature 334 (1988), 395-402). The antigen-specific chains of the TcR do not possess signalling domains but instead are coupled to the conserved multisubunit signaling apparatus CD3 (Call, Cell 111 (2002), 967-979, Alarcon, Immunol. Rev. 191 (2003), 38-46, Malissen Immunol. Rev. 191 (2003), 7-27). The mechanism by which TcR ligation is directly communicated to the signalling apparatus remains a fundamental question in T cell biology (Alarcon, loc. cit.; Davis, Cell 110 (2002), 285-287). It seems clear that sustained T cell responses involve coreceptor engagement, TcR oligomerization, and a higher order arrangement of TcR-pMHC complexes in the immunological synapse (Davis & van der Merwe, Curr. Biol. 11 (2001), R289-R291, Davis, Nat. Immunol. 4 (2003), 217-224). However very early TcR signalling occurs in the absence of these events and may involve a ligand-induced conformational change in CD3 epsilon (Alarcon, loc. cit., Davis (2002), loc. cit., Gil, J. Biol. Chem. 276 (2001), 11174-11179, Gil, Cell 109 (2002), 901-912). The epsilon, gamma, delta and zeta subunits of the signaling complex associate with each other to form a CD3 epsilon-gamma heterodimer, a CD3 epsilon-delta. heterodimer, and a CD3 zeta-zeta homodimer (Call, loc. cit.). Various studies have revealed that the CD3 molecules are important for the proper cell surface expression of the alpha beta TcR and normal T cell development (Berkhout, J. Biol. Chem. 263 (1988), 8528-8536, Wang, J. Exp. Med. 188 (1998), 1375-1380, Kappes, Curr. Opin. Immunol. 7 (1995), 441-447). The solution structure of the ectodomain fragments of the mouse CD3 epsilon gamma heterodimer showed that the epsilon gamma subunits are both C2-set Ig domains that interact with each other to form an unusual side-to-side dimer configuration (Sun, Cell 105 (2001), 913-923). Although the cysteine-rich stalk appears to play an important role in driving CD3 dimerization (Su, loc. cit., Borroto, J. Biol. Chem. 273 (1998), 12807-12816), interaction by means of the extracellular domains of CD3 epsilon and CD3 gamma is sufficient for assembly of these proteins with TcR beta (Manolios, Eur. J. Immunol. 24 (1994), 84-92, Manolios & Li, Immunol. Cell Biol. 73 (1995), 532-536). Although still controversial, the dominant stoichiometry of the TcR most likely comprises one alpha beta TcR, one CD3 epsilon gamma heterodimer, one CD3 epsilon delta heterodimer and one CD3 zeta zeta homodimer (Call, loc. cit.). Given the central role of the human CD3 epsilon gamma heterodimer in the immune response, the crystal structure of this complex bound to the therapeutic antibody OKT3 has recently been elucidated (Kjer-Nielsen, PNAS 101, (2004), 7675-7680).

A number of therapeutic strategies modulate T cell immunity by targeting TcR signaling, particularly the anti-human CD3 monoclonal antibodies (mAbs) that are widely used clinically in immunosuppressive regimes. The CD3-specific mouse mAb OKT3 was the first mAb licensed for use in humans (Sgro, Toxicology 105 (1995), 23-29) and is widely used clinically as an immunosuppressive agent in transplantation (Chatenoud, Clin. Transplant 7 (1993), 422-430, Chatenoud, Nat. Rev. Immunol. 3 (2003), 123-132, Kumar, Transplant. Proc. 30 (1998), 1351-1352), type 1 diabetes (Chatenoud (2003), ioc. cit), and psoriasis (Utset, J. Rheumatol. 29 (2002), 1907-1913). Moreover, anti-CD3 mAbs can induce partial T cell signalling and clonal anergy (Smith, J. Exp. Med. 185 (1997), 1413-1422). OKT3 has been described in the literature as a potent T cell mitogen (Van Wauve, J. Immunol. 124 (1980), 2708-18) as well as a potent T cell killer (Wong, Transplantation 50 (1990), 683-9). OKT3 exhibits both of these activities in a time-dependent fashion; following early activation of T cells leading to cytokine release, upon further administration OKT3 later blocks all known T cell functions. It is due to this later blocking of T cell function that OKT3 has found such wide application as an immunosuppressant in therapy regimens for reduction or even abolition of allograft tissue rejection.

OKT3 reverses allograft tissue rejection most probably by blocking the function of all T cells, which play a major role in acute rejection. OKT3 reacts with and blocks the function of the CD3 complex in the membrane of human T cells, which is associated with the antigen recognition structure of T cells (TCR) and is essential for signal transduction. Which subunit of the TCR/CD3 is bound by OKT3 has been the subject of multiple studies. Though some evidence has pointed to a specificity of OKT3 for the epsilon-subunit of the TCR/CD3 complex (Tunnacliffe, Int. Immunol. 1 (1989), 546-50; Kjer-Nielsen, PNAS 101, (2004), 7675-7680). Further evidence has shown that OKT3 binding of the TCR/CD3 complex requires other subunits of this complex to be present (Salmeron, J. Immunol. 147 (1991), 3047-52).

Other well known antibodies specific for the CD3 molecule are listed in Tunnacliffe, Int. Immunol. 1 (1989), 546-50. As indicated above, such CD3 specific antibodies are able to induce various T cell responses such as lymphokine production (Von Wussow, J. Immunol. 127 (1981), 1197; Palacious, J. Immunol. 128 (1982), 337), proliferation (Van Wauve, J. Immunol. 124 (1980), 2708-18) and suppressor-T cell induction (Kunicka, in "Lymphocyte Typing II" 1 (1986), 223). That is, depending on the experimental conditions, CD3 specific monoclonal antibody can either inhibit or induce cytotoxicity (Leewenberg, J. Immunol. 134 (1985), 3770; Phillips, J. Immunol. 136 (1986) 1579; Platsoucas, Proc. Natl. Acad. Sci. USA 78 (1981), 4500; Itoh, Cell. Immunol. 108 (1987), 283-96; Mentzer, J. Immunol. 135 (1985), 34; Landegren, J. Exp. Med. 155 (1982), 1579; Choi (2001), Eur. J. Immunol. 31, 94-106; Xu (2000), Cell Immunol. 200, 16-26; Kimball (1995), Transpl. Immunol. 3, 212-221).

Although many of the CD3 antibodies described in the art have been reported to recognize the CD3 epsilon subunit of the CD3 complex, most of them bind in fact to conformational epitopes and, thus, only recognize CD3 epsilon in the native context of the TCR. Conformational epitopes are characterized by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The conformational epitopes bound by CD3 epsilon antibodies described in the art may be separated in two groups. In the major group, said epitopes are being formed by two CD3 subunits, e.g. of the CD3 epsilon chain and the CD3 gamma or

WO 2007/033230 discloses antibodies against the sequence EMGGITQTPYKVSISGT of CD3 which corresponds to residues 6-22 of human CD3epsilon.

CD3 delta chain. For example, it has been found in several studies that the most widely used CD3 epsilon monoclonal antibodies OKT3, WT31, UCHT1, 7D6 and Leu-4 did not bind to cells singly transfected with the CD3-epsilon chain. However, these antibodies stained cells doubly transfected with a combination of CD3 epsilon plus either CD3 gamma or CD3 delta (Tunnacliffe, loc. cit.; Law, Int. Immunol. 14 (2002), 389-400; Salmeron, J. Immunol. 147 (1991), 3047-52; Coulie, Eur. J. Immunol. 21 (1991), 1703-9). In a second smaller group, the conformational epitope is being formed within the CD3 epsilon subunit itself. A member of this group is for instance mAb APA 1/1 which has been raised against denatured CD3 epsilon (Risueno, Blood 106 (2005), 601-8). Taken together, most of the CD3 epsilon antibodies described in the art recognize conformational epitopes located on two or more subunits of CD3. The discrete amino acid residues forming the three-dimensional structure of these epitopes may hereby be located either on the CD3 epsilon subunit itself or on the CD3 epsilon subunit and other CD3 subunits such as CD3 gamma or CD3 delta.

Another problem with respect to CD3 antibodies is that many CD3 antibodies have been found to be species-specific. Anti-CD3 monoclonal antibodies - as holds generally true for any other monoclonal antibodies - function by way of highly specific recognition of their target molecules. They recognize only a single site, or epitope, on their target CD3 molecule. For example, one of the most widely used and best characterized monoclonal antibodies specific for the CD3 complex is OKT-3. This antibody reacts with chimpanzee CD3 but not with the CD3 homolog of other primates, such as macaques, or with dog CDR3 (Sandusky et al., J. Med. Primatol. 15 (1986), 441-451). The anti-CD3 monoclonal antibody UCHT-1 is also reactive with CD3 from chimpanzee but not with CD3 from macaques (own data). On the other hand, there are also examples of monoclonal antibodies, which recognize macaque antigens, but not their human counterparts. One example of this group is monoclonal antibody FN-18 directed to CD3 from macaques (Uda et al., J. Med. Primatol. 30 (2001), 141-147). Interestingly, it has been found that peripheral lymphocytes from about 12% of cynomolgus monkeys lacked reactivity with anti-rhesus monkey CD3 monoclonal antibody (FN-18) due to a polymorphism of the CD3 antigen in macaques. Uda et al. described a substitution of two amino acids in the CD3 sequence of cynomolgus monkeys, which are not reactive with FN-18 antibodies, as compared to CD3 derived from animals, which are reactive with FN-18 antibodies (Uda et al., J Med Primatol. 32 (2003), 105-10; Uda et al., J Med Primatol. 33 (2004), 34-7).

While this discriminatory ability, i.e. the species specificity, inherent to CD3 monoclonal antibodies and fragments thereof is a significant impediment to their development as therapeutic agents for the treatment of human diseases. In order to obtain market approval any new candidate medication must pass through rigorous testing. This testing can be subdivided into preclinical and clinical phases: Whereas the latter - further subdivided into the generally known clinical phases I, II and III - is performed in human patients, the former is performed in animals. The aim of preclinical testing is to prove that the drug candidate has the desired activity and most importantly is safe. Only when the safety in animals and possible effectiveness of the drug candidate has been established in preclinical testing this drug candidate will be approved for clinical testing in humans by the respective regulatory authority. Drug candidates can be tested for safety in animals in the following three ways, (i) in a relevant species, i.e. a species where the drug candidates can recognize the ortholog antigens, (ii) in a transgenic animal containing the human antigens and (iii) by use of a surrogate for the drug candidate that can bind the ortholog antigens present in the animal. Limitations of transgenic animals are that this technology is typically limited to rodents. Between rodents and man there are significant differences in the physiology and the safety results cannot be easily extrapolated to humans. The limitations of a surrogate for the drug candidate are the different composition of matter compared to the actual drug candidate and often the animals used are rodents with the limitation as discussed above. Therefore, preclinical data generated in rodents are of limited predictive power with respect to the drug candidate. The approach of choice for safety testing is the use of a relevant species, preferably a lower primate. The limitation now of the CD3 binding molecules suitable for therapeutic intervention in man described in the art is that the relevant species are higher primates, in particular chimpanzees. Chimpanzees are considered as endangered species and due to their human-like nature, the use of such animals for drug safety testing has been banned in Europe and is highly restricted elsewhere.

The present invention relates to a polypeptide comprising a first binding domain which is an antibody capable of binding to an epitope of human and Callithrix jacchus, Saguinus oedipus or Saimiri sciureus CD3ε chain, wherein the epitope is part of an amino acid sequence comprised in the group consisting of SEQ ID NO:2, 4, 6, or 8 and comprises at least the amino acid sequence Gln-Asp-Gly-Asn-Glu, and a second binding domain capable of binding to EGFR, Her2/neu or IgE of a human and/or a non-chimpanzee primate. Sequences as shown in SEQ ID NOs. 2, 4, 6 and 8 and fragments thereof are context independent CD3 epitopes.

The advantage of the present invention is the provision of a polypeptide comprising a, preferably human, binding domain which is an antibody exhibiting cross-species specificity to human and non-chimpanzee primate CD3ε (epsilon) chain, which can be used both for preclinical evaluation of safety, activity and/or pharmacokinetic profile of these, preferably human, binding domains in primates and - in the identical form - as drugs in humans. The same molecule can be used in preclinical animal studies as well as in clinical studies in humans. This leads to highly comparable results and a much-increased predictive power of the animal studies compared to species-specific surrogate molecules. In the present invention, an N-terminal 1-27 amino acid residue polypeptide fragment of the extracellular domain of CD3 epsilon was surprisingly identified which - in contrast to all other known epitopes of CD3 epsilon described in the art - maintains its three-dimensional structural integrity when taken out of its native environment in the CD3 complex (and fused to a heterologous amino acid sequence such as EpCAM or an immunoglobulin Fc part).

The context-independence of the CD3 epitope provided in this invention corresponds to the first 27 N-terminal amino acids of CD3 epsilon or functional fragments of this 27 amino acid stretch. The phrase "context-independent," as used herein in relation to the CD3 epitope means that binding of the herein described inventive binding molecules/ antibody molecules does not lead to a change or modification of the conformation, sequence, or structure surrounding the antigenic determinant or epitope. In contrast, the CD3 epitope recognized by a conventional CD3 binding molecule (e.g. as disclosed in WO 99/54440 or WO 04/106380) is localized on the CD3 epsilon chain C-terminal to the N-terminal 1-27 amino acids of the context-independent epitope, where it only takes the correct conformation if it is embedded within the rest of the epsilon chain and held in the right position by heterodimerization of the epsilon chain with either the CD3 gamma or delta chain.

Anti-CD3 binding molecules as part of a bispecific binding molecule as provided herein and generated (and directed) against a context-independent CD3 epitope provide for a surprising clinical improvement with regard to T cell redistribution and, thus, a more favourable safety profile. Without being bound by theory, since the CD3 epitope is context-independent, forming an autonomous selfsufficient subdomain without much influence on the rest of the CD3 complex, the CD3 binding molecules provided herein induce less allosteric changes in CD3 conformation than the conventional CD3 binding molecules (like molecules provided in WO 99/54440), which recognize context-dependent CD3 epitopes.

The context independence of the CD3 epitope of CD3 binding molecules of the invention as part of a bispecific binbing molecule is associated with less T cell redistribution during the starting phase of treatment with CD3 binding molecules of the invention resulting in a better safety profile of CD3 binding molecules of the invention compared to conventional CD3 binding molecules known in the art, which recognize context dependent CD3 epitopes. Particularly, because T cell redistribution during the starting phase of treatment with CD3 binding molecules is a major risk factor for CNS adverse events, the CD3 binding molecules of the invention by recognizing a context independent rather than a context dependent CD3 epitope have a substantial safety advantage over the CD3 binding molecules known in the art. Patients with such CNS adverse events related to T cell redistribution during the starting phase of treatment with conventional CD3 binding molecules usually suffer from confusion and disorientation, in some cases also from urinary incontinence. Confusion is a change in mental status in which the patient is not able to think with his or her usual level of clarity. The patient usually has difficulties to concentrate and thinking is not only blurred and unclear but often significantly slowed down. Patients with CNS adverse events related to T cell redistribution during the starting phase of treatment with conventional CD3 binding molecules may also suffer from loss of memory. Frequently, the confusion leads to the loss of ability to recognize people and/or places, or tell time and the date. Feelings of disorientation are common in confusion, and the decision-making ability is impaired. CNS adverse events related to T cell redistribution during the starting phase of treatment with conventional CD3 binding molecules may further comprise blurred speech and/or word finding difficulties. This disorder may impair both, the expression and understanding of language as well as reading and writing. Besides urinary incontinence, also vertigo and dizziness may accompany CNS adverse events related to T cell redistribution during the starting phase of treatment with conventional CD3 binding molecules in some patients.

The maintenance of the three-dimensional structure within the mentioned 27 amino acid N-terminal polypeptide fragment of CD3 epsilon can be used for the generation of, preferably human, binding domains which bind to the N-terminal CD3 epsilon polypeptide fragment *in vitro* and to the native (CD3 epsilon subunit of the) CD3 complex on T cells *in vivo* with the same binding affinity. These data strongly indicate that the N-terminal fragment as described herein forms a tertiary conformation, which is similar to its structure normally existing *in vivo*. A very sensitive test for the importance of the structural integrity of the amino acid 1-27 of the N-terminal polypeptide fragment of CD3 epsilon was performed. Individual amino acids of amino acids 1-27 of the N-terminal polypeptide fragment of CD3 epsilon were changed to alanine (alanine scanning) to test the sensitivity of the amino acids 1-27 of the N-terminal polypeptide fragment of CD3 epsilon for minor disruptions. CD3 specific antibody molecules as part of a bispecific binbing molecule of the invention were used to test for binding to the alanine-mutants of amino acids 1-27 of the N-terminal polypeptide fragment of CD3 epsilon (see appended Example 5). Individual exchanges of the first five amino acid residues at the very N-terminal end of the fragment and two of the amino acids at positions 23 and 25 of the amino acids 1-27 of the N-terminal polypeptide fragment of CD3 epsilon were critical for binding of the antibody molecules. The substitution of amino acid residues in the region of position 1-5 comprising the residues Q (Glutamine at position 1), D (Aspartic acid at position 2), G (Glycine at position 3), N (Asparagine at position 4), and E (Glutamic acid at position 5) to Alanine abolished binding of the, preferably human, binding molecules of the invention to said fragment. While, for at least some of the, preferably human, binding molecules of the invention, two amino acid residues at the C-terminus of the mentioned fragment T (Threonine at position 23) and I (Isoleucine at position 25) reduced the binding energy to the, preferably human, binding molecules of the invention.

Unexpectedly, it has been found that the thus isolated, preferably human, binding molecules not only recognize the human N-terminal fragment of CD3 epsilon, but also the corresponding homologous fragments of CD3 epsilon of various primates, including New-World Monkeys (Marmoset, Callithrix jacchus; Saguinus oedipus; Saimiri sciureus) and Old-World Monkeys (Macaca fascicularis, also known as Cynomolgus Monkey; or Macaca mulatta, also known as Rhesus Monkey). Thus, multi-primate specificity, of the CD3-binding molecules of the invention was detected. The following sequence analyses confirmed that human and primates share a highly homologous sequence stretch at the N-terminus of the extracellular domain of CD3 epsilon.

It has been found in the present invention as defined in the claims that it is possible to generate, preferably human, binding molecules specific for CD3 epsilon wherein the identical molecule can be used in preclinical animal testing, as well as clinical studies and even in therapy in human. This is due to the unexpected identification of, preferably human, binding molecules, which, in addition to binding to human CD3 epsilon (and due to genetic similarity likely to the chimpanzee counterpart), also bind to the homologs of said antigens of non-chimpanzee primates, including New-World Monkeys and Old-World Monkeys. As shown in the following Examples, said CD3 epsilon specific, preferably human, binding molecules can be integrated into bispecific single chain antibodies in order to generate therapeutics against various diseases, including but not limited to cancer or immunological disorders. Thus, the need to construct a surrogate CD3 epsilon binding domain or a bispecific single chain antibody including the same for testing in a phylogenetic distant (from humans) species disappears. As a result, the very same molecule can be used in animal preclinical testing as is intended to be administered to humans in clinical testing as well as following market approval and therapeutic drug administration. The ability to use the same molecule for preclinical animal testing as in later administration to humans virtually eliminates, or at least greatly reduces, the danger that the data obtained in preclinical animal testing have limited applicability to the human case. In short, obtaining preclinical safety data in animals using the same molecule as will actually be administered to humans does much to ensure the applicability of the data to a human-relevant scenario. In contrast, in conventional approaches using surrogate molecules, said surrogate molecules have to be molecularly adapted to the animal test system used for preclinical safety assessment. Thus, the molecule to be used in human therapy in fact differs in sequence and also likely in structure from the surrogate molecule used. in preclinical testing in pharmacokinetic parameters and/or biological activity, with the consequence that data obtained in preclinical animal testing have limited applicability / transferability to the human case. The use of surrogate molecules requires the construction, production, purification and characterization of a completely new construct. This leads to additional development costs and time necessary to obtain that molecule. In sum, surrogates have to be developed separately in addition to the actual drug to be used in human therapy, so that two lines of development for two molecules have to be carried out. Therefore, a major advantage of the human binding molecule or a antibody-based constructs exhibiting cross-species specificity described herein is that the identical molecule can be used for therapeutics in humans and in preclinical animal testing.

It is preferred for polypeptide of the invention that the first binding domain which is an antibody capable of binding to an epitope of the human and non-chimpanzee primate CD3 epsilon chain is of human origin.

In addition, due to the human origin of the human binding molecules of the invention the generation of an immune reaction against said binding molecules is excluded to the maximum possible extent upon administration of the binding molecules to human patients.

Another major advantage of the, preferably human, CD3 epsilon specific human binding molecules as part of a bispecific binding molecule of the invention is their applicability for preclinical testing in various primates. The behavior of a drug candidate in animals should ideally be indicative of the expected behavior of this drug candidate upon administration to humans. As a result, the data obtained.from such preclinical testing should therefore generally have a high predictive power for the human case. However, as learned from the tragic outcome of the recent Phase I clinical trial on TGN1412 (a CD28 monoclonal antibody), a drug candidate may act differently in a primate species than in humans: Whereas in preclinical testing of said antibody no or only limited adverse effects have been observed in animal studies performed with cynomolgus monkeys, six human patients developed multiple organ failure upon administration of said antibody (Lancet 368 (2006), 2206-7). The results of these not-desired negative events suggest that it may not be sufficient to limit preclinical testing to only one (primate) species. The fact that the CD3 epsilon specific human binding molecules of the invention bind to a series of New-World and Old-World Monkeys may help to overcome the problems faced in the case mentioned above. Accordingly, the present invention as defined in the claims provides means and methods for minimizing species differences in effects when drugs for human therapy are being developed and tested.

With the, preferably human, cross-species specific CD3 epsilon binding domain as part of a bispecific binbing molecule of the invention it is also no longer necessary to adapt the test animal to the drug candidate intended for administration to humans, such as e.g. the creation of transgenic animals. The CD3 epsilon specific, preferably human, binding molecules (or bispecific single chain antibodies containing the same), exhibiting cross-species specificity according to the uses and the methods of invention can be directly used for preclinical testing in non-chimpanzee primates, without any genetic manipulation of the animals. As well known to those skilled in the art, approaches in which the test animal is adapted to the drug candidate always bear the risk that the results obtained in the preclinical safety testing are less representative and predictive for humans due to the modification of the animal. For example, in transgenic animals, the proteins encoded by the transgenes are often highly over-expressed. Thus, data obtained for the biological activity of an antibody against this protein antigen may be limited in their predictive value for humans in which the protein is expressed at much lower, more physiological levels.

A further advantage of the uses of the CD3 epsilon specific, preferably human, binding molecules (or bispecific single chain antibodies containing the same) exhibiting cross-species specificity is the fact that chimpanzees as an endangered species are avoided for animal testing. Chimpanzees are the closest relatives to humans and were recently grouped into the family of hominids based on the genome sequencing data (Wildman et al., PNAS 100 (2003), 7181). Therefore, data obtained with chimpanzee is generally considered to be highly predictive for humans. However, due to their status as endangered species, the number of chimpanzees, which can be used for medical experiments, is highly restricted. As stated above, maintenance of chimpanzees for animal testing is therefore both costly and ethically problematic. The uses of CD3 epsilon specific, preferably human, binding molecules of the invention (or bispecific single chain antibodies containing the same) avoids both ethical objections and financial burden during preclinical testing without prejudicing the quality, i.e. applicability, of the animal testing data obtained. In light of this, the uses of CD3 epsilon specific, preferably human, binding molecules (or bispecific single chain antibodies containing the same) provides for a reasonable alternative for studies in chimpanzees.

A further advantage of the CD3 epsilon specific, preferably human, binding molecules of the invention (or bispecific single chain antibodies containing the same) is the ability of extracting multiple blood samples when using it as part of animal preclinical testing, for example in the course of pharmacokinetic animal studies. Multiple blood extractions can be much more readily obtained with a non-chimpanzee primate than with lower animals, e.g. a mouse. The extraction of multiple blood samples allows continuous testing of blood parameters for the determination of the biological effects induced by the, preferably human, binding molecules (or bispecific single chain antibody) of the invention. Furthermore, the extraction of multiple blood samples enables the researcher to evaluate the pharmacokinetic profile of the, preferably human, binding molecule (or bispecific single chain antibody) as defined herein. In addition, potential side effects, which may be induced by said, preferably human, binding molecule (or bispecific single chain antibody) reflected in blood parameters can be measured in different blood samples extracted during the course of the administration of said antibody. This allows the determination of the potential toxicity profile of the, preferably human, binding molecule (or bispecific single chain antibody) as defined herein.

The advantages of the preferably human, binding molecules (or Bispecific single chain antibodies) as defined herein exhibiting cross-species specificity may be briefly summarized as follows:

First, the, preferably human, binding molecules (or bispecific single chain antibodies) as defined herein used in preclinical testing is the same as the one used in human therapy. Thus, it is no longer necessary to develop two independent molecules, which may differ in their pharmacokinetic properties and biological activity. This is highly advantageous in that e.g. the pharmacokinetic results are more directly transferable and applicable to the human setting than e.g. in conventional surrogate approaches.

Second, the uses of the, preferably human, binding molecules (or bispecific single chain antibodies) as defined herein for the preparation of therapeutics in human is less cost- and labor-intensive than surrogate approaches.

Third, the, preferably human, binding molecules (or bispecific single chain antibodies) as defined herein can be used for preclinical testing not only in one primate species, but in a series of different primate species, thereby limiting the risk of potential species differences between primates and human.

Fourth, chimpanzee as an endangered species for animal testing is avoided.

Fifth, multiple blood samples can be extracted for extensive pharmacokinetic studies.

Sixth, due to the human origin of the, preferably human, binding molecules according to a preferred embodiment of the invention the generation of an immune reaction against said binding molecules is minimalized when administered to human patients. Induction of an immune response with antibodies specific for a drug candidate derived from a non-human species as e.g. a mouse leading to the development of human-anti-human antibodies (HAMAs) against therapeutic molecules of murine origin is excluded.

The term "protein" is well known in the art and describes biological compounds. Proteins comprise one or more amino acid chains (polypeptides), whereby the amino acids are bound among one another via a peptide bond. The term "polypeptide" as used herein describes a group of molecules, which consist of more than 30 amino acids. In accordance with the invention, the group of polypeptides comprises "proteins" as long as the proteins consist of a single polypeptide. Also in line with the definition the term "polypeptide" describes fragments of proteins as long as these fragments consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently termed homo- or heterodimers, homo- or heterotrimers etc. An example for a heteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art.

As used herein, "human" and "man" refers to the species *Homo sapiens.* As far as the medical uses of the constructs described herein are concerned, human patients are to be treated with the same molecule.

The term "human origin" as used in the context with the molecules of the invention describes molecules derivable from human libraries or having a structure/sequence corresponding to the human equivalent. Accordingly, proteins having an amino acid sequence corresponding to the analog human sequence, e.g. an antibody fragment having an amino acid sequences in the framework corresponding to the human germ line sequences, are understood as molecules of human origin.

As used herein, a "non-chimpanzee primate" or "non-chimp primate" or grammatical variants thereof refers to any primate other than chimpanzee, i.e. other than an animal of belonging to the genus *Pan,* and including the species *Pan paniscus* and *Pan troglodytes,* also known as *Anthropopithecus troglodytes* or *Simia satyrus.* A "primate", "primate species", "primates" or grammatical variants thereof denote/s an order of eutherian mammals divided into the two suborders of prosimians and anthropoids and comprising man, apes, monkeys and lemurs. Specifically, "primates" as used herein comprises the suborder *Strepsirrhini* (non-tarsier prosimians), including the infraorder *Lemuriformes* (itself including the superfamilies Cheirogoleidae and *Lemuroidge*), the infraorder *Chiromyiformes* (itself including the family *Daubentoniidae*) and the infraorder *Lorisiformes* (itself including the families *Lorisidae* and *Galagidae*). "Primates" as used herein also comprises the suborder *Haplorrhini,* including the infraorder *Tarsiiformes* (itself including the family *Tarsiidae*), the infraorder *Simiiformes* (itself including the *Platyrrhini,* or New-World monkeys, and the *Catarrhini,* including the *Cercopithecidae,* or Old-World Monkeys).

The non-chimpanzee primate species may be understood within the meaning of the invention to be a lemur, a tarsier, a gibbon, a marmoset (belonging to New-World Monkeys of the family *Cebidae*) or an Old-World Monkey (belonging to the superfamily *Cercopithecoidea*).

As used herein, an "Old-World Monkey" comprises any monkey falling in the superfamily *Cercopithecoidea,* itself subdivided into the families: the *Cercopithecinae,* which are mainly African but include the diverse genus of macaques which are Asian and North African; and the *Colobinae,* which include most of the Asian genera but also the African colobus monkeys.

Specifically, within the subfamily *Cercopithecinae,* an advantageous non-chimpanzee primate may be from the Tribe *Cercopithecini,* within the genus *Allenopithecus* (Allen's Swamp Monkey, *Allenopithecus nigroviridis*); within the genus *Miopithecus* (Angolan Talapoin, *Miopithecus talapoin*; Gabon Talapoin, *Miopithecus ogouensis*); within the genus *Erythrocebus* (Patas Monkey, *Erythrocebus patas*); within the genus *Chlorocebus* (Green Monkey, *Chlorocebus sabaceus*; Grivet, *Chlorocebus aethiops*; Bale Mountains Vervet, *Chlorocebus djamdjamensis*; Tantalus Monkey, *Chlorocebus tantalus*; Vervet Monkey, *Chlorocebus pygerythrus*; Malbrouck, *Chlorocebus cynosuros*); or within the genus *Cercopithecus* (Dryas Monkey or Salongo Monkey, *Cercopithecus dryas*; Diana Monkey, *Cercopithecus diana*; Roloway Monkey, *Cercopithecus roloway*; Greater Spot-nosed Monkey, *Cercopithecus nictitans*; Blue Monkey, *Cercopithecus mitis*; Silver Monkey, *Cercopithecus doggetti*; Golden Monkey, *Cercopithecus kandti*; Sykes's Monkey, *Cercopithecus albogularis*; Mona Monkey, *Cercopithecus mona*; Campbell's Mona Monkey, *Cercopithecus campbelli*; Lowe's Mona Monkey, *Cercopithecus lowei*; Crested Mona Monkey, *Cercopithecus pogonias*; Wolfs Mona Monkey, *Cercopithecus wolfi*; Dent's Mona Monkey, *Cercopithecus denti*; Lesser Spot-nosed Monkey, *Cercopithecus petaurista*; White-throated Guenon, *Cercopithecus erythrogaster,* Sclater's Guenon, *Cercopithecus sclateri*; Red-eared Guenon, *Cercopithecus erythrotis*; Moustached Guenon, *Cercopithecus cephus*; Red-tailed Monkey, *Cercopithecus ascanius*; L'Hoest's Monkey, *Cercopithecus lhoesti*; Preuss's Monkey, *Cercopithecus preussi*; Sun-tailed Monkey, *Cercopithecus solatus*; Hamlyn's Monkey or Owl-faced Monkey, *Cercopithecus hamlyni*; De Brazza's Monkey, *Cercopithecus neglectus*).

Alternatively, an advantageous non-chimpanzee primate, also within the subfamily *Cercopithecinae* but within the Tribe *Papionini*, may be from within the genus *Macaca* (Barbary Macaque, *Macaca sylvanus*; Lion-tailed Macaque, *Macaca silenus;* Southern Pig-tailed Macaque or Beruk, *Macaca nemestrina*; Northern Pig-tailed Macaque, *Macaca leonina*; Pagai Island Macaque or Bokkoi, *Macaca pagensis*; Siberut Macaque, *Macaca siberu*; Moor Macaque, *Macaca maura*; Booted Macaque, *Macaca ochreata*; Tonkean Macaque, *Macaca tonkeana*; Heck's Macaque, *Macaca hecki;* Gorontalo Macaque, *Macaca nigriscens*; Celebes Crested Macaque or Black "Ape", *Macaca nigra*; Cynomolgus monkey or Crab-eating Macaque or Long-tailed Macaque or Kera, *Macaca fascicularis*; Stump-tailed Macaque or Bear Macaque, *Macaca arctoides*; Rhesus Macaque, *Macaca mulatta;* Formosan Rock Macaque, *Macaca cyclopis*; Japanese Macaque, *Macaca fuscata*; Toque Macaque, *Macaca sinica*; Bonnet Macaque, *Macaca radiata*; Barbary Macaque, *Macaca sylvanus*; Assam Macaque, *Macaca assamensis*; Tibetan Macaque or Milne-Edwards' Macaque, *Macaca thibetana*; Arunachal Macaque or Munzala, *Macaca munzala*); within the genus *Lophocebus* (Gray-cheeked Mangabey, *Lophocebus albigena*; *Lophocebus albigena albigena; Lophocebus albigena osmani; Lophocebus albigena johnstoni*; Black Crested Mangabey, *Lophocebus aterrimus*; Opdenbosch's Mangabey, *Lophocebus opdenboschi*; Highland Mangabey, *Lophocebus kipunji*); within the genus *Papio* (Hamadryas Baboon, *Papio hamadryas*; Guinea Baboon, *Papio papio*; Olive Baboon, *Papio anubis*; Yellow Baboon, *Papio cynocephalus*; Chacma Baboon, *Papio ursinus*); within the genus *Theropithecus* (Gelada, *Theropithecus gelada*); within the genus *Cercocebus* (Sooty Mangabey, *Cercocebus atys*; *Cercocebus atys atys; Cercocebus. atys lunulatus;* Collared Mangabey, *Cercocebus torquatus*; Agile Mangabey, *Cercocebus agilis*; Golden-bellied Mangabey, *Cercocebus chrysogaster,* Tana River Mangabey, *Cercocebus galeritus*; Sanje Mangabey, *Cercocebus sanjei*); or within the genus *Mandrillus* (Mandrill, *Mandrillus sphinx*; Drill, *Mandrillus leucophaeus*).

Most preferred is *Macaca fascicularis* (also known as Cynomolgus monkey and, therefore, in the Examples named "Cynomolgus") and *Macaca mulatta* (rhesus monkey, named "rhesus").

Within the subfamily *Colobinae,* an advantageous non-chimpanzee primate may be from the African group, within the genus *Colobus* (Black Colobus, *Colobus satanas*; Angola Colobus, *Colobus angolensis*; King Colobus, *Colobus polykomos*; Ursine Colobus, *Colobus vellerosus*; Mantled Guereza, *Colobus guereza*); within the genus *Piliocolobus* (Western Red Colobus, *Piliocolobus badius*; *Piliocolobus badius badius*; *Piliocolobus badius temminckii*; *Piliocolobus badius waldronae*; Pennant's Colobus, *Piliocolobus pennantii*; *Piliocolobus pennantii pennantii; Piliocolobus pennantii epieni*; *Piliocolobus pennantii bouvieri*; Preuss's Red Colobus, *Piliocolobus preussi*; Thollon's Red Colobus, *Piliocolobus tholioni*, Central African Red Colobus, *Piliocolobus foai*; *Piliocolobus foai foai*; *Piliocolobus foai elliott, Piliocolobus foai oustaleti*; *Piliocolobus foai semlikiensis*; *Piliocolobus foai parmentierorum*; Ugandan Red Colobus, *Piliocolobus tephrosceles*; Uzyngwa Red Colobus, *Piliocolobus gordonorum*; Zanzibar Red Colobus, *Piliocolobus kirkii*; Tana River Red Colobus, *Piliocolobus rufomitratus*); or within the genus *Procolobus* (Olive Colobus, *Procolobus verus*). Within the subfamily *Colobinae*, an advantageous non-chimpanzee primate may alternatively be from the Langur (leaf monkey) group, within the genus *Semnopithecus* (Nepal Gray Langur, *Semnopithecus schistaceus*; Kashmir Gray Langur, *Semnopithecus ajax*; Tarai Gray Langur, *Semnopithecus hector*, Northern Plains Gray Langur, *Semnopithecus entellus*; Black-footed Gray Langur, *Semnopithecus hypoleucos*; Southern Plains Gray Langur, *Semnopithecus dussumieri*; Tufted Gray Langur, *Semnopithecus priam*); within the *T. vetulus* group or the genus *Trachypithecus* (Purple-faced Langur, *Trachypithecus vetulus*; Nilgiri Langur, *Trachypithecus johnii*); within the *T. cristatus* group of the genus *Trachypithecus* (Javan Lutung, *Trachypithecus auratus*; Silvery Leaf Monkey or Silvery Lutung, *Trachypithecus cristatus*; Indochinese Lutung, *Trachypithecus germaini*; Tenasserim Lutung, *Trachypithecus barbel*); within the *T. obscurus* group of the genus *Trachypithecus* (Dusky Leaf Monkey or Spectacled Leaf Monkey, *Trachypithecus obscurus*; Phayre's Leaf Monkey, *Trachypithecus phayrei*); within the *T. pileatus* group of the genus *Trachypithecus* (Capped Langur, *Trachypithecus pileatus*; Shortridge's Langur, *Trachypithecus shortridgei*; Gee's Golden Langur, *Trachypithecus gee,*); within the *T. francoisi* group of the genus *Trachypithecus* (Francois' Langur, *Trachypithecus francoisi*; Hatinh Langur, *Trachypithecus hatinhensis*; White-headed Langur, *Trachypithecus potiocephalus;* Laotian Langur, *Trachypithecus laotum*; Delacour's Langur, *Trachypithecus delacouri*; Indochinese Black Langur, *Trachypithecus ebenus*); or within the genus *Presbytis* (Sumatran Surili, *Presbytis melalophos*; Banded Surili, *Presbytis femoralis*; Sarawak Surili, *Presbytis chrysomelas*; White-thighed Surili, *Presbytis siamensis*; White-fronted Surili, *Presbytis frontata*; Javan Surili, *Presbytis comata*; Thomas's Langur, *Presbytis thomasi*; Hose's Langur, *Presbytis hosei*; Maroon Leaf Monkey, *Presbytis rubicunda*; Mentawai Langur or Joja, *Presbytis potenziani*; Natuna Island Surili, *Presbytis natunae*).

Within the subfamily *Colobinae,* an advantageous non-chimpanzee primate may alternatively be from the Odd-Nosed group, within the genus *Pygathrix* (Red-shanked Douc, *Pygathrix nemaeus*; Black-shanked Douc, *Pygathrix nigripes*; Gray-shanked Douc, *Pygathrix cinerea*); within the genus *Rhinopithecus* (Golden Snub-nosed Monkey, *Rhinopithecus roxellana;* Black Snub-nosed Monkey, *Rhinopithecus bieti*; Gray Snub-nosed Monkey, *Rhinopithecus brelicht,* Tonkin Snub-nosed Langur, *Rhinopithecus avunculus*); within the genus *Nasalis* (Proboscis Monkey, *Nasalis larvatus*); or within the genus *Simias* (Pig-tailed Langur, *Simias concolor*).

As used herein, the term "marmoset" denotes any New-World Monkeys of the genus *Callithrix,* for example belonging to the Atlantic marmosets of subgenus *Callithrix* (sic!) (Common Marmoset, *Callithrix (Callithrix) jacchus*; Black-tufted Marmoset, *Callithrix (Callithrix) penicillata*; Wied's Marmoset, *Callithrix (Callithrix) kuhlii*; White-headed Marmoset, *Callithrix (Callithrix) geoffroyi*; Buffy-headed Marmoset, *Callithrix (Callithrix) flaviceps*; Buffy-tufted Marmoset, *Callithrix (Callithrix) aurita)*; belonging to the Amazonian marmosets of subgenus *Mico* (Rio Acari Marmoset, *Callithrix (Mico) acariensis*; Manicore Marmoset, *Callithrix (Mico) manicorensis*; Silvery Marmoset, *Callithrix (Mico) argentata*; White Marmoset, *Callithrix (Mico) leucippe*; Emilia's Marmoset, *Callithrix (Mico) emiliae*; Black-headed Marmoset, *Callithrix (Mico) nigriceps*; Marca's Marmoset, *Callithrix (Mico)marcai*; Black-tailed Marmoset, *Callithrix (Mico) melanura*; Santarem Marmoset, *Callithrix (Mico) humeralifera*; Maués Marmoset, *Callithrix (Mico) mauesi*; Gold-and-white Marmoset, *Callithrix (Mico) chrysoleuca*; Hershkovitz's Marmoset, *Callithrix (Mico) intermedia*; Satéré Marmoset, *Callithrix (Mico) sateret)*; Roosmalens' Dwarf Marmoset belonging to the subgenus Callibella (*Callithrix (Callibelia) humilis*); or the Pygmy Marmoset belonging to the subgenus. Cebuella *(Callithrix (Cebuella) pygmaea)*.

Other genera of the New-World Monkeys comprise tamarins of the genus *Saguinus* (comprising the *S. oedipus-group,* the *S. midas* group, the *S. nigricollis* group, the *S. mystax* group, the *S. bicolor* group and the *S. inustus* group) and squirrel monkeys of the genus Saimiri (e.g. *Saimiri sciureus, Saimiri oerstedii, Saimiri ustus, Saimiri boliviensis, Saimiri vanzolini*)

The term "binding domain" characterizes in connection with the present invention a domain of a polypeptide which specifically binds/interacts with a given target structure/antigen/epitope. Thus, the binding domain is an "antigen-interaction-site". The term "antigen-interaction-site" defines, in accordance with the present invention, a motif of a polypeptide, which is able to specifically interact with a specific antigen or a specific group of antigens, e.g. the identical antigen in different species. Said binding/interaction is also understood to define a "specific recognition". The term "specifically recognizing" means in accordance with this invention that the antibody molecule is capable of specifically interacting with and/or binding to at least two, preferably at least three, more preferably at least four amino acids of an antigen, e.g. the human CD3 antigen as defined herein. Such binding may be exemplified by the specificity of a "lock-and-key-principle". Thus, specific motifs in the amino acid sequence of the binding domain and the antigen bind to each other as a result of their primary, secondary or tertiary structure as well as the result of secondary modifications of said structure. The specific interaction of the antigen-interaction-site with its specific antigen may result as well in a simple binding of said site to the antigen. Moreover, the specific interaction of the antigen-interaction-site with its specific antigen may alternatively result in the initiation of a signal, e.g. due to the induction of a change of the conformation of the antigen, an oligomerization of the antigen, etc. A preferred example of a binding- domain in line with the present invention is an antibody. The binding domain may be a monoclonal or polyclonal antibody or derived from a monoclonal or polyclonal antibody.

The term "antibody" comprises derivatives or functional fragments thereof which still retain the binding specificity. Techniques for the production of antibodies are well known in the art and described, e.g. in Harlow and Lane "Antibodies, A Laboratory Manual", Cold Spring Harbor Laboratory Press, 1988 and Harlow and Lane "Using Antibodies: A Laboratory Manual" Cold Spring Harbor Laboratory Press, 1999. The term "antibody" also comprises immunogiobulins (ig's) of different classes (i.e. IgA, IgG, IgM, IgD and IgE) and subclasses (such as IgG1, IgG2 etc.). These antibodies can be used, for example, for the immunoprecipitation, affinity purification and immunolocalization of the polypeptides or fusion proteins of the invention as well as for the monitoring of the presence and amount of such polypeptides, for example, in cultures of recombinant prokaryotes or eukaryotic cells or organisms.

The definition of the term "antibody" also includes embodiments such as chimeric, single chain and humanized antibodies, as well as antibody fragments, like, inter alia, Fab fragments. Antibody fragments or derivatives further comprise F(ab')$_2$, Fv, scFv fragments or single domain antibodies, single variable domain antibodies or immunoglobulin single variable domain comprising merely one variable domain, which might be VH or VL, that specifically bind an antigen or epitope independently of other V regions or domains; see, for example, Harlow and Lane (1988) and (1999), loc. cit. Such immunoglobulin single variable domain encompasses not only an isolated antibody single variable domain polypeptide, but also larger polypeptides that comprise one or more monomers of an antibody single variable domain polypeptide sequence.

Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies specific for elected polypeptide(s). Also, transgenic animals may be used to express humanized antibodies specific for polypeptides and fusion proteins of this invention. For the preparation of monoclonal antibodies, any technique, providing antibodies produced by continuous cell line cultures can be used. Examples for such techniques include the hybridoma technique (Köhler and Milstein Nature 256 (1975), 495-497), the trioma technique, the human B-cell hybridoma technique (Kozbor, Immunology Today 4 (1983), 72) and the EBV-hybridoma technique to produce human monoclonal antibodies (Cole et al., Monoclonal Antibodies and Cancer Therapy, Alan R. Liss, Inc. (1985), 77-96). Surface plasmon resonance as employed in the BIAcore system can be used to increase the efficiency of phage antibodies which bind to an epitope of a target polypeptide, such as CD3 epsilon (Schier, Human Antibodies Hybridomas 7 (1996), 97-105; Malmborg, J. Immunol. Methods 183 (1995), 7-13). It is also envisaged in the context of this invention that the term "antibody" comprises antibody constructs, which may be expressed in a host as described herein below, e.g. antibody constructs which may be transfected and/or transduced via, inter alia, viruses or plasmid vectors.

The term "specific interaction" as used in accordance with the present invention means that the binding (domain) molecule does not or does not significantly cross-react with polypeptides which have similar structure as those bound by the binding molecule, and which might be expressed by the same cells as the polypeptide of interest. Cross-reactivity of a panel of binding molecules under investigation may be tested, for example, by assessing binding of said panel of binding molecules under conventional conditions (see, e.g., Harlow and Lane, Antibodies: A Laboratory Manual, Cold Spring Harbor Laboratory Press, 1988 and Using Antibodies: A Laboratory Manual, Cold Spring Harbor Laboratory Press, 1999). Examples for the specific interaction of a binding domain with a specific antigen comprise the specificity of a ligand for its receptor. Said definition particularly comprises the interaction of ligands, which induce a signal upon binding to its specific receptor. Examples for said interaction, which is also particularly comprised by said definition, is the interaction of an antigenic determinant (epitope) with the binding domain (antigenic binding site) of an antibody.

The term "cross-species specificity" or "interspecies specificity" as used herein means binding of a binding domain described herein to the same target molecule in humans and non-chimpanzee primates. Thus, "cross-species specificity" or "interspecies specificity" is to be understood as an interspecies reactivity to the same molecule X expressed in different species, but not to a molecule other than X. Cross-species specificity of a monoclonal antibody recognizing e.g. human CD3 epsilon, to a non-chimpanzee primate CD3 epsilon, e.g. macaque CD3 epsilon, can be determined, for instance, by FACS analysis. The FACS analysis is carried out in a way that the respective monoclonal antibody is tested for binding to human and non-chimpanzee primate cells, e.g. macaque cells, expressing said human and non-chimpanzee primate CD3 epsilon antigens, respectively. An appropriate assay is shown in the following examples.

As used herein, CD3 epsilon denotes a molecule expressed as part of the T cell receptor and has the meaning as typically ascribed to it in the prior art. In human, it encompasses in individual or independently combined form all known CD3 subunits, for example CD3 epsilon, CD3 delta, CD3 gamma, CD3 zeta, CD3 alpha and CD3 beta. The non-chimpanzee primate CD3 antigens as referred to herein are, for example, *Macaca fascicularis* CD3 and *Macaca mulatta* CD3. In *Macaca fascicularis,* it encompasses CD3 epsilon FN-18 negative and CD3 epsilon FN-18 positive, CD3 gamma and CD3 delta. In *Macaca mulatta,* it encompasses CD3 epsilon, CD3 gamma and CD3 delta. Preferably, said CD3 as used herein is CD3 epsilon.

The human CD3 epsilon is indicated in GenBank Accession No.NM_000733 and comprises SEQ ID NO. 1. The human CD3 gamma is indicated in GenBank Accession NO. NM_000073. The human CD3 delta is indicated in GenBank Accession No. NM_000732.

The CD3 epsilon "FN-18 negative" of *Macaca fascicularis* (i.e. CD3 epsilon not recognized by monoclonal antibody FN-18 due to a polymorphism as set forth above) is indicated in GenBank Accession No. AB073994.

The CD3 epsilon "FN-18 positive" of *Macaca fascicularis* (i.e. CD3 epsilon recognized by monoclonal antibody FN-18) is indicated in GenBank Accession No. AB073993. The CD3 gamma of *Macaca fascicularis* is indicated in GenBank Accession No. AB073992. The CD3 delta of *Macaca fascicularis* is indicated in GenBank Accession No. AB073991.

The nucleic acid sequences and amino acid sequences of the respective CD3 epsilon, gamma and delta homologs of *Macaca mulatta* can be identified and isolated by recombinant techniques described in the art (Sambrook et al. Molecular Cloning: A Laboratory Manual; Cold Spring Harbor Laboratory Press, 3 rd edition 2001). This applies mutatis mutandis to the CD3 epsilon, gamma and delta homologs of other non-chimpanzee primates as defined herein. The identification of the amino acid sequence of Callithrix jacchus, Saimiri sciureus und Saguinus oedipus is described in the appended examples. The amino acid sequence of the extracellular domain of the CD3 epsilon of *Callithrix jacchus* is depicted in SEQ ID NO: 3, the one of *Saguinus oedipus* is depicted in SEQ ID NO: 5 and the one of *Saimiri sciureus* is depicted in SEQ ID NO: 7.

In line with the above the term "epitope" defines an antigenic determinant, which is specifically bound/identified by a binding moiecuie as defined above. The binding domain or molecules may specifically bind to/interact with conformational or continuous epitopes, which are unique for the target structure, e.g. the human and non-chimpanzee primate CD3 epsilon chain. A conformational or discontinuous epitope is characterized for polypeptide antigens by the presence of two or more discrete amino acid residues which are separated in the primary sequence, but come together on the surface of the molecule when the polypeptide folds into the native protein/antigen (Sela, (1969) Science 166, 1365 and Laver, (1990) Cell 61, 553-6). The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more discrete amino acid residues, which are present in a single linear segment of a polypeptide chain. Within the present invention, a "context-dependent" CD3 epitope refers to the conformation of said epitope. Such a context-dependent epitope, localized on the epsilon chain of CD3, can only develop its correct conformation if it is embedded within the rest of the epsilon chain and held in the right position by heterodimerization of the epsilon chain with either CD3 gamma or delta chain. In contrast, a context-independent CD3 epitope as provided herein refers to an N-terminal 1-27 amino acid residue polypeptide or a functional fragment thereof of CD3 epsilon. This N-terminal 1-27 amino acid residue polypeptide or a functional fragment thereof maintains its three-dimensional structural integrity and correct conformation when taken out of its native environment in the CD3 complex.The context- independency of the N-terminal 1-27 amino acid residue polypeptide or a functional fragment thereof, which is part of the extracellular domain of CD3 epsilon, represents, thus, an epitope which is completely different to the epitopes of CD3 epsilon described in connection with a method for the preparation of human binding molecules in WO 2004/106380. Said method used solely expressed recombinant CD3 epsilon. The conformation of this solely expressed recombinant CD3 epsilon differed from that adopted in its natural form, that is, the form in which the CD3-epsilon subunit of the TCR/CD3 complex exists as part of a noncovalent complex with either the CD3-delta or the CD3-gamma subunit of the TCR/CD3 complex. When such solely expressed recombinant CD3-epsilon protein is used as an antigen for selection of antibodies from an antibody library, antibodies specific for this antigen are identified from the library although such a library does not contain antibodies with specificity for self-antigens/autoantigens. This is due to the fact that solely expressed recombinant CD3-epsilon protein does not exist in vivo; it is not an autoantigen. Consequently, subpopulations of B cells expressing antibodies specific for this protein have not been depleted in vivo; an antibody library constructed from such B cells would contain genetic material for antibodies specific for solely expressed recombinant CD3-epsilon protein.

However, since the context-independent N-terminal 1-27 amino acid residue polypeptide or a functional fragment thereof is an epitope, which folds in its native form, binding domains in line with the present invention cannot be identified by methods based on the approach described in WO 04/106380. Therefore, it could be verified in tests that binding molecules as disclosed in WO 04/106380 are not capable of binding to the N-terminal 1-27 amino acid residues of the CD3 epsilon chain. Hence, conventional anti-CD3 binding molecules or anti-CD3 antibody molecules (e.g. as disclosed in WO 99/54440) bind CD3 epsilon chain at a position which is more C-terminally located than the context-independent N-terminal 1-27 amino acid residue polypeptide or a functional fragment provided herein . Prior art antibody molecules OKT3 and UCHT-1 have also a specificity for the epsilon-subunit of the TCR/CD3 complex between amino acid residues 35 to 85 and, accordingly, the epitope of these antibodies is also more C-terminally located. In addition, UCHT-1 binds to the CD3 epsilon chain in a region between amino acid residues 43 to 77 (Tunnacliffe, Int. Immunol. 1 (1989), 546-50; Kjer-Nielsen, PNAS 101, (2004), 7675-7680; Salmeron, J. Immunol. 147 (1991), 3047-52). Therefore, prior art anti-CD3 molecules do not bind to and are not directed against the herin defined context-independent N-terminal 1-27 amino acid residue epitope (or a functional fragment thereof).

For the generation of a, preferably human, binding domain comprised in a polypeptide of the invention, e.g. in a bispecific single chain antibody as defined herein, e.g. monoclonal antibodies binding to both the human and non-chimpanzee primate CD3 epsilon (e.g. macaque CD3 epsilon) can be used.

In a preferred embodiment of the polypeptide of the invention, the non-chimpanzee primate is an old world monkey. In a more preferred embodiment of the polypeptide, the old world monkey is a monkey of the Papio genus Macaque genus. Most preferably, the monkey of the Macaque genus is Assamese macaque (*Macaca assamensis*), Barbary macaque (*Macaca sylvanus*), Bonnet macaques (*Macaca radiata*), Booted or Sulawesi-Booted macaque (*Macaca ochreata*), Sulawesi-crested macaque (*Macaca nigra*), Formosan rock macaque (*Macaca cyclopsis*), Japanese snow macaque or Japanese macaque (*Macaca fuscata*), Cynomologus monkey or crab-eating macaque or long-tailed macaque or Java macaque (*Macaca fascicularis*), Lion-tailed macaque (*Macaca silenus*), Pigtailed macaque (*Macaca nemestrina*), Rhesus macaque (*Macaca mulatta*), Tibetan macaque (*Macaca thibetana*), Tonkean macaque (*Macaca tonkeana*), Toque macaque (*Macaca sinica*), Stump-tailed macaque or Red-faced macaque or Bear monkey (*Macaca arctoides*), or Moor macaque (*Macaca maurus*). Most preferably, the monkey of the Papio genus is *Hamadryas Baboon, Papio hamadryas*; *Guinea Baboon, Papio papio; Olive Baboon, Papio anubis; Yellow Baboon, Papio cynocephalus; Chacma Baboon, Papio ursinus*.

In an alternatively preferred embodiment of the polypeptide of the invention, the non-chimpanzee primate is a new world monkey. In a more preferred embodiment of the polypeptide, the new world monkey is a monkey of the Callithrix genus (marmoset), the Saguinus genus or the Saimiri genus. Most preferably, the monkey of the Callithrix genus is *Callithrix jacchus,* the monkey of the Saguinus genus is *Saguinus oedipus* and the monkey of the Saimiri genus is *Saimiri sciureus.*

As described herein above the polypeptide of the invention binds with the first binding domain to an epitope of human and non-chimpanzee primate CD3ε (epsilon) chain, wherein the epitope is part of an amino acid sequence comprised in the group consisting of 27 amino acid residues as depicted in SEQ ID NOs. 2, 4, 6, or 8.

In line with the present invention it is preferred for the polypeptide of the invention that said epitope is part of an amino acid sequence comprising 26, 25, 24, 23, 22, 21, 20, 19, 18, 17, 16, 15, 14, 13, 12, 11, 10, 9, 8, 7, 6 or 5 amino acids.

More preferably, wherein said epitope comprises at least the amino acid sequence Gln-Asp-Gly-Asn-Glu (Q-D-G-N-E-D).

Within the present invention, a "functional fragment of the N-terminal 1-27 amino acid residues" means that said functional fragment is still a context-independent epitope maintaining its three-dimensional structural integrity when taken out of its native environment in the CD3 complex (and fused to a heterologous amino acid sequence such as EpCAM or an immunoglobulin Fc part, e.g. as shown in Example 3.1). The maintenance of the three-dimensional structure within the 27 amino acid N-terminal polypeptide or functional fragment thereof of CD3 epsilon can be used for the generation of binding domains which bind to the N-terminal CD3 epsilon polypeptide fragment *in vitro* and to the native (CD3 epsilon subunit of the) CD3 complex on T cells *in vivo* with the same binding affinity. Within the present invention, a functional fragment of the N-terminal 1-27 amino acid residues means that CD3 binding molecules provided herein can still bind to such functional fragments in a context-independent manner. The person skilled in the art is aware of methods for epitope mapping to determine which amino acid residues of an epitope are recognized by such anti-CD3 binding molecules (e.g. alanin scanning or pep spot analysis).

In a preferred embodiment of the invention, the polypeptide of the invention comprises a (first) binding domain as defined herein and a second binding domain capable of binding to a cell surface antigen.

The term "cell surface antigen" as used herein denotes a molecule, which is displayed on the surface of a cell. In most cases, this molecule will be located in or on the plasma membrane of the cell such that at least part of this molecule remains accessible from outside the cell in tertiary form. A non-limiting example of a cell surface molecule, which is located *in* the plasma membrane is a transmembrane protein comprising, in its tertiary conformation, regions of hydrophilicity and hydrophobicity. Here, at least one hydrophobic region allows the cell surface molecule to be embedded, or inserted in the hydrophobic plasma membrane of the cell while the hydrophilic regions extend on either side of the plasma membrane into the cytoplasm and extracellular space, respectively. Non-limiting examples of cell surface molecules which are located on the plasma membrane are proteins which have been modified at a cysteine residue to bear a palmitoyl group, proteins modified at a C-terminal cysteine residue to bear a famesyl group or proteins which have been modified at the C-terminus to bear a glycosyl phosphatidyl inositol ("GPI") anchor. These groups allow covalent attachment of proteins to the outer surface of the plasma membrane, where they remain accessible for recognition by extracellular molecules such as antibodies. Examples of cell surface antigens include EGFR, EGFRvIII, , MCSP, Carbonic anhydrase IX (CAIX), CD30, CD33, Her2/neu, IgE, CD44v6 and Muc-1. Additionally, examples for corresponding cell surface antibodies comprise antigens which are characteristic for a specific disease or ailment, i.e. cancer, autoimmune diseases or infections diseases including viral infections. Accordingly, the term "cell surface antigens" explicitly includes viral proteins such as native, unprocessed viral proteins exposed on the surface of infected cells (described inter alia for envelop proteins of Hepatitis virus B, C and HIV-1).

One defense function of cytotoxic T cells is the destruction of virus-infected cells, therefore, the unique property of the bispecific binding molecules of the invention to activate and redirect cytotoxic T cells irrespecitve of their autochthonous specificity has a great impact on the broad field of chronic virus infections. For the majority of these infections elimination of persistently infected cells is the only chance for cure. Currently, adoptive T cell therapies are currently being developed against chronic CMV and EBV infections (Rooney, C.M., et al., Use of gene-modified virus-specific T lymphocytes to control Epstein-Barr-virus-related lymphoproliferation. Lancet, 1995. 345 (8941): p. 9-13; Walter, E.A., et al., Reconstitution of cellular immunity against cytomegalovirus in recipients of allogeneic bone marrow by transfer of T-cell clones from the donor. N Engl J Med, 1995. 333 (16): p. 1038-44).

Chronic hepatitis B infection is clearly one of the most interesting and rewarding indications. Worldwide between 350 and 400 million people are infected with HBV. Current treatment of chronic HBV hepatitis rests on interferon $\gamma$ and nucleosid or nucleotide analogues, a long term therapy with considerable side-effects such as induction of hepatitis flares, fever, myalgias, thrombocytopenia and depression. Although there are now more than 4 approved therapeutic regimens, elimination of the virus is rarely achieved. A persistent inflammation in chronic hepatitis B leads to liver cirrhosis and hepatocellular carcinoma in more than 25% of patients. Moreover, up to 40 % of patients with chronic hepatitis B will die from serious complications, accounting for 0.6 to 1.0 million deaths per year worldwide

HBV, the prototype of the Hepadnaviruses is an enveloped virus whose relaxed circular (rc) genome is reverse transcribed into an RNA pregenome. After infection the rc DNA is imported into the hepatocyte nucleus where it is completed to a covalently closed circular DNA (cccDNA) containing four overlapping reading frames. It serves as transcription template for the pregenomic RNA and three subgenomic RNA. The RNA pregenome functions as mRNA for translation of the viral core and polymerase protein. Infected cells produce continuously HBV surface protein (HBsAg) from the cccDNA even when HBV replication is stopped. HBsAg consists of the small surface (S) proteins with very few portions of middle and large (L) surface proteins. Both the HBV S and L are targeted to the Endoplasmatic Reticulum (ER) membrane from where they are transported in membrane vesicles via the trans golgi organelle to the plasma membrane (Gorelick, F.S. and C. Shugrue, Exiting the endoplasmic reticulum. Mol Cell Endocrinol, 2001. 177 (1-2): p. 13-8). S and L proteins are permanently expressed on the surface of HBV replicating hepatocytes as shown recently (Chu, C.M. and Y.F. Liaw, Membrane staining for hepatitis B surface antigen on hepatocytes: a sensitive and specific marker of active viral replication in hepatitis B. J Clin Pathol, 1995. 48(5): p. 470-3).

Prototype viruses that expose envelope proteins at the cell surface are Hepatitis virus B (HBV), Hepatitis virus C (HCV) and HIV-1 both of which represent an enormous burden of disease globally. For the HIV-1 virus indication, it has recently been shown that T cells modified by a chimeric TCR with an Fv antibody construct directed at the gp120 envelope protein can kill HIV-1 infected target cells (Masiero, S., et al., T-cell engineering by a chimeric T-cell receptor with antibody-type specificity for the HIV-1 gp120. Gene Ther, 2005. 12 (4): p. 299-310). Of the hepadna viruses, hepatitis virus B (HBV) expresses the envelope protein complex HBsAg which is continuously produced from episomal cccDNA even when HBV replication subsides.

The expression as intact S and L HBV proteins on the cell surface makes them accessible for antibodies which are the hallmark of seroconversion when patiens recover from the acute phase of infections and change from circulating HBsAg to antiHBs. If seroconversion does not occur, up to 30 % of hepatocytes continue to express HBV S protein also after highly active antiviral therapy of long duration. Thus beyond T lymphocytes recognizing specifically intracellularly processed HBV peptides and presented by MHC molecules at the cell surface other forms of T cell engagement are feasible aimed at intact surface protein such as S and L antigens accessible in the outer cell membrane. Using single chain antibody fragments recognizing hepatitis B virus small (S) and large (L) envelope proteins, artificial T-cell receptors have been generated which allow directing grafted T-cells to infected hepatocytes and upon antigen contact activation of these T-cells to secrete cytokines and kill infected hepatocytes.

The limitation of this approach is, (i) that T-cells need to be manipulated in vitro, (ii) retroviruses used to transfer the T-cell receptors may cause insertional mutagenesis in the T-cells, and (iii) that once T-cells have been transferred the cytotoxic response cannot be limited.

To overcome these limitations, bispecific single chain antibody molecules comprising a first domain with a binding specificity for the human and the non-chimpanzee primate CD3 epsilon antigen (as provided herin in context of this invention) as well as a second domain with a binding specificity for HBV or HCV envelop proteins of infected hepatocytes may be generated and are within the scope of this invention. Within the present invention it is further preferred that the second binding domain binds to the human cell surface antigen and/or the non-chimpanzee primate counterparts of the human cell surface antigens selected from EGFR, Her2/neu or IgE.

For the generation of the second binding domain of the polypeptide of the invention, e.g. bispecific single chain antibodies as defined herein, monoclonal antibodies binding to both of the respective human and/or non-chimpanzee primate cell surface antigens can be utilized. Appropriate binding domains for the bispecific polypeptide as defined herein e.g. can be derived from cross-species specific monoclonal antibodies by recombinant methods described in the art. A monoclonal antibody binding to a human cell surface antigen and to the homolog of said cell surface antigen in a non-chimpanzee primate can be tested by FACS assays as set forth above. It is evident to those skilled in the art that cross-species specific antibodies can also be generated by hybridoma techniques described in the literature (Milstein and Köhler, Nature 256 (1975), 495-7). For example, mice may be alternately immunized with human and non-chimpanzee primate CD33. From these mice, cross-species specific antibody-producing hybridoma cells are isolated via hybridoma technology and analysed by FACS as set forth above. The generation and analysis of bispecific polypeptides such as bispecific single chain antibodies exhibiting cross-species specificity, as described herein is shown in the following examples. The advantages of the bispecific single chain antibodies exhibiting cross-species specificity include the points enumerated below.

It is particularly preferred for the polypeptide of the invention that the first binding domain capable of binding to an epitope of human and non-chimpanzee primate CD3ε chain comprises a VL region comprising CDR-L1, CDR-L2 and CDR-L3 selected from:

(a) CDR-L1 as depicted in SEQ ID NO. 27, CDR-L2 as depicted in SEQ ID NO. 28 and CDR-L3 as depicted in SEQ ID NO. 29;
(b) CDR-L1 as depicted in SEQ ID NO. 117, CDR-L2 as depicted in SEQ ID NO. 118 and CDR-L3 as depicted in SEQ ID NO. 119; and
(c) CDR-L1 as depicted in SEQ ID NO. 153, CDR-L2 as depicted in SEQ ID NO. 154 and CDR-L3 as depicted in SEQ ID NO. 155.

The variable regions, i.e. the variable light chain ("L" of "VL") and the variable heavy chain ("H" or "VH") are understood in the art to provide the binding domain of an antibody. This variable regions harbor the complementary determining regions.

The term "complementary determining region" (CDR) is well known in the art to dictate the antigen specificity of an antibody. The term "CDR-L" or "L CDR" refers to CDRs in the VL, whereas the term "CDR-H" or "H CDR" refers to the CDRs in the VH.

In an alternatively preferred embodiment of the polypeptide of the invention the first binding domain capable of binding to an epitope of human and non-chimpanzee primate CD3e chain comprises a VH region comprising CDR-H 1, CDR-H2 and CDR-H3 selected from:

(a) CDR-H1 as depicted in SEQ ID NO. 12, CDR-H2 as depicted in SEQ ID NO. 13 and CDR-H3 as depicted in SEQ ID NO. 14;
(b) CDR-H1 as depicted in SEQ ID NO. 30, CDR-H2 as depicted in SEQ ID NO. 31 and CDR-H3 as depicted in SEQ ID NO. 32;

(c) CDR-H1 as depicted in SEQ ID NO. 48, CDR-H2 as depicted in SEQ ID NO. 49 and CDR-H3 as depicted in SEQ ID NO. 50;

(d) CDR-H1 as depicted in SEQ ID NO. 66, CDR-H2 as depicted in SEQ ID NO. 67 and CDR-H3 as depicted in SEQ ID NO. 68;

(e) CDR-H1 as depicted in SEQ ID NO. 84, CDR-H2 as depicted in SEQ ID NO. 85 and CDR-H3 as depicted in SEQ ID NO. 86;

(f) CDR-H1 as depicted in SEQ ID NO. 102, CDR-H2 as depicted in SEQ ID NO. 103 and CDR-H3 as depicted in SEQ ID NO. 104;

(g) CDR-H1 as depicted in SEQ ID NO. 120, CDR-H2 as depicted in SEQ ID NO. 121 and CDR-H3 as depicted in SEQ ID NO. 122;

(h) CDR-H1 as depicted in SEQ ID NO. 138, CDR-H2 as depicted in SEQ ID NO. 139 and CDR-H3 as depicted in SEQ ID NO. 140;

(i) CDR-H1 as depicted in SEQ ID NO. 156, CDR-H2 as depicted in SEQ ID NO. 157 and CDR-H3 as depicted in SEQ ID NO. 158; and

(j) CDR-H1 as depicted in SEQ ID NO. 174, CDR-H2 as depicted in SEQ ID NO. 175 and CDR-H3 as depicted in SEQ ID NO. 176.

It is further preferred that the first binding domain capable of binding to an epitope of human and non-chimpanzee primate CD3ε chain comprises a VL region selected from the group consisting of a VL region as depicted in SEQ ID NO. 35, 39, 125, 129, 161 or 165.

It is alternatively preferred that the first binding domain capable of binding to an epitope of human and non-chimpanzee primate CD3ε chain comprises a VH region selected from the group consisting of a VH region as depicted in SEQ ID NO. 15, 19, 33, 37, 51, 55, 69, 73, 87, 91, 105, 109, 123, 127, 141, 145, 159, 163, 177 or 181.

More preferably, the polypeptide of the invention is characterized by the first binding domain capable of binding to an epitope of human and non-chimpanzee primate CD3ε chain, which comprises a VL region and a VH region selected from the group consisting of:

(a) a VL region as depicted in SEQ ID NO. 17 or 21 and a VH region as depicted in SEQ ID NO. 15 or 19;
(b) a VL region as depicted in SEQ ID NO. 35 or 39 and a VH region as depicted in SEQ ID NO. 33 or 37;
(c) a VL region as depicted in SEQ ID NO. 53 or 57 and a VH region as depicted in SEQ ID NO. 51 or 55;
(d) a VL region as depicted in SEQ ID NO. 71 or 75 and a VH region as depicted in SEQ ID NO. 69 or 73;
(e) a VL region as depicted in SEQ ID NO. 89 or 93 and a VH region as depicted in SEQ ID NO. 87 or 91;
(f) a VL region as depicted in SEQ ID NO. 107 or 111 and a VH region as depicted in SEQ ID NO. 105 or 109;
(g) a VL region as depicted in SEQ ID NO. 125 or 129 and a VH region as depicted in SEQ ID NO. 123 or 127;
(h) a VL region as depicted in SEQ ID NO. 143 or 147 and a VH region as depicted in SEQ ID NO. 141 or 145;
(i) a VL region as depicted in SEQ ID NO. 161 or 165 and a VH region as depicted in SEQ ID NO. 159 or 163; and
(j) a VL region as depicted in SEQ ID NO. 179 or 183 and a VH region as depicted in SEQ ID NO. 177 or 181.

According to a preferred embodiment of the polypeptide of the invention the pairs of VH-regions and VL-regions are in the format of a single chain antibody (scFv). The VH and VL regions are arranged in the order VH-VL or VL-VH. It is preferred that the VH-region is positioned N-terminally to a linker sequence. The VL-region is positioned C-terminally of the linker sequence.

A preferred embodiment of the above described polypeptide of the invention is characterized by the first binding domain capable of binding to an epitope of human and non-chimpanzee primate CD3ε chain comprising an amino acid sequence selected from the group consisting of SEQ ID NOs: 23, 25, 41, 43, 59, 61, 77, 79, 95, 97, 113, 115, 131, 133, 149, 151, 167, 169, 185 or 187.

The invention further relates to an above described polypeptide, wherein the second binding domain binds to a cell surface antigen, which is preferably a tumor antigen.

The term "tumor antigen" as used herein may be understood as those antigens that are presented on tumor cells. These antigens can be presented on the cell surface with an extracellular part, which is often combined with a transmembrane and cytoplasmic part of the molecule. These antigens can sometimes be presented only by tumor cells and never by the normal ones. Tumor antigens can be exclusively expressed on tumor cells or might represent a tumor specific mutation compared to normal cells. In this case, they are called tumor-specific antigens. More common are antigens that are presented by tumor cells and normal cells, and they are called tumor-associated antigens. These tumor-associated antigens can be overexpressed compared to normal cells or are accessible for antibody binding in tumor cells due to the less compact structure of the tumor tissue compared to normal tissue. Non-limiting examples of tumor antigens as used herein are EGFR (Liu, Br. J. Cancer 82/12 (2000), 1991-1999; Bonner, Semin. Radiat. Oncol. 12 (2002), 11-20; Kiyota, Oncology 63/1 (2002), 92-98; Kuan, Brain Tumor Pathol. 17/2 (2000), 71-78),.

EGFR (also known as c-erb1 or HER1) belongs to the erbB receptor tyrosine kinase family. When activated by binding of a ligand from the EGF family of growth factors, EGFR homodimerizes or heterodimerizes with a second EGFR or another member of the erbB receptor family, respectively, initiating a signaling cascade through mitogen-activated protein kinases and other transcription factors leading to proliferation, differentiation and repair (Olayioye, EMBO J. 19 (2000), 3159-67). EGFR is overexpressed in many epithelial cancers, including colorectal, breast, lung, and head and neck cancers (Mendelsohn, J. Clin. Oncol. 21 (2003), 2787-99; Mendelsohn, J. Clin. Oncol. 20 (18, Suppl.) (2002), 1S-13S; Prewett, Clin. Cancer Res. 8 (2002), 994-1003). Overexpression and/or mutation of EGFR in malignant cells leads to constitutive activation of kinase activity resulting in proliferation, angiogenesis, invasion, metastasis, and inhibition of apoptosis (Mendelsohn (2003, loc. cit.; Ciardiello, Clin. Cancer Res. 7 (2001), 2958-70; Perez-Soler, Oncologist 9 (2004), 58-67). Monoclonal antibodies that target the extracellular ligand binding domain or the intracellular tyrosine kinase signaling cascade of EGFR have been shown efficacy as antitumor target (Laskin, Cancer Treat. Review 30 (2004), 1-17). For example, cetuximab (Erbitux) a humanized monoclonal antibody to EGFR, which competitively inhibits the extracellular domain of EGFR to inhibit ligand activation of the receptor, was approved by the Food and Drug Administration (FDA) in 2004 for the treatment of metastatic colon cancer in combination with the topoisomerase inhibitor irinotecan.

In a preferred embodiment of the invention the polypeptide is a bispecific single chain antibody molecule. The herein above described problems with regard to the development of surrogate molecules for preclinical studies is further aggravated, if the drug candidate is a bispecific antibody, e.g. a bispecific single chain antibody. Such a bispecific antibody requires that both antigens recognized are cross-species specific with a given animal species to allow for safety testing in such animal.

As also noted herein above, the present invention provides polypeptides comprising a first binding domain which is an antibody capable of binding to an epitope of human and non-chimpanzee primate CD3ε chain and a second binding domain capable of binding to a cell surface antigen selected from EGFR, Her2/neu or IgE, wherein the second binding domain preferably also binds to a cell surface antigen of a human and/or a non-chimpanzee primate. The advantage of bispecific single chain antibody molecules as drug candidates fulfilling the requirements of the preferred polypeptide of the invention is the use of such molecules in preclinical animal testing as well as in clinical studies and even for therapy in human. In a preferred embodiment of the cross-species specific bispecific single chain antibodies of the invention the second binding domain capable of binding to a cell surface antigen is of human origin. In a cross-species specific bispecific molecule according to the invention the binding domain capable of binding to an epitope of human and non-chimpanzee primate CD3 epsilon chain is located in the order VH-VL or VL-VH at the N-terminus or the C-terminus of the bispecific molecule. Examples for cross-species specific bispecific molecules according to the invention in different arrangements of the VH- and the VL-chain in the first and the second binding domain are described in the appended examples.

As used herein, a "bispecific single chain antibody" denotes a single polypeptide chain comprising two binding domains. Each binding domain comprises one variable region from an antibody heavy chain ("VH region"), wherein the VH region of the first binding domain specifically binds to the CD3ε molecule, and the VH region of the second binding domain specifically binds to a cell surface antigen, as defined in more detail below. The two binding domains are optionally linked to one another by a short polypeptide spacer. A non-limiting example for a polypeptide spacer is Gly-Gly-Gly-Gly-Ser (G-G-G-G-S) and repeats thereof. Each binding domain may additionally comprise one variable region from an antibody light chain ("VL region"), the VH region and VL region within each of the first and second binding domains being linked to one another via a polypeptide linker, for example of the type disclosed and claimed in EP 623679 B1, but in any case long enough to allow the VH region and VL region of the first binding domain and the VH region and VL region of the second binding domain to pair with one another such that, together, they are able to specifically bind to the respective first and second molecules.

According to a preferred embodiment of the invention an above characterized bispecific single chain antibody molecule comprises a group of the following sequences as CDR H1, CDR H2, CDR H3, CDR L1, CDR L2 and CDR L3 in the second binding domain selected from SEQ ID NO: 441 - 446, SEQ ID NO: 453 - 458, SEQ ID NO: 463 - 468, SEQ ID NO: 481 - 486.

A particularly preferred embodiment of the invention concerns an above characterized polypeptide, wherein the bispecific single chain antibody molecule comprises a sequence selected from:

(a) an amino acid sequence as depicted in any of SEQ ID NOs:389, 391, 393, 395, 397, 399, 409, 411, 413, 415, 417, 419, 429, 431, 433, 435, 437, 439, 447, 449, 451, 469, 471, 473, 475, 477, 479, 495, 497, 499, 501, 503 and 505; and

(b) an amino acid sequence encoded by a nucleic acid sequence as depicted in any of SEQ ID NOs: 390, 392, 394, 396, 398, 400, 410, 412, 414, 416, 418, 420, 430, 432, 434, 436, 438, 440, 448, 450, 452, 470, 472, 474, 476, 478, 480, 496, 498, 500, 502, 504 and 506.

In a preferred embodiment of the invention, the bispecific single chain antibodies are cross-species specific for CD3 epsilon and for the tumor antigen recognized by their second binding domain.

In an alternative embodiment the present invention provides a nucleic acid sequence encoding an above described polypeptide of the invention.

The present invention also relates to a vector comprising the nucleic acid molecule of the present invention.

Many suitable vectors are known to those skilled in molecular biology, the choice of which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook et al. (loc cit.) and Ausubel, Current Protocols in Molecular Biology, Green Publishing Associates and Wiley Interscience, N.Y. (1989), (1994). Alternatively, the polynucleotides and vectors of the invention can be reconstituted into liposomes for delivery to target cells. As discussed in further details below, a cloning vector was used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT. Preferably said vector comprises a nucleic acid sequence which is a regulatory sequence operably linked to said nucleic acid sequence defined herein.

The term "regulatory sequence" refers to DNA sequences, which are necessary to effect the expression of coding sequences to which they are ligated. The nature of such control sequences differs depending upon the host organism. In prokaryotes, control sequences generally include promoter, ribosomal binding site, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

Thus, the recited vector is preferably an expression vector. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are weii known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, e.g., the P$_L$, *lac*, *trp* or tac promoter in *E. coli,* and examples of regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells.

Beside elements, which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art; see also the appended Examples. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pCDM8, pRc/CMV, pcDNA1, pcDNA3 (In-vitrogene), pEF-DHFR, pEF-ADA or pEF-neo (Mack et al. PNAS (1995) 92, 7021-7025 and Raum et al. Cancer Immunol Immunother (2001) 50(3), 141-150) or pSPORT1 (GIBCO BRL).

Preferably, the expression control sequences will be eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the invention may follow; see, e.g., the appended examples.

An alternative expression system, which can be used to express a cell cycle interacting protein is an insect system. In one such system, *Autographa californica* nuclear polyhedrosis virus (AcNPV) is used as a vector to express foreign genes in *Spodoptera frugiperda* cells or in *Trichoplusia* larvae. The coding sequence of a recited nucleic acid molecule may be cloned into a nonessential region of the virus, such as the polyhedrin gene, and placed under control of the polyhedrin promoter. Successful insertion of said coding sequence will render the polyhedrin gene inactive and produce recombinant virus lacking coat protein coat. The recombinant viruses are then used to infect *S. frugiperda* cells or *Trichoplusia* larvae in which the protein of the invention is expressed (Smith, J. Virol. 46 (1983), 584; Engelhard, Proc. Nat. Acad. Sci. USA 91 (1994), 3224-3227).

Additional regulatory elements may include transcriptional as well as translational enhancers. Advantageously, the above-described vectors of the invention comprise a selectable and/or scorable marker.

Selectable marker genes useful for the selection of transformed cells and, e.g., plant tissue and plants are well known to those skilled in the art and comprise, for example, antimetabolite resistance as the basis of selection for dhfr, which confers resistance to methotrexate (Reiss, Plant Physiol. (Life Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, PI. Sci. 116 (1996), 59-72; Scikantha, J. Bact. 178 (1996), 121), green fluorescent protein (Gerdes, FEBS Lett. 389 (1996), 44-47) or ß-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used alone or as part of a vector to express the polypeptide of the invention in cells, for, e.g., purification but also for gene therapy purposes. The nucleic acid molecules or vectors containing the DNA sequence(s) encoding any one of the above described polypeptide of the invention is introduced into the cells which in turn produce the polypeptide of interest. Gene therapy, which is based on introducing therapeutic genes into cells by ex-vivo or in-vivo techniques is one of the most important applications of gene transfer. Suitable vectors, methods or gene-delivery systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Verma, Nature 389 (1994), 239; Isner, Lancet 348 (1996), 370-374; Muhlhauser, Circ. Res. 77 (1995), 1077-1086; Onodera, Blood 91 (1998), 30-36; Verma, Gene Ther. 5 (1998), 692-699; Nabel, Ann. N.Y. Acad. Sci. 811 (1997), 289-292; Verzeletti, Hum. Gene Ther. 9 (1998), 2243-51; Wang, Nature Medicine 2 (1996), 714-716; WO 94/29469; WO 97/00957, US 5,580,859; US 5,589,466; or Schaper, Current Opinion in Biotechnology 7 (1996), 635-640. The recited nucleic acid molecules and vectors may be designed for direct introduction or for introduction via liposomes, or viral vectors (e.g., adenoviral, retroviral) into the cell. Preferably, said cell is a germ line cell, embryonic cell, or egg cell or derived there from, most preferably said cell is a stem cell. An example for an embryonic stem cell can be, inter alia, a stem cell as described in Nagy, Proc. Natl. Acad. Sci. USA 90 (1993), 8424-8428.

The invention also provides for a host cell transformed or transfected with a vector of the invention. Said host cell may be produced by introducing the above described vector of the invention or the above described nucleic acid molecule of the invention into the host cell. The presence of at least one vector or at least one nucleic acid molecule in the host cell may mediate the expression of a gene encoding the above described single chain antibody constructs.

The described nucleic acid molecule or vector of the invention, which is introduced in the host cell may either integrate into the genome of the host cell or it may be maintained extrachromosomally.

The host cell can be any prokaryote or eukaryotic cell.

The term "prokaryote" is meant to include all bacteria, which can be transformed or transfected with DNA or RNA molecules for the expression of a protein of the invention. Prokaryotic host cells may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host cell employed in a recombinant production procedure, the protein encoded by the polynucleotide of the present invention may be glycosylated or may be non-glycosylated. Especially preferred is the use of a plasmid or a virus containing the coding sequence of the polypeptide of the invention and genetically fused thereto an N-terminal FLAG-tag and/or C-terminal His-tag. Preferably, the length of said FLAG-tag is about 4 to 8 amino acids, most preferably 8 amino acids. An above described polynucleotide can be used to transform or transfect the host cell using any of the techniques commonly known to those of ordinary skill in the art. Furthermore, methods for preparing fused, operably linked genes and expressing them in, e.g., mammalian cells and bacteria are well-known in the art (Sambrook, loc cit.).

Preferably, the host cell is a bacterium or an insect, fungal, plant or animal cell.

It is particularly envisaged that the recited host cell may be a mammalian cell. Particularly preferred host cells comprise CHO cells, COS cells, myeloma cell lines like SP2/0 or NS/0. As illustrated in the appended examples, particularly preferred are CHO-cells as hosts.

More preferably said host cell is a human cell or human cell line, e.g. per.c6 (Kroos, Biotechnol. Prog., 2003, 19:163-168).

In a further embodiment, the present invention thus relates to a process for the production of a polypeptide of the invention, said process comprising culturing a host cell of the invention under conditions allowing the expression of the polypeptide of the invention and recovering the produced polypeptide from the culture.

The transformed host cells can be grown in fermentors and cultured according to techniques known in the art to achieve optimal cell growth. The polypeptide of the invention can then be isolated from the growth medium, cellular lysates, or cellular membrane fractions. The isolation and purification of the, e.g., microbially expressed polypeptides of the invention may be by any conventional means such as, for example, preparative chromatographic separations and immunological separations such as those involving the use of monoclonal or polyclonal antibodies directed, e.g., against a tag of the polypeptide of the invention or as described in the appended examples.

The conditions for the culturing of a host cell, which allow the expression are known in the art to depend on the host cell system and the expression system/vector used in such process. The parameters to be modified in order to achieve conditions allowing the expression of a recombinant polypeptide are known in the art. Thus, suitable conditions can be determined by the person skilled in the art in the absence of further inventive input.

Once expressed, the polypeptide of the invention can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure polypeptides of at least about 90 to 95% homogeneity are preferred, and 98 to 99% or more homogeneity are most preferred, for pharmaceutical uses. Once purified, partially or to homogeneity as desired, the polypeptide of the invention may then be used therapeutically (including extracorporeally) or in developing and performing assay procedures. Furthermore, examples for methods for the recovery of the polypeptide of the invention from a culture are described in detail in the appended examples.

Furthermore, the invention provides for a composition comprising a polypeptide of the invention or a polypeptide as produced by the process disclosed above. Preferably, said composition is a pharmaceutical composition.

In accordance with the invention, the term "pharmaceutical composition" relates to a composition for administration to a patient, preferably a human patient. The particular preferred pharmaceutical composition of this invention comprises binding molecules directed against and generated against context-independent CD3 epitopes. Preferably, the pharmaceutical composition comprises suitable formulations of carriers, stabilizers and/or excipients. In a preferred embodiment, the pharmaceutical composition comprises a composition for parenteral, transdermal, intraluminal, intraarterial, intrathecal and/or intranasal administration or by direct injection into tissue. It is in particular envisaged that said composition is administered to a patient via infusion or injection. Administration of the suitable compositions may be effected by different ways, e.g., by intravenous, intraperitoneal, subcutaneous intramuscular, topical or intradermal administration. In particular, the present invention provides for the suitable composition for an uninterrupted administration. As a non-limiting example, uninterrupted, i.e. continuous administration may be realized by a small pump system worn by the patient for metering the influx of therapeutic agent into the body of the patient. The pharmaceutical composition comprising the binding molecules directed against and generated against context-independent CD3 epitopes of the invention can be administered by using said pump systems. Such pump systems are generally known in the art, and commonly rely on periodic exchange of cartridges containing the therapeutic agent to be infused. When exchanging the cartridge in such a pump system, a temporary interruption of the otherwise uninterrupted flow of therapeutic agent into the body of the patient may ensue. In such a case, the phase of administration prior to cartridge replacement and the phase of administration following cartridge replacement would still be considered within the meaning of the pharmaceutical means and methods of the invention together make up one "uninterrupted administration" of such therapeutic agent.

The continuous or uninterrupted administration of these binding molecules directed against and generated against context-independent CD3 epitopes of this invention may be intravenuous or subcutaneous by way of a fluid delivery device or small pump system including a fluid driving mechanism for driving fluid out of a reservoir and an actuating mechanism for actuating the driving mechanism. Pump systems for subcutaneous administration may include a needle or a cannula for penetrating the skin of a patient and delivering the suitable composition into the patient's body. Said pump systems may be directly fixed or attached to the skin of the patient independently of a vein, artery or blood vessel, thereby allowing a direct contact between the pump system and the skin of the patient. The pump system can be attached to the skin of the patient for 24 hours up to several days. The pump system may be of small size with a reservoir for small volumes. As a non-limiting example, the volume of the reservoir for the suitable pharmaceutical composition to be administered can be between 0.1 and 50 ml.

The continuous administration may be transdermal by way of a patch worn on the skin and replaced at intervals. One of skill in the art is aware of patch systems for drug delivery suitable for this purpose. It is of note that transdermal administration is especially amenable to uninterrupted administration, as exchange of a first exhausted patch can advantageously be accomplished simultaneously with the placement of a new, second patch, for example on the surface of the skin immediately adjacent to the first exhausted patch and immediately prior to removal of the first exhausted patch. Issues of flow interruption or power cell failure do not arise.

The composition of the present invention, comprising in particular binding molecules directed against and generated against context-independent CD3 epitopes may further comprise a pharmaceutically acceptable carrier. Examples of suitable pharmaceutical carriers are well known in the art and include solutions, e.g. phosphate buffered saline solutions, water, emulsions, such as oil/water emulsions, various types of wetting agents, sterile solutions, liposomes, etc. Compositions comprising such carriers can be formulated by well known conventional methods. Formulations can comprise carbohydrates, buffer solutions, amino acids and/or surfactants. Carbohydrates may be non-reducing sugars, preferably trehalose, sucrose, octasulfate, sorbitol or xylitol. Such formulations may be used for continuous administrations which may be intravenuous or subcutaneous with and/or without pump systems. Amino acids may be charged amino acids, preferably lysine, lysine acetate, arginine, glutamate and/or histidine. Surfactants may be detergents, preferably with a molecular weight of >1.2 KD and/or a polyether, preferably with a molecular weight of >3 KD. Non-limiting examples for preferred detergents are Tween 20, Tween 40, Tween 60, Tween 80 or Tween 85. Non-limiting examples for preferred polyethers are PEG 3000, PEG 3350, PEG 4000 or PEG 5000. Buffer systems used in the present invention can have a preferred pH of 5-9 and may comprise citrate, succinate, phosphate, histidine and acetate. The compositions of the present invention can be administered to the subject at a suitable dose which can be determined e.g. by dose escalating studies by administration of increasing doses of the polypeptide of the invention exhibiting cross-species specificity .described herein to non-chimpanzee primates, for instance macaques. As set forth above, the polypeptide of the invention exhibiting cross-species specificity described herein can be advantageously used in identical form in preclinical testing in non-chimpanzee primates and as drug in humans. These compositions can also be administered in combination with other proteinaceous and non-proteinaceous drugs. These drugs may be administered simultaneously with the composition comprising the polypeptide of the invention as defined herein or separately before or after administration of said polypeptide in timely defined intervals and doses. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, inert gases and the like. In addition, the composition of the present invention might comprise proteinaceous carriers, like, e.g., serum albumin or immunoglobulin, preferably of human origin. It is envisaged that the composition of the invention might comprise, in addition to the polypeptide of the invention defined herein, further biologically active agents, depending on the intended use of the composition. Such agents might be drugs acting on the gastro-intestinal system, drugs acting as cytostatica, drugs preventing hyperurikemia, drugs inhibiting immunoreactions (e.g. corticosteroids), drugs modulating the inflammatory response, drugs acting on the circulatory system and/or agents such as cytokines known in the art.

The biological activity of the pharmaceutical composition defined herein can be determined for instance by cytotoxicity assays, as described in the following examples, in WO 99/54440 or by Schlereth et al. (Cancer Immunol. Immunother. 20 (2005), 1 - 12). "Efficacy" or "in vivo efficacy" as used herein refers to the response to therapy by the pharmaceutical composition of the invention, using e.g. standardized NCI response criteria. The success or *in vivo* efficacy of the therapy using a pharmaceutical composition of the invention refers to the effectiveness of the composition for its intended purpose, i.e. the ability of the composition to cause its desired effect, i.e. depletion of pathologic cells, e.g. tumor cells. The *in vivo* efficacy may be monitored by established standard methods for the respective disease entities including, but not limited to white blood cell counts, differentials, Fluorescence Activated Cell Sorting, bone marrow aspiration. In addition, various disease specific clinical chemistry parameters and other established standard methods may be used. Furthermore, computer-aided tomography, X-ray, nuclear magnetic resonance tomography (e.g. for National Cancer Institute-criteria based response assessment [Cheson BD, Horning SJ, Coiffier B, Shipp MA, Fisher RI, Connors JM, Lister TA, Vose J, Grillo-Lopez A, Hagenbeek A, Cabanillas F, Klippensten D, Hiddemann W, Castellino R, Harris NL, Armitage JO, Carter W, Hoppe R, Canellos GP. Report of an international workshop to standardize response criteria for non-Hodgkin's lymphomas. NCI Sponsored International Working Group. J Clin Oncol. 1999 Apr;17(4):1244]), positron-emission tomography scanning, white blood cell counts, differentials, Fluorescence Activated Cell Sorting, bone marrow aspiration, lymph node biopsies/histologies, and various lymphoma specific clinical chemistry parameters (e.g. lactate dehydrogenase) and other established standard methods may be used.

Another major challenge in the development of drugs such as the pharmaceutical composition of the invention is the predictable modulation of pharmacokinetic properties. To this end, a pharmacokinetic profile of the drug candidate, i.e. a profile of the pharmacokinetic parameters that effect the ability of a particular drug to treat a given condition, is established. Pharmacokinetic parameters of the drug influencing the ability of a drug for treating a certain disease entity include, but are not limited to: half-life, volume of distribution, hepatic first-pass metabolism and the degree of blood serum binding. The efficacy of a given drug agent can be influenced by each of the parameters mentioned above.

"Half-life" means the time where 50% of an administered drug are eliminated through biological processes, e.g. metabolism, excretion, etc.

By "hepatic first-pass metabolism" is meant the propensity of a drug to be metabolized upon first contact with the liver, i.e. during its first pass through the liver. "Volume of distribution" means the degree of retention of a drug throughout the various compartments of the body, like e.g. intracellular and extracellular spaces, tissues and organs, etc. and the distribution of the drug within these compartments.

"Degree of blood serum binding" means the propensity of a drug to interact with and bind to blood serum proteins, such as albumin, leading to a reduction or loss of biological activity of the drug.

Pharmacokinetic parameters also include bioavailability, lag time (Tlag), Tmax, absorption rates, more onset and/or Cmax for a given amount of drug administered. "Bioavailability" means the amount of a drug in the blood compartment.

"Lag time" means the time delay between the administration of the drug and its detection and measurability in blood or plasma.

"Tmax" is the time after which maximal blood concentration of the drug is reached, and "Cmax" is the blood concentration maximally obtained with a given drug. The time to reach a blood or tissue concentration of the drug which is required for its biological effect is influenced by all parameters. Pharmacokinetic parameters of bispecific single chain antibodies, a preferred embodiment of the polypeptide of the invention, exhibiting cross-species specificity, which may be determined in preclinical animal testing in non-chimpanzee primates as outlined above are also set forth e.g. in the publication by Schlereth et al. (Cancer Immunol. Immunother. 20 (2005), 1 - 12).

The term "toxicity" as used herein refers to the toxic effects of a drug manifested in adverse events or severe adverse events. These side events might refer to a lack of tolerability of the drug in general and/or a lack of local tolerance after administration. Toxicity could also include teratogenic or carcinogenic effects caused by the drug.

The term "safety", "in vivo safety" or "tolerability" as used herein defines the administration of a drug without inducing severe adverse events directly after administration (local tolerance) and during a longer period of application of the drug. "Safety", "in vivo safety" or "tolerability" can be evaluated e.g. at regular intervals during the treatment and follow-up period. Measurements include clinical evaluation, e.g. organ manifestations, and screening of laboratory abnormalities. Clinical evaluation may be carried out and deviating to normal findings recorded/coded according to NCI-CTC and/or MedDRA standards. Organ manifestations may include criteria such as allergy/immunology, blood/bone marrow, cardiac arrhythmia, coagulation and the like, as set forth e.g. in the Common Terminology Criteria for adverse events v3.0 (CTCAE). Laboratory parameters which may be tested include for instance haematology, clinical chemistry, coagulation profile and urine analysis and examination of other body fluids such as serum, plasma, lymphoid.or spinal fluid, liquor and the like. Safety can thus be assessed e.g. by physical examination, imaging techniques (i.e. ultrasound, x-ray, CT scans, Magnetic Resonance Imaging (MRI), other measures with technical devices (i.e. electrocardiogram), vital signs, by measuring laboratory parameters and recording adverse events. For example, adverse events in non-chimpanzee primates in the uses and methods according to the invention may be examined by histopathological and/or histochemical methods.

The term "effective and non-toxic dose" as used herein refers to a tolerable dose of the bispecific single chain antibody as defined herein which is high enough to cause depletion of pathologic cells, tumor elimination, tumor shrinkage or stabilization of disease without or essentially without major toxic effects. Such effective and non-toxic doses may be determined e.g. by dose escalation studies described in the art and should be below the dose inducing severe adverse side events (dose limiting toxicity, DLT).

The above terms are also referred to e.g. in the Preclinical safety evaluation of biotechnology-derived pharmaceuticals S6; ICH Harmonised Tripartite Guideline; ICH Steering Committee meeting on July 16, 1997.

Moreover, the invention relates to a pharmaceutical composition comprising a polypeptide of this invention (i.e. a polypeptide comprising at least one binding domain capable of binding to an epitope of human and non-chimpanzee primate CD3 epsilon chain, wherein the epitope is part of an amino acid sequence comprised in the group consisting of SEQ ID NOs. 2, 4, 6, or 8 in accordance with this invention or produced according to the process according to the invention) for the prevention, treatment or amelioration of a disease selected from a proliferative disease, a tumorous disease, or an immunological disorder. Preferably, said pharmaceutical composition further comprises suitable formulations of carriers, stabilizers and/or excipients.

A further aspect of the invention relates to a use of a polypeptide as defined herein above or produced according to a process defined herein above, for the preparation of a pharmaceutical composition for the prevention, treatment or amelioration of a disease. Preferably, said disease is a proliferative disease, a tumorous disease, or an immunological disorder. It is further preferred that said tumorous disease is a malignant disease, preferably cancer.

In another preferred embodiment of use of the polypeptide of the invention said pharmaceutical composition is suitable to be administered in combination with an additional drug, i.e. as part of a co-therapy. In said co-therapy, an active agent may be optionally included in the same pharmaceutical composition as the polypeptide of the invention, or may be included in a separate pharmaceutical composition. In this latter case, said separate pharmaceutical composition is suitable for administration prior to, simultaneously as or following administration of said pharmaceutical composition comprising the polypeptide of the invention. The additional drug or pharmaceutical composition may be a non-proteinaceous compound or a proteinaceous compound. In the case that the additional drug is a proteinaceous compound, it is advantageous that the proteinaceous compound be capable of providing an activation signal for immune effector cells.

Preferably, said proteinaceous compound or non-proteinaceous compound may be administered simultaneously or non-simultaneously with the polypeptide of the invention, a nucleic acid molecule as defined hereinabove, a vector as defined as defined hereinabove, or a host as defined as defined hereinabove.

Another aspect of the invention relates to a pharmaceutical composition of the invention for use in a method for the treatment or amelioration of a disease in a subject in the need thereof said method comprising the step of administration of an effective amount of a pharmaceutical composition of the invention. Preferably, said disease is a proliferative disease, a tumorous disease, or an immunological disorder. Even more preferred, said tumorous disease is a malignant disease, preferably cancer.

In another preferred embodiment said pharmaceutical composition is suitable to be administered in combination with an additional drug, i.e. as part of a co-therapy. In said co-therapy, an active agent may be optionally included in the same pharmaceutical composition as the polypeptide of the invention, or may be included in a separate pharmaceutical composition. In this latter case, said separate pharmaceutical composition is suitable for administration prior to, simultaneously as or following administration of said pharmaceutical composition comprising the polypeptide of the invention. The additional drug or pharmaceutical composition may be a non-proteinaceous compound or a proteinaceous compound. In the case that the additional drug is a proteinaceous compound, it is advantageous that the proteinaceous compound be capable of providing an activation signal for immune effector cells.

Preferably, said proteinaceous compound or non-proteinaceous compound may be administered simultaneously or non-simultaneously with the polypeptide of the invention, a nucleic acid molecule as defined hereinabove, a vector as defined as defined hereinabove, or a host as defined as defined hereinabove.

It is preferred for the above described method of the invention that said subject is a human.

In a further aspect, the invention relates to a kit comprising a polypeptide of the invention, a nucleic acid molecule of the invention, a vector of the invention, or a host of the invention.

The present disclosure further provides the following list of items:

Item 1. A method for the identification of (a) polypeptide(s) comprising a cross-species specific binding domain capable of binding to an epitope of human and non-chimpanzee primate CD3 epsilon (CD3ε), the method comprising the steps of:

(a) contacting the polypeptide(s) with an N-terminal fragment of the extracellular domain of CD3ε of maximal 27 amino acids comprising the amino acid sequence Gln-Asp-Gly-Asn-Glu-Glu-Met-Gly (SEQ ID NO. 381) or Gln-Asp-Gly-Asn-Glu-Glu-Ile-Gly (SEQ ID NO. 382), fixed via its C-terminus to a solid phase;
(b) eluting the bound polypeptide(s) from said fragment; and
(c) isolating the polypeptide(s) from the eluate of (b).

It is preferred that the polypeptide(s) identified by the above method of the invention are of human origin.

The present "method for the identification of (a) polypeptide(s)" is understood as a method for the isolation of one or more different polypeptides with the same specificity for the fragment of the extracellular domain of CD3ε comprising at its N-terminus the amino acid sequence Gln-Asp-Gly-Asn-Glu-Glu-Met-Gly (SEQ ID NO. 381) or Gln-Asp-Gly-Asn-Glu-Glu-Ile-Gly (SEQ ID NO. 382) from a plurality of polypeptide candidates as well as a method for the purification of a polypeptide from a solution. Non-limiting embodiments of a method of the isolation of one or more different polypeptides with the same specificity for the fragment of the extracellular domain of CD3ε comprise methods for the selection of antigen-specific binding entities, e.g. panning methods as commonly used for hybridoma screening, screening of transiently/stably transfected clones of eukaryotic host cells or in phage display methods. A non-limiting example for the latter method for the purification of a polypeptide from a solution is e.g. the purification of a recombinantly expressed polypeptide from a culture supernatant or a preparation from such culture.

As stated above the fragment used in this method is a N-terminal fragment of the extracellular domain of the primate CD3ε molecule. The amino acid sequence of the extracellular domain of the CD3ε molecule of different primates is depicted in SEQ ID NOs: 1, 3, 5 and 7. The two forms of the N-terminal octamer are depicted in SEQ ID NOs: 381 and 382. It is preferred that this N-terminus is freely available for binding of the polypeptides to be identified by the method of the invention. The term "freely available" is understood in the context of the invention as free of additional motives such as a His-tag. The interference of such a His-tag with a binding molecule identified by this method is described in the appended Example 6.

According to the method mentioned above said fragment is fixed via its C-terminus to a solid phase. The person skilled in the art will easily and without any inventive ado elect a suitable solid phase support dependent from the used embodiment of the method of the invention. Examples for a solid support comprise but are not limited to matrices like beads (e.g. agarose beads, sepharose beads, polystyrol beads, dextran beads), plates (culture plates or MultiWell plates) as well as chips known e.g. from Biacore®. The selection of the means and methods for the fixation/immobilization of the fragment to said solid support depend on the election of the solid support. A commonly used method for the fixation/immobilization is a coupling via an N-hydroxysuccinimide (NHS) ester. The chemistry underlying this coupling as well as alternative methods for the fixation/immobilization are known to the person skilled in the art, e.g. from Hermanson "Bioconjugate Techniques", Academic Press, Inc. (1996). For the fixation to/immobilization on chromatographic supports the following means are commonly used: NHS-activated sepharose (e.g. HiTrap-NHS of GE Life Science - Amersham), CnBr-activated sepharose (e.g. GE Life Science Amersham), NHS-activated dextran beads (Sigma) or activated polymethacrylate. These reagents may also be used in a batch approach. Moreover, dextran beads comprising iron oxide (e.g. available from Miltenyi) may be used in a batch approach. These beads may be used in combination with a magnet for the separation of the beads from a solution. Polypeptides can be immobilized on a Biacore chip (e.g. CM5 chips) by the use of NHS activated carboxymethyldextran. Further examples for an appropriate solid support are amine reactive MultiWell plates (e.g. Nunc Immobilizer™ plates).

According to the method mentioned above said fragment of the extracellular domain of CD3 epsilon can be directly coupled to the solid support or via a stretch of amino acids, which might be a linker or another protein/polypeptide moiety. Alternatively, the extracellular domain of CD3 epsilon can be indirectly coupled via one or more adaptor molecule(s).

Means and methods for the eluation of a peptide bound to an immobilized epitope are well known in the art. The same holds true for methods for the isolation of the identified polypeptide(s) from the eluate.

In line with the disclosure of a method for the isolation of one or more different polypeptides with the same specificity for the fragment of the extracellular domain of CD3ε comprising at its N-terminus the amino acid sequence Gln-Asp-Gly-Asn-Glu-Glu-X-Gly from a plurality of polypeptide candidates may comprise one or more steps of the following methods for the selection of antigen-specific entities:

CD3ε specific binding molecules can be selected from antibody derived repertoires. A phage display library can be constructed based on standard procedures, as for example disclosed in "Phage Display: A Laboratory Manual"; Ed. Barbas, Burton, Scott & Silverman; Cold Spring Harbor Laboratory Press, 2001. The format of the antibody fragments in the antibody library can be scFv, but may generally also be a Fab fragment or even a single domain antibody fragment. For the isolation of antibody fragments naïve antibody fragment libraries may be used. For the selection of potentially low immunogenic binding entities in later therapeutic use, human antibody fragment libraries may be favourable for the direct selection of human antibody fragments. In some cases they may form the basis for synthetic antibody libraries (Knappik et al. J Mol. Biol. 2000, 296:57 ff). The corresponding format may be Fab, scFv (as described below) or domain antibodies (dAbs, as reviewed in Holt et al., Trends Biotechnol. 2003, 21:484 ff).

It is also known in the art that in many cases there is no immune human antibody source available against the target antigen. Therefore animals are immunized with the target antigen and the respective antibody libraries isolated from animal tissue as e.g. spleen or PBMCs. The N-terminal fragment may be biotinylated or covalently linked to proteins like KLH or bovine serum albumin (BSA). According to common approaches rodents are used for immunization. Some immune antibody repertoires of non-human origin may be especially favourable for other reasons, e.g. for the presence of single domain antibodies (VHH) derived from cameloid species (as described in Muyldermans, J Biotechnol. 74:277; De Genst et al. Dev Como Immunol. 2006, 30:187 ff). Therefore a corresponding format of the antibody library may be Fab, scFv (as described below) or single domain antibodies (VHH).

In one possible approach ten weeks old F1 mice from balb/c x C57black crossings can be immunized with whole cells e.g. expressing transmembrane EpCAM N-terminally displaying as translational fusion the N-terminal amino acids 1 to 27 of the mature CD3ε chain. Alternatively, mice can be immunized with 1-27 CD3 epsilon-Fc fusion protein (a corresponding approach is described in the appended Example 2). After booster immunization(s), blood samples can be taken and antibody serum titer against the CD3-positive T cells can be tested e.g. in FACS analysis.

Usually, serum titers are significantly higher in immunized than in non-immunized animals. immunized animals may form the basis for the construction of immune antibody libraries. Examples of such libraries comprise phage display libraries. Such libraries may be generally constructed based on standard procedures, as for example disclosed in "Phage Display: A Laboratory Manual"; Ed. Barbas, Burton, Scott & Silverman; Cold Spring Harbor Laboratory Press, 2001.

The non-human antibodies can also be humanized via phage display due to the generation of more variable antibody libraries that can be subsequently enriched for binders during selection.

In a phage display approach any one of the pools of phages that displays the antibody libraries forms a basis to select binding entities using the respective antigen as target molecule. The central step in which antigen specific, antigen bound phages are isolated is designated as panning. Due to the display of the antibody fragments on the surface of the phages, this general method is called phage display. One preferred method of selection is the use of small proteins such as the filamentous phage N2 domain translationally fused to the N-terminus of the scFv displayed by the phage. Another display method known in the art, which may be used to isolate binding entities is the ribosome display method (reviewed in Groves & Osboum, Expert Opin Biol Ther. 2005, 5:125 ff; Lipovsek & Pluckthun, J Immunol Methods 2004, 290:52 ff).

In order to demonstrate binding of scFv phage particles to a 1-27 CD3ε-Fc fusion protein a phage library carrying the cloned scFv-repertoire can be harvested from the respective culture supernatant by PEG (polyethyleneglycole). ScFv phage particles may be incubated with immobilized CD3ε Fc fusion protein. The immobilized CD3ε Fc fusion protein may be coated to a solid phase. Binding entities can be eluted and the eluate can be used for infection of fresh uninfected bacterial hosts. Bacterial hosts successfully transduced with a phagemid copy, encoding a human scFv-fragment, can be selected again for carbenicillin resistance and subsequently infected with e.g. VCMS 13 helper phage to start the second round of antibody display and in vitro selection. A total of 4 to 5 rounds of selections is carried out, normally.

The binding of isolated binding entities can be tested on CD3epsilon positive Jurkat cells, HPBall cells, PBMCs or transfected eukaryotic cells that carry the N-terminal CD3ε sequence fused to surface displayed EpCAM using a flow cytometric assay (see appended Example 4).

Item 2. The method of item 1, wherein the polypeptide(s) comprise(s) the identified binding domain as a first binding domain and a second binding domain capable of binding to a cell surface antigen.

For the generation of the second binding domain of the polypeptide identified by the method mentioned above, e.g. bispecific single chain antibodies as defined herein, monoclonal antibodies binding to both of the respective human and non-chimpanzee primate cell surface antigens can be used. Appropriate binding domains for the bispecific polypeptide as defined herein e.g. can be derived from cross-species specific monoclonal antibodies by recombinant methods described in the art. A monoclonal antibody binding to a human cell surface antigen and to the homolog of said cell surface antigen in a non-chimpanzee primate can be tested by FACS assays as set forth above. Hybridoma techniques as described in the literature (Milstein and Köhler, Nature 256 (1975), 495-7) can also be used for the generation of cross-species specific antibodies. For example, mice may be alternately immunized with human and non-chimpanzee primate CD33. From these mice, cross-species specific antibody-producing hybridoma cells can be isolated via hybridoma technology and analysed by FACS as set forth above. The generation and analysis of bispecific polypeptides such as bispecific single chain antibodies exhibiting cross-species specificity as described herein is shown in the following Examples. The advantages of the bispecific single chain antibodies exhibiting cross-species specificity include the points enumerated below.

Item 3. The method of item 2, wherein the second binding domain binds to a cell surface antigen of a human and/or a non-chimpanzee primate.

Item 4. The method of any of items 1 to 3, wherein the first binding domain is an antibody.

Item 5. The method of item 4, wherein the antibody is a single chain antibody.

Item 6. The method of any of items 2 to 5, wherein the second binding domain is an antibody.

Item 7. The method of any of items 1 to 6, wherein the fragment of the extracellular domain of CD3ε consists of one or more fragments of a polypeptide having an amino acid sequence of any one depicted in SEQ ID NOs.2, 4, 6 or 8.

Item 8. The method of item 7, wherein said fragment is 8, 9, 10, 11, 12, 13, 14, 15, 16, 17, 18, 19, 20, 21, 22, 23, 24, 25, 26, 27 amino acid residues in length.

Item 9. The method of any of items 1 to 8, wherein the method of identification is a method of screening a plurality of polypeptides comprising a cross-species specific binding domain capable of binding to an epitope of human and non-chimpanzee primate CD3ε.

Item 10. The method of any of items 1 to 8, wherein the method of identification is a method of purification/isolation of a polypeptide comprising a cross-species specific binding domain capable of binding to an epitope of human and non-chimpanzee primate CD3ε.

Item 11. Use of an N-terminal fragment of the extracellular domain of CD3ε of maximal 27 amino acids comprising the amino acid sequence Gln-Asp-Gly-Asn-Glu-Glu-Met-Gly (SEQ ID NO. 381) or Gln-Asp-Gly-Asn-Glu-Glu-Ile-Gly (SEQ ID NO. 382) for the generation of a cross-species specific binding domain.
In line with said use of the invention it is preferred that the generated cross-species specific binding domain is of human origin.

Item 12. Use according to item 11, wherein the cross-species specific binding domain is an antibody.

Item 13. Use according to item 12, wherein the antibody is a single chain antibody.

Item 14. Use according to item 12 to 13, wherein the antibody is a bispecific antibody.

These and other embodiments are disclosed and encompassed by the description and Examples of the present invention. Recombinant techniques and methods in immunology are described e.g. in Sambrook et al. Molecular Cloning: A Laboratory Manual; Cold Spring Harbor Laboratory Press, 3rd edition 2001; Lefkovits; Immunology Methods Manual; The Comprehensive Sourcebook of Techniques; Academic Press, 1997; Golemis; Protein-Protein Interactions: A Molecular Cloning Manual; Cold Spring Laboratory Press, 2002. Further literature concerning any one of the antibodies, methods, uses and compounds to be employed in accordance with the present invention may be retrieved from public libraries and databases, using for example electronic devices. For example, the public database "Medline", available on the Internet, may be utilized, for example under http://www.ncbi.nlm.nih.gov/PubMed/medline.html. Further databases and addresses such as http://www.ncbi.nlm.nih.gov/ or listed at the EMBL-services homepage under http://www.embl.de/services/index.html are known to the person skilled in the art and can also be obtained using, e. g., http://www. google.com.
The figures show:

**Figure 1**
Fusion of the N-terminal amino acids 1-27 of primate CD3 epsilon to a heterologous soluble protein.

**Figure 2**
The figure shows the average absorption values of quadruplicate samples measured in an ELISA assay detecting the presence of a construct consisting of the N-terminal amino acids 1-27 of the mature human CD3 epsilon chain fused to the hinge and Fc gamma portion of human IgG1 and a C-terminal 6 Histidine tag in a supernatant of transiently transfected 293 cells. The first column labeled "27 aa huCD3E" shows the average absorption value for the construct, the second column labeled "irrel. SN" shows the average value for a supernatant of 293 cells transfected with an irrelevant construct as negative control. The comparison of the values obtained for the construct with the values obtained for the negative control clearly demonstrates the presence of the recombinant construct.

**Figure 3**
The figure shows the average absorption values of quadruplicate samples measured in an ELISA assay detecting the binding of the cross species specific anti-CD3 binding molecules in form of crude preparations of periplasmatically expressed single-chain antibodies to a construct comprising the N-terminal 1-27 amino acids of the mature human CD3 epsilon chain fused to the hinge and Fc gamma portion of human IgG1 and a C-terminal His6 tag. The columns show from left to right the average absorption values for the specificities designated as A2J HLP, I2C HLP, E2M HLP, F7O HLP, G4H HLP, H2C HLP, E1L HLP, F12Q HLP, F6A HLP and H1E HLP. The rightmost column labelled "neg. contr." shows the average absorption value for the single-chain preparation of a murine anti-human CD3 antibody as negative control. The comparison of the values obtained for the anti-CD3 specificities with the values obtained for the negative control clearly demonstrates the strong binding of the anti-CD3 specificities to the N-terminal 1-27 amino acids of the mature human CD3 epsilon chain.

**Figure 4**
Fusion of the N-terminal amino acids 1-27 of primate CD3 epsilon to a heterologous membrane bound protein.

**Figure 5**
Histogram overlays of different transfectants tested in a FACS assay detecting the presence of recombinant transmembrane fusion proteins consisting of cynomolgus EpCAM and the N-terminal 1-27 amino acids of the human, marmoset, tamarin, squirrel monkey and domestic swine CD3 epsilon chain respectively. The histogram overlays from left to right and top to bottom show the results for the transfectants expressing the constructs comprising the human 27 mer, marmoset 27 mer, tamarin 27 mer, squirrel monkey 27 mer and swine 27 mer respectively. In the individual overlays the thin line represents a sample incubated with PBS with 2% FCS instead of anti-Flag M2 antibody as negative control and the bold line shows a sample incubated with the anti-Flag M2 antibody. For each construct the overlay of the histograms shows binding of the anti-Flag M2 antibody to the transfectants, which clearly demonstrates the expression of the recombinant constructs on the transfectants.

**Figure 6**

Histogram overlays of different transfectants tested in a FACS assay detecting the binding of the cross-species specific anti-CD3 binding molecules in form of crude preparations of periplasmatically expressed single-chain antibodies to the N-terminal amino acids 1-27 of the human, marmoset, tamarin and squirrel monkey CD3 epsilon chain respectively fused to cynomolgus EpCAM.

**Figure 6A:**

The histogram overlays from left to right and top to bottom show the results for the transfectants expressing the 1-27 CD3-EpCAM comprising the human 27 mer tested with the CD3 specific binding molecules designated H2C HLP, F12Q HLP, E2M HLP and G4H HLP respectively.

**Figure 6B:**

The histogram overlays from left to right and top to bottom show the results for the transfectants expressing the 1-27 CD3-EpCAM comprising the marmoset 27 mer tested with the CD3 specific binding molecules designated H2C HLP, F12Q HLP, E2M HLP and G4H HLP respectively.

**Figure 6C:**

The histogram overlays from left to right and top to bottom show the results for the transfectants expressing the 1-27 CD3-EpCAM comprising the tamarin 27 mer tested with the CD3 specific binding molecules designated H2C HLP, F12Q HLP, E2M HLP and G4H HLP respectively.

**Figure 6D:**

The histogram overlays from left to right and top to bottom show the results for the transfectants expressing the 1-27 CD3-EpCAM comprising the squirrel monkey 27 mer tested with the CD3 specific binding molecules designated H2C HLP, F12Q HLP, E2M HLP and G4H HLP respectively.

**Figure 6E:**

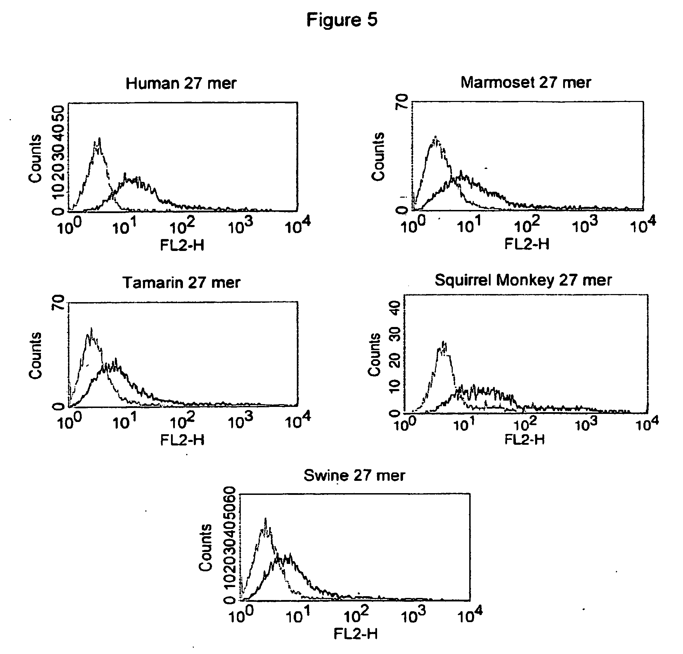

The histogram overlays from left to right and top to bottom show the results for the transfectants expressing the 1-27 CD3-EpCAM comprising the swine 27 mer tested with the CD3 specific binding molecules designated H2C HLP, F12Q HLP, E2M HLP and G4H HLP respectively.
In the individual overlays the thin line represents a sample incubated with a single-chain preparation of a murine anti-human CD3-antibody as negative control and the bold line shows a sample incubated with the respective anti-CD3 binding molecules indicated. Considering the lack of binding to the swine 27 mer transfectants and the expression levels of the constructs shown in Figure 5 the overlays of the histograms show specific and strong binding of the tested anti-CD3 specificities of the fully cross-species specific human bispecific single chain antibodies to cells expressing the recombinant transmembrane fusion proteins comprising the N-terminal amino acids 1-27 of the human, marmoset, tamarin and squirrel monkey CD3 epsilon chain respectively fused to cynomolgus EpCAM and show therefore multi primate cross-species specificity of the anti-CD3 binding molecules.

**Figure 7**

FACS assay for detection of human CD3 epsilon on transfected murine EL4 T cells. Graphical analysis shows an overlay of histograms. The bold line shows transfected cells incubated with the anti-human CD3 antibody UCHT-1. The thin line represents ceiis incubated with a mouse IgG1 isotype control. Binding of the anti CD3 antibody UCHT1 clearly shows expression of the human CD3 epsilon chain on the cell surface of transfected murine EL4 T cells.

**Figure 8**

Binding of cross-species specific anti CD3 antibodies to alanine-mutants in an alanine scanning experiment. In the individual Figures the columns show from left to right the calculated binding values in arbitrary units in logarithmic scale for the wild-type transfectant (WT) and for all alanine-mutants from the position 1 to 27. The binding values are calculated using the following formula:

$$value\_Sample(x,y) = \frac{Sample(x,y) - neg\_Contr.(x)}{(UCHT-1(x) - neg\_Contr.(x)) * \dfrac{WT(y) - neg\_Contr.(wt)}{UCHT-1(wt) - neg\_Contr.(wt)}}$$

In this equation *value_Sample* means the value in arbitrary units of binding depicting the degree of binding of a specific anti-CD3 antibody to a specific alanine-mutant as shown in the Figure, *Sample* means the geometric mean fluorescence value obtained for a specific anti-CD3 antibody assayed on a specific alanine-scanning transfectant, *neg_Contr.* means the geometric mean fluorescence value obtained for the negative control assayed on a specific alanine-mutant, *UCHT*-1 means the geometric mean fluorescence value obtained for the UCHT-1 antibody assayed on a specific alanine-mutant, *WT* means the geometric mean fluorescence value obtained for a specific anti-CD3 antibody assayed on the wild-type transfectant, *x* specifies the respective transfectant, y specifies the respective anti-CD3 antibody and *wt* specifies that the respective transfectant is the wild-type. Individual alanine-mutant positions are labelled with the single letter code of the wild-type amino acid and the number of the position.

**Figure 8A:**
The figure shows the results for cross-species specific anti CD3 antibody A2J HLP expressed as chimeric IgG molecule. Reduced binding activity is observed for mutations to alanine at position 4 (asparagine), at position 23 (threonine) and at position 25 (isoleucine). Complete loss of binding is observed for mutations to alanine at position 1 (glutamine), at position 2 (aspartate), at position 3 (glycine) and at position 5 (glutamate).

**Figure 8B:**
The figure shows the results for cross-species specific anti CD3 antibody E2M HLP, expressed as chimeric IgG molecule. Reduced binding activity is observed for mutations to alanine at position 4 (asparagine), at position 23 (threonine) and at position 25 (isoleucine). Complete loss of binding is observed for mutations to alanine at position 1 (glutamine), at position 2 (aspartate), at position 3 (glycine) and at position 5 (glutamate).

**Figure 8C:**
The figure shows the results for cross-species specific anti CD3 antibody H2C HLP, expressed as chimeric IgG molecule. Reduced binding activity is observed for mutations to alanine at position 4 (asparagine). Complete loss of binding is observed for mutations to alanine glutamine at position 1 (glutamine), at position 2 (aspartate), at position 3 (glycine) and at position 5 (glutamate).

**Figure 8D:**
shows the results for cross-species specific anti CD3 antibody F12Q HLP, tested as periplasmatically expressed single-chain antibody. Complete loss of binding is observed for mutations to alanine at position 1 (glutamine), at position 2 (aspartate), at position 3 (glycine) and at position 5 (glutamate).

**Figure 9**
FACS assay detecting the binding of the cross-species specific anti-CD3 binding molecule H2C HLP to human CD3 with and without N-terminal His6 tag.
Histogram overlays are performed of the EL4 cell line transfected with wild-type human CD3 epsilon chain (left histogram) or the human CD3 epsilon chain with N-terminal His6 tag (right histogram) tested in a FACS assay detecting the binding of cross-species specific binding molecule H2C HLP. Samples are incubated with an appropriate isotype control as negative control (thin line), anti-human CD3 antibody UCHT-1 as positive control (dotted line) and cross-species specific anti-CD3 antibody H2C HLP in form of a chimeric IgG molecule (bold line).
Histogram overlays show comparable binding of the UCHT-1 antibody to both transfectants as compared to the isotype control demonstrating expression of both recombinant constructs. Histogram overlays also show binding of the anti-CD3 binding molecule H2C HLP only to the wild-type human CD3 epsilon chain but not to the His6-human CD3 epsilon chain. These results demonstrate that a free N-terminus is essential for binding of the cross-species specific anti-CD3 binding molecule H2C HLP.

**Figure 10**
Saturation binding of EGFR-21-63 LH x H2C on human CD3 positive PBMC to determine the KD value of CD3 binding on cells by FACS analysis. The assay is performed as described in Example 7.

**Figure 11**

FACS binding analysis of designated cross-species specific bispecific single chain constructs to CHO cells transfected with human EGFR, human CD3+ T cell line HPB-ALL, CHO cells transfected with cynomolgus EGFR and a macaque T cell line 4119 LnPx. The FACS staining is performed as described in Example 12. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 12**

Cytotoxicity activity induced by designated cross-species specific EGFR specific single chain constructs redirected to indicated target cell lines. A) and B) Stimulated CD4-/CD56- human PBMCs are used as effector cells, CHO cells transfected with human EGFR as target cells. The assay is performed as described in Example 13.

**Figure 13**

Cytotoxicity activity induced by designated cross-species specific EGFR specific single chain constructs redirected to indicated target cell lines. A) and B) The macaque T cell line 4119 LnPx are used as effector cells, CHO cells transfected with cynomolgus EGFR as target cells. The assay is performed as described in Example 13.

**Figure 14**

FACS binding analysis of designated cross-species specific bispecific single chain constructs to CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 17. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 15**

FACS binding analysis of designated cross-species specific bispecific single chain constructs CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 17. The thick line represents cells incubated with 2 μg/ml purified protein that are subsequently incubated with the anti-his antibody and the PE labeled detection antibody. The thin histogram line reflects the negative control: cells only incubated with the anti-his antibody and the detection antibody.

**Figure 16**

FACS binding analysis of designated cross-species specific bispecific single chain constructs CHO cells transfected with the human MCSP D3, human CD3+ T cell line HPB-ALL, CHO cells transfected with cynomolgus MCSP D3 and a macaque T cell line 4119 LnPx. The FACS staining is performed as described in Example 17. The thick line represents cells incubated with 2 μg/ml purified monomeric protein that are subsequently incubated with the anti-his antibody and the PE labeled detection antibody. The thin histogram line reflects the negative control: cells only incubated with the anti-his antibody and the detection antibody.

**Figure 17**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 18.

**Figure 18**

Cytotoxicity activity induced by designated cross-species specific MCSP specific single chain constructs redirected to indicated target cell lines. A) and B) The macaque T cell line 4119 LnPx are used as effector cells, CHO cells transfected with cynomolgus MCSP D3 as target cells. The assay is performed as described in Example 18.

**Figure 19**

Cytotoxicity activity induced by designated cross-species specific MCSP specific single chain constructs redirected to indicated target cell lines. A) and B) Stimulated CD4-/CD56- human PBMCs are used as effector cells, CHO cells transfected with human MCSP D3 as target cells. The assay is performed as described in Example 18.

**Figure 20**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 18.

**Figure 21**

Cytotoxicity activity induced by designated cross-species specific MCSP specific single chain constructs redirected to indicated target cell lines. A) Stimulated CD4-/CD56- human PBMCs are used as effector cells, CHO cells transfected with human MCSP D3 as target cells. B) The macaque T cell line 4119 LnPx are used as effector cells, CHO cells transfected with cynomolgus MCSP D3 as target cells. The assay is performed as described in Example 18.

**Figure 22**

Plasma stability of MCSP and CD3 cross-species specific bispecific single chain antibodies tested by the measurement of cytotoxicity activity induced by samples of the designated single chain constructs incubated with 50% human plasma at 37°C and 4°C for 24 hours respectively or with addition of 50% human plasma immediately prior to cytotoxicity testing or without addition of plasma. CHO cells transfected with human MCSP are used as target cell line and stimulated CD4-/CD56- human PBMCs are used as effector cells. The assay is performed as described in Example 19.

**Figure 23**

FACS binding analysis of designated cross-species specific bispecific single chain constructs to CHO cells transfected with human HER2, the human CD3+ T cell line HPB-ALL, CHO cells transfected with macaque HER2 and the macaque T cell line 4119LnPx respectively. The FACS staining is performed as described in Example 23.4. The bold lines represent cells incubated with 2 μg/ml purified bispecific single chain construct. The thin lines reflect the negative controls. PBS with 2% FCS was used as negative control. For each cross-species specific bispecific single chain construct the overlay of the histograms shows specific binding of the construct to human and macaque HER2 and human and macaque CD3.

**Figure 24**

The diagrams show results of chromium release assays measuring cytotoxic activity induced by designated cross-species specific HER2 specific single chain constructs redirected to the indicated target cell lines. Effector cells were also used as indicated. The assays are performed as described in Example 23.5. The diagrams clearly demonstrate for each construct shown the potent recruitment of cytotoxic activity of human and macaque effector cells against human and macaque HER2 transfected CHO cells, respectively.

**Figure 25**

CD3 specific ELISA analysis of periplasmic preparations containing Flag tagged scFv protein fragments from selected clones. Periplasmic preparations of soluble scFv protein fragments were added to wells of an ELISA plate, which had been coated with soluble human CD3 epsilon (aa 1-27)-Fc fusion protein and had been additionally blocked with PBS 3% BSA. Detection was performed by a monoclonal anti Flag-Biotin-labeled antibody followed by peroxidase-conjugated Streptavidin. The ELISA was developed by an ABTS substrate solution. The OD values (y axis) were measured at 405 nm by an EUSA reader. Clone names are presented on the x axis.

**Figure 26**

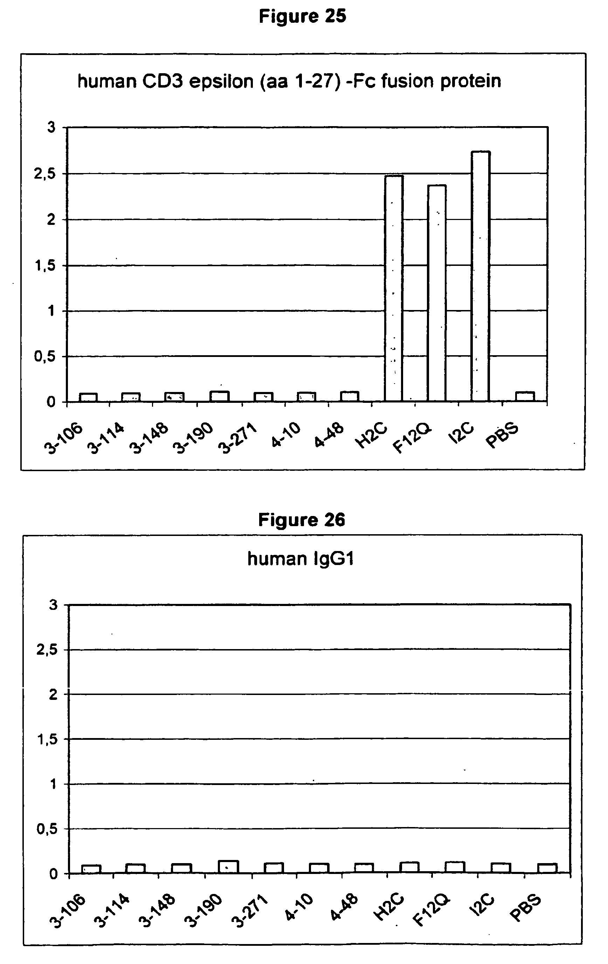

ELISA analysis of periplasmic preparations containing Flag tagged scFv protein fragments from selected clones. The same periplasmic preparations of soluble scFv protein fragments as in Figure 25 were added to wells of an ELISA plate which had not been coated with human CD3 epsilon (aa 1-27)-Fc fusion protein but with hulgG1 (Sigma) and blocked with 3% BSA in PBS.

Detection was performed by a monoclonal anti Flag-Biotin-labeled antibody followed by peroxidase-conjugated Streptavidin. The ELISA was developed by an ABTS substrate solution. The OD values (y axis) were measured at 405 nm by an ELISA reader. Clone names are presented on the x axis.

The present invention is additionally described by way of the following illustrative non-limiting examples that provide a better understanding of the present invention and of its many advantages.

**EXAMPLES**

**1. Identification of CD3epsilon sequences from blood samples of non-human primates**

Blood samples of the following non-human primates were used for CD3epsilon-identification: *Callithrix jacchus, Saguinus oedipus* and *Saimiris scivreus.* Fresh heparin-treated whole blood samples were prepared for isolating total cellular RNA according to manufacturer's protocol (QIAamp RNA Blood Mini Kit, Qiagen). The extracted mRNA was transcribed into cDNA according to published protocols. In brief, 10 μl of precipitated RNA was incubated with 1.2 μl of 10x hexanucleotide mix (Roche) at 70 °C for 10 minutes and stored on ice. A reaction mix consisting of 4 μl of 5x superscript II buffer, 0.2 μl of 0.1M dithiothreitole, 0.8 μl of superscript II (Invitrogen), 1.2 μl of desoxyribonucleoside triphosphates (25 μM), 0.8 μl of RNase Inhibitor (Roche) and 1.8 μl of DNase and RNase free water (Roth) was added. The reaction mix was incubated at room temperature for 10 minutes followed by incubation at 42 °C for 50 minutes and at 90 °C for 5 minutes. The reaction was cooled on ice before adding 0.8 μl of RNaseH (1 U/μl, Roche) and incubated for 20 minutes at 37 °C.

The first-strand cDNAs from each species were subjected to separate 35-cycle polymerase chain reactions using Taq DNA polymerase (Sigma) and the following primer combination designed on database research: forward primer 5'-AGAGTTCTGGGCCTCTGC-3' (SEQ ID NO: 377); reverse primer 5'-CGGATGGGCTCATAGTCTG-3' (SEQ ID NO: 378);. The amplified 550 bp-bands were gel purified (Gel Extraction Kit, Qiagen) and sequenced (Sequiserve, Vaterstetten/Germany, see sequence listing).

CD3epsilon *Callithrix jacchus*

Nucleotides

```
CAGGACGGTAATGAAGAAATGGGTGATACTACACAGAACCCATATAAAGTTTCCATCTCAGG
AACCACAGTAACACTGACATGCCCTCGGTATGATGGACATGAAATAAAATGGCTCGTAAATA
GTCAAAACAAAGAAGGTCATGAGGACCACCTGTTACTGGAGGACTTTTCGGAAATGGAGCAA
AGTGGTTATTATGCCTGCCTCTCCAAAGAGACTCCCGCAGAAGAGGCGAGCCATTATCTCTA
CCTGAAGGCAAGAGTGTGTGAGAACTGCGTGGAGGTGGAT
```

**Amino acids**

```
QDGNEEMGDTTQNPYKVSISGTTVTLTCPRYDGHEIKWLVNSQNKEGHEDHLLLEDFSEMEQ
SGYYACLSKETPAEEASHYLYLKARVCENCVEVD
```

**CD3epsilon *Saguinus oedipus***

**Nucleotides**

```
CAGGACGGTAATGAAGAAATGGGTGATACTACACAGAACCCATATAAAGTTTCCATCTCAGG
AACCACAGTAACACTGACATGCCCTCGGTATGATGGACATGAAATAAAATGGCTTGTAAATA
GTCAAAACAAAGAAGGTCATGAGGACCACCTGTTACTGGAGGATTTTTCGGAAATGGAGCAA
AGTGGTTATTATGCCTGCCTCTCCAAAGAGACTCCCGCAGAAGAGGCGAGCCATTATCTCTA
CCTGAAGGCAAGAGTGTGTGAGAACTGCGTGGAGGTGGAT
```

**Amino acids**

QDGNEEMGDTTQNPYKVSISGTTVTLTCPRYDGHEIKWLVNSQNKEGHEDHLLLEDFSEMEQ
SGYYACLSKETPAEEASHYLYLKARVCENCVEVD

**CD3epsilon *Saimiris ciureus***

**Nucleotides**

CAGGACGGTAATGAAGAGATTGGTGATACTACCCAGAACCCATATAAAGTTTCCATCTCAGG
AACCACAGTAACACTGACATGCCCTCGGTATGATGGACAGGAAATAAAATGGCTCGTAAATG
ATCAAAACAAAGAAGGTCATGAGGACCACCTGTTACTGGAAGATTTTTCAGAAATGGAACAA
AGTGGTTATTATGCCTGCCTCTCCAAAGAGACCCCCACAGAAGAGGCGAGCCATTATCTCTA
CCTGAAGGCAAGAGTGTGTGAGAACTGCGTGGAGGTGGAT

**Amino acids**

QDGNEEIGDTTQNPYKVSISGTTVTLTCPRYDGQEIKWLVNDQNKEGHEDHLLLEDFSEMEQ
SGYYACLSKETPTEEASHYLYLKARVCENCVEVD

**2. Generation of cross-species specific single chain antibody fragments (scFv) binding to the N-terminal amino acids 1-27 of CD3epsilon of man and different non-chimpanzee primates**

**2.1. Immunization of mice using the N-terminus of CD3epsilon separated from its native CD3-context by fusion to a heterologous soluble protein**

Ten weeks old F1 mice from balb/c x C57black crossings were immunized with the CD3epsilon-Fc fusion protein carrying the most N-terminal amino acids 1-27 of the mature CD3epsilon chain (1-27 CD3-Fc) of man and/or saimiris sciureus. To this end 40 $\mu$g of the 1-27 CD3-Fc fusion protein with 10 nmol of a thioate-modified CpG-Oligonucleotide (5'-tccatgacgttcctgatgct-3') in 300 ul PBS were injected per mouse intra-peritoneally. Mice receive booster immunizations after 21, 42 and optionally 63 days in the same way. Ten days after the first booster immunization, blood samples were taken and antibody serum titer against 1-27 CD3-Fc fusion protein iwa tested by ELISA. Additionally, the titer against the CD3-positive human T cell line HPBall was tested in flow cytometry according to standard protocols. Serum titers were significantly higher in immunized than in non-immunized animals.

**2.2. Generation of an immune murine antibody scFv library: Construction of a combinatorial antibody library and phage display**

Three days after the last injection the murine spleen cells were harvested for the preparation of total RNA according to standard protocols.
A library of murine immunoglobuline (Ig) light chain (kappa) variable region (VK) and Ig heavy chain variable region (VH) DNA-fragments was constructed by RT-PCR on murine spleen RNA using VK-and VH specific primer. cDNA was synthesized according to standard protocols.
The primers were designed in a way to give rise to a 5'-Xhol and a 3'-BstEII recognition site for the amplified heavy chain V-fragments and to a 5'-Sacl and a 3'-Spel recognition site for amplified VK DNA fragments.
For the PCR-amplification of the VH DNA-fragments eight different 5'-VH-family specific primers (MVH1(GC) AG GTG CAG CTC GAG GAG TCA GGA CCT; MVH2 GAG GTC CAG CTC GAG CAG TCT GGA CCT; MVH3 CAG GTC CAA CTC GAG CAG CCT GGG GCT; MVH4 GAG GTT CAG CTC GAG CAG TCT GGG GCA; MVH5 GA(AG) GTG AAG CTC GAG GAG TCT GGA GGA; MVH6 GAG GTG AAG CTT CTC GAG TCT GGA GGT; MVH7 GAA GTG AAG CTC GAG GAG TCT GGG GGA; MVH8 GAG GTT CAG CTC GAG CAG TCT GGA GCT) were each combined with one 3'-VH primer (3'MuVHBstEII tga gga gac ggt gac cgt ggt ccc ttg gcc cca g); for the PCR amplification of the VK-chain fragments seven different 5'-VK-family specific primers (MUVK1 CCA GTT CCG AGC TCG TTG TGA CTC AGG AAT CT; MUVK2 CCA GTT CCG AGC TCG TGT TGA CGC AGC CGC CC; MUVK3 CCA GTT CCG AGC TCG TGC TCA CCC AGT CTC CA; MUVK4 CCA GTT CCG AGC TCC AGA TGA CCC AGT CTC CA; MUVK5 CCA GAT GTG AGC TCG TGA TGA CCC AGA CTC CA; MUVK6 CCA GAT GTG AGC TCG TCA TGA CCC AGT CTC CA; MUVK7 CCA GTT CCG AGC TCG TGA TGA CAC AGT CTC CA) were each combined with one 3'-VK primer (3'MuVkHindIII/ BsiW1 tgg tgc act agt cgt acg ttt gat ctc aag ctt ggt ccc).

The following PCR program was used for amplification: denaturation at 94°C for 20 sec; primer annealing at 52°C for 50 sec and primer extension at 72°C for 60 sec and 40 cycles, followed by a 10 min final extension at 72°C.

450 ng of the kappa light chain fragments (SacI-SpeI digested) were ligated with 1400 ng of the phagemid pComb3H5Bhis (SacI-SpeI digested; large fragment). The resulting combinatorial antibody library was then transformed into 300 ul of electrocompetent Escherichia coli XL1 Blue cells by electroporation (2.5 kV, 0.2 cm gap cuvette, 25 uFD, 200 Ohm, Biorad gene-pulser) resulting in a library size of more than $10^7$ independent clones. After one hour of phenotype expression, positive transformants were selected for carbenicilline resistance encoded by the pComb3H5BHis vector in 100 ml of liquid super broth (SB)-culture over night. Cells were then harvested by centrifugation and plasmid preparation was carried out using a commercially available plasmid preparation kit (Qiagen).

2800 ng of this plasmid-DNA containing the VK-library (XhoI-BstEII digested; large fragment) were ligated with 900 ng of the heavy chain V-fragments (XhoI-BstEII digested) and again transformed into two 300 ul aliquots of electrocompetent E. coli XL1 Blue cells by electroporation (2.5 kV, 0.2 cm gap cuvette, 25 uFD, 200 Ohm) resulting in a total VH-VK scFv (single chain variable fragment) library size of more than $10^7$ independent clones.

After phenotype expression and slow adaptation to carbenicillin, the E. coli cells containing the antibody library were transferred into SB-Carbenicillin (50 ug/mL) selection medium. The E. coli cells containing the antibody library wass then infected with an infectious dose of $10^{12}$ particles of helper phage VCSM13 resulting in the production and secretion of filamentous M13 phage, wherein phage particle contains single stranded pComb3H5BHis-DNA encoding a murine scFv-fragment and displayed the corresponding scFv-protein as a translational fusion to phage coat protein III. This pool of phages displaying the antibody library was later used for the selection of antigen binding entities.

## 2.3. Phage display based selection of CD3-specific binders

The phage library carrying the cloned scFv-repertoire was harvested from the respective culture supernatant by PEG8000/NaCl precipitation and centrifugation. Approximately $10^{11}$ to $10^{12}$ scFv phage particles were resuspended in 0.4 ml of PBS/0.1% BSA and incubated with $10^5$ to $10^7$ Jurkat cells (a CD3-positive human T-cell line) for 1 hour on ice under slow agitation. These Jurkat cells were grown beforehand in RPMI medium enriched with fetal calf serum (10 %), glutamine and penicillin/streptomycin, harvested by centrifugation, washed in PBS and resuspended in PBS/1 % FCS (containing Na Azide). scFv phage which do not specifically bind to the Jurkat cells were eliminated by up to five washing steps with PBS/1 % FCS (containing Na Azide). After washing, binding entities were eluted from the cells by resuspending the cells in HCl-glycine pH 2.2 (10 min incubation with subsequent vortexing) and after neutralization with 2 M Tris pH 12, the eluate was used for infection of a fresh uninfected E. coli XL1 Blue culture (OD600 > 0.5). The E. coli culture containing E. coli cells successfully transduced with a phagemid copy, encoding a human scfv-fragment, were again selected for carbenicillin resistance and subsequently infected with VCMS 13 helper phage to start the second round of antibody display and in vitro selection. A total of 4 to 5 rounds of selections were carried out, normally.

## 2.4. Screening for CD3-specific binders

Plasmid DNA corresponding to 4 and 5 rounds of panning was isolated from E. coli cultures after selection. For the production of soluble scFv-protein, VH-VL-DNA fragments were excised from the plasmids (Xhol-SpeI). These fragments were cloned via the same restriction sites in the plasmid pComb3H5BFlag/His differing from the original pComb3H5BHis in that the expression construct (e.g. scFv) includes a Flag-tag (TGD YKDDDDK) between the scFv and the His6-tag and the additional phage proteins were deleted. After ligation, each pool (different rounds of panning) of plasmid DNA was transformed into 100 $\mu$l heat shock competent E. coli TG1 or XLI blue and plated onto carbenicillin LB-agar. Single colonies were picked into 100 ul of LB carb (50 ug/ml).

E. coli transformed with pComb3H5BHis containing a VL-and VH-segment produce soluble scFv in sufficient amounts after excision of the gene III fragment and induction with 1 mM IPTG. Due to a suitable signal sequence, the scFv-chain was exported into the periplasma where it folds into a functional conformation.

Single E. coli TG1 bacterial colonies from the transformation plates were picked for periplasmic small scale preparations and grown in SB-medium (e.g. 10 ml) supplemented with 20 mM MgCl2 and carbenicillin 50μg/ml (and re-dissolved in PBS (e.g. 1 ml) after harvesting. By four rounds of freezing at -70°C and thawing at 37°C, the outer membrane of the bacteria was destroyed by temperature shock and the soluble periplasmic proteins including the scFvs were released into the supernatant. After elimination of intact cells and cell-debris by centrifugation, the supernatant containing the human anti-human CD3-scFvs was collected and used for further examination.

### 2.5. Identification of CD3-specific binders

Binding of the isolated scFvs was tested by flow cytometry on eukaryotic cells, which on their surface express a heterologous protein displaying at its N-terminus the first 27 N-terminal amino acids of CD3epsilon.

As described in Example 4, the first amino acids 1-27 of the N-terminal sequence of the mature CD3 epsilon chain of the human T cell receptor complex (amino acid sequence: QDGNEEMGGITQTPYKVSISGTTVILT) were fused to the N-terminus of the transmembrane protein EpCAM so that the N-terminus was located at the outer cell surface. Additionally, a FLAG epitope was inserted between the N-terminal 1-27 CD3epsilon sequence and the EpCAM sequence. This fusion product was expressed in human embryonic kidney (HEK) and chinese hamster ovary (CHO) cells.

Eukaryotic cells displaying the 27 most N-terminal amino acids of mature CD3epsilon of other primate species were prepared in the same way for Saimiri sciureus (Squirrel monkey) (CD3epsilon N-terminal amino acid sequence: QDGNEEIGDTTQNPYKVSISGTTVTLT), for Callithrix jacchus (CD3epsilon N-terminal amino acid sequence: QDGNEEMGDTTQNPYKVSISGTTVTLT) and for Saguinus oedipus (CD3epsilon N-terminal amino acid sequence: QDGNEEMGDTTQNPYKVSISGTTVTLT).

For flow cytometry $2,5x10^5$ cells are incubated with 50 ul supernatant or with 5 μg/ml of the purified constructs in 50 μl PBS with 2% FCS. The binding of the constructs was detected with an anti-His antibody (Penta-His Antibody, BSA free, Qiagen GmbH, Hilden, FRG) at 2 μg/ml in 50 μl PBS with 2% FCS. As a second step reagent a R-Phycoerythrin-conjugated affinity purified F(ab')2 fragment, goat anti-mouse IgG (Fc-gamma fragment specific), diluted 1:100 in 50 μl PBS with 2% FCS (Dianova, Hamburg, FRG) was used. The samples were measured on a FACSscan (BD biosciences, Heidelberg, FRG).

Binding was always confirmed by flowcytometry as described in the foregoing paragraph on primary T cells of man and different primates (e.g. saimiris sciureus, callithrix jacchus, saguinus oedipus).

### 2.6. Generation of human/humanized equivalents of non-human CD3epsilon specific scFvs

The VH region of the murine anti-CD3 scFv was aligned against human antibody germline amino acid sequences. The human antibody germline VH sequence was chosen which has the closest homology to the non-human VH and a direct alignment of the two amino acid sequences was performed. There were a number of framework residues of the non-human VH that differ from the human VH framework regions ("different framework positions"). Some of these residues may contribute to the binding and activity of the antibody to its target.

To construct a library that contain the murine CDRs and at every framework position that differs from the chosen human VH sequence both possibilities (the human and the maternal murine amino acid residue), degenerated oligonucleotides were synthesized. These oligonucleotides incorporate at the differing positions the human residue with a probability of 75 % and the murine residue with a probability of 25 %. For one human VH e.g. six of these oligonucleotides had to be synthesized that overlap in a terminal stretch of approximately 20 nucleotides. To this end every second primer was an antisense primer. Restriction sites needed for later cloning within the oligonucleotides were deleted.

These primers may have a length of 60 to 90 nucleotides, depending on the number of primers that were needed to span over the whole V sequence.

These e.g. six primers were mixed in equal amounts (e.g. 1 μl of each primer (primer stocks 20 to 100 μM) to a 20 μl PCR reaction) and added to a PCR mix consisting of PCR buffer, nucleotides and Taq polymerase. This mix was incubated at 94 °C for 3 minutes, 65 °C for 1 minute, 62°C for 1 minute, 59 °C for 1 minute, 56 °C for 1 minute, 52 °C for 1 minute, 50 °C for 1 minute and at 72°C for 10 minutes in a PCR cycler. Subsequently the product was run in an agarose gel electrophoresis and the product of a size from 200 to 400 isolated from the gel according to standard methods.

This PCR product was then used as a template for a standard PCR reaction using primers that incorporate N-terminal and C-terminal suitable cloning restriction sites. The DNA fragment of the correct size (for a VH approximately 350 nucleotides) was isolated by agarose gel electrophoresis according to standard methods. In this way sufficient VH DNA fragment was amplified. This VH fragment was now a pool of VH fragments that have each one a different amount of human and murine residues at the respective differing framework positions (pool of humanized VH). The same procedure was performed for the VL region of the murine anti-CD3 scFv (pool of humanized VL).

The pool of humanized VH was then combined with the pool of humanized VL in the phage display vector pComb3H5Bhis to form a library of functional scFvs from which - after display on filamentous phage - anti-CD3 binders were selected, screened, identified and confirmed as described above for the parental non-human (murine) anti-CD3 scFv. Single clones were then analyzed for favorable properties and amino acid sequence. Those scFvs which were closest in amino acid sequence homology to human germline V-segments are preferred particularly those wherein at least one CDR among CDR I and II of VH and CDR I and II of VLkappa or CDR I and II of VLlambda shows more than 80% amino acid sequence identity to the closest respective CDR of all human germline V-segments. Anti-CD3 scFvs were converted into recombinant bispecific single chain antibodies as described in the following Examples 10 and 16 and further characterized.

## 3. Generation of a recombinant fusion protein of the N-terminal amino acids 1-27 of the human CD3 epsilon chain fused to the Fc-part of an IgG1 (1-27 CD3-Fc).

### 3.1. Cloning and expression of 1-27 CD3-Fc

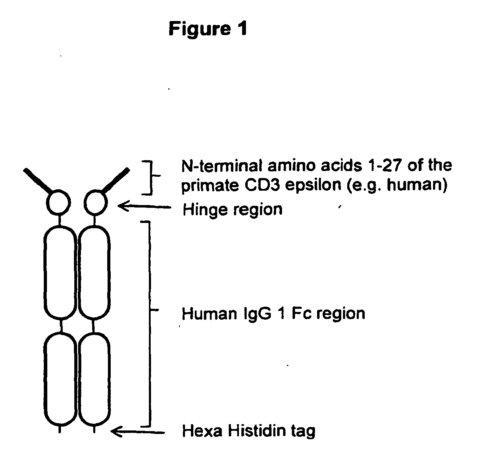

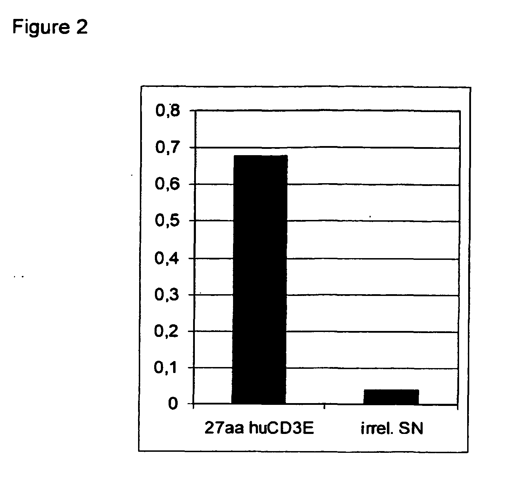

The coding sequence of the 1-27 N-terminal amino acids of the human CD3 epsilon chain fused to the hinge and Fc gamma region of human immunoglobulin IgG1 as well as an 6 Histidine Tag were obtained by gene synthesis according to standard protocols (cDNA sequence and amino acid sequence of the recombinant fusion protein are listed under SEQ ID NOs 350 and 349). The gene synthesis fragment was designed as to contain first a Kozak site for eukaryotic expression of the construct, followed by an 19 amino acid immunoglobulin leader peptide, followed in frame by the coding sequence of the first 27 amino acids of the extracellular portion of the mature human CD3 epsilon chain, followed in frame by the coding sequence of the hinge region and Fc gamma portion of human IgG1, followed in frame by the coding sequence of a 6 Histidine tag and a stop codon (Figure 1). The gene synthesis fragment was also designed as to introduce restriction sites at the beginning and at the end of the cDNA coding for the fusion protein. The introduced restriction sites, EcoRI at the 5' end and SalI at the 3' end, are utilized in the following cloning procedures. The gene synthesis fragment was cloned via EcoRI and SalI into a plasmid designated pEF-DHFR (pEF-DHFR is described in Mack et al. Proc. Natl. Acad. Sci. USA 92 (1995) 7021-7025) following standard protocols. A sequence verified plasmid was used for transfection in the FreeStyle 293 Expression System (Invitrogen GmbH, Karlsruhe, Germany) according to the manufacturer's protocol. After 3 days cell culture supernatants of the transfectants were harvested and tested for the presence of the recombinant construct in an ELISA assay. Goat anti-human IgG, Fc-gamma fragment specific antibody (obtained from Jackson ImmunoResearch Europe Ltd., Newmarket, Suffolk, UK) was diluted in PBS to 5 $\mu$g/ml and coated with 100 $\mu$l per well onto a MaxiSorp 96-well ELISA plate (Nunc GmbH & Co. KG, Wiesbaden, Germany) over night at 4 °C. Wells were washed with PBS with 0,05 % Tween 20 (PBS/Tween and blocked with 3 % BSA in PBS (bovine Albumin, fraction V, Sigma-Aldrich Chemie GmbH, Taufkirchen, Germany) for 60 minutes at room temperature (RT). Subsequently, wells were washed again PBS/Tween and then incubated with cell culture supernatants for 60 minutes at RT. After washing wells were incubated with a peroxidase conjugated anti-His6 antibody (Roche Diagnostics GmbH, Roche Applied Science, Mannheim, Germany) diluted 1:500 in PBS with 1 % BSA for 60 minutes at RT. Subsequently, wells were washed with 200 $\mu$l PBS/Tween and 100 $\mu$l of the SIGMAFAST OPD (SIGMAFAST OPD [o-Phenylenediamine dihydrochloride] substrate solution (Sigma-Aldrich Chemie GmbH, Taufkirchen, Germany) was added according to the manufacturer's protocol. The reaction was stopped by adding 100 $\mu$l 1 M $H_2SO_4$. Color reaction was measured on a PowerWaveX microplate spectrophotometer (BioTek Instruments, Inc., Winooski, Vermont, USA) at 490 nm and subtraction of background absorption at 620 nm. As shown in Figure 2 presence of the construct as compared to irrelevant supernatant of mock-transfected HEK 293 cells used as negative control was clearly detectable.

### 3.2. Binding assay of cross-species specific single chain antibodies to 1-27 CD3-Fc.

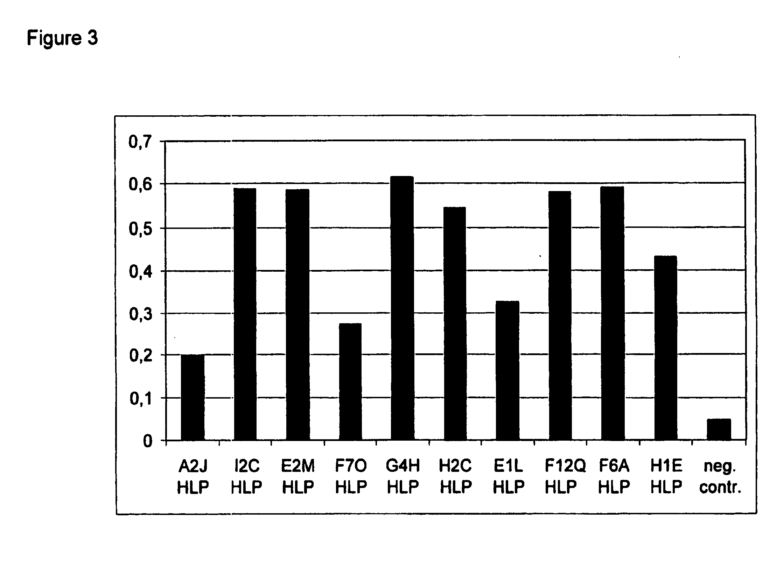

Binding of crude preparations of periplasmatically expressed cross-species specific single chain antibodies specific for CD3 epsilon to 1-27 CD3-Fc was tested in an ELISA assay. Goat anti-human IgG, Fc-gamma fragment specific antibody (Jackson ImmunoResearch Europe Ltd., Newmarket, Suffolk, UK) was diluted in PBS to 5 $\mu$g/ml and coated with 100 $\mu$l per well onto a MaxiSorp 96-well ELISA plate (Nunc GmbH & Co. KG, Wiesbaden, Germany) over night at 4 °C. Wells were washed with PBS with 0,05 % Tween 20 (PBS/Tween and blocked with PBS with 3 % BSA (bovine Albumin, fraction V, Sigma-Aldrich Chemie GmbH, Taufkirchen, Germany) for 60 minutes at RT. Subsequently, wells were washed with PBS/Tween and incubated with supernatants of cells expressing the 1-27 CD3-Fc construct for 60 minutes at RT. Wells were washed with PBS/Tween and incubated with crude preparations of periplasmatically expressed cross-species specific single-chain antibodies as described above for 60 minutes at room temperature. After washing with PBS/Tween wells were incubated with peroxidase conjugated anti-Flag M2 antibody (Sigma-Aldrich Chemie GmbH, Taufkirchen, Germany) diluted 1:10000 in PBS with 1 % BSA for 60 minutes at RT. Wells were washed with PBS/Tween and incubated with 100 $\mu$l of the SIGMAFAST OPD (OPD [o-Phenylenediamine dihydrochloride] substrate solution (Sigma-Aldrich Chemie GmbH, Taufkirchen, Germany) according to the manufacturer's protocol. Color reaction was stopped with 100 $\mu$l 1 M $H_2SO_4$ and measured on a PowerWaveX microplate spectrophotometer (BioTek Instruments, Inc., Winooski, Vermont, USA) at 490 nm and subtraction of background absorption at 620 nm. Strong binding of cross-species specific human single chain antibodies specific for CD3 epsilon to the 1-27 CD3-Fc construct compared to a murine anti CD3 single-chain antibody was observed (Figure 3).

**4. Generation of recombinant transmembrane fusion proteins of the N-terminal amino acids 1-27 of CD3 epsilon from different non-chimpanzee primates fused to EpCAM from cynomolgus monkey (1-27 CD3-EpCAM).**

**4.1. Cloning and expression of 1-27 CD3-EpCAM**

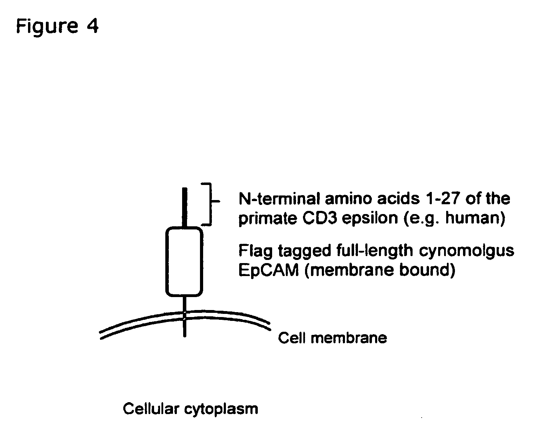

CD3 epsilon was isolated from different non-chimpanzee primates (marmoset, tamarin, squirrel monkey) and swine. The coding sequences of the 1-27 N-terminal amino acids of CD3 epsilon chain of the mature human, common marmoset (*Callithrix jacchus*), cottontop tamarin (*Saguinus oedipus),* common squirrel monkey (*Saimiri sciureus*) and domestic swine (*Sus scrofa;* used as negative control) fused to the N-terminus of Flag tagged cynomolgus EpCAM were obtained by gene synthesis according to standard protocols. cDNA sequence and amino acid sequence of the recombinant fusion proteins are listed under SEQ ID NOs 351 to 360). The gene synthesis fragments were designed as to contain first a BsrGI site to allow fusion in correct reading frame with the coding sequence of a 19 amino acid immunoglobulin leader peptide already present in the target expression vector, which is followed in frame by the coding sequence of the N-terminal 1-27 amino acids of the extracellular portion of the mature CD3 epsilon chains, which is followed in frame by the coding sequence of a Flag tag and followed in frame by the coding sequence of the mature cynomolgus EpCAM transmembrane protein (Figure 4). The gene synthesis fragments were also designed to introduce a restriction site at the end of the cDNA coding for the fusion protein. The introduced restriction sites BsrGI at the 5' end and SalI at the 3' end, were utilized in the following cloning procedures. The gene synthesis fragments were then cloned via BsrGI and SalI into a derivative of the plasmid designated pEF DHFR (pEF-DHFR is described in Mack et al. Proc. Natl. Acad. Sci. USA 92 (1995) 7021-7025), which already contained the coding sequence of the 19 amino acid immunoglobulin leader peptide following standard protocols. Sequence verified plasmids were used to transiently transfect 293-HEK cells using the MATra-A Reagent (IBA GmbH, Göttingen, Germany) and 12 $\mu$g of plasmids DNA for adherent 293-HEK cells in 175 ml cell culture flasks according to the manufacturers protocol. After 3 days of cell culture the transfectants were tested for cell surface expression of the recombinant transmembrane protein via an FACS assay according to standard protocols. For that purpose a number of $2,5\times10^5$ cells were incubated with the anti-Flag M2 antibody (Sigma-Aldrich Chemie GmbH, Taufkirchen, Germany) at 5 $\mu$g/ml in PBS with 2% FCS. Bound antibody was detected with an R-Phycoerythrin-conjugated affinity purified F(ab')2 fragment, goat anti-mouse IgG, Fc-gamma fragment specific 1:100 in PBS with 2% FCS (Jackson ImmunoResearch Europe Ltd., Newmarket, Suffolk, UK). The samples were measured on a FACScalibur (BD biosciences, Heidelberg, Germany). Expression of the Flag tagged recombinant transmembrane fusion proteins consisting of cynomolgus EpCAM and the 1-27 N-terminal amino acids of the human, marmoset, tamarin, squirrel monkey and swine CD3 epsilon chain respectively on transfected cells was clearly detectable (Figure 5).

**4.2. Binding of cross-species specific anti-CD3 single chain antibodies to the 1-27 CD3-EpCAM**

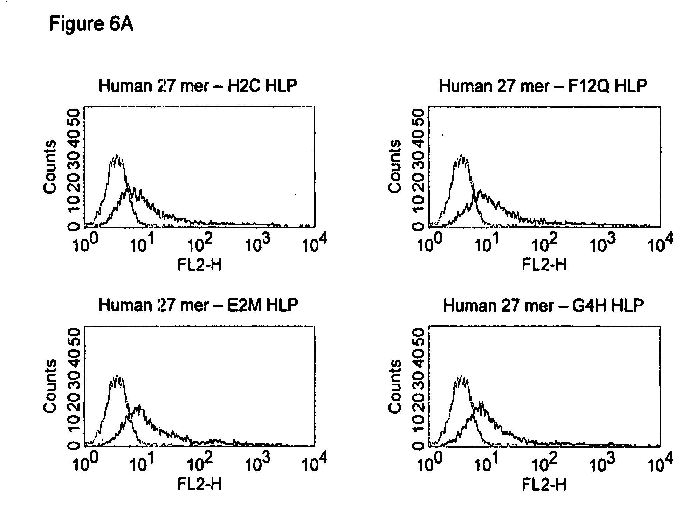

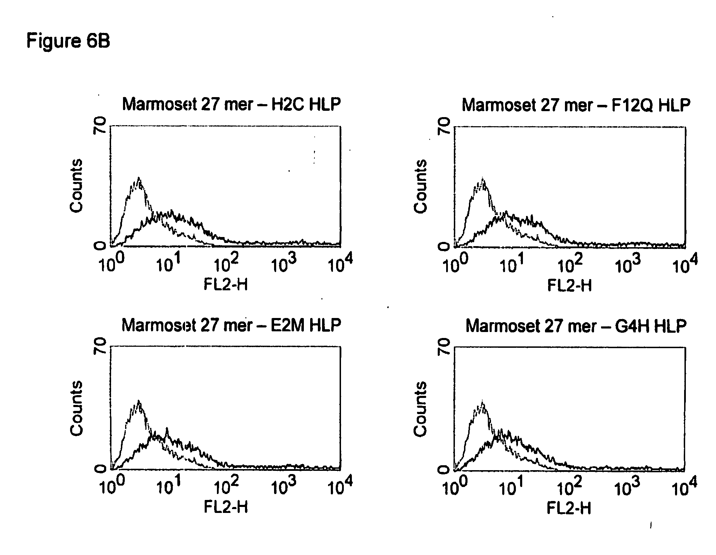

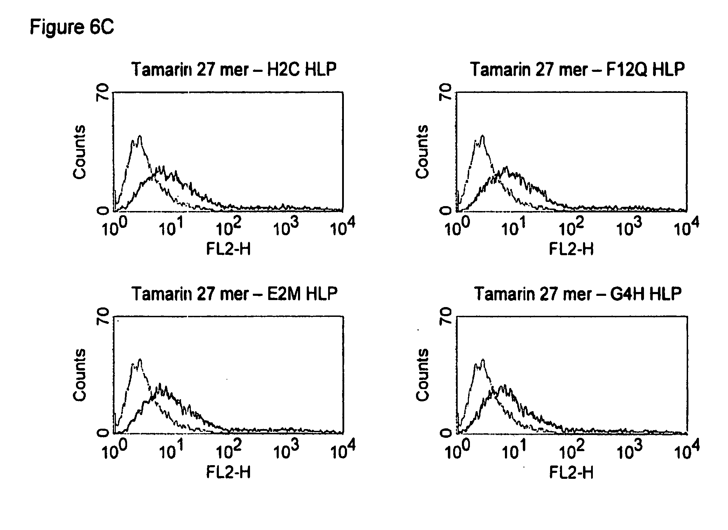

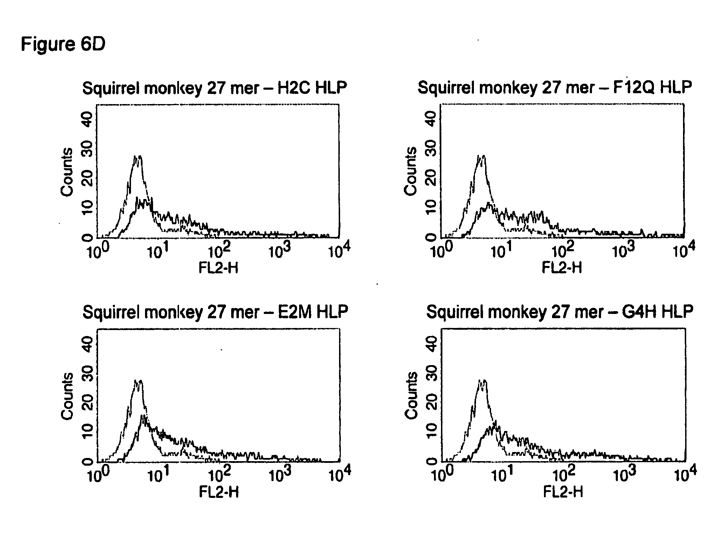

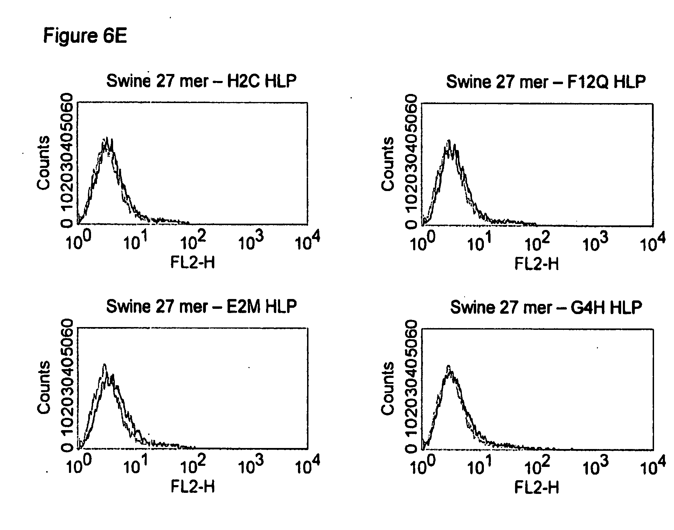

Binding of crude preparations of periplasmatically expressed cross-species specific anti CD3 single-chain antibodies to the 1-27 N-terminal amino acids of the human, marmoset, tamarin and squirrel monkey CD3 epsilon chains respectively fused to cynomolgus Ep-CAM was tested in an FACS assay according to standard protocols. For that purpose a number of $2,5\times10^5$ cells were incubated with crude preparations of periplasmatically expressed cross-species specific anti CD3 single-chain antibodies (preparation was performed as described above and according to standard protocols) and a single-chain murine anti-human CD3 antibody as negative control. As secondary antibody the Penta-His antibody (Qiagen GmbH, Hildesheim, Germany) was used at 5 $\mu$g/ml in 50 $\mu$l PBS with 2% FCS. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). The samples were measured on a FACScalibur (BD biosciences, Heidelberg, Germany). As shown in Figures 6 (A to E) binding of single chain antibodies to the transfectants expressing the recombinant transmembrane fusion proteins consisting of the 1-27 N-terminal amino acids of CD3 epsilon of the human, marmoset, tamarin or squirrel monkey fused to cynomolgus EpCAM was observed. No binding of cross-species specific single chain antibodies was observed to a fusion protein consisting of the 1-27 N-terminal CD3 epsilon of swine fused to cynomolgus EpCAM used as negative control. Multi-primate cross-species specificity of the anti-CD3 single chain antibodies was shown. Signals obtained with the anti Flag M2 antibody and the cross-species specific single chain antibodies were comparable, indicating a strong binding activity of the cross-species specific single chain antibodies to the N-terminal amino acids 1-27 of CD3 epsilon.

**5. Binding analysis of cross-species specific anti-CD3 single chain antibodies by alanine-scanning of mouse cells transfected with the human CD3 epsilon chain and its alanine mutants**

**5.1. Cloning and expression of human wild-type CD3 epsilon**

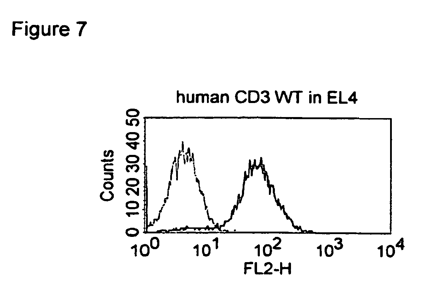

The coding sequence of the human CD3 epsilon chain was obtained by gene synthesis according to standard protocols (cDNA sequence and amino acid sequence of the human CD3 epsilon chain are listed under SEQ ID NOs 362 and 361). The gene synthesis fragment was designed as to contain a Kozak site for eukaryotic expression of the construct and restriction sites at the beginning and the end of the cDNA coding for human CD3 epsilon. The introduced restriction sites EcoRI at the 5' end and SaII at the 3' end, were utilized in the following cloning procedures. The gene synthesis fragment was then cloned via EcoRI and SaII into a plasmid designated pEF NEO following standard protocols. pEF NEO was derived of pEF DHFR (Mack et al. Proc. Natl. Acad. Sci. USA 92 (1995) 7021-7025) by replacing the cDNA of the DHFR with the cDNA of the neomycin resistance by conventional molecular cloning. A sequence verified plasmid was used to transfect the murine T cell line EL4 (ATCC No. TIB-39) cultivated in RPMI with stabilized L-glutamine supplemented with 10 % FCS, 1% penicillin/streptomycin, 1% HEPES, 1% pyruvate, 1% non-essential amino acids (all Biochrom AG Berlin, Germany) at 37 °C, 95 % humidity and 7 % $CO_2$. Transfection was performed with the SuperFect Transfection Reagent (Qiagen GmbH, Hilden, Germany) and 2 µg of plasmid DNA according to the manufacturer's protocol. After 24 hours the cells were washed with PBS and cultivated again in the aforementioned ceii culture medium with 600 µg/ml G418 for selection (PAA Laboratories GmbH, Pasching, Austria). 16 to 20 days after transfection the outgrowth of resistant cells was observed. After additional 7 to 14 days cells were tested for expression of human CD3 epsilon by FACS analysis according to standard protocols. $2,5x10^5$ cells were incubated with anti-human CD3 antibody UCHT-1 (BD biosciences, Heidelberg, Germany) at 5 µg/ml in PBS with 2% FCS. The binding of the antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). The samples were measured on a FACSCalibur (BD biosciences, Heidelberg, Germany). Expression of human wild-type CD3 on transfected EL4 cells is shown in Figure 7.

**5.2. Cloning and expression of the cross-species specific anti-CD3 single chain antibodies as IgG1 antibodies**

In order to provide improved means of detection of binding of the cross-species specific single chain anti-CD3 antibodies H2C HLP, A2J HLP and E2M HLP were converted into IgG1 antibodies with murine IgG1 and human lambda constant regions. cDNA sequences coding for the heavy and light chains of respective IgG antibodies were obtained by gene synthesis according to standard protocols. The gene synthesis fragments for each specificity were designed as to contain first a Kozak site to allow eukaryotic expression of the construct, which is followed by an 19 amino acid immunoglobulin leader peptide (SEQ ID NOs 364 and 363), which is followed in frame by the coding sequence of the respective heavy chain variable region or respective light chain variable region, followed in frame by the coding sequence of the heavy chain constant region of murine IgG1 (SEQ ID NOs 366 and 365) or the coding sequence of the human lambda light chain constant region (SEQ ID NO 368 and 367), respectively. Restriction sites were introduced at the beginning and the end of the cDNA coding for the fusion protein. Restriction sites EcoRI at the 5' end and SaII at the 3' end were used for the following cloning procedures. The gene synthesis fragments were cloned via EcoRI and SaII into a plasmid designated pEF DHFR (Mack et al. Proc. Natl. Acad. Sci. USA 92 (1995) 7021-7025) for the heavy chain constructs and pEF ADA (pEF ADA is described in Raum et al., Cancer immunoi immunother., 50(3), (2001), 141-50) for the light chain constructs) according to standard protocols. Sequence verified plasmids were used for co-transfection of respective light and heavy chain constructs in the FreeStyle 293 Expression System (Invitrogen GmbH, Karlsruhe, Germany) according to the manufacturer's protocol. After 3 days cell culture supernatants of the transfectants were harvested and used for the alanine-scanning experiment.

**5.3. Cloning and expression of alanine mutants of human CD3 epsilon for alanine-scanning**

27 cDNA fragments coding for the human CD3 epsilon chain with an exchange of one codon of the wild-type sequence of human CD3 epsilon into a codon coding for alanine (GCC) for each amino acid of amino acids 1-27 of the extracellular domain of the mature human CD3 epsilon chain respectively were obtained by gene synthesis. Except for the exchanged codon the cDNA fragments were identical to the aforementioned human wild-type CD3 cDNA fragment. Only one codon was replaced in each construct compared to the human wild-type CD3 cDNA fragment described above. Restriction sites EcoRI and SaII were introduced into the cDNA fragments at identical positions compared to the wild-type construct. All alanine-scanning constructs were cloned into pEF NEO and sequence verified plasmids were transfected into EL4 cells. Transfection and selection of transfectants was performed as described above. As result a panel of expressed constructs was obtained wherein the first amino acid of the human CD3 epsilon chain, glutamine (Q, Gln) at position 1 was replaced by alanine. The last amino acid replaced by alanine was the threonine (T, Thr) at position 27 of mature human wild-type CD3 epsilon. For each amino acid between glutamine 1 and threonine 27 respective transfectants with an exchange of the wild-type amino acid into alanine were generated.

## 5.4. Alanine-scanning experiment

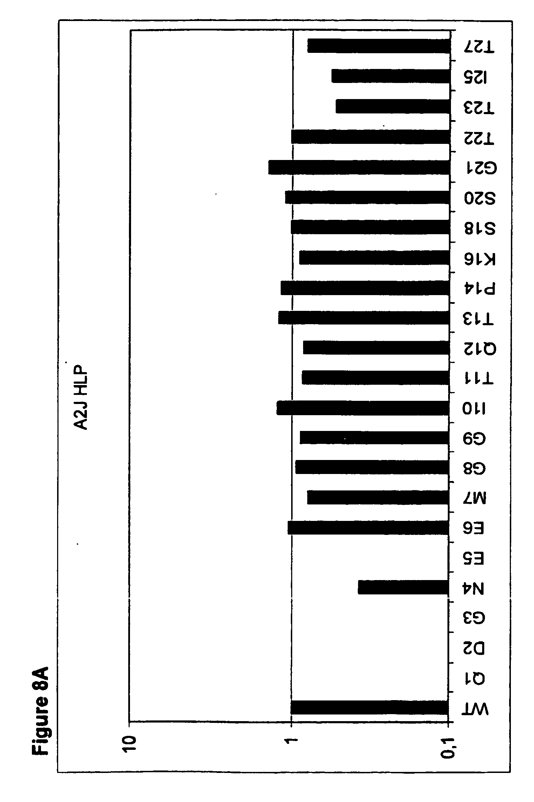

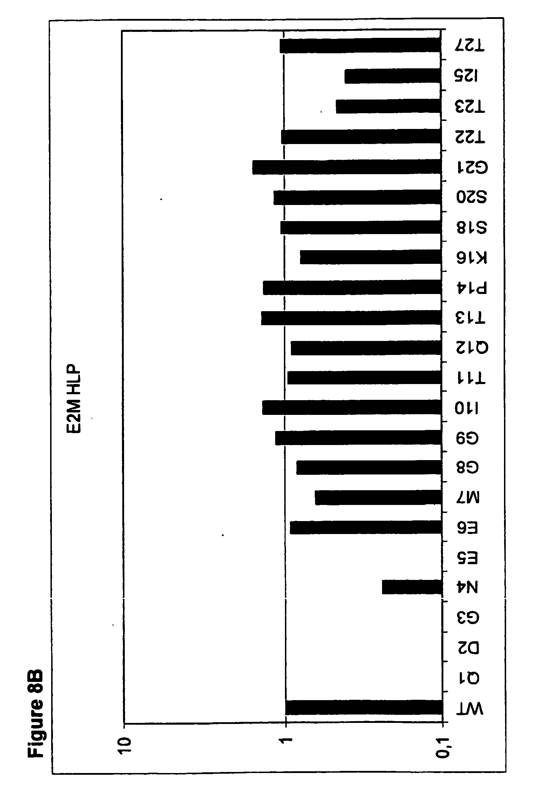

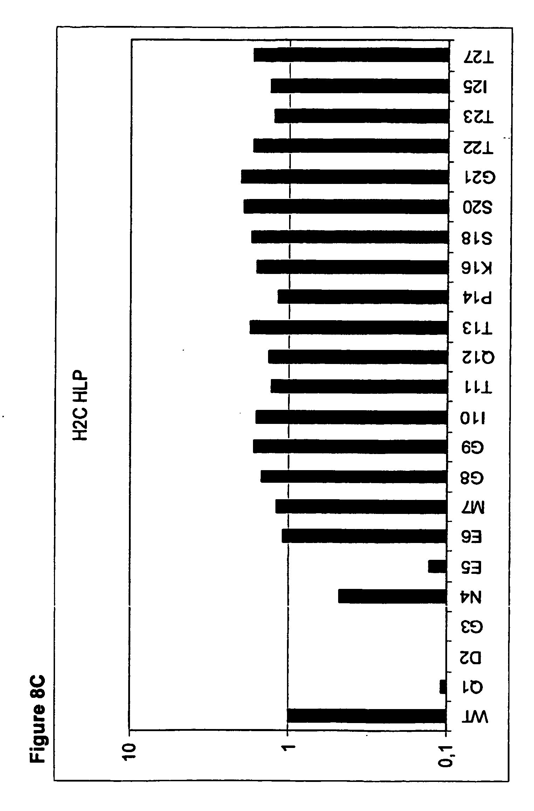

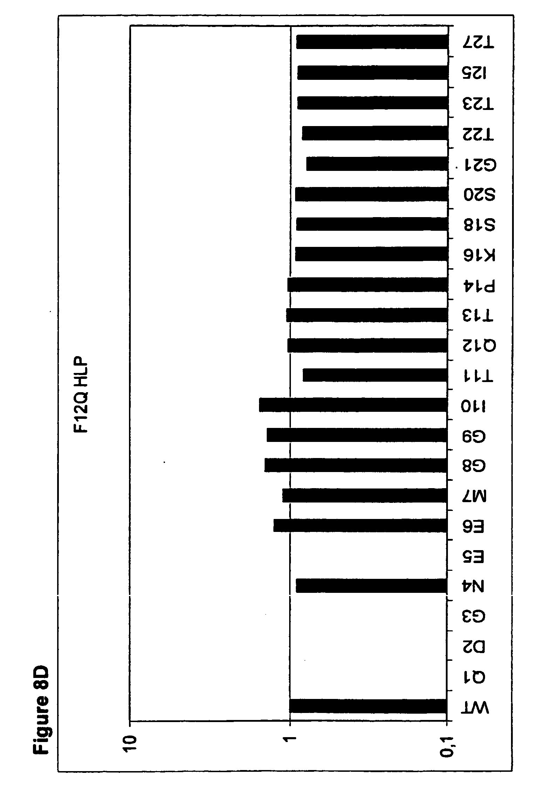

Chimeric IgG antibodies as described in 2) and cross-species specific single chain antibodies specific for CD3 epsilon were tested in alanine-scanning experiment. Binding of the antibodies to the EL4 cell lines transfected with the alanine-mutant constructs of human CD3 epsilon as described in 3) was tested by FACS assay according to standard protocols. $2,5 \times 10^5$ cells of the respective transfectants were incubated with 50 $\mu$l of cell culture supernatant containing the chimeric IgG antibodies or with 50 $\mu$l of crude preparations of periplasmatically expressed single-chain antibodies. For samples incubated with crude preparations of periplasmatically expressed single-chain antibodies the anti-Flag M2 antibody (Sigma-Aldrich Chemie GmbH, Taufkirchen, Germany) was used as secondary antibody at 5 $\mu$g/ml in 50 $\mu$l PBS with 2% FCS. For samples incubated with the chimeric IgG antibodies a secondary antibody was not necessary. For all samples the binding of the antibody molecules was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACSCalibur (BD biosciences, Heidelberg, Germany). Differential binding of chimeric IgG molecules or cross-species specific single-chain antibodies to the EL4 cell lines transfected with the alanine-mutants of human CD3 epsilon was detected. As negative control either an isotype control or a crude preparation of a periplasmatically expressed single-chain antibody of irrelevant specificity was used respectively. UCHT-1 antibody was used as positive control for the expression level of the alanine-mutants of human CD3 epsilon. The EL4 cell lines transfected with the alanine-mutants for the amino acids tyrosine at position 15, valine at position 17, isoleucine at position 19, valine at position 24 or leucine at position 26 of the mature CD3 epsilon chain were not evaluated due to very low expression levels (data not shown). Binding of the cross-species specific single chain antibodies and the single chain antibodies in chimeric IgG format to the EL4 cell lines transfected with the alanine-mutants of human CD3 epsilon is shown in Figure 8 (A-D) as relative binding in arbitrary units with the geometric mean fluorescence values of the respective negative controls subtracted from all respective geometric mean fluorescence sample values. To compensate for different expression levels all sample values for a certain transfectant were then divided through the geometric mean fluorescence value of the UCHT-1 antibody for the respective transfectant. For comparison with the wild-type sample value of a specificity all sample values of the respective specificity were finally divided through the wild-type sample value, thereby setting the wild-type sample value to 1 arbitrary unit of binding.
The calculations used are shown in detail in the following formula:

$$value\_Sample(x,y) = \frac{Sample(x,y) - neg\_Contr.(x)}{(UCHT-1(x) - neg\_Contr.(x)) * \dfrac{WT(y) - neg\_Contr.(wt)}{UCHT-1(wt) - neg\_Contr.(wt)}}$$

In this equation *value_Sample* means the value in arbitrary units of binding depicting the degree of binding of a specific anti-CD3 antibody to a specific alanine-mutant as shown in Figure 8 (A-D), *Sample* means the geometric mean fluorescence value obtained for a specific anti-CD3 antibody assayed on a specific alanine-scanning transfectant, *neg_Contr.* means the geometric mean fluorescence value obtained for the negative control assayed on a specific alanine-mutant, *UCHT-1* means the geometric mean fluorescence value obtained for the UCHT-1 antibody assayed on a specific alanine-mutant, *WT* means the geometric mean fluorescence value obtained for a specific anti-CD3 antibody assayed on the wild-type transfectant, *x* specifies the respective transfectant, y specifies the respective anti-CD3 antibody and *wt* specifies that the respective transfectant is the wild-type.
As can be seen in Figure 8 (A-D) the IgG antibody A2J HLP showed a pronounced loss of binding for the amino acids asparagine at position 4, threonine at position 23 and isoleucine at position 25 of the mature CD3 epsilon chain. A complete loss of binding of IgG antibody A2J HLP was observed for the amino acids glutamine at position 1, aspartate at position 2, glycine at position 3 and glutamate at position 5 of the mature CD3 epsilon chain. IgG antibody E2M HLP showed a pronounced loss of binding for the amino acids asparagine at position 4, threonine at position 23 and isoleucine at position 25 of the mature CD3 epsilon chain. IgG antibody E2M HLP showed a complete loss of binding for the amino acids glutamine at position 1, aspartate at position 2, glycine at position 3 and glutamate at position 5 of the mature CD3 epsilon chain. IgG antibody H2C HLP showed an intermediate loss of binding for the amino acid asparagine at position 4 of the mature CD3 epsilon chain and it showed a complete loss of binding for the amino acids glutamine at position 1, aspartate at position 2, glycine at position 3 and glutamate at position 5 of the mature CD3 epsilon chain. Single chain antibody F12Q HLP showed an essentially complete loss of binding for the amino acids glutamine at position 1, aspartate at position 2, glycine at position 3 of the mature CD3 epsilon chain and glutamate at position 5 of the mature CD3 epsilon chain.

**6. Binding analysis of the cross-species specific anti-CD3 binding molecule H2C HLP to the human CD3 epsilon chain with and without N-terminal His6 tag transfected into the murine T cell line EL4**

**6.1. Cloning and expression of the human CDS epsilon chain with N-terminal six histidine tag (His6 tag)**

A cDNA fragment coding for the human CD3 epsilon chain with a N-terminal His6 tag was obtained by gene synthesis. The gene synthesis fragment was designed as to contain first a Kozak site for eukaryotic expression of the construct, which is followed in frame by the coding sequence of a 19 amino acid immunoglobulin leader peptide, which is followed in frame by the coding sequence of a His6 tag which is followed in frame by the coding sequence of the mature human CD3 epsilon chain (the cDNA and amino acid sequences of the construct are listed as SEQ ID NOs 380 and 379). The gene synthesis fragment was also designed as to contain restriction sites at the beginning and the end of the cDNA. The introduced restriction sites EcoRI at the 5' end and SalI at the 3' end, were used in the following cloning procedures. The gene synthesis fragment was then cloned via EcoRI and SalI into a plasmid designated pEF-NEO (as described above) following standard protocols. A sequence verified plasmid was used to transfect the murine T cell line EL4. Transfection and selection of the transfectants were performed as described above. After 34 days of cell culture the transfectants were used for the assay described below.

**6.2. Binding of the cross-species specific anti-CD3 binding molecule H2C HLP to the human CD3 epsilon chain with and without N-terminal His6 tag**

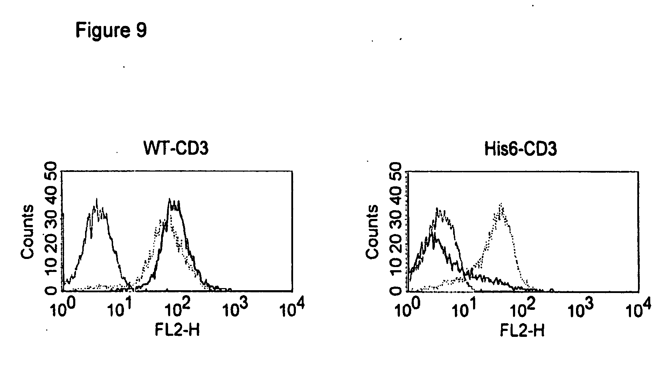

A chimeric IgG antibody with the binding specificity H2C HLP specific for CD3 epsilon was tested for binding to human CD3 epsilon with and without N-terminal His6 tag. Binding of the antibody to the EL4 cell lines transfected the His6-human CD3 epsilon and wild-type human CD3 epsilon respectively was tested by an FACS assay according to standard protocols. $2,5 \times 10^5$ cells of the transfectants were incubated with 50 $\mu$l of cell culture supernatant containing the chimeric IgG antibody or 50 $\mu$l of the respective control antibodies at 5$\mu$g/ml in PBS with 2% FCS. As negative control an appropriate isotype control and as positive control for expression of the constructs the CD3 specific antibody UCHT-1 were used respectively. The binding of the antibodies was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACSCalibur (BD biosciences, Heidelberg, Germany). Compared to the EL4 cell line transfected with wild-type human CD3 epsilon a clear loss of binding of the chimeric igG with binding specificity H2C HLP to human-CD3 epsilon with an N-terminal His6 tag was detected. These results showed that a free N-terminus of CD3 epsilon is essential for binding of the cross-species specific anti-CD3 binding specificity H2C HLP to the human CD3 epsilon chain (Figure 9).

**7. Determination of the binding constant KD of bispecific single chain antibody cross-species specific for primate EGFR and primate CD3 (EGFR LH x H2C HLP) to the fusion protein 1-27 CD3-Fc by Plasmon Surface Resonance measurement compared to binding to CD3 expressing PBMC measured by a Fluorescence Activated Cell Sorter (FACS)**

**7.1. Plasmon Surface Resonance Measurement**

To determine the binding affinity of the fully cross-species specific bispecific single chain antibody EGFR-21-63 LH x H2C HLP to amino acids 1-27 of the N-terminus of the human CD3 epsilon chain a Surface Plasmon Resonance measurement was performed with a recombinant fusion protein consisting of the N-terminal amino acids 1-27 of the mature human CD3 epsilon chain fused to a Fc-part of human IgG1 (1-27 CD3-Fc). To this end a Biacore Carboxymethyl-Dextran CM5 chip (Biacore, Uppsala, Sweden) was installed on a Biacore 2000® system (Biacore, Uppsala, Sweden). One flow cell was activated by a *N*-(3-Dimethylaminopropyl)-*N'*-ethylcarbodiimide hydrochloride / N-Hydroxysuccinimide solution according to standard procedures. A solution. of the fusion protein 1-27 CD3-Fc was added afterwards resulting in stable covalent linkage of the protein to the dextran layer of the Biacore chip. Unbound protein was removed by extensive washing followed by blocking of unreacted remaining NHS-activated carboxy groups by adding an ethanolamine solution. Success of protein coupling was confirmed by a higher signal measured as Response Units compared to the signal prior to coupling. A reference cell was prepared as described but without adding a protein solution.

Purified bispecific antibody EGFR-21-63 LH x H2C HLP was extensively dialyzed against HBS-EP buffer (Biacore, Uppsala, Sweden) in a Slide-A-Lyzer® Mini Dialysis Unit (Pierce, Rockford-II, USA). Protein concentration after dialysis was determined by UV280 nm absorption resulting in a concentration of 43 $\mu$g/ml.

The protein solution was transferred into a 96 well plate and serially diluted with HBS-EP buffer at a 1:1 ratio to 10 further wells.

Surface Plasmon Resonance Measurements were done by separately sampling all 11 wells. The flow cells were regenerated with acetate buffer between measurements to release bound protein.

Binding signals of bispecific antibody molecules were obtained by subtraction of the signal of the reference cell from the signal of the measurement cell conjugated with the 1-27 CD3-Fc protein. Association and dissociation curves were measured as Response Units and recorded. The binding constants were calculated using the Biacore® curve fitting software based on the Langmuir model.

The calculated binding constant KD over the first five concentrations was determined to be 1,52 x $10^{-7}$ M.

## 7.2. Determination of CD3 binding constant by FACS measurement

In order to test the affinity of the cross-species specific bispecific antibody molecules with regard to the binding strength to native human CD3, an additional saturation FACS binding analysis was performed. The chosen bispecific antibody molecule EGFR-21-63 LH x H2C HLP was used to set up a dilution row with a factor of 1:1.5 and a starting concentration of 63.3 μg/ml. The bispecific antibody molecule was incubated at these different concentrations with 1.25 x $10^5$ human PBMCs each for 1 hour a 4°C followed by two washing steps in PBS at 4°C. The detection of the bound bispecific antibody molecules was carried out by using a Penta-His antibody (Qiagen GmbH, Hildesheim, Germany) at 5 μg/ml in 50 μl PBS with 2% FCS. After incubation for 45 minutes at 4°C and two washing steps the binding of the Penta-His antibody was detected with an R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Flow cytometry was performed on a FACS-Canto II apparatus, the FACS Diva software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002). The acquired fluorescence intensity mean values were plotted as a function of the used bispecific antibody molecule concentration and analyzed by the biomathematical software Prism in a one side binding analysis (hyperbola). The software calculated the corresponding KD value that described the binding of a ligand (the bispecific antibody molecule) to a receptor (the CD3 positive PBMC subtraction) that follows the law of mass action. The underlying formula is as follows: Y = Bmax x X / (Kd+X) with Bmax being the maximal binding. KD is the concentration of ligand required to reach half-maximal binding. The FACS staining was carried out in duplicates, the $R^2$ values were better than 0.95.

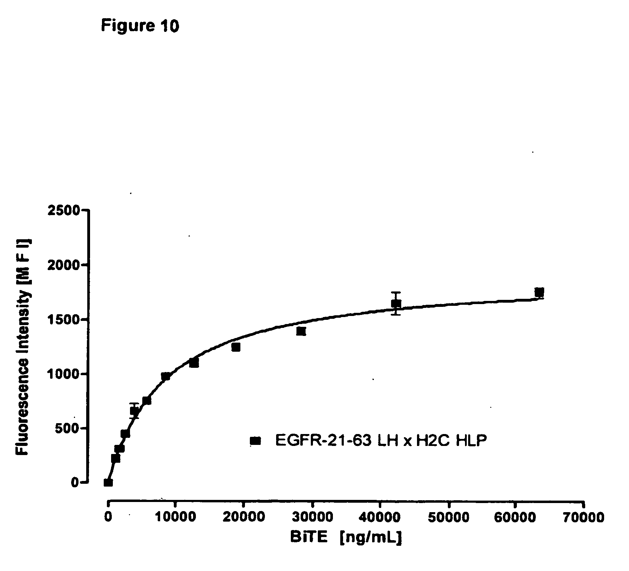

The determined half-maximal binding for the bispecific antibody molecule EGFR-21-63 LH x H2C HLP was reached at a concentration of 8472 ng/ml which corresponds to 154 nM (1.54 x $10^{-7}$ M) at a given molecular mass of 55000 Dalton (Figure 10). Thus, the affinity of EGFR-21-63 LH x H2C HLP to the N-terminal amino acids 1-27 of the human CD3 epsilon chain separated from their native CD3-context proved to be equal to the affinity of EGFR-21-63 LH x H2C HLP to native CD3 on intact T cells.

## 8. Generation of CHO cells transfected with human EGFR

The cell line positive for human EGFR, A431 (epidermoid carcinoma cell line, CRL-1555, American Type Culture Collection, Rockville, MD) was used to obtain the total RNA that was isolated according to the instructions of the kit manual (Qiagen, RNeasy Mini Kit, Hilden, Germany). The obtained RNA was used for cDNA synthesis by random-primed reverse transcription. For cloning of the full length sequence of the human EGFR antigen the following oligonucleotides were used:

    5' EGFR AG Xbal 5'-GGTCTAGAGCATGCGACCCTCCGGGACGGCCGGG-3'
    3' EGFR AG Sall 5'-TTTTAAGTCGACTCATGCTCCAATAAATTCACTGCT-3'

The coding sequence was amplified by PCR (denaturation at 94°C for 5 min, annealing at 58°C for 1 min, elongation at 72°C for 2 min for the first cycle; denaturation at 94°C for 1 min, annealing at 58°C for 1 min, elongation at 72°C for 2 min for 30 cycles; terminal extension at 72°C for 5 min). The PCR product was subsequently digested with Xbal and Sall, ligated into the appropriately digested expression vector pEF-DHFR (Raum et al., Cancer Immunol. Immunother. 2001; 50: 141-150), and transformed into E.coli. The aforementioned procedures were carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence (SEQ ID 370, Amino acid sequence SEQ ID 369) was transfected into DHFR deficient CHO cells for eukaryotic expression of the construct. Eukaryotic protein expression in DHFR deficient CHO cells was performed as described by Kaufmann R.J. (1990) Methods Enzymol. 185, 537-566. Gene amplification of the construct was induced by increasing concentrations of methothrexate (MTX) to a final concentration of up to 20 nM MTX.

### 9. Generation of CHO cells expressing the extracellular domain of cynomolgus EGFR

The cDNA sequence of the extracellular domain of cynomolgus EGFR was obtained by a set of two PCRs on cynomolgus monkey colon cDNA (Cat# C1534090-Cy-BC; obtained from BioCat GmbH, Heidelberg, Germany) using the following reaction conditions: 1 cycle at 94°C for 3 minutes followed by 35 cycles with 94°C for 1 minute, 53°C for 1 minute and 72°C for 2 minutes followed by a terminal cycle of 72°C for 3 minutes. The following primers were used:

1. forward primer: 5'- CGCTCTGCCCGGCGAGTCGGGC -3' reverse primer: 5'- CCGTCTTCCTCCATCTCATAGC -3'
2. forward primer: 5'- ACATCCGGAGGTGACAGATCACGGCTCGTGC -3' reverse primer: 5'- CAGGATATCCGAACGATGTGGCGCCTTCGC -3'

Those PCRs generated two overlapping fragments (A: 1-869, B: 848-1923), which were isolated and sequenced according to standard protocols using the PCR primers, and thereby provided a 1923 bp portion of the cDNA sequence of cynomolgus EGFR from the third nucleotide of codon +1 of the mature protein to the 21$^{st}$ codon of the transmembrane domain. To generate a construct for expression of cynomolgus EGFR a cDNA fragment was obtained by gene synthesis according to standard protocols (the cDNA and amino acid sequence of the construct is listed under SEQ ID Nos 372 and 371). In this construct the coding sequence for cynomolgus EGFR from amino acid +2 to +641 of the mature EGFR protein was fused into the coding sequence of human EGFR replacing the coding sequence of the amino acids +2 to +641. The gene synthesis fragment was also designed as to contain a Kozak site for eukaryotic expression of the construct and restriction sites at the beginning and the end of the cDNA coding for essentially the extracellular domain of cynomolgus EGFR fused to the transmembrane and intracellular domains of human EGFR. Furthermore a conservative mutation was introduced at amino acid 627 (4$^{th}$ amino acid of the transmembrane domain) mutating valine into leucine to generate a restriction site (SphI) for cloning purposes. The introduced restriction sites XbaI at the 5' end and SaiI at the 3' end, were utilised in the following cloning procedures. The gene synthesis fragment was then cloned via XbaI and SalI into a plasmid designated pEF-DHFR (pEF-DHFR is described in Mack et al. Proc. Natl. Acad. Sci. USA 92 (1995) 7021-7025). A sequence verified clone of this plasmid was used to transfect CHO/dhfr- cells as described above.

### 10. Generation of EGFR and CD3 cross-species specific bispecific single chain molecules

#### 10.1. Cloning of cross-species specific binding molecules

Generally, bispecific single chain antibody molecules, each comprising a domain with a binding specificity cross-species specific for human and non-chimpanzee primate CD3epsilon as well as a domain with a binding specificity cross-species specific for human and non-chimpanzee primate EGFR, were designed as set out in the following Table 1:

Table 1: Formats of anti-CD3 and anti-EGFR cross-species specific bispecific single chain antibody molecules

| SEQ ID (nucl/prot) | Formats of protein constructs (N → C) |
| --- | --- |
| 294/293 | EGFR-21-63 LH x H2C HL |
| 296/295 | EGFR-21-63 LH x H2C HLP |
| 302/301 | EGFR-21-63 LH x A2J HLP |
| 298/297 | EGFR-21-63 LH x H1E HLP |
| 306/305 | EGFR-21-63 LH x E2M HLP |
| 308/307 | EGFR-21-63 LH x F7O HLP |
| 390 / 389 | EGFR1 HL x I2C HL |
| 392 / 391 | EGFR1 LH x I2C HL |
| 394 / 393 | EGFR1 HL x F12Q HL |
| 396 / 395 | EGFR1 LH x F12Q HL |
| 398 / 397 | EGFR1 HL x H2C HL |
| 400 / 399 | EGFR1 LH x H2C HL |

(continued)

| SEQ ID (nucl/prot) | Formats of protein constructs (N → C) |
|---|---|
| 448 / 447 | EGFR1 HL x H2C HL |
| 450 / 449 | EGFR1 HL x F12Q LH |
| 452 / 451 | EGFR1 HL x I2C HL |
| 410 / 409 | EGFR2 HL x I2C HL |
| 412 / 411 | EGFR2 LH x I2C HL |
| 414 / 413 | EGFR2 HL x F12Q HL |
| 416 / 415 | EGFR2 LH x F12Q HL |
| 418 / 417 | EGFR2 HL x H2C HL |
| 420 / 419 | EGFR2 LH x H2C HL |

The aforementioned constructs containing the variable light-chain (L) and variable heavy-chain (H) domains cross-species specific for human and cynomolgus EGFR were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence was transfected into dihydrofolate reductase (DHFR) deficient Chinese hamster ovary (CHO) cells for eukaryotic expression of the construct.

The constructs were transfected stably or transiently into DHFR-deficient CHO-cells (ATCC No. CRL 9096) by electroporation or alternatively into HEK 293 (human embryonal kidney cells, ATCC Number: CRL-1573) in a transient manner according to standard protocols.

**10.2. Expression and purification of the bispecific single chain antibody molecules**

The bispecific single chain antibody molecules were expressed in chinese hamster ovary cells (CHO). Eukaryotic protein expression in DHFR deficient CHO cells was performed as described by Kaufmann R.J. (1990) Methods Enzymol. 185, 537-566. Gene amplification of the constructs was induced by increasing final concentrations of MTX up to 20 nM. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein was stored at -20°C. Alternatively, constructs were transiently expressed in HEK 293 cells. Transfection was performed with 293fectin reagent (Invitrogen, #12347-019) according to the manufacturer's protocol.

Äkta® Explorer System (GE Health Systems) and Unicorn® Software were used for chromatography. Immobilized metal affinity chromatography ("IMAC") was performed using a Fractogel EMD chelate® (Merck) which was loaded with ZnCl2 according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazol) according to the following:

    Step 1: 20% buffer B in 6 column volumes
    Step 2: 100% buffer B in 6 column volumes

Eluted protein fractions from step 2 were pooled for further purification. All chemicals were of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrat, 200 mM Lysin, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein was >95% as determined by SDS-PAGE.

The bispecific single chain antibody has a molecular weight of about 52 kDa under native conditions as determined by gel filtration in PBS. All constructs were purified according to this method.

Western Blot was performed using an Optitran® BA-S83 membrane and the Invitrogen Blot Module according to the protocol provided by the manufacturer. The antibodies used were directed against the His Tag (Penta His, Qiagen) and Goat-anti-mouse Ig labeled with alkaline phosphatase (AP) (Sigma), and BCIP/NBT (Sigma) as substrate. A single band was detected at 52 kD corresponding to the purified bispecific single chain antibody.

## 11. Determination of the binding constant KD of fully cross-species specific bispecific single chain antibodies to the fusion protein 1-27 CD3-Fc by Surface Plasmon Resonance measurement

To determine the binding affinities of bispecific single chain antibody molecules cross-species specific to primate EGFR and primate CD3 to the amino acids 1-27 of the N-terminus of the mature human CD3 epsilon chain a Surface Plasmon Resonance measurement was performed with a recombinant fusion protein consisting of the N-terminal amino acids 1-27 of the human CD3 epsilon chain fused to a Fc-part of human IgG1 (1-27 CD3-Fc). To this end a Biacore Carboxymethyl-Dextran CM5 chip (Biacore, Uppsala, Sweden) was installed on a Biacore 2000® system (Biacore, Uppsala, Sweden). A flow cell was activated by a N-(3-Dimethylaminopropyl)-N'-ethylcarbodiimide hydrochloride / N-Hydroxysuccinimide solution according to standard procedures. A solution of the fusion protein 1-27 CD3-Fc was added afterwards resulting in stable covalent linkage of the protein to the dextran layer of the Biacore chip. Unbound protein was removed by extensive washing followed by blocking of remaining unreacted NHS-activated carboxy groups by adding an ethanolamine solution. Success of protein coupling was confirmed by detection of a higher signal measured as Response Units compared to the signal prior to coupling. A reference cell was prepared as described but without adding the protein solution.

Purified bispecific single chain antibodies listed below were adjusted to 5 $\mu$g/ml with HBS-EP buffer (Biacore, Uppsala, Sweden) and transferred into a 96 well plate each at a volume of 150 $\mu$l.

Surface Plasmon Resonance Measurements were performed for all samples and the flow cells were regenerated with acetate buffer between measurements to release bound protein (all according to standard protocols).

Binding signals of the bispecific single chain antibodies were obtained by subtraction of the signal of the reference cell from the signal of the measurement cell conjugated with the 1-27 CD3-Fc protein.

Association and dissociation curves were measured as Response Units and recorded. The binding constants were calculated using the Biacore® curve-fitting software based on the Langmuir model. The calculated affinities for the tested fully cross-species specific bispecific single chain molecules to the N-terminal amino acids 1-27 of the human CD3 epsilon are given as KD values below and range from $2{,}54 \times 10^{-6}$ M to $2{,}49 \times 10^{-7}$ M. "LH" refers to an arrangement of variable domains in the order VL-VH. "HL" refers to an arrangement of variable domains in the order VH-VL. G4H, F70, A2J, E1L, E2M, H 1 E and F6A refer to different cross-species specific CD3 binding molecules.

| Bispecific antibody molecule | KD(M) |
|---|---|
| EGFR LH x F70 HLP | $1.01 \times 10^{-6}$ |
| EGFR LH x A2J HLP | $2.49 \times 10^{-7}$ |
| EGFR LH x E2M HLP | $2.46 \times 10^{-6}$ |
| EGFR LH x H1E HLP | $2.54 \times 10^{-6}$ |

## 12. Flow cytometric binding analysis of the EGFR and CD3 cross-species specific bispecific antibodies

In order to test the functionality of the cross-species specific Bispecific antibody constructs with regard to binding capability to human and cynomolgus EGFR and CD3, respectively, a FACS analysis was performed. For this purpose CHO cells transfected with human EGFR as described in Example 8 and human CD3 positive T cell leukemia cell line HPB-ALL (DSMZ, Braunschweig, ACC483) were used to test the binding to human antigens. The binding reactivity to cynomolgus antigens was tested by using the generated cynomolgus EGFR transfectant described in Example 9 and a macaque T cell line 4119LnPx (kindly provided by Prof Fickenscher, Hygiene Institute, Virology, Erlangen-Nuernberg;

published in Knappe A, et al., and Fickenscher H., Blood 2000, 95, 3256-61) 200.000 cells of the respective cell population were incubated for 30 min on ice with 50 $\mu$l of the purified protein of the cross-species specific bispecific antibody constructs (2 $\mu$g/ml). Alternatively, the cell culture supernatant of transiently produced proteins was used. The cells were washed twice in PBS and binding of the construct was detected with a murine Penta His antibody (Qiagen; diluted 1:20 in 50 $\mu$l PBS with 2% FCS). After washing, bound anti His antibodies were detected with an Fc gamma-specific antibody (Dianova) conjugated to phycoerythrin, diluted 1:100 in PBS with 2% FCS. Fresh culture medium was used as a negative control.

Flow cytometry was performed on a FACS-Calibur apparatus, the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

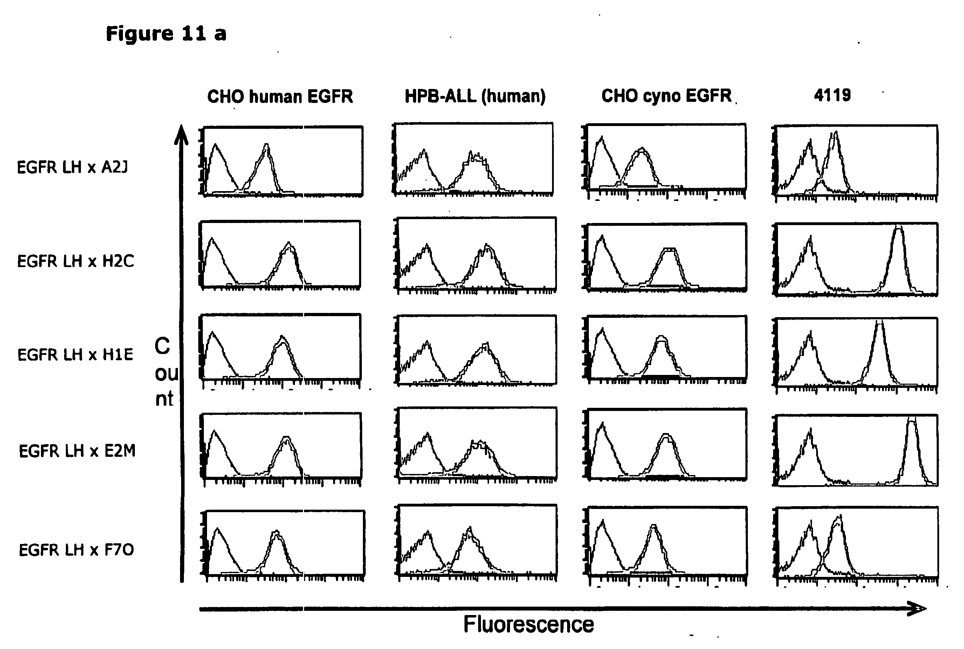

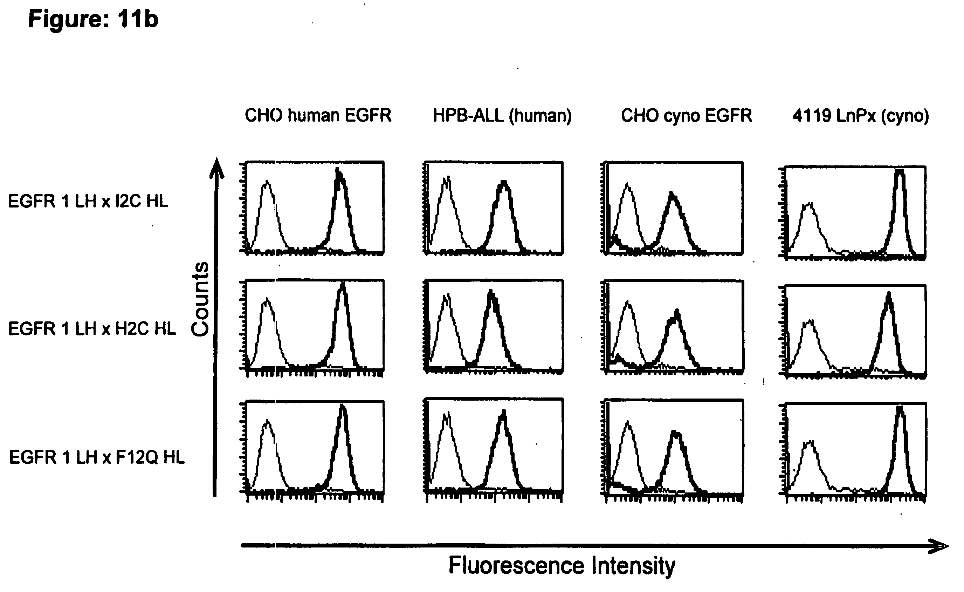

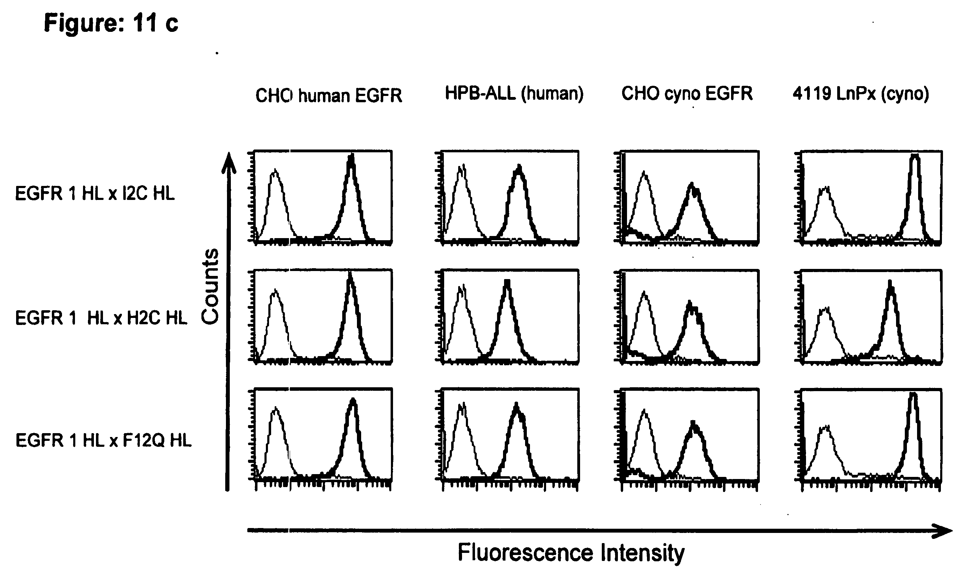

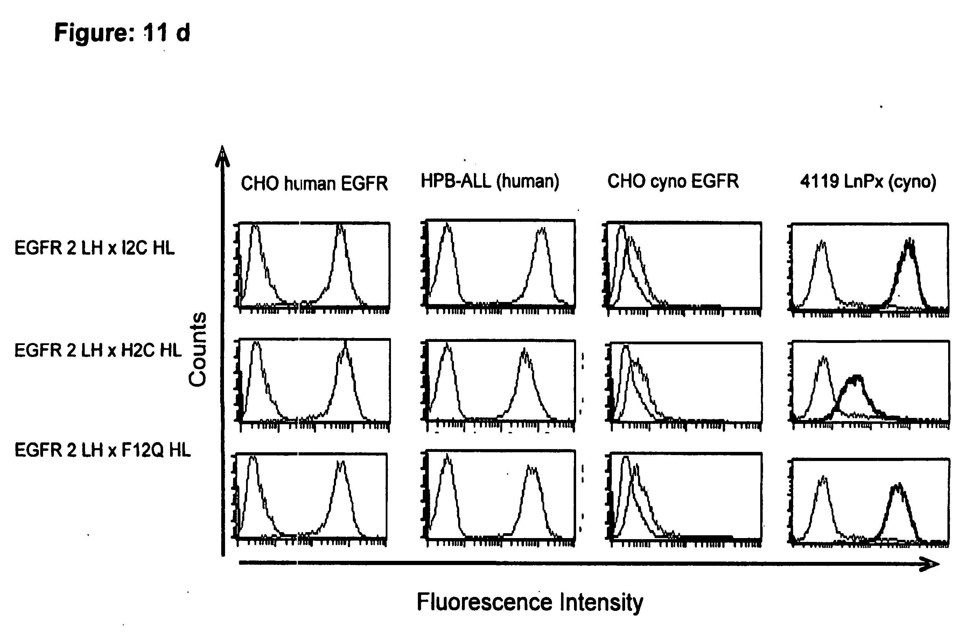

The binding ability of several bispecific single chain molecules which are specific for EGFR and cross-species specific for human and non-chimpanzee primate CD3 were clearly detectable as shown in Figure 11. In the FACS analysis, all constructs showed binding to CD3 and EGFR compared to culture medium and first and second detection antibody as the negative controls. Cross-species specificity of the bispecific antibody to human and cynomolgus CD3 and EGFR antigens was demonstrated.

## 13. Bioactivity of EGFR and CD3 cross-species specific bispecific single chain antibodies

Bioactivity of the generated bispecific single chain antibodies was analyzed by chromium 51 ($^{51}$Cr) release in vitro cytotoxicity assays using the EGFR positive cell lines described in Examples 8 and 9. As effector cells stimulated human CD8 positive T cells or the macaque T cell line 4119LnPx were used, respectively.

Generation of the stimulated CD8+ T cells was performed as follows:

A Petri dish (145 mm diameter, Greiner) was pre-coated with a commercially available anti-CD3 specific antibody in a final concentration of 1 $\mu$g/ml for 1 hour at 37°C. Unbound protein was removed by one washing step with PBS. The fresh PBMCs were isolated from peripheral blood (30 - 50 ml human blood) by Ficoll gradient centrifugation according to standard protocols. 3 - 5 x $10^7$ PBMCs were added to the precoated petri dish in 120 ml of RPMI 1640 / 10% FCS / IL-2 20 U/ml (Proleukin, Chiron) and stimulated for 2 days. At the third day the cells were collected, washed once with RPMI 1640. IL-2 was added to a final concentration of 20 U/ml and cultivated again for one day. CD8+ cytotoxic T lymphocytes (CTLs) were isolated by depletion of CD4+ T cells and CD56+ NK cells.

Target cells were washed twice with PBS and labeled with 11,1 MBq $^{51}$Cr in a final volume of 100$\mu$l RPMI with 50% FCS for 45 minutes at 37°C. Subsequently the labeled target cells were washed 3 times with 5 ml RPMI and then used in the cytotoxicity assay. The assay was performed in a 96 well plate in a total volume of 250 $\mu$l supplemented RPMI (as above) with an E:T ratio of 10:1. 1 $\mu$g/ml of the cross-species specific bispecific single chain antibody molecules and 20 threefold dilutions thereof were applied. Alternatively cell culture supernatant of transiently produced proteins was serially diluted in 1:2 steps. The assay time is 18 hours and cytotoxicity was measured as relative values of released chromium in the supernatant related to the difference of maximum lysis (addition of Triton-X) and spontaneous lysis (without effector cells). All measurements were done in quadruplicates. Measurement of chromium activity in the supernatants was performed with a Wizard 3" gammacounter (Perkin Elmer Life Sciences GmbH, Köln, Germany). Analysis of the experimental data was performed with Prism 4 for Windows (version 4.02, GraphPad Software Inc., San Diego, California, USA). Sigmoidal dose response curves typically had $R^2$ values >0.90 as determined by the software. $EC_{50}$ values calculated by the analysis program were used for comparison of bioactivity.

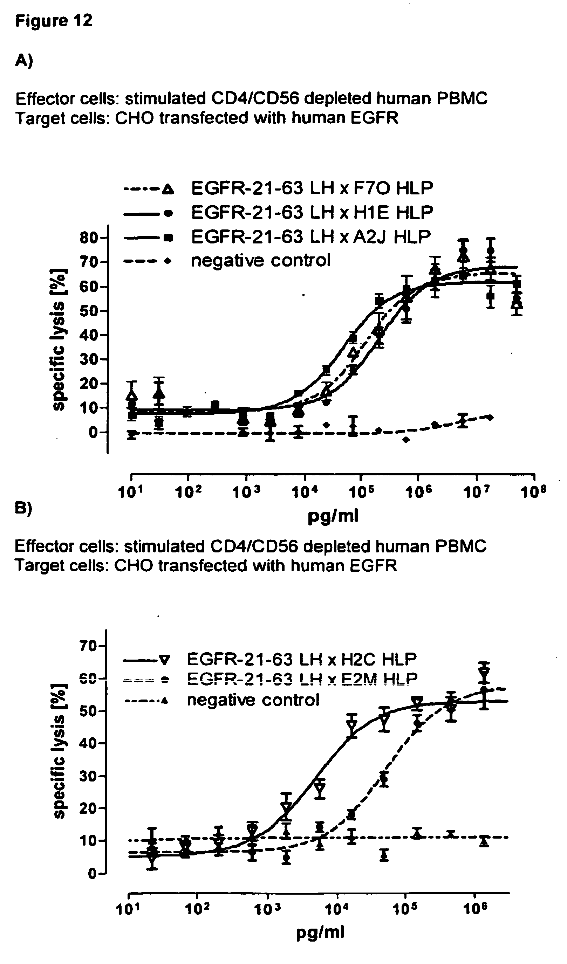

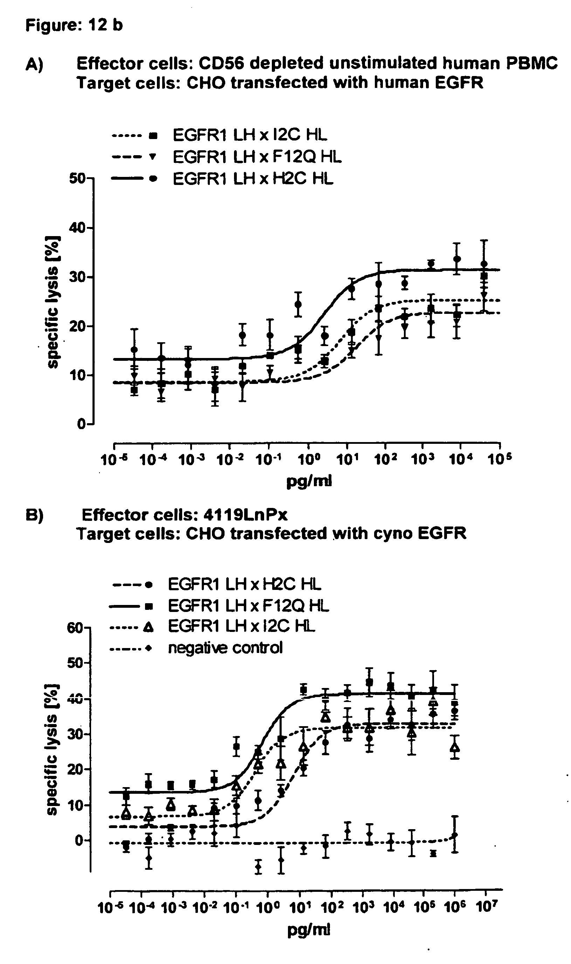

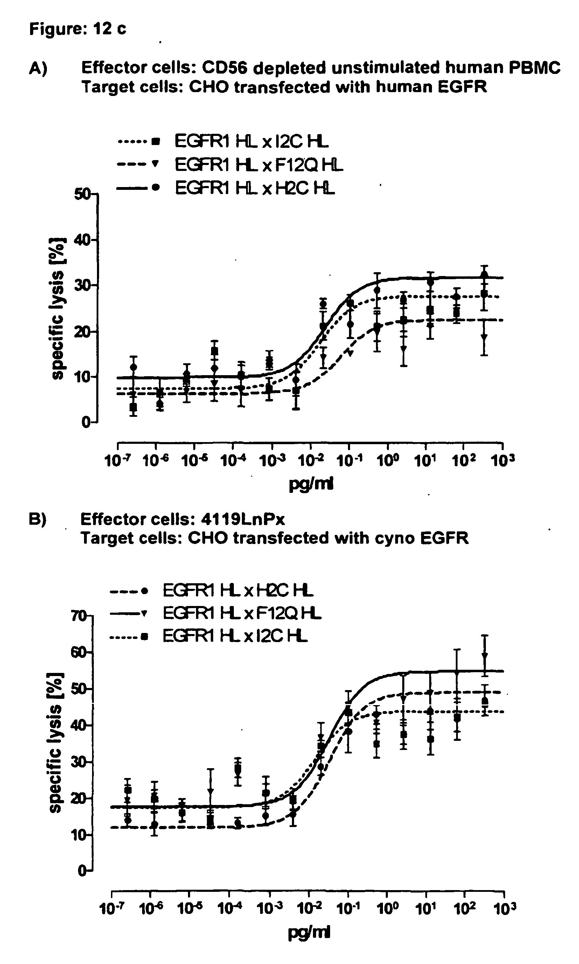

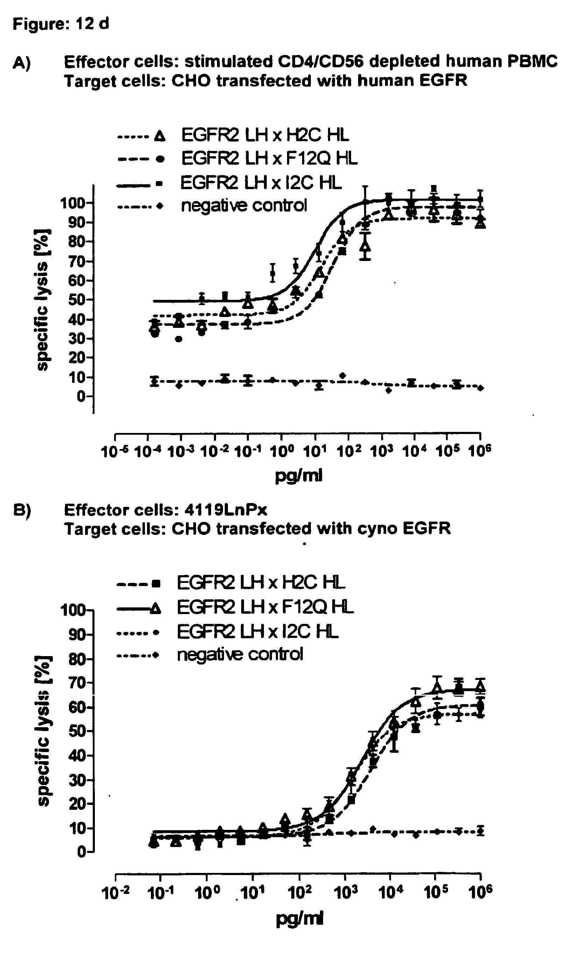

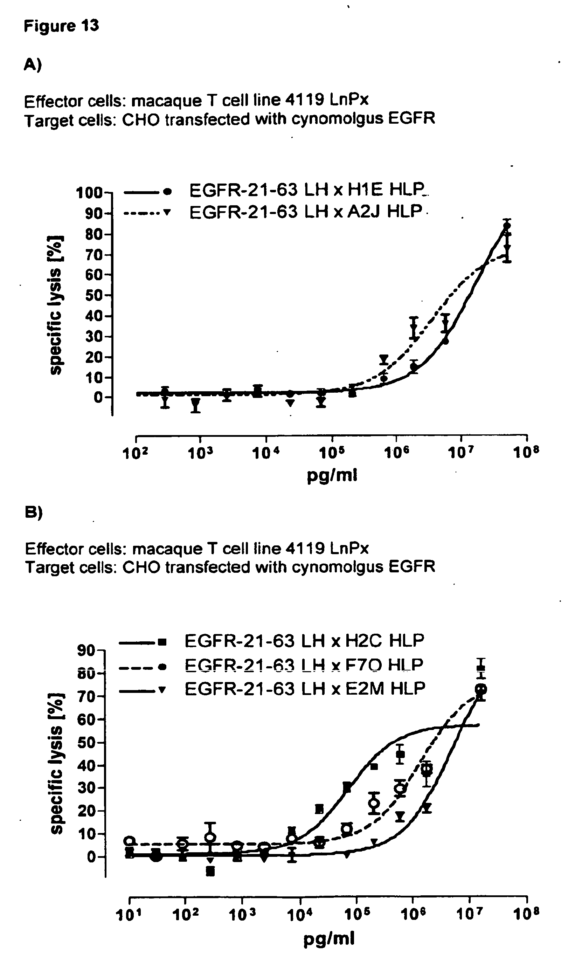

As shown in Figures 12 and 13, all of the generated cross-species specific bispecific single chain antibody constructs revealed cytotoxic activity against human EGFR positive target cells elicited by human CD8+ cells and cynomolgus EGFR positive target cells elicited by the macaque T cell line 4119LnPx. A bispecific single chain antibody with different target specificity was used as negative control.

## 14. Cloning and expression of the C-terminal, transmembrane and truncated extracellular domains of human MCSP

The coding sequence of the C-terminal, transmembrane and truncated extracellular domain of human MCSP (amino acids 1538 - 2322) was obtained by gene synthesis according to standard protocols (cDNA sequence and amino acid sequence of the recombinant construct for expression of the C-terminal, transmembrane and truncated extracellular domain of human MCSP (designated as human D3) are listed under SEQ ID NOs 374 and 373). The gene synthesis fragment was designed as to contain first a Kozak site to allow eukaryotic expression of the construct followed by the coding sequence of an 19 amino acid immunoglobulin leader peptide followed in frame by a FLAG tag, followed in frame by a sequence containing several restriction sites for cloning purposes and coding for a 9 amino acid artificial linker (SRTRSGSQL), followed in frame by the coding sequence of the C-terminal, transmembrane and truncated extracellular domain of human MCSP and a stop codon. Restriction sites were introduced at the beginning and at the end of the DNA fragment. The restriction sites EcoRI at the 5' end and Sall at the 3' end were used in the following cloning procedures. The fragment was digested with EcoRI and Sall and cloned into pEF-DHFR (pEF-DHFR is described in Mack et al. Proc. Natl. Acad. Sci. USA 92 (1995) 7021-7025) following standard protocols. A sequence verified plasmid was used to transfect CHO/dhfr- cells (ATCC No. CRL 9096). Cells were cultivated in RPMI 1640 with stabilized glutamine, supplemented with 10% FCS, 1% penicillin/streptomycin (all obtained from Biochrom AG Berlin, Germany) and nucleosides from a stock solution of cell culture grade reagents (Sigma-Aldrich Chemie GmbH, Taufkirchen, Germany) to a final concentration of 10 $\mu$g/ml Adenosine, 10 $\mu$g/ml Deoxyadenosine and 10 $\mu$g/ml Thymidine, in an incubator at 37°C, 95% humidity and 7% $CO_2$. Transfection was performed using the PolyFect Transfection Reagent (Qiagen GmbH, Hilden, Germany) and 5 $\mu$g of plasmid DNA according to the manufacturer's protocol. After cultivation for 24 hours cells were washed once with PBS and cultivated again in RPMI 1640 with stabilized glutamine and 1% penicillin/streptomycin. Thus the cell culture medium did not contain nucleosides and thereby selection was applied on the transfected cells. Approximately 14 days after transfection the outgrowth of resistant cells was observed. After an additional 7 to 14 days the transfectants were tested for expression of the construct by FACS analysis. 2,5x10[5] cells were incubated with 50 $\mu$l of an anti-Flag-M2 antibody (Sigma-Aldrich Chemie GmbH, Taufkirchen, Germany) diluted to 5 $\mu$g/ml in PBS with 2% FCS. The binding of the antibody was detected with a R-Phycoerythrin-conjugated affinity purified F(ab')2 fragment, goat anti-mouse IgG, Fc-gamma fragment specific diluted 1:100 in PBS with 2% FCS (ImmunoResearch Europe Ltd., Newmarket, Suffolk, UK). The samples were measured on a FACScalibur (BD biosciences, Heidelberg, Germany).

**15. Cloning and expression of the C-terminal, transmembrane and truncated extracellular domains of macaque MCSP**

The cDNA sequence of the C-terminal, transmembrane and truncated extracellular domains of macaque MCSP (designated as macaque D3) was obtained by a set of three PCRs on macaque skin cDNA (Cat No. C1534218-Cy-BC; BioCat GmbH, Heidelberg, Germany) using the following reaction conditions: 1 cycle at 94°C, 3 min., 40 cycles with 94°C for 0,5 min., 52°C for 0,5 min. and 72°C for 1,75 min., terminal cycle of 72°C for 3 min.. The following primers were used:

> forward primer: 5'-GATCTGGTCTACACCATCGAGC-3'
> reverse primer: 5'-GGAGCTGCTGCTGGCTCAGTGAGG-3'
> forward primer: 5'- TTCCAGCTGAGCATGTCTGATGG-3'
> reverse primer: 5'- CGATCAGCATCTGGGCCCAGG-3'
> forward primer: 5'- GTGGAGCAGTTCACTCAGCAGGACC-3'
> reverse primer: 5'- GCCTTCACACCCAGTACTGGCC-3'

Those PCRs generated three overlapping fragments (A: 1-1329, B: 1229-2428, C: 1782-2547) which were isolated and sequenced according to standard protocols using the PCR primers and thereby provided a 2547 bp portion of the cDNA sequence of macaque MCSP (the cDNA sequence and amino acid sequence of this portion of macaque MCSP are listed under SEQ ID NOs 376 and 375) from 74 bp upstream of the coding sequence of the C-terminal domain to 121 bp downstream of the stop codon. Another PCR using the following reaction conditions: 1 cycle at 94°C for 3 min, 10 cycles with 94°C for 1 min, 52°C for 1 min and 72°C for 2,5 min, terminal cycle of 72°C for 3 min was used to fuse the PCR products of the aforementioned reactions A and B. The following primers are used:

> forward primer: 5'-tcccgtacgagatctggatcccaattggatggcggactcgtgctgttctcacacagagg-3'
> reverse primer: 5'-agtgggtcgactcacacccagtactggccattcttaagggcaggg-3'

The primers for this PCR were designed to introduce restriction sites at the beginning and at the end of the cDNA fragment coding for the C-terminal, transmembrane and truncated extracellular domains of macaque MCSP. The introduced restriction sites Mfel at the 5' end and Sall at the 3' end, were used in the following cloning procedures. The PCR fragment was then cloned via Mfel and Sall into a Bluescript plasmid containing the EcoRI/Mfel fragment of the aforementioned plasmid pEF-DHFR (pEF-DHFR is described in Raum et al. Cancer Immunol Immunother 50 (2001) 141-150) by replacing the C-terminal, transmembrane and truncated extracellular domains of human MCSP. The gene synthesis fragment contained the coding sequences of the immunoglobulin leader peptide and the Flag tag as well as the artificial linker (SRTRSGSQL) in frame to the 5' end of the cDNA fragment coding for the C-terminal, transmembrane and truncated extracellular domains of macaque MCSP. This vector was used to transfect CHO/dhfr- cells (ATCC No. CRL 9096). Cells were cultivated in RPMI 1640 with stabilized glutamine supplemented with 10% FCS, 1% penicillin/streptomycin (all from Biochrom AG Berlin, Germany) and nucleosides from a stock solution of ceii cuiiure grade reagents (Sigma-Aldrich Chemie GmbH, Taufkirchen, Germany) to a final concentration of 10 $\mu$g/ml Adenosine, 10 $\mu$g/ml Deoxyadenosine and 10 $\mu$g/ml Thymidine, in an incubator at 37°C, 95% humidity and 7% CO2. Transfection was performed with PolyFect Transfection Reagent (Qiagen GmbH, Hilden, Germany) and 5 $\mu$g of plasmid DNA according to the manufacturer's protocol. After cultivation for 24 hours cells were washed once with PBS and cultivated again in RPMI 1640 with stabilized glutamine and 1% peniciltin/streptomycin. Thus the cell culture medium did not contain nucleosides and thereby selection was applied on the transfected cells. Approximately 14 days after transfection the outgrowth of resistant cells is observed. After an additional 7 to 14 days the transfectants were tested for expression of the recombinant construct via FACS. 2,5x10$^5$ cells were incubated with 50 $\mu$l of an anti-Flag-M2 antibody (Sigma-Aldrich Chemie GmbH, Taufkirchen, Germany) diluted to 5 $\mu$g/ml in PBS with 2% FCS. Bound antibody was detected with a R-Phycoerythrin-conjugated affinity purified F(ab')2 fragment, goat anti-mouse IgG, Fc-gamma fragment specific, diluted 1:100 in PBS with 2% FCS (Jackson ImmunoResearch Europe Ltd., Newmarket, Suffolk, UK). Samples were measured on a FACScalibur (BD biosciences, Heidelberg, Germany).

## 16. Generation and characterisation of MCSP and CD3 cross-species specific bispecific single chain molecules

Bispecific single chain antibody molecules each comprising a binding domain cross-species specific for human and non-chimpanzee primate CD3 epsilon as well as a binding domain cross-species-specific for human and non-chimpanzee primate MCSP, are designed as set out in the following Table 2:

Table 2: Formats of MCSP and CD3 cross-species specific bispecific single chain antibodies

| SEQ ID (nucl/prot) | Formats of protein constructs (N → C) |
| --- | --- |
| 310/309 | MCSP-G4 HL x H2C HL |
| 312/311 | MCSP-G4 HL x F12Q HL |
| 314/313 | MCSP-G4 HL x I2C HL |
| 316/315 | MCSP-G4 HLP x F6A HLP . |
| 318/317 | MCSP-G4 HLP x H2C HLP |
| 322/321 | MCSO-G4 HLP x G4H HLP |
| 326/325 | MCSP-G4 HLP x E1 L HLP |
| 328/327 | MCSP-G4 HLP x E2M HLP |
| 332/331 | MCSP-G4 HLP x F12Q HL |
| 334/333 | MCSP-G4 HLP x I2C HL |
| 336/335 | MCSP-D2 HL x H2C HL |
| 338/337 | MCSP-D2 HL x F12Q HL |
| 340/339 | MCSP-D2 HL x I2C HL |
| 342/341 | MCSP-D2 HLP x H2C HLP |
| 344/343 | MCSP-F9 HL x H2C HL |
| 346/345 | MCSP-F9 HLP x H2C HLP |
| 348/347 | MCSP-F9 HLP x G4H HLP |

The aforementioned constructs containing the variable heavy-chain (VH) and variable light-chain (VL) domains cross-species specific for human and macaque MCSP D3 and the VH and VL domains cross-species specific for human and macaque CD3 were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a histidine$_6$-tag and a stop codon. The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). The constructs were transfected stably or transiently into DHFR-deficient CHO-cells (ATCC No. CRL 9096) by electroporation or alternatively into HEK 293 (human embryonal kidney cells, ATCC Number: CRL-1573) in a transient manner according to standard protocols.

Eukaryotic protein expression in DHFR deficient CHO cells was performed as described by Kaufmann R.J. (1990) Methods Enzymol. 185, 537-566. Gene amplification of the constructs was induced by addition of increasing concentrations of methothrexate (MTX) up to final concentrations of 20 nM MTX. After two passages of stationary culture the cells were grown in roller bottles with nucleoside-free HyQ PF CHO liquid soy medium (with 4.0 mM L-Glutamine with 0.1% Pluronic F - 68; HyClone) for 7 days before harvest. The cells were removed by centrifugation and the supernatant containing the expressed protein is stored at - 20°C.

Äkta® Explorer System (GE Health Systems) and Unicorn® Software were used for chromatography. Immobilized metal affinity chromatography ("IMAC") was performed using a Fractogel EMD chelate® (Merck) which was loaded with $ZnCl_2$ according to the protocol provided by the manufacturer. The column was equilibrated with buffer A (20 mM sodium phosphate buffer pH 7.2, 0.1 M NaCl) and the cell culture supernatant (500 ml) was applied to the column (10 ml) at a flow rate of 3 ml/min. The column was washed with buffer A to remove unbound sample. Bound protein was eluted using a two step gradient of buffer B (20 mM sodium phosphate buffer pH 7.2, 0.1 M NaCl, 0.5 M Imidazole) according to the following:

Step 1: 20% buffer B in 6 column volumes
Step 2: 100% buffer B in 6 column volumes

Eluted protein fractions from step 2 were pooled for further purification. All chemicals are of research grade and purchased from Sigma (Deisenhofen) or Merck (Darmstadt).

Gel filtration chromatography was performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) were subjected to standard SDS-PAGE and Western Blot for detection. Prior to purification, the column was calibrated for molecular weight determination (molecular weight marker kit, Sigma MW GF-200). Protein concentrations were determined using OD280 nm.

Purified bispecific single chain antibody protein was analyzed in SDS PAGE under reducing conditions performed with pre-cast 4-12% Bis Tris gels (Invitrogen). Sample preparation and application were performed according to the protocol provided by the manufacturer. The molecular weight was determined with MultiMark protein standard (Invitrogen). The gel was stained with colloidal Coomassie (Invitrogen protocol). The purity of the isolated protein is >95% as determined by SDS-PAGE.

The bispecific single chain antibody has a molecular weight of about 52 kDa under native conditions as determined by gel filtration in phosphate buffered saline (PBS). All constructs were purified according to this method.

Western Blot was performed using an Optitran® BA-S83 membrane and the Invitrogen Blot Module according to the protocol provided by the manufacturer. For detection of the bispecific single chain antibody protein antibodies an anti-His Tag antibody was used (Penta His, Qiagen). A Goat-anti-mouse Ig antibody labeled with alkaline phosphatase (AP) (Sigma) was used as secondary antibody and BCIP/NBT (Sigma) as substrate. A single band was detected at 52 kD corresponding to the purified bispecific single chain antibody.

Alternatively, constructs were transiently expressed in DHFR deficient CHO cells. In brief, 4 x 105 cells per construct were cultivated in 3 ml RPMI 1640 all medium with stabilized glutamine supplemented with 10% fetal calf serum, 1% penicillin/streptomycin and nucleosides from a stock solution of cell culture grade reagents (Sigma-Aldrich Chemie GmbH, Taufkirchen, Germany) to a final concentration of 10 μg/ml Adenosine, 10 μg/ml Deoxyadenosine and 10 μg/ml Thymidine, in an incubator at 37°C, 95% humidity and 7% CO2 one day before transfection. Transfection was performed with Fugene 6 Transfection Reagent (Roche, # 11815091001) according to the manufacturer's protocol. 94 μl OptiMEM medium (Invitrogen) and 6 μl Fugene 6 are mixed and incubated for 5 minutes at room temperature. Subsequently, 1.5 μg DNA per construct were added, mixed and incubated for 15 minutes at room temperature. Meanwhile, the DHFR deficient CHO cells were washed with 1x PBS and resuspended in 1.5 ml RPMI 1640 all medium. The transfection mix was diluted with 600 μl RPMI 1640 all medium, added to the cells and incubated overnight at 37°C, 95% humidity and 7% CO2. The day after transfection the incubation volume of each approach was extended to 5 ml RPMI 1640 all medium. Supernatant was harvested after 3 days of incubation.

## 17. Flow cytometric binding analysis of the MCSP and CD3 cross-species specific bispecific antibodies

In order to test the functionality of the cross-species specific bispecific antibody constructs regarding the capability to bind to human and macaque MCSP D3 and CD3, respectively, a FACS analysis was performed. For this purpose CHO cells transfected with human MCSP D3 (as described in Example 14) and the human CD3 positive T cell leukemia cell line HPB-ALL (DSMZ, Braunschweig, ACC483) were used to test the binding to human antigens. The binding reactivity to macaque antigens was tested by using the generated macaque MCSP D3 transfectant (described in Example 15) and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines were incubated for 30 min on ice with 50 μl of the purified protein of the cross-species specific bispecific antibody constructs (2 μg/ml) or cell culture supernatant of transfected cells expressing the cross-species specific bispecific antibody constructs. The cells were washed twice in PBS with 2% FCS and binding of the construct was detected with a murine anti-His antibody (Penta His antibody; Qiagen; diluted 1:20 in 50 μl PBS with 2% FCS). After washing, bound anti-His antibodies were detected with an Fc gamma-specific antibody (Dianova) conjugated to phycoerythrin, diluted 1:100 in PBS with 2% FCS. Supernatant of untransfected CHO cells was used as negative control for binding to the T cell lines. A single chain construct with irrelevant target specificity was used as negative control for binding to the MCSP-D3 transfected CHO cells.

Flow cytometry was performed on a FACS-Calibur apparatus; the CellQuest software was used to acquire and analyze the data (Becton Dickinson biosciences, Heidelberg). FACS staining and measuring of the fluorescence intensity were performed as described in Current Protocols in Immunology (Coligan, Kruisbeek, Margulies, Shevach and Strober, Wiley-Interscience, 2002).

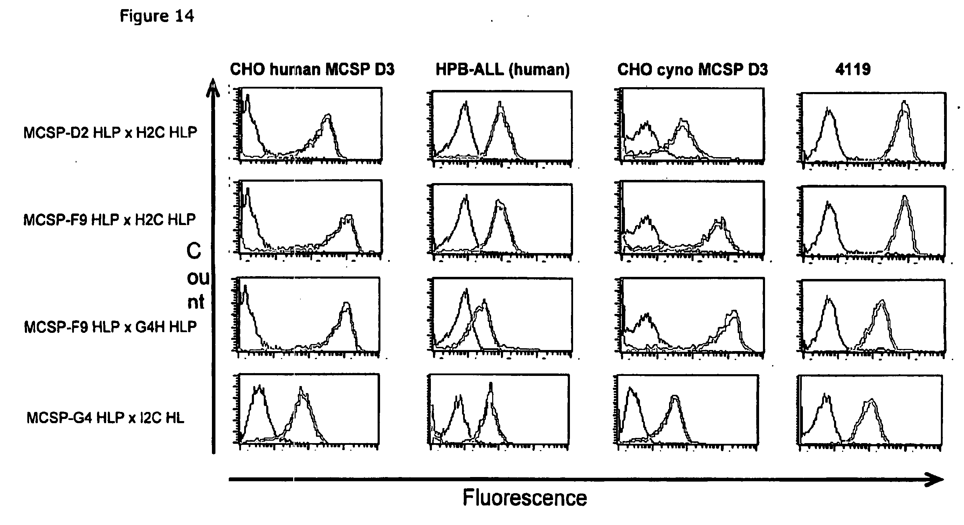

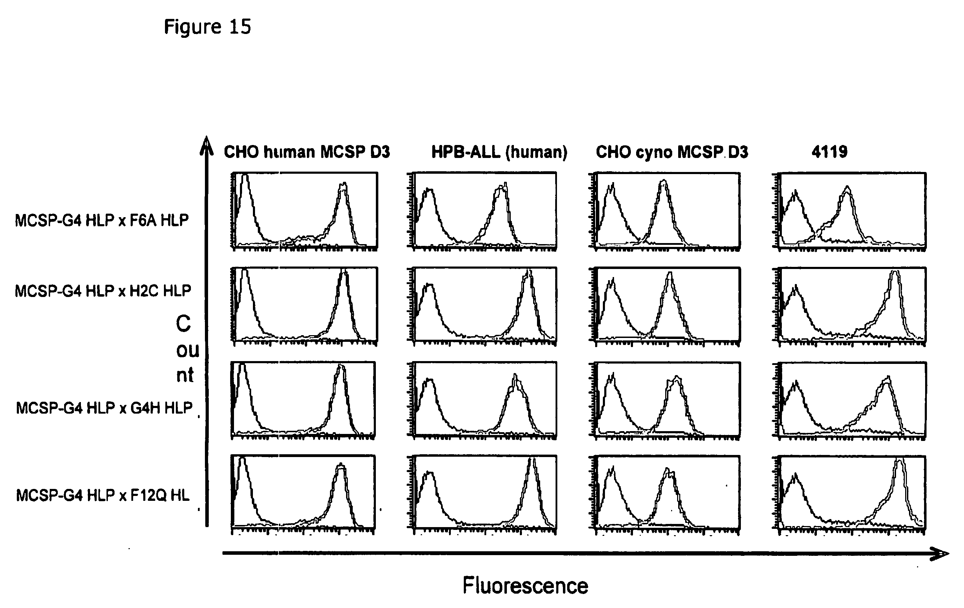

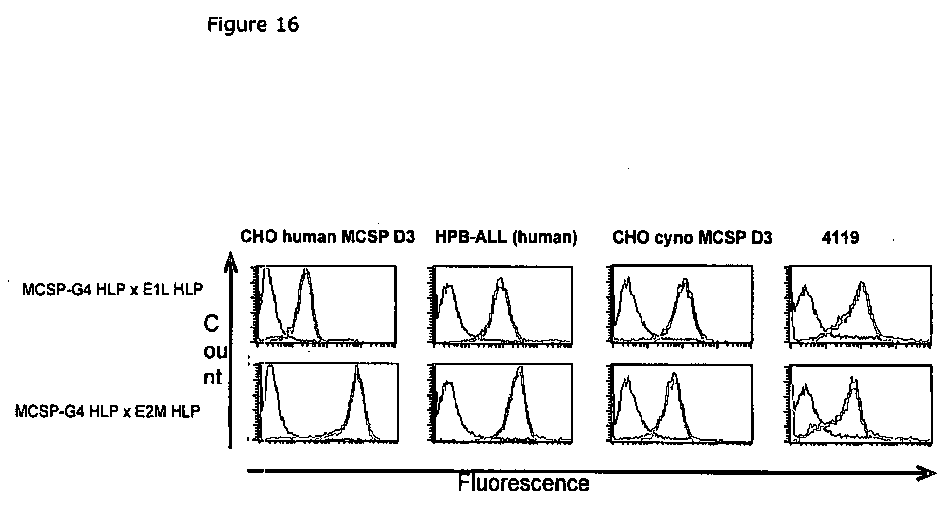

The bispecific binding of the single chain molecules listed above; which are cross-species specific for MCSP D3 and cross-species specific for human and macaque CD3 was clearly detectable as shown in Figures 14, 15, 16 and 58. In the FACS analysis all constructs showed binding to CD3 and MCSP D3 as compared to the respective negative controls. Cross-species specificity of the bispecific antibodies to human and macaque CD3 and MCSP D3 antigens was demonstrated.

## 18. Bioactivity of MCSP and CD3 cross-species specific bispecific single chain antibodies

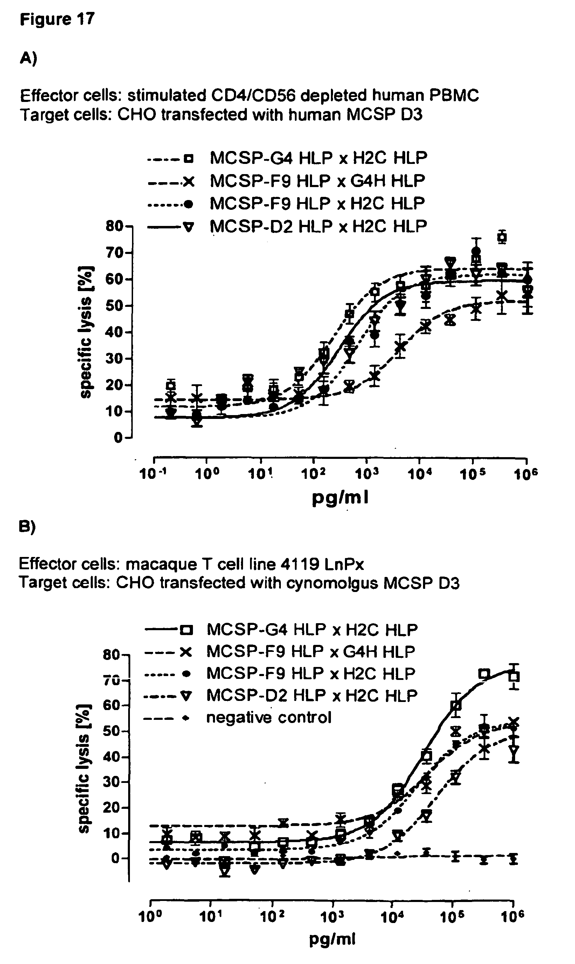

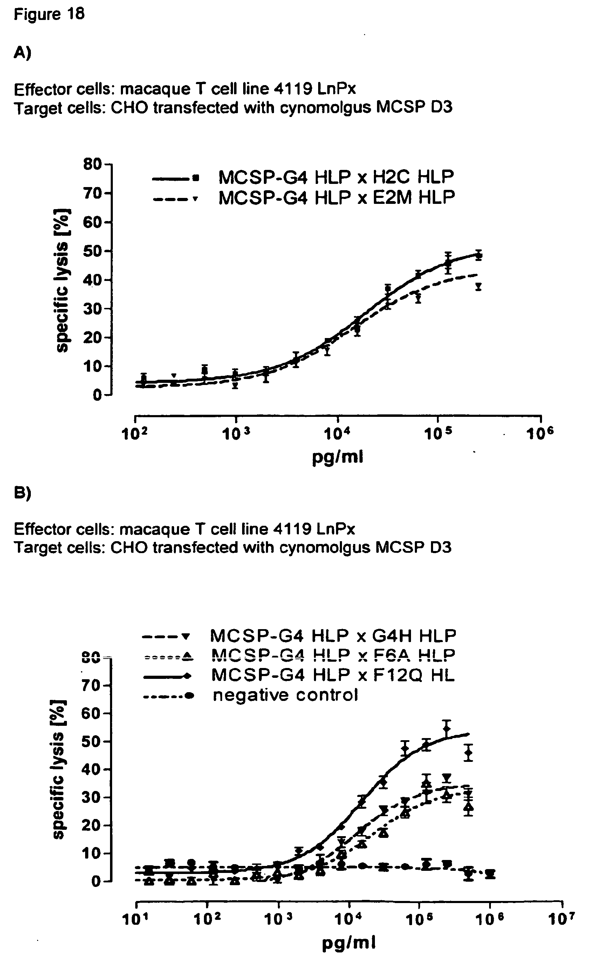

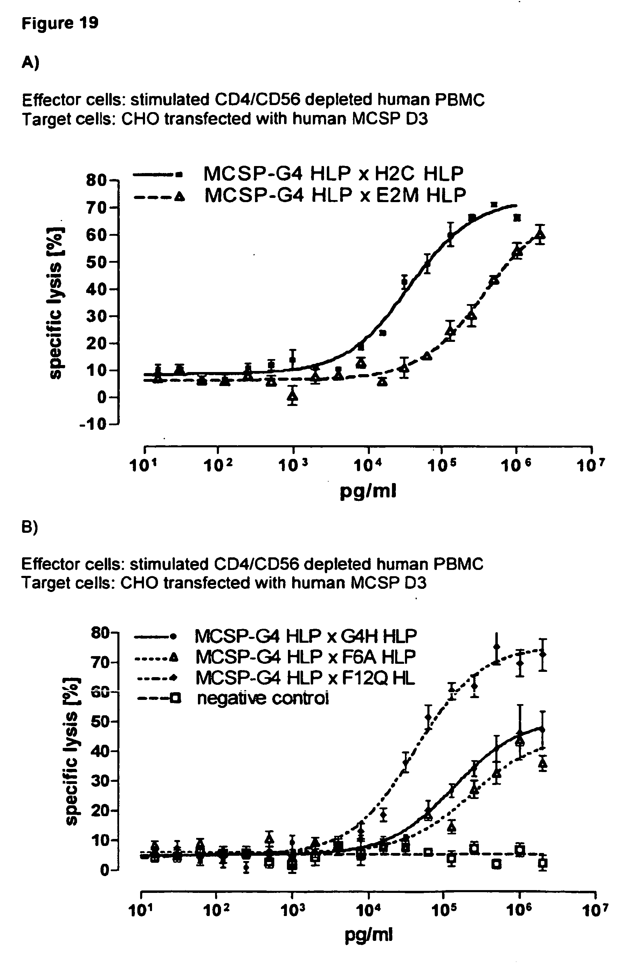

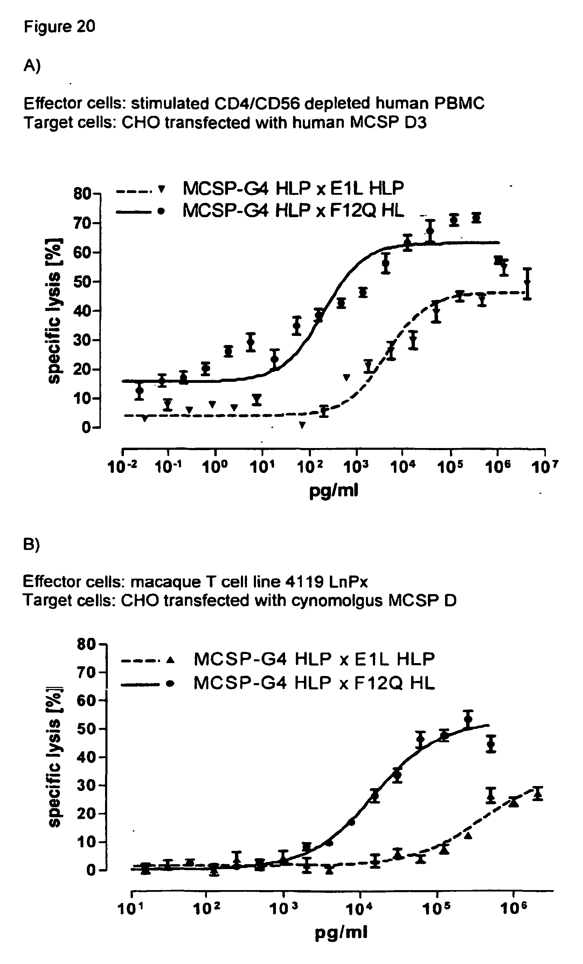

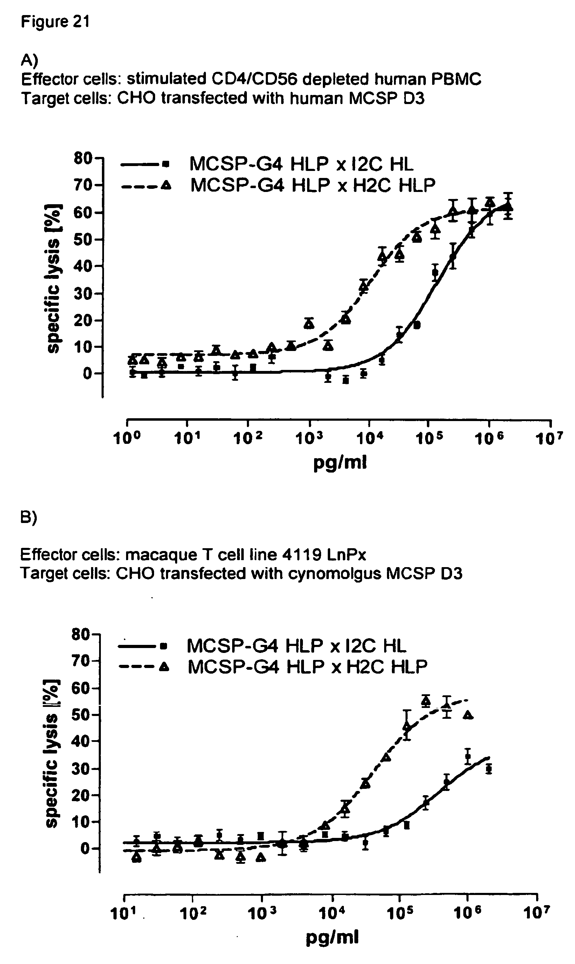

As shown in Figures 17 to 21, all of the generated cross-species specific bispecific single chain antibody constructs revealed cytotoxic activity against human MSCP positive target cells elicited by human CD8+ cells and cynomolgus MSCP positive target cells elicited by the macaque T cell line 4119LnPx. A bispecific single chain antibody with different target specificity is used as negative control.

## 19. Plasma stability of MCSP and CD3 cross-species specific bispecific single chain antibodies

Stability of the generated bispecific single chain antibodies in human plasma was analyzed by incubation of the bispecific single chain antibodies in 50% human Plasma at 37°C and 4°C for 24 hours and subsequent testing of bioactivity. Bioactivity was studied in a chromium 51 ($^{51}$Cr) release in vitro cytotoxicity assay using a MCSP positive CHO cell line (expressing MCSP as cloned according to example 14 or 15) as target and stimulated human CD8 positive T cells as effector cells.

$EC_{50}$ values calculated by the analysis program as described above are used for comparison of bioactivity of bispecific single chain antibodies incubated with 50% human plasma for 24 hours at 37°C and 4°C respectively with bispecific single chain antibodies without addition of plasma or mixed with the same amount of plasma immediately prior to the assay.

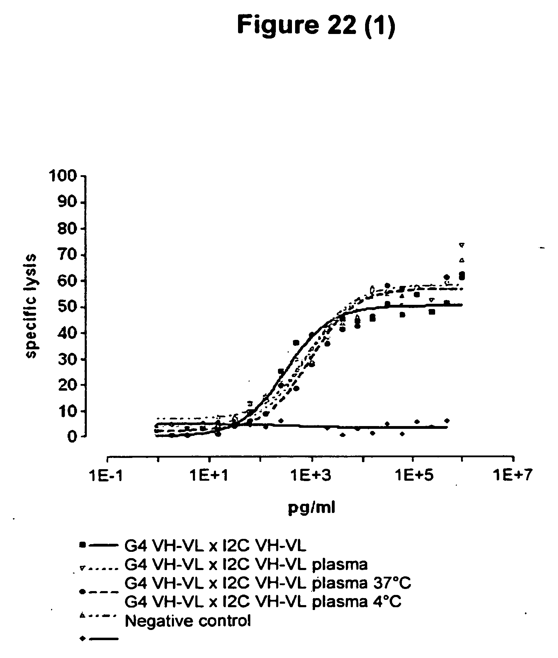

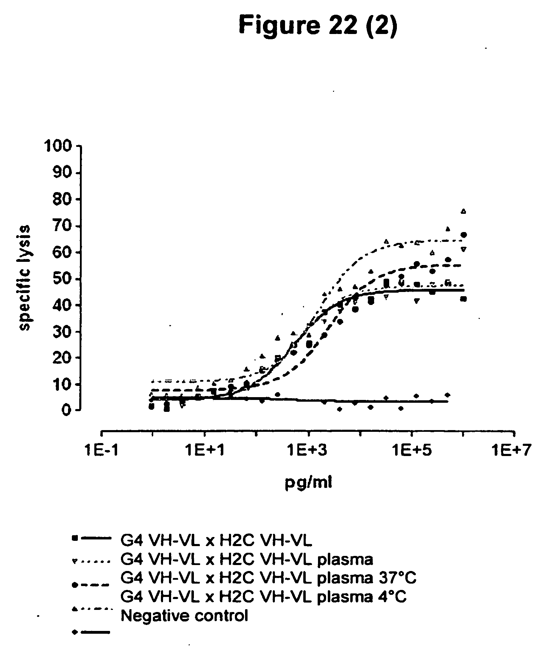

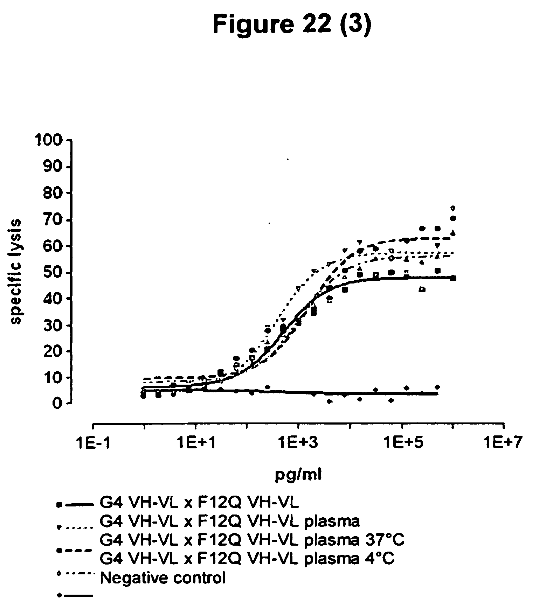

As shown in Figure 22 and Table 3 the bioactivity of the G4 H-L x I2C H-L, G4 H-L x H2C H-L and G4 H-L x F12Q H-L bispecific antibodies was not significantly reduced as compared with the controls without the addition of plasma or with addition of plasma immediately before testing of bioactivity.

Table 3: bioactivity of the bispecific antibodies without or with the addition of Plasma

| Construct | Without plasma | With plasma | Plasma 37°C | Plasma 4°C |
|---|---|---|---|---|
| G4 H-L x I2C H-L | 300 | 796 | 902 | 867 |
| G4 H-L x H2C H-L | 496 | 575 | 2363 | 1449 |
| G4 H-L x F12Q H-L | 493 | 358 | 1521 | 1040 |

## 20. Generation of human EGFR and CD3 cross-species specific bispecific single chain molecules

Bispecific single chain antibody molecules with a binding domain cross-species specific for human and cynomolgus CD3 as well as a binding domain cross-species-specific for human EGFR, are designed as set out in the following Table 4:

Table 4: Formats of EGFR and CD3 cross-species specific Bispecific single chain antibodies

| SEQ ID (nucl/prot) | Formats of protein constructs (N → C) |
|---|---|
| 390/389 | EGFR H-L x I2C H L |
| 392/391 | EGFR L-H x I2C H L |
| 394/393 | EGFR H-L x F12Q H L |
| 396/395 | EGFR L-H x F12Q H L |
| 398/397 | EGFR H-L x H2C H L |
| 400/399 | EGFR L-H x H2C H L |
| 448 / 447 | EGFR HL x H2C HL |
| 450 / 449 | EGFR HL x F12Q LH |
| 452 / 451 | EGFR HL x I2C HL |

The aforementioned constructs containing the variable light-chain (L) and variable heavy-chain (H) domains cross-species specific for human and cynomolgus EGFR were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon.

The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). The constructs were stably transfected into DHFR-deficient CHO-cells (ATCC No. CRL 9096) as well as produced and purified as described in example 10.

### 21. Generation of human EGFR and CD3 cross-species specific bispecific single chain molecules

Bispecific single chain antibody molecules with a binding domain cross-species specific for human and cynomolgus CD3 as well as a binding domain cross-species-specific for human EGFR, are designed as set out in the following Table 5:

Table 5: Formats of EGFR and CD3 cross-species specific bispecific single chain antibodies

| SEQ ID (nucl/prot) | Formats of protein constructs (N → C) |
|---|---|
| 410/4099 | EGFR H-L x I2C H L |
| 412/411 | EGFR L-H x I2C H L |
| 414/413 | EGFR H-L x F12Q H L |
| 416/415 | EGFR L-H x F12Q H L |
| 418/417 | EGFR H-L x H2C H L |
| 420/419 | EGFR L-H x H2C H L |

The aforementioned constructs containing the variable light-chain (L) and variable heavy-chain (H) domains cross-species specific for human and cynomolgus EGFR were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon.

The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). The constructs were stably transfected into DHFR-deficient CHO-cells (ATCC No. CRL 9096) as well as produced and purified as described in example 10.

**22. Generation of human Her2/neu and CD3 cross-species specific bispecific single chain molecules**

Bispecific single chain antibody molecules with a binding domain cross-species specific for human and cynomolgus CD3 as well as a binding domain cross-species-specific for human Her2/neu, are designed as set out in the following Table 6:

Table 6: Formats of Her2/neu and CD3 cross-species specific bispecific single chain antibodies

| SEQ ID (nucl/prot) | Formats of protein constructs (N → C) |
|---|---|
| 430/439 | HER2/neu VH-VL x I2C VH VL |
| 432/431 | HER2/neu VL-VH x I2C VH VL |
| 434/433 | HER2/neu VH-VL x F12Q VH VL |
| 436/435 | HER2/neu VL-VH x F12Q VH VL |
| 438/437 | HER2/neu VH-VL x H2C VH VL |
| 440/439 | HER2/neu VL-VH x H2C VH VL |

The aforementioned constructs containing the variable light-chain (L) and variable heavy-chain (H) domains cross-species specific for human and cynomolgus HER2/neu were obtained by gene synthesis. The gene synthesis fragments were designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon.

The gene synthesis fragment was also designed as to introduce suitable N- and C-terminal restriction sites. The gene synthesis fragment was cloned via these restriction sites into a plasmid designated pEF-DHFR (pEF-DHFR is described in Raum et al. Cancer Immunol Immunother 50 (2001) 141-150) according to standard protocols (Sambrook, Molecular Cloning; A Laboratory Manual, 3rd edition, Cold Spring Harbour Laboratory Press, Cold Spring Harbour, New York (2001)). The constructs were stably transfected into DHFR-deficient CHO-cells (ATCC No. CRL 9096) as well as produced and purified as described in example 10.

**23.1. Generation of CHO cells expressing human HER2**

The coding sequence of human HER2 as published in GenBank (Accession number X03363) is obtained by gene synthesis according to standard protocols. The gene synthesis fragment is designed as to contain the coding sequence of the human HER2 protein including its leader peptide (the cDNA and amino acid sequence of the construct is listed under SEQ ID Nos 459 and 460). The gene synthesis fragment is also designed as to introduce restriction sites at the beginning and at the end of the fragment. The introduced restriction sites, XbaI at the 5' end and SalI at the 3' end, are utilised in the following cloning procedures. The gene synthesis fragment is cloned via XbaI and SalI into a plasmid designated pEFDHFR (pEFDHFR is described in Raum et al. Cancer Immunol Immunother 50 (2001) 141-150) following standard protocols. The aforementioned procedures are carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence is transfected into DHFR deficient CHO cells for eukaryotic expression of the construct. Eukaryotic protein expression in DHFR deficient CHO cells is performed as described by Kaufmann R.J. (1990) Methods Enzymol. 185, 537-566. Gene amplification of the construct is induced by increasing concentrations of methotrexate (MTX) to a final concentration of up to 20 nM MTX.

**23.2. Generation of CHO cells expressing the extracellular domain of macaque Her2**

The coding sequence of human Her2 as described above is modified to encode the amino acids 123 to 1038 of the macaque Her2 protein as published in GenBank (Accession number XP_001090430). The coding sequence for this chimeric protein is obtained by gene synthesis according to standard protocols (the cDNA and amino acid sequence of the construct is listed under SEQ ID Nos 461 and 462). The gene synthesis fragment is also designed as to contain a Kozak site for eukaryotic expression of the construct and restriction sites at the beginning and the end of the fragment. The introduced restriction sites XbaI at the 5' end and SalI at the 3' end, are utilized in the following cloning procedures.

The gene synthesis fragment is then cloned via XbaI and SalI into a plasmid designated pEFDHFR (pEFDHFR is described in Raum et al. Cancer Immunol Immunother 50 (2001) 141-150). A sequence verified clone of this plasmid is used to transfect CHO/dhfr- cells as described above.

**23.3. Generation of HER2 and CD3 cross-species specific bispecific single chain molecules**

**3.1. Cloning of cross-species specific binding molecules**

Generally, bispecific single chain antibody molecules, each comprising a domain with a binding specificity cross-species specific for human and macaque CD3epsilon as well as a domain with a binding specificity cross-species specific for human and macaque HER2, are designed as set out in the following Table 7:

Table 7: Formats of anti-CD3 and anti-HER2 cross-species specific bispecific single chain antibody molecules

| SEQ ID (nucl/prot) | Formats of protein constructs (N → C) |
| --- | --- |
| 432 / 431 | Her2 LH x I2C HL |
| 436 / 435 | Her2 LH x F12Q HL |
| 440 / 439 | Her2 LH x H2C HL |
| 430 / 429 | Her2 HL x I2C HL |
| 434 / 433 | Her2 HL x F12Q HL |
| 438 / 437 | Her2 HL x H2C HL |
| 480 / 479 | I2C HL x Her2 LH |
| 478 / 477 | F12Q HL x Her2 LH |
| 476 / 475 | H2C HL x Her2 LH |

The aforementioned constructs containing the variable light-chain (L) and variable heavy-chain (H) domains cross-species specific for human and macaque HER2 and the CD3 specific VH and VL combinations cross-species specific for human and macaque CD3 are obtained by gene synthesis. The gene synthesis fragments are designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment is also designed as to introduce suitable restriction sites at the beginning and at the end of the fragment. The introduced restriction sites are utilised in the following cloning procedures. The gene synthesis fragment is cloned via these restriction sites into a plasmid designated pEFDHFR (pEFDHFR is described in Raum et al. Cancer Immunol Immunother 50 (2001) 141-150) following standard protocols. The aforementioned procedures are carried out according to standard protocols (Sambrook, Molecular Cloning; A Laboratory Manual, 3rd edition, Cold Spring Harbour Laboratory Press, Cold Spring Harbour, New York (2001)). A clone with sequence-verified nucleotide sequence is transfected into DHFR deficient CHO cells for eukaryotic expression of the construct. Eukaryotic protein expression in DHFR deficient CHO cells is performed as described by Kaufmann R.J. (1990) Methods Enzymol. 185, 537-566. Gene amplification of the construct is induced by increasing concentrations of methothrexate (MTX) to a final concentration of up to 20 nM MTX.

**3.2. Expression and purification of the bispecific single chain antibody molecules**

The bispecific single chain antibody molecules are expressed in Chinese hamster ovary cells (CHO). Eukaryotic protein expression in DHFR deficient CHO cells is performed as described by Kaufmann R.J. (1990) Methods Enzymol. 185, 537-566. Gene amplification of the constructs is induced by addition of increasing concentrations of MTX up to final concentrations of 20 nM MTX. After two passages of stationary culture the cells are grown in roller bottles with nucleoside-free HyQ PF CHO liquid soy medium (with 4.0 mM L-Glutamine with 0.1% Pluronic F - 68; HyClone) for 7 days before harvest. The cells are removed by centrifugation and the supernatant containing the expressed protein is stored at -80°C. Transfection is performed with 293fectin reagent (Invitrogen, #12347-019) according to the manufacturer's protocol.

Akta® Explorer System (GE Health Systems) and Unicorn® Software are used for chromatography. Immobilized metal affinity chromatography ("IMAC") is performed using a Fractogel EMD chelate® (Merck) which is loaded with $ZnCl_2$ according to the protocol provided by the manufacturer. The column is equilibrated with buffer A (20 mM sodium phosphate buffer pH 7.2, 0.1 M NaCl) and the cell culture supernatant (500 ml) is applied to the column (10 ml) at a flow rate of 3 ml/min. The column is washed with buffer A to remove unbound sample. Bound protein is eluted using a two step gradient of buffer B (20 mM sodium phosphate buffer pH 7.2, 0.1.M NaCl, 0.5 M Imidazole) according to the following:

Step 1: 20% buffer B in 6 column volumes
Step 2: 100% buffer B in 6 column volumes

Eluted protein fractions from step 2 are pooled for further purification. All chemicals are of research grade and purchased from Sigma (Deisenhofen) or Merck (Darmstadt).

Gel filtration chromatography is performed on a HiLoad 16/60 Superdex 200 prep grade column (GE/Amersham) equilibrated with Equi-buffer (25 mM Citrate, 200 mM Lysine, 5% Glycerol, pH 7.2). Eluted protein samples (flow rate 1 ml/min) are subjected to standard SDS-PAGE and Western Blot for detection. Prior to purification, the column is calibrated for molecular weight determination (molecular weight marker kit, Sigma MW GF-200). Protein concentrations are determined using OD280 nm.

Purified bispecific single chain antibody protein is analyzed in SDS PAGE under reducing conditions performed with pre-cast 4-12% Bis Tris gels (Invitrogen). Sample preparation and application are performed according to the protocol provided by the manufacturer. The molecular weight is determined with MultiMark protein standard (Invitrogen). The gel is stained with colloidal Coomassie (Invitrogen protocol). The purity of the isolated protein is >95% as determined by SDS-PAGE.

The bispecific single chain antibody has a molecular weight of about 52 kDa under native conditions as determined by gel filtration in PBS. All constructs are purified according to this method.

Western Blot is performed using an Optitran® BA-S83 membrane and the Invitrogen Blot Module according to the protocol provided by the manufacturer. For detection of the bispecific single chain antibody protein antibodies an anti-His Tag antibody is used (Penta His, Qiagen). A Goat-anti-mouse Ig antibody labeled with alkaline phosphatase (AP) (Sigma) is used as secondary antibody and BCIP/NBT (Sigma) as substrate. A single band is detected at 52 kD corresponding to the purified bispecific single chain antibody.

### 23.4. Flow cytometric binding analysis of the HER2 and CD3 cross-species specific bispecific antibodies

In order to test the functionality of the cross-species specific Bispecific antibody constructs regarding the capability to bind to human and macaque HER2 and CD3, respectively, a FACS analysis is performed. For this purpose CHO cells transfected with human HER2 as described in Example 23.1 and the human CD3 positive T cell leukemia cell line HPB-ALL (DSMZ, Braunschweig, ACC483) are used to test the binding to human antigens. The binding reactivity to macaque antigens is tested by using the generated macaque HER2 transfectant described in Example 23.2 and a macaque T cell line 4119LnPx (kindly provided by Prof. Fickenscher, Hygiene Institute, Virology, Erlangen-Nuernberg; published in Knappe A, et al., and Fickenscher H., Blood 2000, 95, 3256-61). 200.000 cells of the respective cell lines are incubated for 30 min on ice with 50 $\mu$l of the purified protein of the cross-species specific bispecific antibody constructs (2 $\mu$g/ml). The cells are washed twice in PBS with 2% FCS and binding of the construct is detected with a murine anti-His antibody (Penta His antibody; Qiagen; diluted 1:20 in 50 $\mu$l PBS with 2% FCS). After washing, bound anti-His antibodies are detected with an Fc gamma-specific antibody (Dianova) conjugated to phycoerythrin, diluted 1:100 in PBS with 2% FCS. PBS with 2% FCS is used as negative control for binding to the T cell lines as well as to the HER2 transfected CHO cells.

Flow cytometry is performed on a FACS-Calibur apparatus; the CellQuest software is used to acquire and analyze the data (Becton Dickinson biosciences, Heidelberg). FACS staining and measuring of the fluorescence intensity are performed as described in Current Protocols in Immunology (Coligan, Kruisbeek, Margulies, Shevach and Strober, Wiley-Interscience, 2002).

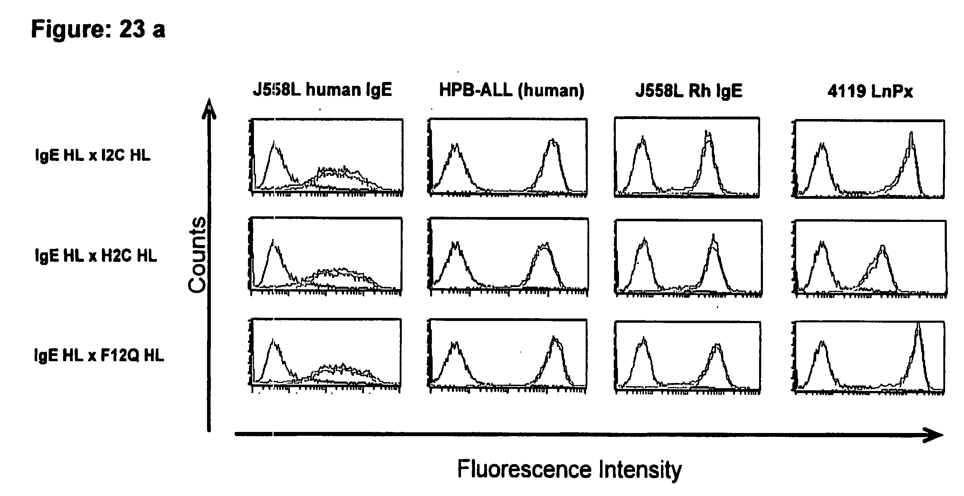

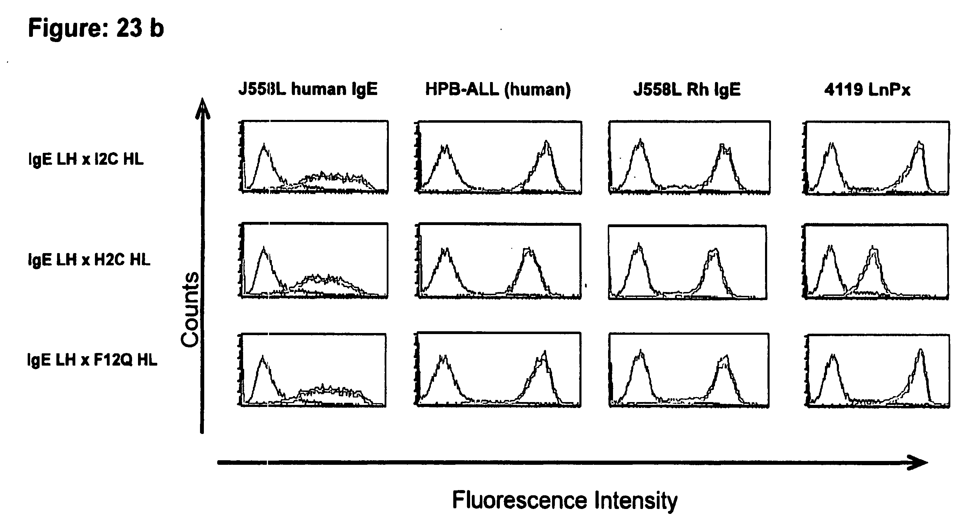

The bispecific binding of the single chain molecules listed above, which are cross-species specific for HER2 and cross-species specific for human and non-chimpanzee primate CD3 is clearly detectable as shown in Figure 23. In the FACS analysis all constructs show binding to CD3 and HER2 as compared to the respective negative controls. Cross-species specificity of the bispecific antibodies to human and macaque CD3 and HER2 antigens is demonstrated.

### 23.5. Bioactivity of HER2 and CD3 cross-species specific bispecific single chain antibodies

Bioactivity of the generated bispecific single chain antibodies is analyzed by chromium 51 ($^{51}$Cr) release in vitro cytotoxicity assays using the HER2 positive cell lines described in Examples 23.1 and 23.2. As effector cells stimulated human CD4/CD56 depleted PBMC or the macaque T cell line 4119LnPx are used as specified in the respective figures.

Generation of the stimulated CD4/CD56 depleted PBMC is performed as follows: A Petri dish (85 mm diameter, Nunc) is coated with a commercially available anti-CD3 specific antibody (e.g. OKT3, Othoclone) in a final concentration

of 1 μg/ml for 1 hour at 37°C. Unbound protein is removed by one washing step with PBS. The fresh PBMC are isolated from peripheral blood (30 - 50 ml human blood) by Ficoll gradient centrifugation according to standard protocols. 3 - 5 x 10[7] PBMC are added to the precoated petri dish in 50 ml of RPMI 1640 with stabilized glutamine / 10% FCS / IL-2 20 U/ml (Proleukin, Chiron) and stimulated for 2 days. On the third day the cells are collected and washed once with RPMI 1640. IL-2 is added to a final concentration of 20 U/ml and the cells are cultivated again for one day in the same cell culture medium as above. By depletion of CD4+ T cells and CD56+ NK cells according to standard protocols CD8+ cytotoxic T lymphocytes (CTLs) are enriched. Target cells are washed twice with PBS and labelled with 11.1 MBq $^{51}$Cr in a final volume of 100 μl RPMI with 50% FCS for 45 minutes at 37°C. Subsequently the labelled target cells are washed 3 times with 5 ml RPMI and then used in the cytotoxicity assay. The assay is performed in a 96 well plate in a total volume of 250μl supplemented RPMI (as above) with E:T ratios of 1:1 or 10:1, which are specified in the respective figures. 1 μg/ml of the cross-species specific bispecific single chain antibody molecules and 15 - 21 fivefold dilutions thereof are applied. The assay time is 18 hours and cytotoxicity is measured as relative values of released chromium in the supernatant related to the difference of maximum lysis (addition of Triton-X) and spontaneous lysis (without effector cells). All measurements are done in quadruplicates. Measurement of chromium activity in the supernatants is performed with a Wizard 3" gamma counter (Perkin Elmer Life Sciences GmbH, Köln, Germany). Analysis of the experimental data is performed with Prism 4 for Windows (version 4.02, GraphPad Software Inc., San Diego, California, USA). Sigmoidal dose response curves typically have $R^2$ values >0.90 as determined by the software. $EC_{50}$ values calculated by the analysis program are used for comparison of bioactivity.

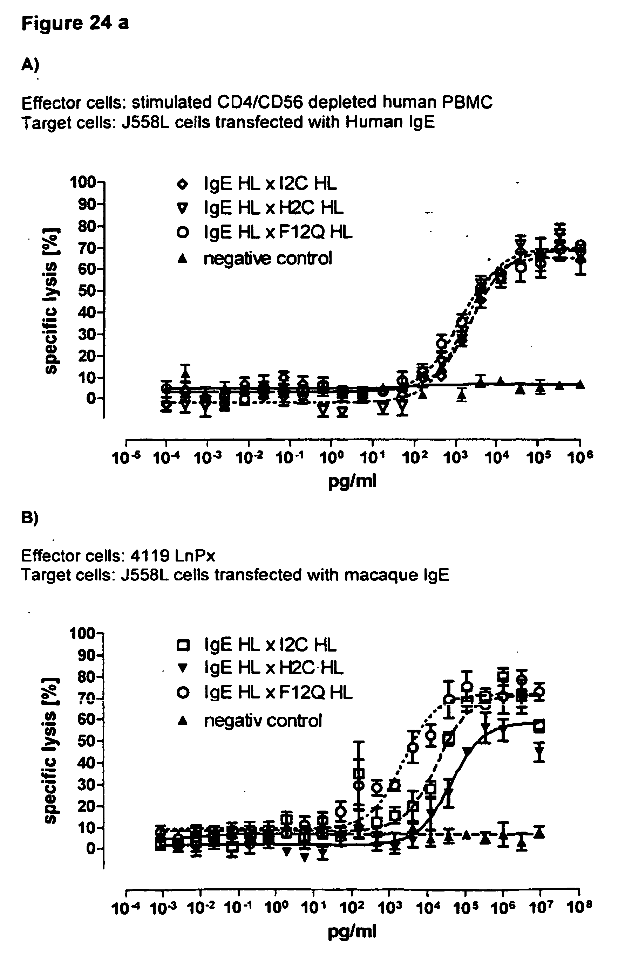

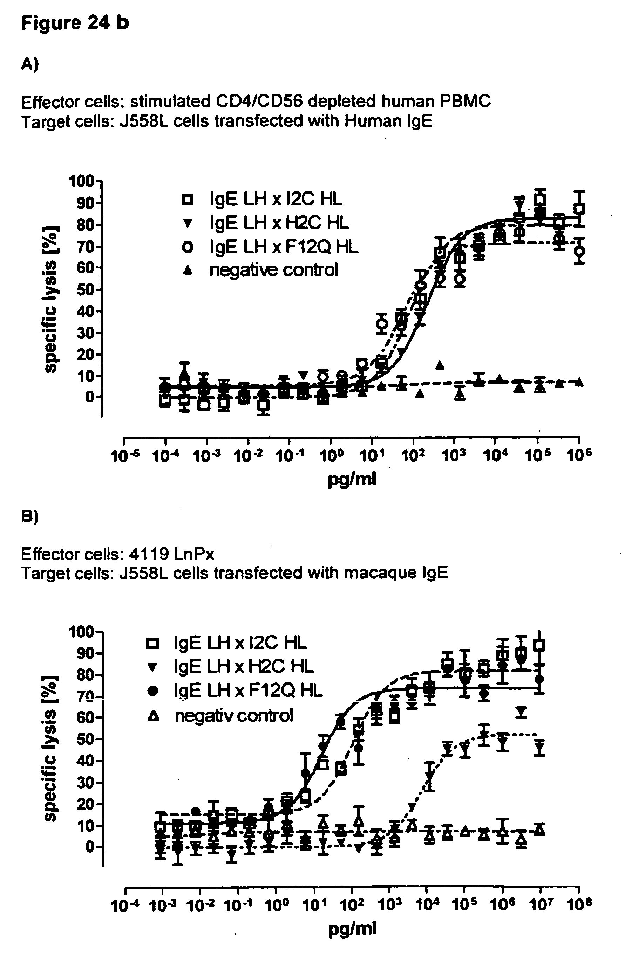

As shown in Figure 24 cross-species specific bispecific single chain antibody constructs demonstrate cytotoxic activity against human HER2 positive target cells elicited by stimulated human CD4/CD56 depleted PBMC and against macaque HER2 positive target cells elicited by the macaque T cell line 4119LnPx.

**Example 24: Cloning and expression of the human and macaque membrane bound form of IgE**

The mouse cell line J558L (obtained from Interlab Project, Istituto Nazionale per la Ricerca sul Cancro, Genova, Italy, ECACC 88032902), a spontaneous heavy chain-loss-variant myeloma cell line that synsizes and secretes a lambda light chain, was used to be complemented by a membrane bound heavy chain variant of the human and macaque IgE, respectively. In order to generate such constructs synthetic molecules were obtained by gene synthesis according to standard protocols (the nucleotide sequences of the constructs are listed under SEQ ID Nos 507 and 508). In these constructs the coding sequence for human and macaque c epsilon chain was fused to the human transmembrane region of IgE, respectively. The built in specificity of the VH chain is directed against the hapten (4-hydroxy-3-nitro-phenyl) acetyl) (NP). The gene synthesis fragment was also designed as to contain a Kozak site for eukaryotic expression of the construct and an immunoglobulin leader and restriction sites at the beginning and the end of the DNA. The introduced restriction sites EcoRI at the 5' end and SalI at the 3' end were utilised during the cloning step into the expression plasmid designated pEFDHFR. After sequence verification (macaque: XM_001116734 macaca mulatta Ig epsilon C region, mRNA; human: NC_000014 Homo sapiens chromosome 14, complete sequence, National Center for Biotechnology Information, http://www.ncbi.nlm.nih.gov/entrez ) the plasmids were used to transfect CHO/dhfr-cells as described above. Eukaryotic protein expression in DHFR deficient CHO cells is performed as described by Kaufmann R.J. (1990) Methods Enzymol. 185, 537-566. Gene amplification of the construct is induced by increasing concentrations of methothrexate (MTX) to a final concentration of up to 20 nM MTX.

**Example 25: Generation of IgE and CD3 cross-species specific bispecific single chain molecules**

Generally, bispecific single chain antibody molecules, each comprising a domain with a binding specificity for the human and the cynomolgus CD3 antigen as well as a domain with a binding specificity for the human and the macaque IgE antigen, were designed as set out in the following Table 8:

Table 8: Formats of anti-CD3 and anti-IgE cross-species specific Bispecific single chain antibody molecules

| SEQ ID (nucl/prot) | Formats of protein constructs (N → C) |
|---|---|
| 496/495 | IgE HL x H2C HL |
| 498/497 | IgE HL x F12Q HL |
| 500 / 499 | IgE HL x 12C HL |
| 502 / 501 | IgE LH x H2C HL |
| 504 / 503 | IgE LH x F12Q HL |

(continued)

| SEQ ID (nucl/prot) | Formats of protein constructs (N → C) |
|---|---|
| 506 / 505 | IgE LH x I2C HL |

The aforementioned constructs containing the variable light-chain (L) and variable heavy-chain (H) domains cross-species specific for human and macaque IgE and the CD3 specific VH and VL combinations cross-species specific for human and macaque CD3 are obtained by gene synthesis. The gene synthesis fragments are designed as to contain first a Kozak site for eukaryotic expression of the construct, followed by a 19 amino acid immunoglobulin leader peptide, followed in frame by the coding sequence of the respective bispecific single chain antibody molecule, followed in frame by the coding sequence of a 6 histidine tag and a stop codon. The gene synthesis fragment is also designed as to introduce suitable restriction sites at the beginning and at the end of the fragment. The introduced restriction sites are utilised in the following cloning procedures. The gene synthesis fragment is cloned via these restriction sites into a plasmid designated pEFDHFR following standard protocols. A clone with sequence-verified nucleotide sequence is transfected into DHFR deficient CHO cells for eukaryotic expression of the construct. Eukaryotic protein expression in DHFR deficient CHO cells is performed as described by Kaufmann R.J. (1990) Methods Enzymol. 185, 537-566. Gene amplification of the construct is induced by increasing concentrations of methothrexate (MTX) to a final concentration of up to 20 nM MTX. Alternatively the constructs are transfected into DHFR-deficient CHO-cells in a transient manner according to standard protocols.

The FACS binding experiments were performed with the human IgE transfected J558L cell line to assess the binding capability to the human IgE. The cross-species specificity to macaque IgE positive cells was tested by deploying the J558L cells transfected with the macaque IgE. The same changes in cell lines apply to the cytotoxicity assays performed with the IgE and CD3 cross-species specific bispecific single chain antibodies. Apart from this the assays were performed as described in examples 4 and 5.

As depicted in Figure 23, the generated IgE and CD3 cross-species specific bispecific single chain antibodies demonstrated binding to both the human and cynomolgus antigens and proved to be fully cross-species specific.

As shown in Figure 24, all of the generated cross-species specific bispecific single chain antibody constructs revealed cytotoxic activity against human IgE positive target cells elicited by human CD8+ cells and macaque IgE positive target cells elicited by the macaque T cell line 4119LnPx. As a negative control, an irrelevant bispecific single chain antibody has been used.

**Example 26: Specific binding of scFv clones to the N-terminus of human CD3 epsilon**

**26.1. Bacterial expression of scFv constructs in E. coli XL1 Blue**

As previously mentioned, *E. coli* XL1 Blue transformed with pComb3H5Bhis/Flag containing a VL- and VH-segment produce soluble scFv in sufficient amounts after excision of the gene III fragment and induction with 1 mM IPTG. The scFv-chain is exported into the periplasma where it folds into a functional conformation.

The following scFv clones were chosen for this experiment:

i) ScFvs 4-10, 3-106, 3-114, 3-148, 4-48, 3-190 and 3-271 as described in WO 2004/106380.
ii) ScFvs from the human anti-CD3epsilon binding clones H2C. F12Q and 12C as described herein.

For periplasmic preparations, bacterial cells transformed with the respective scFv containing plasmids allowing for periplasmic expression were grown in SB-medium supplemented with 20 mM MgCl$_2$ and carbenicillin 50 $\mu$g/ml and redissolved in PBS after harvesting. By four rounds of freezing at -70°C and thawing at 37°C, the outer membrane of the bacteria was destroyed by osmotic shock and the soluble periplasmic proteins including the scFvs were released into the supernatant. After elimination of intact cells and cell-debris by centrifugation, the supernatant containing the human anti-human CD3-scFvs was collected and used for further examination. These crude supernatants containing scFv will be further termed periplasmic preparations (PPP).

**26.2. Binding of scFvs to human CD3 epsilon (aa 1-27)- Fc fusion protein**

ELISA experiments were carried out by coating the human CD3 epsilon (aa 1-27)- Fc fusion protein to the wells of 96 well plastic plates (Nunc, maxisorb) typically at 4° C over night. The antigen coating solution was then removed, wells washed once with PBS/0.05 % Tween 20 and subsequently blocked with PBS/3 % BSA for at least one hour. After removal of the blocking solution, PPPs and control solutions were added to the wells and incubated for typically one hour at room temperature. The wells were then washed three times with PBS/0.05 % Tween 20. Detection of scFvs bound to immobilized antigen was carried out using a Biotin-labeled anti FLAG-tag antibody (M2 anti Flag-Bio, Sigma, typically at a final concentration of 1 $\mu$g/ml PBS) and detected with a peroxidase-labeled Streptavidine (Dianova, 1 $\mu$g/ml PBS). The signal was developed by adding ABTS substrate solution and measured at a wavelength of 405 nm. Unspecific binding of the test-samples to the blocking agent and/or the human IgG1 portion of the human CD3 epsilon (aa 1-27)-Fc fusion protein was examined by carrying out the identical assay with the identical reagents and identical timing on ELISA plates which were coated with human IgG1 (Sigma). PBS was used as a negative control.

As shown in Figure 25, scFvs H2C, F12Q and I2C show strong binding signals on human CD3 epsilon (aa 1-27)- Fc fusion protein. The human scFvs 3-106, 3-114, 3-148, 3-190, 3-271, 4-10 and 4-48 (as described in WO 2004/106380) do not show any significant binding above negative control level.

To exclude the possibility that the positive binding of scFvs H2C, F12Q and 12C to wells coated with human CD3 epsilon (aa 1-27)- Fc fusion protein might be due to binding to BSA (used as a blocking agent) and/or the human IgG1 Fc-gamma-portion of the human CD3 epsilon (aa 1-27)- Fc fusion protein, a second ELISA experiment was performed in paralle. In this second ELISA experiment, all parameters were identical to those in the first ELISA experiment, except that in the second ELISA experiment human IgG1 (Sigma) was coated instead of human CD3 epsilon (aa 1-27)-Fc fusion protein. As shown in Figure 26, none of the scFvs tested showed any significant binding to BSA and/or human IgG1 above background level. Taken together, these results allow the conclusion that the scFvs 4-10, 3-271, 3-148, 3-190, 4-48, 3-106 and 3-114 do not bind specifically to the human CD3 epsilon (aa 1-27)- region, whereas the scFvs H2C, F12Q and I2C clearly show specific binding to the N-terminal 27 amino acids of human CD3 epsilon.

| SEQ ID NO. | DESIGNATION | SOURCE | TYPE | UENCE |
|---|---|---|---|---|
| 1 | Human CD3ε extracellular domain | human | aa | QDGNEEMGGITQTPYKVSISGTTVILTCPQYPGSEILWQHNDKNIGGDEDDKNIGSDEDHLSLKE FSELEQSGYYVCYPRGSKPEDANFYLYLRARVCENCMEMD |
| 2 | Human CD3ε 1-27 | human | aa | QDGNEEMGGITQTPYKVSISGTTVILT |
| 3 | *Callithrix jacchus* CD3ε extracellular domain | *Callithrix jacchus* | aa | QDGNEEMGDTTQNPYKVSISGTTVTLTCPRYDGHEIKWLVNSQNKEGHEDHLLLEDFSEMEQSGY YACLSKETPAEEASHYLYLKARVCENCVEVD |
| 4 | *Callithrix jacchus* CD3ε 1-27 | *Callithrix jacchus* | aa | QDGNEEMGDTTQNPYKVSISGTTVTLT |
| 5 | *Saguinus oedipus* CD3ε extracellular domain | *Saguinus oedipus* | aa | QDGNEEMGDTTQNPYKVSISGTTVTLTCPRYDGHEIKWLVNSQNKEGHEDHLLLEDFSEMEQSGY YACLSKETPAEEASHYLYLKARVCENCVEVD |
| 6 | *Saguinus oedipus* CD3ε 1-27 | *Saguinus oedipus* | aa | QDGNEEMGDTTQNPYKVSISGTTVTLT |
| 7 | *Saimiri sciureus* CD3ε extracellular domain | *Saimiri sciureus* | aa | QDGNEEIGDTTQNPYKVSISGTTVTLTCPRYDGQEIKWLVNDQNKEGHEDHLLLEDFSEMEQSGY YACLSKETPTEEASHYLYLKARVCENCVEVD |
| 8 | *Saimiri sciureus* CD3ε 1-27 | *Saimiri sciureus* | aa | QDGNEEIGDTTQNPYKVSISGTTVTLT |
| 9 | CDR-L1 of F6A | artificial | aa | GSSTGAVTSGYYPN |
| 10 | CDR-L2 of F6A | artificial | aa | GTKFLAP |

| 11 | CDR-L3 of F6A | artificial | aa | ALWYSNRWV |
|---|---|---|---|---|
| 12 | CDR-H1 of F6A | artificial | aa | IYAMN |
| 13 | CDR-H2 of F6A | artificial | aa | RIRSKYNNYATYYADSVKS |
| 14 | CDR-H3 of F6A | artificial | aa | HGNFGNSYVSFFAY |
| 15 | VH of F6A | artificial | aa | EVQLVESGGGLVQPGGSLKLSCAASGFTFNIYAMNWVRQAPGKGLEWVARIRSKYNNYATYYADS VKSRFTISRDDSKNTAYLQMNNLKTEDTAVYYCVRHGNFGNSYVSFFAYWGQGTLVTVSS |
| 16 | VH of F6A | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATATCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TATCCTTCTTCGCTTACTGGGGCCAAGGGACTCTGGTCACCGTCTCCTCA |
| 17 | VL of F6A | artificial | aa | QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 18 | VL of F6A | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 19 | VH-P of F6A | artificial | aa | EVQLLESGGGLVQPGGSLKLSCAASGFTFNIYAMNWVRQAPGKGLEWVARIRSKYNNYATYYADS VKSRFTISRDDSKNTAYLQMNNLKTEDTAVYYCVRHGNFGNSYVSFFAYWGQGTLVTVSS |
| 20 | VH-P of F6A | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATATCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TATCCTTCTTCGCTTACTGGGGCCAAGGGACTCTGGTCACCGTCTCCTCA |
| 21 | VL-P of F6A | artificial | aa | ELVVTQEPSLTVSPGGTVTLTCGSSTGAVTSGYYPNWVQQKPGQAPRGLIGGTKFLAPGTPARFS |

EP 2 155 788 B1

| | | | | |
|---|---|---|---|---|
| | | | | GSLLGGKAALTLSGVQPEDEAEYYCALWYSNRWVFGGGTKLTVL |
| 22 | VL-P of F6A | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 23 | VH-VL of F6A | artificial | aa | EVQLVESGGGLVQPGGSLKLSCAASGFTFNIYAMNWVRQAPGKGLEWVARIRSKYNNYATYYADS VKSRFTISRDDSKNTAYLQMNNLKTEDTAVYYCVRHGNFGNSYVSFFAYWGQGTLVTVSSGGGGS GGGGSGGGGSQTVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 24 | VH-VL of F6A | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATATCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TATCCTTCTTCGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 25 | VH-VL-P of F6A | artificial | aa | EVQLLESGGGLVQPGGSLKLSCAASGFTFNIYAMNWVRQAPGKGLEWVARIRSKYNNYATYYADS VKSRFTISRDDSKNTAYLQMNNLKTEDTAVYYCVRHGNFGNSYVSFFAYWGQGTLVTVSSGGGGS GGGGSGGGGSELVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 26 | VH-VL-P of F6A | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATATCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TATCCTTCTTCGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT |

EP 2 155 788 B1

EP 2 155 788 B1

| | | | | ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
|---|---|---|---|---|
| 27 | CDR-L1 of H2C | artificial | aa | GSSTGAVTSGYYPN |
| 28 | CDR-L2 of H2C | artificial | aa | GTKFLAP |
| 29 | CDR-L3 of H2C | artificial | aa | ALWYSNRWV |
| 30 | CDR-H1 of H2C | artificial | aa | KYAMN |
| 31 | CDR-H2 of H2C | artificial | aa | RIRSKYNNYATYYADSVKD |
| 32 | CDR-H3 of H2C | artificial | aa | HGNFGNSYISYWAY |
| 33 | VH of H2C | artificial | aa | EVQLVESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSS |
| 34 | VH of H2C | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA ·TATCCTACTGGGCTTACTGGGGCCAAGGGACTCTGGTCACCGTCTCCTCA |
| 35 | VL of H2C | artificial | aa | QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 36 | VL of H2C | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 37 | VH-P of H2C | artificial | aa | EVQLLESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSS |

| 38 | VH-P of H2C | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCA |
|---|---|---|---|---|
| 39 | VL-P of H2C | artificial | aa | ELVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 40 | VL-P of H2C | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 41 | VH-VL of H2C | artificial | aa | EVQLVESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSQTVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 42 | VH-VL of H2C . | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA . |
| 43 | VH-VL-P of H2C | artificial | aa | EVQLLESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSELVVTQEPSLTVSPGGTVTLTCGSSTGAVTSGYYPNWVQQKPGQAPRGLIGGTKF |

| | | | | |
|---|---|---|---|---|
| | | | | LAPGTPARFSGSLLGGKAALTLSGVQPEDEAEYYCALWYSNRWVFGGGTKLTVL |
| 44 | VH-VL-P of H2C | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 45 | CDR-L1 of H1E | artificial | aa | GSSTGAVTSGYYPN |
| 46 | CDR-L2 of H1E | artificial | aa | GTKFLAP |
| 47 | CDR-L3 of H1E | artificial | aa | ALWYSNRWV |
| 48 | CDR-H1 of H1E | artificial | aa | SYAMN |
| 49 | CDR-H2 of H1E | artificial | aa | RIRSKYNNYATYYADSVKG |
| 50 | CDR-H3 of H1E | artificial | aa | HGNFGNSYLSFWAY |
| 51 | VH of H1E | artificial | aa | EVQLVESGGGLEQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYLSFWAYWGQGTLVTVSS |
| 52 | VH of H1E | artificial | nt | GAGGTGCAGCTGGTCGAGTCTGGAGGAGGATTGGAGCAGCCTGGAGGGTCATTGAAACTCTCATG TGCAGCCTCTGGATTCACCTTCAATTCGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACC TATCCTTCTGGGCTTACTGGGGCCAAGGGACTCTGGTCACCGTCTCCTC |
| 53 | VL of H1E | artificial | aa | QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |

EP 2 155 788 B1

EP 2 155 788 B1

| 54 | VL of H1E | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 55 | VH-P of H1E | artificial | aa | EVQLLESGGGLEQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYLSFWAYWGQGTLVTVSS |
| 56 | VH-P of H1E | artificial | nt | GAGGTGCAGCTGCTCGAGTCTGGAGGAGGATTGGAGCAGCCTGGAGGGTCATTGAAACTCTCATG TGCAGCCTCTGGATTCACCTTCAATTCGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACC TATCCTTCTGGGCTTACTGGGGCCAAGGGACTCTGGTCACCGTCTCCTCA |
| 57 | VL-P of H1E | artificial | aa | ELVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 58 | VL-P of H1E | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 59 | VH-VL of H1E | artificial | aa | EVQLVESGGGLEQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYLSFWAYWGQGTLVTVSSGGGGS GGGGSGGGGSQTVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 60 | VH-VL of H1E | artificial | nt | GAGGTGCAGCTGGTCGAGTCTGGAGGAGGATTGGAGCAGCCTGGAGGGTCATTGAAACTCTCATG TGCAGCCTCTGGATTCACCTTCAATTCGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACC TATCCTTCTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT |

| | | | | ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
|---|---|---|---|---|
| 61 | VH-VL-P of H1E | artificial | aa | EVQLLESGGGLEQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYLSFWAYWGQGTLVTVSSGGGGS GGGGSGGGGSSELVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 62 | VH-VL-P of H1E | artificial | nt | GAGGTGCAGCTGCTCGAGTCTGGAGGAGGATTGGAGCAGCCTGGAGGGTCATTGAAACTCTCATG TGCAGCCTCTGGATTCACCTTCAATTCGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACC TATCCTTCTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 63 | CDR-L1 of G4H | artificial | aa | GSSTGAVTSGYYPN |
| 64 | CDR-L2 of G4H | artificial | aa | GTKFLAP |
| 65 | CDR-L3 of G4H | artificial | aa | ALWYSNRWV |
| 66 | CDR-H1 of G4H | artificial | aa | RYAMN |
| 67 | CDR-H2 of G4H | artificial | aa | RIRSKYNNYATYYADSVKG |
| 68 | CDR-H3 of G4H | artificial | aa | HGNFGNSYLSYFAY |
| 69 | VH of G4H | artificial | aa | EVQLVESGGGLVQPGGSLKLSCAASGFTFNRYAMNWVRQAPGKGLEWVARIRSKYNNYATYYADS VKGRFTISRDDSKNTAYLQMNNLKTEDTAVYYCVRHGNFGNSYLSYFAYWGQGTLVTVSS |
| 70 | VH of G4H | artificial | nt | GAGGTGCAGCTGGTCGAGTCTGGAGGAGGATTGGTGCAGCCTGGAGGGTCATTGAAACTCTCATG |

EP 2 155 788 B1

|  | Description | Organism | Type | Sequence |
|---|---|---|---|---|
|  |  |  | nt | TGCAGCCTCTGATTCAATCGCTACGCCATGAACTGGTCCGCCAGGCTCCAGGAAAGGGTTTGGAATGGGTTGCTCGCATAAGAAGTAAATATAATTATGCAACATATTATGCCGATTCAGTGAAAGGGAGGTTCACCATCTCCAGAGATGATTCAAAAAAACACTGCCTATCTACAAATGAACAACTTGAAAACTGAGGACACTGCCGTGTACTACTGTGTGAGACATGGGAACTTCGGTAATAGCTACTTATCCTTACTTCGCTTACTGGGGCCAAGGGACTCTGGTCACCGTCTCCTCA |
| 71 | VL of G4H | artificial | aa | QTVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 72 | VL of G4H | artificial | nt | CAGACTGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTGCCCCCCGTACTCCTGCCAGATTCTCAGGCTCCCTGCTTGGAGGCAAGGCTGCCCTCACCCTCTCAGGGGTGTTCGGTGGTGGAGGAGGCAGAATATTACTGTGCTCTATGGTACAGCAACCGCTGGGTGTTCGGTGGAGGAACCAAACTGACTGTCCTA |
| 73 | VH-P of G4H | artificial | aa | EVQLLESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSS |
| 74 | VH-P of G4H | artificial | nt | GAGGTGCAGCTGCTCGAGTCTGGAGGAGGATTGGTGCAGCCTGGAGGGTCATTGAAACTCTCATGTGCAGCCTCTGATTCAATCGCTACGCCATGAACTGGTCCGCCAGGCTCCAGGAAAGGGTTTGGAATGGGTTGCTCGCATAAGAAGTAAATATAATTATGCAACATATTATGCCGATTCAGTGAAAGGGAGGTTCACCATCTCCAGAGATGATTCAAAAAAACACTGCCTATCTACAAATGAACAACTTGAAAACTGAGGACACTGCCGTGTACTACTGTGTGAGACATGGGAACTTCGGTAATAGCTACTTATCCTTACTTCGCTTACTGGGGCCAAGGGACTCTGGTCACCGTCTCCTCA |
| 75 | VL-P of G4H | artificial | aa | ELVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 76 | VL-P of G4H | artificial | nt | GAGCTCGTTGTGACTCAGGAACCTTCACTCACCGTATCACCTGGTGGAACAGTCACACTCACTTGTGGCTCCTCGACTGGGGCTGTTACATCTGGCTACTACCCAAACTGGGTCCAACAAAAACCAGGTCAGGCACCCCGTGGTCTAATAGGTGGGACTAAGTTCCTGCCCCCCGTACTCCTGCCAGATTCTCAGGCTCCCTGCTTGGAGGCAAGGCTGCCCTCACCCTCTCAGGGGTGTTCGGTGGTGGAGGAGGCAGAATATTACTGTGCTCTATGGTACAGCAACCGCTGGGTGTTCGGTGGAGGAACCAAACTGACTGTCCTA |
| 77 | VH-VL of G4H | artificial | aa | EVQLVESGGGLVQPGGSLKLSCAASGFTFNRYAMNWVRQAPGKGLEWVARIRSKYNNYATYYADSVKGRFTISRDDSKNTAYLQMNNLKTEDTAVYYCVRHGNFGNSYLSYFAYWGQGTLVTVSSGGGGSGGGGSGGGGSQTVVTQEPSLTVSPGGTVTLTCGSSTGAVTSGYYPNWVQQKPGQAPRGLIGGTKF |

EP 2 155 788 B1

| 78 | VH-VL of G4H | artificial | nt | LAPGTPARFSGSLLGGKAALTLSGVQPEDEAEYYCALWYSNRWVFGGGTKLTVL |
|---|---|---|---|---|
| 78 | VH-VL of G4H | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAATCGCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGGGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACT<br>TATCCTACTTCGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 79 | VH-VL-P of G4H | artificial | aa | EVQLLESGGGLVQPGGSLKLSCAASGFTFNRYAMNWVRQAPGKGLEWVARIRSKYNNYATYYADS<br>VKGRFTISRDDSKNTAYLQMNNLKTEDTAVYYCVRHGNFGNSYLSYFAYWGQGTLVTVSSGGGGS<br>GGGGSGGGGSELVVTQEPSLTVSPGGTVTLTCGSSTGAVTSGYYPNWVQQKPGQAPRGLIGGTKF<br>LAPGTPARFSGSLLGGKAALTLSGVQPEDEAEYYCALWYSNRWVFGGGTKLTVL |
| 80 | VH-VL-P of G4H | artificial | nt | GAGGTGCAGCTGCTCGAGTCTGGAGGAGGATTGGTGCAGCCTGGAGGGTCATTGAAACTCTCATG<br>TGCAGCCTCTGGATTCACCTTCAATCGCTACGCCATGAACTGGGTCCGCCAGGCTCCAGGAAAGG<br>GTTTGGAATGGGTTGCTCGCATAAGAAGTAAATATAATAATTATGCAACATATTATGCCGATTCA<br>GTGAAAGGGAGGTTCACCATCTCCAGAGATGATTCAAAAAACACTGCCTATCTACAAATGAACAA<br>CTTGAAAACTGAGGACACTGCCGTGTACTACTGTGTGAGACATGGGAACTTCGGTAATAGCTACT<br>TATCCTACTTCGCTTACTGGGGCCAAGGGACTCTGGTCACCGTCTCCTCAGGTGGTGGTGGTTCT<br>GGCGGCGGCGGCTCCGGTGGTGGTGGTTCTGAGCTCGTTGTGACTCAGGAACCTTCACTCACCGT<br>ATCACCTGGTGGAACAGTCACACTCACTTGTGGCTCCTCGACTGGGGCTGTTACATCTGGCTACT<br>ACCCAAACTGGGTCCAACAAAAACCAGGTCAGGCACCCCGTGGTCTAATAGGTGGGACTAAGTTC<br>CTCGCCCCCGGTACTCCTGCCAGATTCTCAGGCTCCCTGCTTGGAGGCAAGGCTGCCCTCACCCT<br>CTCAGGGGTACAGCCAGAGGATGAGGCAGAATATTACTGTGCTCTATGGTACAGCAACCGCTGGG<br>TGTTCGGTGGAGGAACCAAACTGACTGTCCTA |
| 81 | CDR-L1 of A2J | artificial | aa | RSSTGAVTSGYYPN |
| 82 | CDR-L2 of A2J | artificial | aa | ATDMRPS |
| 83 | CDR-L3 of A2J | artificial | aa | ALWYSNRWV |

| SEQ ID NO | Description | Source | Type | Sequence |
|---|---|---|---|---|
| 84 | CDR-H1 of A2J | artificial | aa | VYAMN |
| 85 | CDR-H2 of A2J | artificial | aa | RIRSKYNNYATYYADSVKK |
| 86 | CDR-H3 of A2J | artificial | aa | HGNFGNSYLSWWAY |
| 87 | VH of A2J | artificial | aa | EVQLVESGGGLVQPGGSLKLSCAASGFTFNVYAMNWVRQAPGKGLEWVARIRSKYNNYATYYADSVKKRFTISRDDSKNTAYLQMNNLKTEDTAVYYCVRHGNFGNSYLSWWAYWGQGTLVTVSS |
| 88 | VH of A2J | artificial | nt | GAGGTGCAGCTGGTCGAGTCTGGAGGAGGATTGGTGCAGCCTGGAGGGTCATTGAAACTCTCATGTGCAGCCTCTGGATTCACCTTCAATGTCTACGCCATGAACTGGGTCCGCCAGGCTCCAGGAAAGGGTTTGGAATGGGTTGCTCGCATAAGAAGTAAATAATAATTATGCAACATATTATGCCGATTCAGTGAAAAAGAGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACTTATCCTGGTGGGCTTACTGGGGCCAAGGGACTCTGGTCACCGTCTCCTCA |
| 89 | VL of A2J | artificial | aa | QTVVTQEPSLTVSPGGTVTLTCRSSTGAVTSGYYPNWVQQKPGQAPRGLIGATDMRPSGTPARFSGSLLGGKAALTLSGVQPEDEAEYYCALWYSNRWVFGGGTKLTVL |
| 90 | VL of A2J | artificial | nt | CAGACTGTTGTGACTCAGGAACCTTCACTCACCGTATCACCTGGTGGAACAGTCACACTCACTTGTCGCTCCTCGACTGGGGCTGTTACATCGGCTACTACCACCAAACTGGGTCCAAACAAAACCAGGTCAGGCACCCCGTGGTCTAATAGGTGCCACTGACATGAGGCCCTCTGGTACTCCTGCCAGATTCCTCAGGCTCCCTGCTTGGAGGCAAGGCTGCCCTCACCCTGCCTCTGGGTGTTCGGTGGTGTGGAGGATGAGGCAGAATATTACTGCTCTATGGTACAGACCACCGCTGGGTGTTCGGTGGTGTGGAGGAACCAAACTGACTGTCCTA |
| 91 | VH-P of A2J | artificial | aa | EVQLLESGGGLVQPGGSLKLSCAASGFTFNVYAMNWVRQAPGKGLEWVARIRSKYNNYATYYADSVKKRFTISRDDSKNTAYLQMNNLKTEDTAVYYCVRHGNFGNSYLSWWAYWGQGTLVTVSS |
| 92 | VH-P of A2J | artificial | nt | GAGGTGCAGCTGCTCGAGTCTGGAGGAGGATTGGTGCAGCCTGGAGGGTCATTGAAACTCTCATGTGCAGCCTCTGGATTCACCTTCAATGTCTACGCCATGAACTGGGTCCGCCAGGCTCCAGGAAAGGGTTTGGAATGGGTTGCTCGCATAAGAAGTAAATAATAATTATGCAACATATTATGCCGATTCAGTGAAAAAGAGAGGTTCACCATCTCCAGAGATGATTCAAAAAACACTGCCTATCTACAAATGAACAACTTGAAAACTGAGGACACTGCCGTGTACTACTGTGTGAGACATGGGAACTTCGGTAATAGCTACTTATCCTGGTGGGCTTACTGGGGCCAAGGGACTCTGGTCACCGTCTCCTCA |
| 93 | VL-P of A2J | artificial | aa | ELVVTQEPSLTVSPGGTVTLTCRSSTGAVTSGYYPNWVQQKPGQAPRGLIGATDMRPSGTPARFSGSLLGGKAALTLSGVQPEDEAEYYCALWYSNRWVFGGGTKLTVL |

| 91 | VL-P of A2J | artificial | nt | GAGCTCGTTGTGACTCAGGAACCTTCACTCACCGTATCACCTGGTGGAACAGTCACACTCACTTG TCGCTCCTCGACTGGGGCTGTTACATCTGGCTACTACCCAAACTGGGTCCAACAAAAACCAGGTC AGGCACCCCGTGGTCTAATAGGTGCCACTGACATGAGGCCCTCTGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 95 | VH-VL of A2J | artificial | aa | EVQLVESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYLSWWAYWGQGTLVTVSSGGGGS GGGGSGGGGSQTVVTQEPSLTVSPGGTVTLTCRSSTGAVTSGYYPNWVQQKPGQAPRGLIGATDM RPSGTPARFSGSLLGGKAALTLSGVQPEDEAEYYCALWYSNRWVFGGGTKLTVL |
| 96 | VH-VL of A2J | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATGTCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACT TATCCTGGTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTCGCTCCTCGACTGGGGCTGTTACATCTGGCTACT ACCCAAACTGGGTCCAACAAAAACCAGGTCAGGCACCCCGTGGTCTAATAGGTGCCACTGACATG AGGCCCTCTGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 97 | VH-VL-P of A2J | artificial | aa | EVQLLESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYLSWWAYWGQGTLVTVSSGGGGS GGGGSGGGGSELVVTQEPSLTVSPGGTVTLTCRSSTGAVTSGYYPNWVQQKPGQAPRGLIGATDM RPSGTPARFSGSLLGGKAALTLSGVQPEDEAEYYCALWYSNRWVFGGGTKLTVL |
| 98 | VH-VL-P of A2J | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATGTCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACT TATCCTGGTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTCGCTCCTCGACTGGGGCTGTTACATCTGGCTACT |

EP 2 155 788 B1

| No. | Name | Source | Type | Sequence |
|---|---|---|---|---|
|  |  |  |  | ACCCAAAACTGGGTCCAACAAAAACCAGGTCAGGCACCCCGTGGTCTAATAGGTGCCACTGACATGAGGCCCTCTGGTACTCCTGCCAGATTCTCAGCTCCCTTGGAGGCAAGGCTGCCCTCACCCTCTCAGGGGTACAGCCAGGCAGAGGCAGAATATTACTGTGCTCTATGGTACAGCAACCGCTGGGTGTTCGGTGGAGGAACCAAACTGACTGTCCTA |
| 99 | CDR-L1 of E1L | artificial | aa | GSSTGAVTSGYYPN |
| 100 | CDR-L2 of E1L | artificial | aa | GTKFLAP |
| 101 | CDR-L3 of E1L | artificial | aa | ALWYSNRWV |
| 102 | CDR-H1 of E1L | artificial | aa | KYAMN |
| 103 | CDR-H2 of E1L | artificial | aa | RIRSKYNNYATYYADSVKS |
| 104 | CDR-H3 of E1L | artificial | aa | HGNFGNSYTSYYAY |
| 105 | VH of E1L | artificial | aa | EVQLVESGGGLVQPGGSLKLSCAASGFTFNKYAMNWVRQAPGKGLEWVARIRSKYNNYATYYADSVKSRFTISRDDSKNTAYLQMNNLKTEDTAVYYCVRHGNFGNSYTSYYAYWGQGTLVTVSS |
| 106 | VH of E1L | artificial | nt | GAGGTGCAGCTGGTCGAGTCTGGAGGAGGATTGGTGCAGCCTGGAGGGTCCGCCAGCTCATGTGCAGCCTCTGGATTCACCTTCAATAAGTACGCCATGAACTGGGTCCGCCAGGCTCCAGGAAAGGGTTTGGAATGGGTTGCTCGCATAAGAAGTAAATATAATAATTATGCAACATATTATGCCGATTCAGTGAAATCGAGGTTCACCATCTCCAGAGATGATTCAAAAAACACTGCTATCTACAAATGAACAACTTGAAAACTGAGGACGCACTGCCGTGTACTACGTGTGAGACAGTGGGAGCATGGGAACTTCGGTAATAGCTACACATCCTTACTACGCTTACTGGGGCAAGGACTCTTGCTCCTCA |
| 107 | VL of E1L | artificial | aa | QTVVTQEPSLTVSPGGTVTLTCGSSTGAVTSGYYPNWVQQKPGQAPRGLIGTKFLAPGTPARFSGSLLGGKAALTLSGVQPEDEAEYYCALWYSNRWVFGGGTKLTVL |
| 108 | VL of E1L | artificial | nt | CAGACTGTTGTGACTCAGGAACCTTCACCTGTGTCACCTGGTGGAACAGTCACACTCACTTGTGGCTCCTCGACTGGGGCGTGTTACATCTGGCTACTACCACCAAACTGGTCCAACAAAAACCAGGTCAGGCACCCCGTGGTCTAATAGGTGCCAAGGCTGCCCTCACCCTGCCCCCCGGTACTCCTGCCAGATTCTCAGGCTCCCTGCTTGGAGGCAAGGCAACCGCTGTACAGCAGAGATGAGGCAGAATATTACTGTGCTCTATGGTACAGCAACCGCTGTTCGGTGGAGGAACCAAACTGACTGTGTCCTA |
| 109 | VH-P of E1L | artificial | aa | EVQLLESGGGLVQPGGSLKLSCAASGFTFNKYAMNWVRQAPGKGLEWVARIRSKYNNYATYYADSVKSRFTISRDDSKNTAYLQMNNLKTEDTAVYYCVRHGNFGNSYTSYYAYWGQGTLVTVSS |

EP 2 155 788 B1

| 110 | VH-P of E1L | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAATCGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA CATCCTACTACGCTTACTGGGGCCAAGGGACTCTGGTCACCGTCTCCTCA |
| 111 | VL-P of E1L | artificial | aa | ELVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 112 | VL-P of E1L | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 113 | VH-VL of E1L | artificial | aa | EVQLVESGGGLVQPGGSLKLSCAASGFTFNKYAMNWVRQAPGKGLEWVARIRSKYNNYATYYADS VKSRFTISRDDSKNTAYLQMNNLKTEDTAVYYCVRHGNFGNSYTSYYAYWGQGTLVTVSSGGGGS GGGGSGGGGSQTVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 114 | VH-VL of E1L | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAATCGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA CATCCTACTACGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 115 | VH-VL-P of E1L | artificial | aa | EVQLLESGGGLVQPGGSLKLSCAASGFTFNKYAMNWVRQAPGKGLEWVARIRSKYNNYATYYADS VKSRFTISRDDSKNTAYLQMNNLKTEDTAVYYCVRHGNFGNSYTSYYAYWGQGTLVTVSSGGGGS GGGGSGGGGSELVVTQEPSLTVSPGGTVTLTCGSSTGAVTSGYYPNWVQQKPGQAPRGLIGGTKF |

| | | | | |
|---|---|---|---|---|
| | | | | LAPGTPARFSGSLLGGKAALTLSGVQPEDEAEYYCALWYSNRWVFGGGTKLTVL |
| 116 | VH-VL-P of E1L | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAATCGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA CATCCTACTACGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 117 | CDR-L1 of E2M | artificial | aa | RSSTGAVTSGYYPN |
| 118 | CDR-L2 of E2M | artificial | aa | ATDMRPS |
| 119 | CDR-L3 of E2M | artificial | aa | ALWYSNRWV |
| 120 | CDR-H1 of E2M | artificial | aa | GYAMN |
| 121 | CDR-H2 of E2M | artificial | aa | RIRSKYNNYATYYADSVKE |
| 122 | CDR-H3 of E2M | artificial | aa | HRNFGNSYLSWFAY |
| 123 | VH of E2M | artificial | aa | EVQLVESGGGLVQPGGSLKLSCAASGFTFNGYAMNWVRQAPGKGLEWVARIRSKYNNYATYYADS VKERFTISRDDSKNTAYLQMNNLKTEDTAVYYCVRHRNFGNSYLSWFAYWGQGTLVTVSS |
| 124 | VH of E2M | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATGGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGAGAGGTTCACCATCTCCAGAGATGATTCAAAAAACACTGCCTATCTACAAATGAACAA CTTGAAAACTGAGGACACTGCCGTGTACTACTGTGTGAGACATAGGAACTTCGGTAATAGCTACT TATCCTGGTTCGCTTACTGGGGCCAAGGGACTCTGGTCACCGTCTCCTCA |
| 125 | VL of E2M | artificial | aa | QTVVTQEPSLTVSPGGTVTLTCRSSTGAVTSGYYPNWVQQKPGQAPRGLIGATDMRPSGTPARFS GSLLGGKAALTLSGVQPEDEAEYYCALWYSNRWVFGGGTKLTVL |

| 126 | VL of E2M | artificial | nt | CAGACTGTTGTGACTCAGGAACCTTCACTCACCGTATCACCTGGTGGAACAGTCACACTCACTTG TCGCTCCTCGACTGGGGCTGTTACATCTGGCTACTACCCAAACTGGGTCCAACAAAAACCAGGTC AGGCACCCCGTGGTCTAATAGGTGCCACTGACATGAGGCCCTCTGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 127 | VH-P of E2M | artificial | aa | EVQLLESGGGLVQPGGSLKLSCAASGFTFNGYAMNWVRQAPGKGLEWVARIRSKYNNYATYYADS VKERFTISRDDSKNTAYLQMNNLKTEDTAVYYCVRHRNFGNSYLSWFAYWGQGTLVTVSS |
| 128 | VH-P of E2M | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATGGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGAGAGGTTCACCATCTCCAGAGATGATTCAAAAAACACTGCCTATCTACAAATGAACAA CTTGAAAACTGAGGACACTGCCGTGTACTACTGTGTGAGACATAGGAACTTCGGTAATAGCTACT TATCCTGGTTCGCTTACTGGGGCCAAGGGACTCTGGTCACCGTCTCCTCA |
| 129 | VL-P of E2M | artificial | aa | ELVVTQEPSLTVSPGGTVTLTCRSSTGAVTSGYYPNWVQQKPGQAPRGLIGATDMRPSGTPARFS GSLLGGKAALTLSGVQPEDEAEYYCALWYSNRWVFGGGTKLTVL |
| 130 | VL-P of E2M | artificial | nt | GAGCTCGTTGTGACTCAGGAACCTTCACTCACCGTATCACCTGGTGGAACAGTCACACTCACTTG TCGCTCCTCGACTGGGGCTGTTACATCTGGCTACTACCCAAACTGGGTCCAACAAAAACCAGGTC AGGCACCCCGTGGTCTAATAGGTGCCACTGACATGAGGCCCTCTGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 131 | VH-VL of E2M | artificial | aa | EVQLVESGGGLVQPGGSLKLSCAASGFTFNGYAMNWVRQAPGKGLEWVARIRSKYNNYATYYADS VKERFTISRDDSKNTAYLQMNNLKTEDTAVYYCVRHRNFGNSYLSWFAYWGQGTLVTVSSGGGGS GGGGSGGGGSQTVVTQEPSLTVSPGGTVTLTCRSSTGAVTSGYYPNWVQQKPGQAPRGLIGATDM RPSGTPARFSGSLLGGKAALTLSGVQPEDEAEYYCALWYSNRWVFGGGTKLTVL |
| 132 | VH-VL of E2M | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATGGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGAGAGGTTCACCATCTCCAGAGATGATTCAAAAAACACTGCCTATCTACAAATGAACAA CTTGAAAACTGAGGACACTGCCGTGTACTACTGTGTGAGACATAGGAACTTCGGTAATAGCTACT TATCCTGGTTCGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT |

EP 2 155 788 B1

| | | | | |
|---|---|---|---|---|
| | | | | ATCACCTGGTGGAACAGTCACACTCACTTGTCGCTCCTCGACTGGGGCTGTTACATCTGGCTACT ACCCAAACTGGGTCCAACAAAAACCAGGTCAGGCACCCCGTGGTCTAATAGGTGCCACTGACATG AGGCCCTCTGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 133 | VH-VL-P of E2M | artificial | aa | EVQLLESGGGLVQPGGSLKLSCAASGFTFNGYAMNWVRQAPGKGLEWVARIRSKYNNYATYYADS VKERFTISRDDSKNTAYLQMNNLKTEDTAVYYCVRHRNFGNSYLSWFAYWGQGTLVTVSSGGGGS GGGGSGGGGSELVVTQEPSLTVSPGGTVTLTCRSSTGAVTSGYYPNWVQQKPGQAPRGLIGATDM RPSGTPARFSGSLLGGKAALTLSGVQPEDEAEYYCALWYSNRWVFGGGTKLTVL |
| 134 | VH-VL-P of E2M | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATGGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGAGAGGTTCACCATCTCCAGAGATGATTCAAAAAACACTGCCTATCTACAAATGAACAA CTTGAAAACTGAGGACACTGCCGTGTACTACTGTGTGAGACATAGGAACTTCGGTAATAGCTACT TATCCTGGTTCGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTCGCTCCTCGACTGGGGCTGTTACATCTGGCTACT ACCCAAACTGGGTCCAACAAAAACCAGGTCAGGCACCCCGTGGTCTAATAGGTGCCACTGACATG AGGCCCTCTGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 135 | CDR-L1 of F7O | artificial | aa | GSSTGAVTSGYYPN |
| 136 | CDR-L2 of F7O | artificial | aa | GTKFLAP |
| 137 | CDR-L3 of F7O | artificial | aa | ALWYSNRWV |
| 138 | CDR-H1 of F7O | artificial | aa | VYAMN |
| 139 | CDR-H2 of F7O | artificial | aa | RIRSKYNNYATYYADSVKK |
| 140 | CDR-H3 of F7O | artificial | aa | HGNFGNSYISWWAY |
| 141 | VH of F7O | artificial | aa | EVQLVESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYISWWAYWGQGTLVTVSS |
| 142 | VH of F7O | artificial | nt | GAGGTGCAGCTGGTCGAGTCTGGAGGAGGATTGGTGCAGCCTGGAGGGTCATTGAAACTCTCATG |

EP 2 155 788 B1

| | | | | |
|---|---|---|---|---|
| | | | | TGCAGCCTCTGGATTCACCTTCAATGTGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTGGTGGGCTTACTGGGGCCAAGGGACTCTGGTCACCGTCTCCTCA |
| 143 | VL of F7O | artificial | aa | QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 144 | VL of F7O | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 145 | VH-P of F7O | artificial | aa | EVQLLESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYISWWAYWGQGTLVTVSS |
| 146 | VH-P of F7O | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATGTGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTGGTGGGCTTACTGGGGCCAAGGGACTCTGGTCACCGTCTCCTCA |
| 147 | VL-P of F7O | artificial | aa | ELVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 148 | VL-P of F7O | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGCTCTATGGTACAGCAACCGCTGGGTGTTCGGTGGAGGAACCAAACTGACTGTCC TA |
| 149 | VH-VL of F7O | artificial | aa | EVQLVESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYISWWAYWGQGTLVTVSSGGGGS GGGGSGGGGSQTVVTQEPSLTVSPGGTVTLTCGSSTGAVTSGYYPNWVQQKPGQAPRGLIGGTKF |

EP 2 155 788 B1

| | | | | LAPGTPARFSGSLLGGKAALTLSGVQPEDEAEYYCALWYSNRWVFGGGTKLTVL |
|---|---|---|---|---|
| 150 | VH-VL of F7O | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATGTGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAAACACTGCCTATCTACAAATGAACAA CTTGAAAACTGAGGACACTGCCGTGTACTACTGTGTGAGACATGGGAACTTCGGTAATAGCTACA TATCCTGGTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 151 | VH-VL-P of F7O | artificial | aa | EVQLLESGGGLVQPGGSLKLSCAASGFTFNVYAMNWVRQAPGKGLEWVARIRSKYNNYATYYADS VKKRFTISRDDSKNTAYLQMNNLKTEDTAVYYCVRHGNFGNSYISWWAYWGQGTLVTVSSGGGGS GGGGSGGGGSELVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVL |
| 152 | VH-VL-P of F7O | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATGTGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAAAGAGGTTCACCATCTCCAGAGATGATTCAAAAAAACACTGCCTATCTACAAATGAACAA CTTGAAAACTGAGGACACTGCCGTGTACTACTGTGTGAGACATGGGAACTTCGGTAATAGCTACA TATCCTGGTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTA |
| 153 | CDR-L1 of F12Q | artificial | aa | GSSTGAVTSGNYPN |
| 154 | CDR-L2 of F12Q | artificial | aa | GTKFLAP |
| 155 | CDR-L3 of F12Q | artificial | aa | VLWYSNRWV |

| 156 | CDR-H1 of F12Q | artificial | aa | SYAMN |
|---|---|---|---|---|
| 157 | CDR-H2 of F12Q | artificial | aa | RIRSKYNNYATYYADSVKG |
| 158 | CDR-H3 of F12Q | artificial | aa | HGNFGNSYVSWWAY |
| 159 | VH of F12Q | artificial | aa | EVQLVESGGGLVQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYVSWWAYWGQGTLVTVSS |
| 160 | VH of F12Q | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TTTCCTGGTGGGCTTACTGGGGCCAAGGGACTCTGGTCACCGTCTCCTCA |
| 161 | VL of F12Q | artificial | aa | QTVVTQEPSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 162 | VL of F12Q | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCAACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGTTCTATGGTACAGCAACCGCTGGGTGTTCGGTGGAGGAACCAAACTGACTGTCC TA |
| 163 | VH-P of F12Q | artificial | aa | EVQLLESGGGLVQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYVSWWAYWGQGTLVTVSS |
| 164 | VH-P of F12Q | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TTTCCTGGTGGGCTTACTGGGGCCAAGGGACTCTGGTCACCGTCTCCTCA |
| 165 | VL-P of F12Q | artificial | aa | ELVVTQEPSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |

| 166 | VL-P of F12Q | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCAACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGTTCTATGGTACAGCAACCGCTGGGTGTTCGGTGGAGGAACCAAACTGACTGTCC TA |
| 167 | VH-VL of F12Q | artificial | aa | EVQLVESGGGLVQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYVSWWAYWGQGTLVTVSSGGGGS GGGGSGGGGSQTVVTQEPSLTVSPGGTVTLTCGSSTGAVTSGNYPNWVQQKPGQAPRGLIGGTKF LAPGTPARFSGSLLGGKAALTLSGVQPEDEAEYYCVLWYSNRWVFGGGTKLTVL |
| 168 | VH-VL of F12Q | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TTTCCTGGTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCAACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGTTCTATGGTACAGCAACCGCTGGG TGTTCGGTGGAGGAACCAAACTGACTGTCCTA |
| 169 | VH-VL-P of F12Q | artificial | aa | EVQLLESGGGLVQPGGSLKLSCAASGFTFNSYAMNWVRQAPGKGLEWVARIRSKYNNYATYYADS VKGRFTISRDDSKNTAYLQMNNLKTEDTAVYYCVRHGNFGNSYVSWWAYWGQGTLVTVSSGGGGS GGGGSGGGGSELVVTQEPSLTVSPGGTVTLTCGSSTGAVTSGNYPNWVQQKPGQAPRGLIGGTKF LAPGTPARFSGSLLGGKAALTLSGVQPEDEAEYYCVLWYSNRWVFGGGTKLTVL |
| 170 | VH-VL-P of F12Q | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAGCTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGGCAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACG TTTCCTGGTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCAACT |

EP 2 155 788 B1

| | | | | |
|---|---|---|---|---|
| | | | | ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGTTCTATGGTACAGCAACCGCTGGG TGTTCGGTGGAGGAACCAAACTGACTGTCCTA |
| 171 | CDR-L1 of I2C | artificial | aa | GSSTGAVTSGNYPN |
| 172 | CDR-L2 of I2C | artificial | aa | GTKFLAP |
| 173 | CDR-L3 of I2C | artificial | aa | VLWYSNRWV |
| 174 | CDR-H1 of I2C | artificial | aa | KYAMN |
| 175 | CDR-H2 of I2C | artificial | aa | RIRSKYNNYATYYADSVKD |
| 176 | CDR-H3 of I2C | artificial | aa | HGNFGNSYISYWAY |
| 177 | VH of I2C | artificial | aa | EVQLVESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSS |
| 178 | VH of I2C | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCA |
| 179 | VL of I2C | artificial | aa | QTVVTQEPSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 180 | VL of I2C | artificial | nt | CAGACTGTTGTGACTCAGGAACCTTCACTCACCGTATCACCTGGTGGAACAGTCACACTCACTTG TGGCTCCTCGACTGGGGCTGTTACATCTGGCAACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGTTCTATGGTACAGCAACCGCTGGGTGTTCGGTGGAGGAACCAAACTGACTGTCC TA |
| 181 | VH-P of I2C | artificial | aa | EVQLLESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSS |

EP 2 155 788 B1

| 182 | VH-P of I2C | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCA |
|---|---|---|---|---|
| 183 | VL-P of I2C | artificial | aa | ELVVTQEPSLTVSPGGTVTLTCGSSTGAVTSGNYPNWVQQKPGQAPRGLIGGTKFLAPGTPARFS GSLLGGKAALTLSGVQPEDEAEYYCVLWYSNRWVFGGGTKLTVL |
| 184 | VL-P of I2C | artificial | nt | GAGCTCGTTGTGACTCAGGAACCTTCACTCACCGTATCACCTGGTGGAACAGTCACACTCACTTG TGGCTCCTCGACTGGGGCTGTTACATCTGGCAACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA ATATTACTGTGTTCTATGGTACAGCAACCGCTGGGTGTTCGGTGGAGGAACCAAACTGACTGTCC TA |
| 185 | VH-VL of I2C | artificial | aa | EVQLVESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSQTVVTQEPSLTVSPGGTVTLTCGSSTGAVTSGNYPNWVQQKPGQAPRGLIGGTKF LAPGTPARFSGSLLGGKAALTLSGVQPEDEAEYYCVLWYSNRWVFGGGTKLTVL |
| 186 | VH-VL of I2C | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCAACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGTTCTATGGTACAGCAACCGCTGGG TGTTCGGTGGAGGAACCAAACTGACTGTCCTA |
| 187 | VH-VL-P of I2C | artificial | aa | EVQLLESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSELVVTQEPSLTVSPGGTVTLTCGSSTGAVTSGNYPNWVQQKPGQAPRGLIGGTKF |

EP 2 155 788 B1

| | | | | |
|---|---|---|---|---|
| | | | | LAPGTPARFSGSLLGGKAALTLSGVQPEDEAEYYCVLWYSNRWVFGGGTKLTVL |
| 188 | VH-VL-P of I2C | artificial | nt | GAGGTGCAGCTGCTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTGAGCTCGTTGTGACTCAGGAACCTTCACTCACCGT ATCACCTGGTGGAACAGTCACACTCACTTGTGGCTCCTCGACTGGGGCTGTTACATCTGGCAACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGTTCTATGGTACAGCAACCGCTGGG TGTTCGGTGGAGGAACCAAACTGACTGTCCTA |
| 189 | CDR-L1 of EGF-R 21-63 | artificial | aa | RASQSVSSSTYSYIH |
| 190 | CDR-L2 of EGF-R 21-63 | artificial | aa | YASNLES |
| 191 | CDR-L3 of EGF-R 21-63 | artificial | aa | QHSWEIPFT |
| 192 | CDR-H1 of EGF-R 21-63 | artificial | aa | DCVII |
| 193 | CDR-H2 of EGF-R 21-63 | artificial | aa | QIYPGTGRSYYNEIFKG |
| 194 | CDR-H3 of EGF-R 21-63 | artificial | aa | STLIHGTWFSY |
| 195 | VH of EGF-R 21-63 | artificial | aa | QVQLQQSGPDLVKPGASVKMSCKASGHTFTDCVIIWVKQRAGQGLEWIGQIYPGTGRSYYNEIFK GKATLTADKSSNTVHIQLSSLTSEDSAVYFCALSTLIHGTWFSYWGQGTLVTVSS |
| 196 | VH of EGF-R 21-63 | artificial | nt | CAGGTTCAGCTGCAGCAGTCTGGACCTGATCTGGTGAAGCCTGGGGCCTCAGTGAAGATGTCCTG CAAGGCTTCTGGACACACTTTCACTGACTGTGTTATAATCTGGGTGAAACAGAGAGCTGGACAGG GCCTTGAGTGGATTGGACAGATTTATCCAGGGACTGGTCGTTCTTACTACAATGAGATTTTCAAG GGCAAGGCCACACTGACTGCAGACAAATCCTCCAACACAGTCCACATTCAACTCAGCAGCCTGAC |

EP 2 155 788 B1

EP 2 155 788 B1

| | | | | |
|---|---|---|---|---|
| | | | | ATCTGAGGACTCTGCGGTCTATTTCTGTGCCCTATCTACTCTTATTCACGGGACCTGGTTTTCTT ATTGGGGCCAAGGGACTCTGGTCACTGTCTCTTCC |
| 197 | VL of EGF-R 21-63 | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIK |
| 198 | VL of EGF-R 21-63 | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAA |
| 199 | VL-VH of EGF-R 21-63 | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSS |
| 200 | VL-VH of EGF-R 21-63 | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCC |
| 201 | CDR-L1 of MCSP-G4 | artificial | aa | KSSQSVLNSSNNRNYLA |
| 202 | CDR-L2 of MCSP-G4 | artificial | aa | WASTRES |
| 203 | CDR-L3 of MCSP-G4 | artificial | aa | QQHYSTPFT |

| 204 | CDR-H1 of MCSP-G4 | artificial | aa | NYYIH |
|------|-------------------|------------|----|-------|
| 205 | CDR-H2 of MCSP-G4 | artificial | aa | WINPNSGATNYAQKFQG |
| 206 | CDR-H3 of MCSP-G4 | artificial | aa | SWVSWFAS |
| 207 | VH of MCSP-G4 | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSS |
| 208 | VH of MCSP-G4 | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCA |
| 209 | VL of MCSP-G4 | artificial | aa | DIVMTQSPDSLAVSLGERATINCKSSQSVLNSSNNRNYLAWYQQKPGQPPKLLIYWASTRESGVP DRFSGSGSGTDFTLTISGLQAEDVAVYYCQQHYSTPFTFGPGTKVDIK |
| 210 | VL of MCSP-G4 | artificial | nt | GATATCGTGATGACCCAGTCTCCAGACTCCCTGGCTGTGTCTCTGGGCGAGAGGGCCACCATCAA CTGCAAGTCCAGCCAGAGTGTTTTAAACAGCTCCAACAATAGGAACTACTTAGCTTGGTACCAGC AGAAACCAGGACAGCCTCCTAAGCTGCTCATTTACTGGGCATCTACCCGGGAATCCGGGGTCCCT GACCGATTCAGTGGCAGCGGGTCTGGGACAGATTTCACTCTCACCATCAGTGGCCTGCAGGCTGA AGATGTGGCAGTTTATTACTGTCAGCAACATTATAGTACTCCATTCACTTTTGGCCCTGGGACCA AAGTGGATATCAAA |
| 211 | VH-P of MCSP-G4 | artificial | aa | EVQLLESGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSS |
| 212 | VH-P of MCSP-G4 | artificial | nt | GAGGTGCAGCTGCTCGAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCA |

EP 2 155 788 B1

| | | | | |
|---|---|---|---|---|
| 213 | VL-P of MCSP-G4 | artificial | aa | ELVMTQSPDSLAVSLGERATINCKSSQSVLNSSNNRNYLAWYQQKPGQPPKLLIYWASTRESGVP DRFSGSGSGTDFTLTISGLQAEDVAVYYCQQHYSTPFTFGPGTKVDIK |
| 214 | VL-P of MCSP-G4 | artificial | nt | GAGCTCGTGATGACCCAGTCTCCAGACTCCCTGGCTGTGTCTCTGGGCGAGAGGGCCACCATCAA CTGCAAGTCCAGCCAGAGTGTTTTAAACAGCTCCAACAATAGGAACTACTTAGCTTGGTACCAGC AGAAACCAGGACAGCCTCCTAAGCTGCTCATTTACTGGGCATCTACCCGGGAATCCGGGGTCCCT GACCGATTCAGTGGCAGCGGGTCTGGGACAGATTTCACTCTCACCATCAGTGGCCTGCAGGCTGA AGATGTGGCAGTTTATTACTGTCAGCAACATTATAGTACTCCATTCACTTTTGGCCCTGGGACCA AAGTGGATATCAAA |
| 215 | VH-VL of MCSP-G4 | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSDIVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIK |
| 216 | VH-VL of MCSP-G4 | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGATATCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAA |
| 217 | VH-VL-P of MCSP-G4 | artificial | aa | EVQLLESGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIK |
| 218 | VH-VL-P of MCSP-G4 | artificial | nt | GAGGTGCAGCTGCTCGAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC |

EP 2 155 788 B1

| Sequence | | type | source | name | SEQ ID |
|---|---|---|---|---|---|
| AAGGAACCTTGGTCACCGTCTCCTCAGTGGTGGTTCTGGCGGCGGCGGCTCCGGTGGTGGTGGTTCTGAGCTCGTGATGACCCAGTCTCCAGACTCCCTGGCTGTGTCTCTGGGCGAGAGGGCCACCATCAACTGCAAGTCCAGCCAGAGTGTTTAAACAGCTCCAACAATAGGAACTACTTAGCTTGGTACCAGCAGAAACCAGGACAGCCTCCTAAGCTGCTCATTTACTGGGCATCTCTCACCATCAGTGGCAGTCCCTGACCGATTCAGTGGCAGCGGGTCTGGGACAGATTTCACTCTCACCATCAGTGGCCTGCAGGCTGAAGATGTGGCAGTTTATTACTGTCAGCAACATTATAGTACTCCATTCACTTTTGGCCCTGGGACCAAAGTGGATATCAAA | | | | | |
| KSSQSVLNSSNNRNYLA | | aa | artificial | CDR-L1 of MCSP-D2 | 219 |
| WASTRES | | aa | artificial | CDR-L2 of MCSP-D2 | 220 |
| QQHYSTPFT | | aa | artificial | CDR-L3 of MCSP-D2 | 221 |
| GYYMH | | aa | artificial | CDR-H1 of MCSP-D2 | 222 |
| WINPNSGGTSYAQKFQG | | aa | artificial | CDR-H2 of MCSP-D2 | 223 |
| SWVSWFAS | | aa | artificial | CDR-H3 of MCSP-D2 | 224 |
| QVQLVQSGAEVKKPGASVKVSCKASGYTFTGYYMHWVRQAPGQGLEWMGWINPNSGGTSYAQKFQGRVTMTRDTSTSTVYMELSNLRSDDTAVYYCAKSWVSWFASWGQGTLVTVSS | | aa | artificial | VH of MCSP-D2 | 225 |
| CAGGTGCAGCTGGTCCAGTCTGGGGCTGAGGTGAAGAAGCCTGGGGCCTCAGTGAAGGTCTCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAAGCTACGCACAGAAGTTCCAGGGCAGAGTCACCATGACCAGGGACACGTCCACGAGCACAGTCTACATGGAGCTGAGCAACCTGAGATCTGACGACACGGCCGTGTATTACTGTGCGAAATCCTGGGTCTCCTGGTTTGCTTCCTGGGGTCAAGGAACCTTGGTCACCGTCTCCTCA | | nt | artificial | VH of MCSP-D2 | 226 |
| DIVMTQSPDSLAVSLGERATINCKSSQSVLNSSNNRNYLAWYQQKPGQPPKLLIYWASTRESGVPDRFSGSGSGTDFTLTISGLQAEDVAVYYCQQHYSTPFTFGPGTKVDIK | | aa | artificial | VL of MCSP-D2 | 227 |
| GATATCGTGATGACCCAGTCTCCAGACTCCCTGGCTGTGTCTCTGGGCGAGAGGGCCACCATCAA | | nt | artificial | VL of MCSP-D2 | 228 |

| | | | | |
|---|---|---|---|---|
| | | | | CTGCAAGTCCAGCCAGAGTGTTTTAAACAGCTCCAACAATAGGAACTACTTAGCTTGGTACCAGC AGAAACCAGGACAGCCTCCTAAGCTGCTCATTTACTGGGCATCTACCCGGGAATCCGGGGTCCCT GACCGATTCAGTGGCAGCGGGTCTGGGACAGATTTCACTCTCACCATCAGTGGCCTGCAGGCTGA AGATGTGGCAGTTTATTACTGTCAGCAACATTATAGTACTCCATTCACTTTTGGCCCTGGGACCA AAGTGGATATCAAA |
| 229 | VH-P of MCSP-D2 | artificial | aa | EVQLLESGAEVKKPGASVKVSCKASGYTFTGYYMHWVRQAPGQGLEWMGWINPNSGGTSYAQKFQ GRVTMTRDTSTSTVYMELSNLRSDDTAVYYCAKSWVSWFASWGQGTLVTVSS |
| 230 | VH-P of MCSP-D2 | artificial | nt | GAGGTGCAGCTGCTCGAGTCTGGGGCTGAGGTGAAGAAGCCTGGGGCCTCAGTGAAGGTCTCCTG CAAGGCTTCTGGATACACCTTCACCGGCTACTATATGCACTGGGTGCGACAGGCCCCTGGACAAG GGCTTGAGTGGATGGGATGGATCAACCCTAACAGTGGTGGCACAAGCTACGCACAGAAGTTCCAG GGCAGAGTCACCATGACTAGGGACACGTCCACGAGCACAGTCTACATGGAGCTGAGCAACCTGAG ATCTGACGACACGGCCGTGTATTACTGTGCGAAATCCTGGGTCTCCTGGTTTGCTTCCTGGGGTC AAGGAACCTTGGTCACCGTCTCCTCA |
| 231 | VL-P of MCSP-D2 | artificial | aa | ELVMTQSPDSLAVSLGERATINCKSSQSVLNSSNNRNYLAWYQQKPGQPPKLLIYWASTRESGVP DRFSGSGSGTDFTLTISGLQAEDVAVYYCQQHYSTPFTFGPGTKVDIK |
| 232 | VL-P of MCSP-D2 | artificial | nt | GAGCTCGTGATGACCCAGTCTCCAGACTCCCTGGCTGTGTCTCTGGGCGAGAGGGCCACCATCAA CTGCAAGTCCAGCCAGAGTGTTTTAAACAGCTCCAACAATAGGAACTACTTAGCTTGGTACCAGC AGAAACCAGGACAGCCTCCTAAGCTGCTCATTTACTGGGCATCTACCCGGGAATCCGGGGTCCCT GACCGATTCAGTGGCAGCGGGTCTGGGACAGATTTCACTCTCACCATCAGTGGCCTGCAGGCTGA AGATGTGGCAGTTTATTACTGTCAGCAACATTATAGTACTCCATTCACTTTTGGCCCTGGGACCA AAGTGGATATCAAA |
| 233 | VH-VL of MCSP-D2 | artificial | aa | QVQLVQSGAEVKKPGASVKVSCKASGYTFTGYYMHWVRQAPGQGLEWMGWINPNSGGTSYAQKFQ GRVTMTRDTSTSTVYMELSNLRSDDTAVYYCAKSWVSWFASWGQGTLVTVSSGGGGSGGGGSGGG GSDIVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIK |
| 234 | VH-VL of MCSP-D2 | artificial | nt | CAGGTGCAGCTGGTCCAGTCTGGGGCTGAGGTGAAGAAGCCTGGGGCCTCAGTGAAGGTCTCCTG CAAGGCTTCTGGATACACCTTCACCGGCTACTATATGCACTGGGTGCGACAGGCCCCTGGACAAG GGCTTGAGTGGATGGGATGGATCAACCCTAACAGTGGTGGCACAAGCTACGCACAGAAGTTCCAG GGCAGAGTCACCATGACTAGGGACACGTCCACGAGCACAGTCTACATGGAGCTGAGCAACCTGAG ATCTGACGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGATATCGTGATGACCCAGTCTCCAGACTCCCTGGCTGTGTCTCTGGGCGAGAGGGCCAC |

| | | | | |
|---|---|---|---|---|
| | | | | CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAA |
| 235 | VH-VL-P of MCSP-D2 | artificial | aa | EVQLLESGAEVKKPGASVKVSCKASGYTFTGYYMHWVRQAPGQGLEWMGWINPNSGGTSYAQKFQ GRVTMTRDTSTSTVYMELSNLRSDDTAVYYCAKSWVSWFASWGQGTLVTVSSGGGGSGGGGSGGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIK |
| 236 | VH-VL-P of MCSP-D2 | artificial | nt | GAGGTGCAGCTGCTCGAGTCTGGGGCTGAGGTGAAGAAGCCTGGGGCCTCAGTGAAGGTCTCCTG CAAGGCTTCTGGATACACCTTCACCGGCTACTATATGCACTGGGTGCGACAGGCCCCTGGACAAG GGCTTGAGTGGATGGGATGGATCAACCCTAACAGTGGTGGCACAAGCTACGCACAGAAGTTCCAG GGCAGAGTCACCATGACTAGGGACACGTCCACGAGCACAGTCTACATGGAGCTGAGCAACCTGAG ATCTGACGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAA |
| 237 | CDR-L1 of MCSP-F9 | artificial | aa | KSSQSVLSSSNNKNYLN |
| 238 | CDR-L2 of MCSP-F9 | artificial | aa | WASTRES |
| 239 | CDR-L3 of MCSP-F9 | artificial | aa | QQHYSVPFT |
| 240 | CDR-H1 of MCSP-F9 | artificial | aa | SSNWWS |
| 241 | CDR-H2 of MCSP-F9 | artificial | aa | TIYYNGNTYYNPSLKS |

EP 2 155 788 B1

| 242 | CDR-H3 of MCSP-F9 | artificial | aa | SWVSWFAS |
|---|---|---|---|---|
| 243 | VH of MCSP-F9 | artificial | aa | QVQLQESGPGLVKPSETLSLTCVVSGGSISSSNWWSWVRQPPGKGLEWLGTIYYNGNTYYNPSLK SRVTISVDTSKNQFSLRLSSVTAADTAVYYCAKSWVSWFASWGQGTLVTVSS |
| 244 | VH of MCSP-F9 | artificial | nt | CAGGTGCAGCTGCAAGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGAGGCTGAGCTCTGTGAC CGCCGCAGACACGGCCGTGTATTACTGTGCGAAATCCTGGGTCTCCTGGTTTGCTTCCTGGGGTC AAGGAACCTTGGTCACCGTCTCCTCA |
| 245 | VL of MCSP-F9 | artificial | aa | DIVMTQSPDSLAVSLGERATINCKSSQSVLSSSNNKNYLNWYQQKPGQPPKLLIYWASTRESGVP DRFSGSGSGTDFTLTISSLQAEDVAVYYCQQHYSVPFTFGPGTKVDIK |
| 246 | VL of MCSP-F9 | artificial | nt | GATATCGTGATGACACAGTCTCCAGACTCCCTGGCTGTGTCTCTGGGCGAGAGGGCCACCATCAA CTGCAAGTCCAGCCAGAGTGTCTTATCCAGCTCCAACAATAAGAACTACTTAAATTGGTACCAGC AGAAACCAGGACAGCCTCCTAAGTTGCTCATTTACTGGGCATCTACCCGGGAATCCGGGGTCCCT GACCGATTCAGTGGCAGCGGGTCTGGGACAGATTTCACTCTCACCATCAGCAGCCTGCAGGCTGA AGATGTGGCAGTTTATTACTGTCAGCAACATTATAGTGTTCCATTCACTTTCGGCCCTGGGACCA AAGTGGATATCAAA |
| 247 | VH-P of MCSP-F9 | artificial | aa | EVQLLESGPGLVKPSETLSLTCVVSGGSISSSNWWSWVRQPPGKGLEWLGTIYYNGNTYYNPSLK SRVTISVDTSKNQFSLRLSSVTAADTAVYYCAKSWVSWFASWGQGTLVTVSS |
| 248 | VH-P of MCSP-F9 | artificial | nt | GAGGTGCAGCTGCTCGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGAGGCTGAGCTCTGTGAC CGCCGCAGACACGGCCGTGTATTACTGTGCGAAATCCTGGGTCTCCTGGTTTGCTTCCTGGGGTC AAGGAACCTTGGTCACCGTCTCCTCA |
| 249 | VL-P of MCSP-F9 | artificial | aa | ELVMTQSPDSLAVSLGERATINCKSSQSVLSSSNNKNYLNWYQQKPGQPPKLLIYWASTRESGVP DRFSGSGSGTDFTLTISSLQAEDVAVYYCQQHYSVPFTFGPGTKVDIK |
| 250 | VL-P of | artificial | nt | GAGCTCGTGATGACACAGTCTCCAGACTCCCTGGCTGTGTCTCTGGGCGAGAGGGCCACCATCAA CTGCAAGTCCAGCCAGAGTGTCTTATCCAGCTCCAACAATAAGAACTACTTAAATTGGTACCAGC |

| SEQ ID NO | Name | Source | Type | Sequence |
|---|---|---|---|---|
| | MCSP-F9 | | | AGAAACCAGGAGCAGCTCCCT GACCGATTCAGTGGCAGCGGGTCTGGGACAGCTTCACTCTCACCATCAGCAGCCTGCAGCCTGA AGATGTGGCAGTTTATTACTGTCAGCAACATTATAGTGTTCCATTCACTTTCGGCCCTGGGACCA AAGTGGATATCAAA |
| 251 | VH-VL of MCSP-F9 | artificial | aa | QVQLQESGPGLVKPSETLSLTCVVSGGSISSSNWWSWVRQPPGKGLEWLGTIYYNGNTYYNPSLK SRVTISVDTSKNQFSLRLSSVTAADTAVYYCAKSWSVSWFASWGQGTLVTVSSGGGGSGGGGSGGG GSDIVMTQSPDSLAVSLGERATINCKSSQSVLSSSNNKNYLNWYQQKPGQPPKLLIYWASTRESG VPDRFSGSGSGTDFTLTISSLQAEDVAVYYCQQHYSVPFTFGPGTKVDIK |
| 252 | VH-VL of MCSP-F9 | artificial | nt | CAGGTGCAGCTGCAAGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGTCGCTGTCTTCCTGGGTC CGCCGCAGACACGGCCGTGTATTACTGTGCGAAATCCTGGTCGGTTTGCTTCTCCGGTGGGGTC AAGGAACCTTGGTCACCGTCTCCTCAGGTGGTTCTGGCGGCGGCGGCTCCGGTGGTGGT GGTTCTGATATCGTGATGACGCCAGAGTCTCCAGACTCCCTGGCCGTGTCTCTGGGCGAGAGGGCCAC CATCAACTGCAAGTCCAGCCAGAGTGTCTTATCCAGCTCCAACAATAAGAACTACTTAAATTGGT ACCAGCAGAAACCAGGACAGCCTCCTAAGCTGCTCATTTACTGGGCATCTCACCTACCGGGA GTCCCTGACCGATTCAGTGGCAGCGGGTCTGGGACAGATTTCACTCTCACCATCAGCAGCCTGCA GGCTGAAGATGTGGCAGTTTATTACTGTCAGCAACATTATAGTGTTCCATTCACTTTCGGCCCCTG GGACCAAAAGTGGATATCAAA |
| 253 | VH-VL-P of MCSP-F9 | artificial | aa | EVQLLESGPGLVKPSETLSLTCVVSGGSISSSNWWSWVRQPPGKGLEWLGTIYYNGNTYYNPSLK SRVTISVDTSKNQFSLRLSSVTAADTAVYYCAKSWSVSWFASWGQGTLVTVSSGGGGSGGGGSGGG GSELVMTQSPDSLAVSLGERATINCKSSQSVLSSSNNKNYLNWYQQKPGQPPKLLIYWASTRESG VPDRFSGSGSGTDFTLTISSLQAEDVAVYYCQQHYSVPFTFGPGTKVDIK |
| 254 | VH-VL-P of MCSP-F9 | artificial | nt | GAGGTGCAGCTGCTCGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGTCTCCTGGTTGCTTCTCCGGGGTC CGCCGCAGACACGGCCGTGTATTACTGTGCGAAATCCTGGTCGGTTTGCTTCTCCGGTGGTGGT AAGGAACCTTGGTCACCGTCTCCTCAGGTGGTGGTTCTGGCGGCGGCGGCTCCGGTGGTGGT CATCAACTGCAAGTCCAGCCAGAGTGTCTTATCCAGCTCCAACAATAAGAACTACTTAAATTGGT ACCAGCAGAAACCAGGACAGCCTCCTAAGCTGCTCATTTACTGGGCATCTCACTCTCACCATCAGCAGATTGGT GTCCCTGACCGATTCAGTGGCAGCGGGTCTGGGACAGATTTCACTCTCACCATCAGCAGCCTGCA |

85

| | | | | GGCTGAAGATGTGGCAGTTTATTACTGTCAGCAACATTATAGTGTTCCATTCACTTTCGGCCCTG GGACCAAAGTGGATATCAAA |
|---|---|---|---|---|
| 255 | CDR-L1 of IgE-D4 | artificial | aa | RASQSVSSNLA |
| 256 | CDR-L2 of IgE-D4 | artificial | aa | DASNRAT |
| 257 | CDR-L3 of IgE-D4 | artificial | aa | QQFGDTLWT |
| 258 | CDR-H1 of IgE-D4 | artificial | aa | SYAMS |
| 259 | CDR-H2 of IgE-D4 | artificial | aa | SISSGNIIYYPDNVKG |
| 260 | CDR-H3 of IgE-D4 | artificial | aa | GRSTYGGFDH |
| 261 | VH of IgE-D4 | artificial | aa | EVQLLESGGGLVQPGGSLRLSCAASGFTFSSYAMSWVRQAPGKGLEWVASISSGNIIYYPDNVKG RFTISRDNSKNTLYLQMNSLRAEDTAVYYCTRGRSTYGGFDHWGQGTTVTVSS |
| 262 | VH of IgE-D4 | artificial | nt | GAGGTGCAGCTGCTCGAGTCTGGGGGAGGCCTTGTGCAGCCTGGAGGGTCTCTGAGGCTCTCCTG TGCAGCCTCTGGATTCACTTTCAGTAGTTATGCCATGTCTTGGGTTCGCCAGGCTCCAGGGAAGG GGCTGGAGTGGGTCGCTTCCATTAGTAGTGGTAATATCATCTACTATCCAGACAATGTGAAGGGC CGATTCACCATCTCTAGAGATAATTCCAAGAACACCCTGTACCTGCAAATGAACAGTCTGAGGGC TGAGGACACGGCCGTGTATTATTGTACTAGAGGCCGCAGTACCTACGGGGGATTTGACCACTGGG GCCAAGGCACCACAGTCACCGTCTCCTCA |
| 263 | VL of IgE-D4 | artificial | aa | EIVLTQSPATLSVSPGERATLSCRASQSVSSNLAWYQQKPGQAPRLLIYDASNRATGIPARFSGS GSGTDFTLTISSLEPEDFAVYYCQQFGDTLWTFGQGTKVEIK |
| 264 | VL of IgE-D4 | artificial | nt | GAGATCGTGTTGACACAGTCTCCAGCCACCCTGTCTGTGTCTCCAGGGGAAAGAGCCACCCTCTC CTGCAGGGCCAGTCAGAGTGTTAGCAGCAACTTAGCCTGGTACCAGCAGAAACCTGGCCAGGCTC CCAGGCTCCTCATCTATGATGCATCCAACAGGGCCACTGGCATCCCAGCCAGGTTCAGTGGCAGT GGGTCTGGGACAGACTTCACTCTCACCATCAGCAGCCTAGAGCCTGAAGATTTTGCAGTTTATTA CTGTCAGCAGTTCGGTGATACACTGTGGACGTTCGGCCAAGGGACCAAGGTGGAGATCAAA |

EP 2 155 788 B1

| | | | | |
|---|---|---|---|---|
| 265 | VH-P of IgE-D4 | artificial | aa | EVQLLESGGGLVQPGGSLRLSCAASGFTFSSYAMSWVRQAPGKGLEWVASISSGNIIYYPDNVKGRFTISRDNSKNTLYLQMNSLRAEDTAVYYCTRGRSTYGGFDHWGQGTTVTVSS |
| 266 | VH-P of IgE-D4 | artificial | nt | GAGGTGCAGCTGCTCGAGTCTGGGGGAGGCCTTGTGCAGCCTGGAGGGTCTCTGAGGCTCTCCTGTGCAGCCTCTGGATTCACTTTCAGTAGTTATGCCATGTCTTGGGTTCGCCAGGCTCCAGGGAAGGGGCTGGAGTGGGTCGCTTCCATTAGTAGTAGTGGTAATATCATCTACTATCCAGACAATGTGAAGGGCCGATTCACCATCTCTAGAGATAATTCCAAGAACACCCTGTACCTGCAAATGAACAGTCTGAGGGCTGAGGACACGGCCGTGTATTATTGTACTAGAGGGCGCAGTACCTACGGGGGATTTGACCACTGGGGCCAAGGCACCACAGTCACCGTCTCCTCA |
| 267 | VL-P of IgE-D4 | artificial | aa | ELVLTQSPATLSVSPGERATLSCRASQSVSSNLAWYQQKPGQAPRLLIYDASNRATGIPARFSGSGSGTDFTLTISSLEPEDFAVYYCQQFGDTLWTFGQGTKVEIK |
| 268 | VL-P of IgE-D4 | artificial | nt | GAGCTCGTTGTTGACACAGTCTCCAGCCACCGTCTGTGTCTCCAGGGGAAAGAGCCACCCTCTCCTGCAGGGCCAGTCAGAGTGTTAGCAGCAACTTAGCCTGGTACCAGCAGAAACCTGGCCAGGCTCCCAGGCTCCTCATCTATGATGCATCCAACAGGGCCACTGGCATCCCAGCCAGGTTCAGTGGCAGTGGGTCTGGGACAGACAGACTTCACTCTCACCATCAGCAGCCTAGAGCCTGAAGATTTTGCAGTTTATTACTGTCAGCAGTTCGGTGATACACTGTGGACGTTCGGCCAAGGGACCAAGGTGGAGATCAAA |
| 269 | VH-VL of IgE-D4 | artificial | aa | EVQLLESGGGLVQPGGSLRLSCAASGFTFSSYAMSWVRQAPGKGLEWVASISSGNIIYYPDNVKGRFTISRDNSKNTLYLQMNSLRAEDTAVYYCTRGRSTYGGFDHWGQGTTVTVSSGGSEIVLTQSPATLSVSPGERATLSCRASQSVSSNLAWYQQKPGQAPRLLIYDASNRATGIPARFSGSGSGTDFTLTISSLEPEDFAVYYCQQFGDTLWTFGQGTKVEIK |
| 270 | VH-VL of IgE-D4 | artificial | nt | GAGGTGCAGCTGCTCGAGTCTGGGGGAGGCCTTGTGCAGCCTGGAGGGTCTCTGAGGCTCTCCTGTGCAGCCTCTGGATTCACTTTCAGTAGTTATGCCATGTCTTGGGTTCGCCAGGCTCCAGGGAAGGGGCTGGAGTGGGTCGCTTCCATTAGTAGTAGTGGTAATATCATCTACTATCCAGACAATGTGAAGGGCCGATTCACCATCTCTAGAGATAATTCCAAGAACACCCTGTACCTGCAAATGAACAGTCTGAGGGCTGAGGACACGGCCACCACAGTCACCGTCTCCTCAGGTGGTTCTGAGATCGTGTTGACACAGTCTCCAGCCACCCTGTCTGTGTCTCCAGGGGAAAGAGCCACCCTCTCCTGCAGGGCCAGTCAGAGTGTTAGCAGCAACTTAGCCTGGTACCAGCAGAAACCTGGCCAGGCTCCCAGGCTCCTCATCTATGATGCATCCAACAGGGCCACTGGCATCCCAGCCAGGTTCAGTGGCAGTGGGTCTGGGACAGTTCGGTGATACACTGTGGACGTTCGGCCAAGGGACCAAGGTGGAGATCAAA |

| 271 | VH-VL-P of IgE-D4 | artificial | aa | EVQLLESGGGLVQPGGSLRLSCAASGFTFSSYAMSWVRQAPGKGLEWVASISSGNIIYYPDNVKG RFTISRDNSKNTLYLQMNSLRAEDTAVYYCTRGRSTYGGFDHWGQGTTVTVSSGGGGSGGGGSGG GGSELVLTQSPATLSVSPGERATLSCRASQSVSSNLAWYQQKPGQAPRLLIYDASNRATGIPARF SGSGSGTDFTLTISSLEPEDFAVYYCQQFGDTLWTFGQGTKVEIK |
| --- | --- | --- | --- | --- |
| 272 | VH-VL-P of IgE-D4 | artificial | nt | GAGGTGCAGCTGCTCGAGTCTGGGGGAGGCCTTGTGCAGCCTGGAGGGTCTCTGAGGCTCTCCTG TGCAGCCTCTGGATTCACTTTCAGTAGTTATGCCATGTCTTGGGTTCGCCAGGCTCCAGGGAAGG GGCTGGAGTGGGTCGCTTCCATTAGTAGTGGTAATATCATCTACTATCCAGACAATGTGAAGGGC CGATTCACCATCTCTAGAGATAATTCCAAGAACACCCTGTACCTGCAAATGAACAGTCTGAGGGC TGAGGACACGGCCGTGTATTATTGTACTAGAGGCCGCAGTACCTACGGGGGATTTGACCACTGGG GCCAAGGCACCACAGTCACCGTCTCCTCAGGTGGTGGTGGTTCTGGCGGCGGCGGCTCCGGTGGT GGTGGTTCTGAGCTCGTGTTGACACAGTCTCCAGCCACCCTGTCTGTGTCTCCAGGGGAAAGAGC CACCCTCTCCTGCAGGGCCAGTCAGAGTGTTAGCAGCAACTTAGCCTGGTACCAGCAGAAACCTG GCCAGGCTCCCAGGCTCCTCATCTATGATGCATCCAACAGGGCCACTGGCATCCCAGCCAGGTTC AGTGGCAGTGGGTCTGGGACAGACTTCACTCTCACCATCAGCAGCCTAGAGCCTGAAGATTTTGC AGTTTATTACTGTCAGCAGTTCGGTGATACACTGTGGACGTTCGGCCAAGGGACCAAGGTGGAGA TCAAA |
| 273 | CDR-L1 of IgE-G9 | artificial | aa | WASQGVSNNLA |
| 274 | CDR-L2 of IgE-G9 | artificial | aa | DAFNRAT |
| 275 | CDR-L3 of IgE-G9 | artificial | aa | QQFGDSLWT |
| 276 | CDR-H1 of IgE-G9 | artificial | aa | SYAMS |
| 277 | CDR-H2 of IgE-G9 | artificial | aa | SISSGNIIYYPDNVKG |
| 278 | CDR-H3 of IgE-G9 | artificial | aa | GRSTYGGFDH |
| 279 | VH of IgE-G9 | artificial | aa | EVQLLESGGGLVQPGGSLRLSCAASGFTFSSYAMSWVRQAPGKGLEWVASISSGNIIYYPDNVKG RFTISRDNSKNTLYLQMNSLRAEDTAVYYCTRGRSTYGGFDHWGQGTTVTVSS |

| | | | | |
|---|---|---|---|---|
| 280 | VH of IgE-G9 | artificial | nt | GAGGTGCAGCTGCTCGAGTCTGGGGGAGGCCTTGTGCAGCCTGGAGGGTCTCTGAGGCTCTCCTG TGCAGCCTCTGGATTCACTTTCAGTAGTTATGCCATGTCTTGGGTTCGCCAGGCTCCAGGGAAGG GGCTGGAGTGGGTCGCTTCCATTAGTAGTGGTAATATCATCTACTATCCAGACAATGTGAAGGGC CGATTCACCATCTCTAGAGATAATTCCAAGAACACCCTGTACCTGCAAATGAACAGTCTGAGGGC TGAGGACACGGCCGTGTATTATTGTACTAGAGGCCGCAGTACCTACGGGGGATTTGACCACTGGG GCCAAGGCACCACAGTCACCGTCTCCTCA |
| 281 | VL of IgE-G9 | artificial | aa | EIVMTQSPATLSVSPGERVTLSCWASQGVSNNLAWYQQRPGQAPRLLIYDAFNRATGIPARFSGS GSGTDFTLTISRLEPEDFAVYYCQQFGDSLWTFGQGTKLEIK |
| 282 | VL of IgE-G9 | artificial | nt | GAGATCGTGATGACACAGTCTCCAGCCACCCTGTCTGTGTCTCCAGGGGAAAGAGTCACCCTCTC CTGCTGGGCCAGTCAGGGTGTGAGCAACAACTTAGCCTGGTACCAGCAGAGACCTGGCCAGGCTC CCAGGCTCCTCATCTATGATGCATTCAACAGGGCCACTGGCATCCCAGCCAGGTTCAGTGGCAGT GGGTCTGGGACAGACTTCACTCTCACCATCAGCAGACTGGAGCCTGAAGATTTTGCGGTGTATTA CTGTCAGCAGTTTGGTGATTCACTTTGGACGTTCGGCCAGGGGACCAAGCTGGAAATCAAA |
| 283 | VH-P of IgE-G9 | artificial | | EVQLLESGGGLVQPGGSLRLSCAASGFTFSSYAMSWVRQAPGKGLEWVASISSGNIIYYPDNVKG RFTISRDNSKNTLYLQMNSLRAEDTAVYYCTRGRSTYGGFDHWGQGTTVTVSS |
| 284 | VH-P of IgE-G9 | artificial | | GAGGTGCAGCTGCTCGAGTCTGGGGGAGGCCTTGTGCAGCCTGGAGGGTCTCTGAGGCTCTCCTG TGCAGCCTCTGGATTCACTTTCAGTAGTTATGCCATGTCTTGGGTTCGCCAGGCTCCAGGGAAGG GGCTGGAGTGGGTCGCTTCCATTAGTAGTGGTAATATCATCTACTATCCAGACAATGTGAAGGGC CGATTCACCATCTCTAGAGATAATTCCAAGAACACCCTGTACCTGCAAATGAACAGTCTGAGGGC TGAGGACACGGCCGTGTATTATTGTACTAGAGGCCGCAGTACCTACGGGGGATTTGACCACTGGG GCCAAGGCACCACAGTCACCGTCTCCTCA |
| 285 | VL-P of IgE-G9 | artificial | | ELVMTQSPATLSVSPGERVTLSCWASQGVSNNLAWYQQRPGQAPRLLIYDAFNRATGIPARFSGS GSGTDFTLTISRLEPEDFAVYYCQQFGDSLWTFGQGTKLEIK |
| 286 | VL-P of IgE-G9 | artificial | | GAGCTCGTGATGACACAGTCTCCAGCCACCCTGTCTGTGTCTCCAGGGGAAAGAGTCACCCTCTC CTGCTGGGCCAGTCAGGGTGTGAGCAACAACTTAGCCTGGTACCAGCAGAGACCTGGCCAGGCTC CCAGGCTCCTCATCTATGATGCATTCAACAGGGCCACTGGCATCCCAGCCAGGTTCAGTGGCAGT GGGTCTGGGACAGACTTCACTCTCACCATCAGCAGACTGGAGCCTGAAGATTTTGCGGTGTATTA CTGTCAGCAGTTTGGTGATTCACTTTGGACGTTCGGCCAGGGGACCAAGCTGGAAATCAAA |
| 287 | VH-VL of IgE-G9 | artificial | aa | EVQLLESGGGLVQPGGSLRLSCAASGFTFSSYAMSWVRQAPGKGLEWVASISSGNIIYYPDNVKG RFTISRDNSKNTLYLQMNSLRAEDTAVYYCTRGRSTYGGFDHWGQGTTVTVSSGGGGSGGGGSGG GGSEIVMTQSPATLSVSPGERVTLSCWASQGVSNNLAWYQQRPGQAPRLLIYDAFNRATGIPARF |

EP 2 155 788 B1

89

| | | | | |
|---|---|---|---|---|
| | | | | SGSGSGTDFTLTISRLEPEDFAVYYCQQFGDSLWTFGQGTKLEIK |
| 288 | VH-VL of IgE-G9 | artificial | nt | GAGGTGCAGCTGCTCGAGTCTGGGGGAGGCCTTGTGCAGCCTGGAGGGTCTCTGAGGCTCTCCTG TGCAGCCTCTGGATTCACTTTCAGTAGTTATGCCATGTCTTGGGTTCGCCAGGCTCCAGGGAAGG GGCTGGAGTGGGTCGCTTCCATTAGTAGTGGTAATATCATCTACTATCCAGACAATGTGAAGGGC CGATTCACCATCTCTAGAGATAATTCCAAGAACACCCTGTACCTGCAAATGAACAGTCTGAGGGC TGAGGACACGGCCGTGTATTATTGTACTAGAGGCCGCAGTACCTACGGGGGATTTGACCACTGGG GCCAAGGCACCACAGTCACCGTCTCCTCAGGTGGTGGTGGTTCTGGCGGCGGCGGCTCCGGTGGT GGTGGTTCTGAGATCGTGATGACACAGTCTCCAGCCACCCTGTCTGTGTCTCCAGGGGAAAGAGT CACCCTCTCCTGCTGGGCCAGTCAGGGTGTGAGCAACAACTTAGCCTGGTACCAGCAGAGACCTG GCCAGGCTCCCAGGCTCCTCATCTATGATGCATTCAACAGGGCCACTGGCATCCCAGCCAGGTTC AGTGGCAGTGGGTCTGGGACAGACTTCACTCTCACCATCAGCAGACTGGAGCCTGAAGATTTTGC GGTGTATTACTGTCAGCAGTTTGGTGATTCACTTTGGACGTTCGGCCAGGGGACCAAGCTGGAAA TCAAA |
| 289 | VH-VL-P of IgE-G9 | artificial | aa | EVQLLESGGGLVQPGGSLRLSCAASGFTFSSYAMSWVRQAPGKGLEWVASISSGNIIYYPDNVKG RFTISRDNSKNTLYLQMNSLRAEDTAVYYCTRGRSTYGGFDHWGQGTTVTVSSGGGGSGGGGSGG GGSELVMTQSPATLSVSPGERVTLSCWASQGVSNNLAWYQQRPGQAPRLLIYDAFNRATGIPARF SGSGSGTDFTLTISRLEPEDFAVYYCQQFGDSLWTFGQGTKLEIK |
| 290 | VH-VL-P of IgE-G9 | artificial | nt | GAGGTGCAGCTGCTCGAGTCTGGGGGAGGCCTTGTGCAGCCTGGAGGGTCTCTGAGGCTCTCCTG TGCAGCCTCTGGATTCACTTTCAGTAGTTATGCCATGTCTTGGGTTCGCCAGGCTCCAGGGAAGG GGCTGGAGTGGGTCGCTTCCATTAGTAGTGGTAATATCATCTACTATCCAGACAATGTGAAGGGC CGATTCACCATCTCTAGAGATAATTCCAAGAACACCCTGTACCTGCAAATGAACAGTCTGAGGGC TGAGGACACGGCCGTGTATTATTGTACTAGAGGCCGCAGTACCTACGGGGGATTTGACCACTGGG GCCAAGGCACCACAGTCACCGTCTCCTCAGGTGGTGGTGGTTCTGGCGGCGGCGGCTCCGGTGGT GGTGGTTCTGAGCTCGTGATGACACAGTCTCCAGCCACCCTGTCTGTGTCTCCAGGGGAAAGAGT CACCCTCTCCTGCTGGGCCAGTCAGGGTGTGAGCAACAACTTAGCCTGGTACCAGCAGAGACCTG GCCAGGCTCCCAGGCTCCTCATCTATGATGCATTCAACAGGGCCACTGGCATCCCAGCCAGGTTC AGTGGCAGTGGGTCTGGGACAGACTTCACTCTCACCATCAGCAGACTGGAGCCTGAAGATTTTGC GGTGTATTACTGTCAGCAGTTTGGTGATTCACTTTGGACGTTCGGCCAGGGGACCAAGCTGGAAA TCAAA |
| 291 | EGF-R 21-63 VL-VH x F6A VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLVQPGGSLKLSCAASGFTFNIYAMNWVRQAPGKGLEWVARIRSKYNNYATYYADSVKSRFTISR |

EP 2 155 788 B1

EP 2 155 788 B1

| | | | | |
|---|---|---|---|---|
| | | | | DDSKNTAYLQMNNLKTEDTAVYYCVRHGNFGNSYVSFFAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 292 | EGF-R 21-63 VL-VH x F6A VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATAT CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAAGCAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACGTATCCTTCTTCGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 293 | EGF-R 21-63 VL-VH x H2C VH-VL | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLVESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SQTVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |

EP 2 155 788 B1

| 294 | EGF-R 21-63 VL-VH x H2C VH-VL | artificial | nt | GACATTGTGCTGACACAGTCTCCTGCTTCCTTACCTGTGTCTCTGGGGCAGAGGGCCACCATCTC<br>ATGCAGGGCCAGCCAAAGTGTCAGTTCATCTACTTATAGTTATATACACTGGTACCAACAGAAAC<br>CAGGACAGCCACCCAAACTCCTCATCACGTATGCATCCAACCTAGAATCTGGGGTCCCTGCCAGG<br>TTCAGTGGCAGTGGGTCTGGGACAGACTTCACCCTCGACATCCATCCTGTGGAGGAGGATGATTC<br>TTCAACATATTACTGTCAGCACAGTTGGGAGATTCCATTTACGTTCGGCTCGGGGACAAAGTTGG<br>AAATAAAAGGTGGTGGTGGTTCTGGCGGCGGCGGCTCCGGTGGTGGTGGTTCTCAGGTTCAGCTG<br>CAGCAGTCTGGACCTGATCTGGTGAAGCCTGGGGCCTCAGTGAAGATGTCCTGCAAGGCTTCTGG<br>ACACACTTTCACTGACTGTGTTATAATCTGGGTGAAACAGAGAGCTGGACAGGGCCTTGAGTGGA<br>TTGGACAGATTTATCCAGGGACTGGTCGTTCTTACTACAATGAGATTTTCAAGGGCAAGGCCACA<br>CTGACTGCAGACAAATCCTCCAACACAGTCCACATTCAACTCAGCAGCCTGACATCTGAGGACTC<br>TGCGGTCTATTTCTGTGCCCTATCTACTCTTATTCACGGGACCTGGTTTTCTTATTGGGGCCAAG<br>GGACTCTGGTCACTGTCTCTTCCGGAGGTGGTGGCTCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 295 | EGF-R 21-63 VL-VH x H2C VH-VL-P | artificial | aa | DIVLTQSPASLPVSLGQRATISCRASQSVSSSTYSYIHWYQQKPGQPPKLLITYASNLESGVPAR<br>FSGSGSGTDFTLDIHPVEEDDSSTYYCQHSWEIPFTFGSGTKLEIKGGGGSGGGGSGGGGSQVQL<br>QQSGPDLVKPGASVKMSCKASGHTFTDCVIIWVKQRAGQGLEWIGQIYPGTGRSYYNEIFKGKAT<br>LTADKSSNTVHIQLSSLTSEDSAVYFCALSTLIHGTWFSYWGQGTLVTVSSGGGGSEVQLLESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 296 | EGF-R 21-63 VL-VH x H2C VH-VL-P | artificial | nt | GACATTGTGCTGACACAGTCTCCTGCTTCCTTACCTGTGTCTCTGGGGCAGAGGGCCACCATCTC<br>ATGCAGGGCCAGCCAAAGTGTCAGTTCATCTACTTATAGTTATATACACTGGTACCAACAGAAAC<br>CAGGACAGCCACCCAAACTCCTCATCACGTATGCATCCAACCTAGAATCTGGGGTCCCTGCCAGG |

EP 2 155 788 B1

EP 2 155 788 B1

| | | | | |
|---|---|---|---|---|
| | | | | TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGACAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 297 | EGF-R 21-63 VL-VH x H1E VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLEQPGGSLKLSCAASGFTFNSYAMNWVRQAPGKGLEWVARIRSKYNNYATYYADSVKGRFTISR DDSKNTAYLQMNNLKTEDTAVYYCVRHGNFGNSYLSFWAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 298 | EGF-R 21-63 VL-VH x H1E VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG |

| | | | | |
|---|---|---|---|---|
| | | | | ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGAGCAGCCTGGAGGGTCATTGAAACTCTCATGTGCAGCCTCTGGATTCACCTTCAATTC GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACCTATCCTTCTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 299 | EGF-R 21-63 VL-VH x G4H VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLVQPGGSLKLSCAASGFTFNRYAMNWVRQAPGKGLEWVARIRSKYNNYATYYADSVKGRFTISR DDSKNTAYLQMNNLKTEDTAVYYCVRHGNFGNSYLSYFAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 300 | EGF-R 21-63 VL-VH x G4H VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG |

| | | | | |
|---|---|---|---|---|
| | | . | | GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATCG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACTTATCCTACTTCGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 301 | EGF-R 21-63 VL-VH x A2J VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLVQPGGSLKLSCAASGFTFNVYAMNWVRQAPGKGLEWVARIRSKYNNYATYYADSVKKRFTISR DDSKNTAYLQMNNLKTEDTAVYYCVRHGNFGNSYLSWWAYWGQGTLVTVSSGGGGSGGGGSGGGG SELVVTQEPSLTVSPGGTVTLTCRSSTGAVTSGYYPNWVQQKPGQAPRGLIGATDMRPSGTPARF SGSLLGGKAALTLSGVQPEDEAEYYCALWYSNRWVFGGGTKLTVL |
| 302 | EGF-R 21-63 VL-VH x A2J VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATGT CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAAAGAGGTTCACCATCTCCAGA |

| | | | | |
|---|---|---|---|---|
| | | | | GATGATTCAAAAAACACTGCCTATCTACAAATGAACAACTTGAAAACTGAGGACACTGCCGTGTA CTACTGTGTGAGACATGGGAACTTCGGTAATAGCTACTTATCCTGGTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTCGCTCCTCGACTGGGGCTGTTACATCTGGCTACTACCCAAACTGGGTCCAACAAAAACCAG GTCAGGCACCCCGTGGTCTAATAGGTGCCACTGACATGAGGCCCTCTGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 303 | EGF-R 21-63 VL-VH x E1L VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLVQPGGSLKLSCAASGFTFNKYAMNWVRQAPGKGLEWVARIRSKYNNYATYYADSVKSRFTISR DDSKNTAYLQMNNLKTEDTAVYYCVRHGNFGNSYTSYYAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 304 | EGF-R 21-63 VL-VH x E1L VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAATCGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACACATCCTACTACGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC |

| | | | | |
|---|---|---|---|---|
| | | | | TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 305 | EGF-R 21-63 VL-VH x E2M VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLVQPGGSLKLSCAASGFTFNGYAMNWVRQAPGKGLEWVARIRSKYNNYATYYADSVKERFTISR DDSKNTAYLQMNNLKTEDTAVYYCVRHRNFGNSYLSWFAYWGQGTLVTVSSGGGGSGGGGSGGGG SELVVTQEPSLTVSPGGTVTLTCRSSTGAVTSGYYPNWVQQKPGQAPRGLIGATDMRPSGTPARF SGSLLGGKAALTLSGVQPEDEAEYYCALWYSNRWVFGGGTKLTVL |
| 306 | EGF-R 21-63 VL-VH x E2M VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATGG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGAGAGGTTCACCATCTCCAGA GATGATTCAAAAAACACTGCCTATCTACAAATGAACAACTTGAAAACTGAGGACACTGCCGTGTA CTACTGTGTGAGACATAGGAACTTCGGTAATAGCTACTTATCCTGGTTCGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTCGCTCCTCGACTGGGGCTGTTACATCTGGCTACTACCCAAACTGGGTCCAACAAAAACCAG GTCAGGCACCCCGTGGTCTAATAGGTGCCACTGACATGAGGCCCTCTGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG |

| | | | | |
|---|---|---|---|---|
| | | | | TCCTA |
| 307 | EGF-R 21-63 VL-VH x F7O VH-VL-P | artificial | aa | DIVLTQSPASLPVSLGQRATISCRASQSVSSSTYSYIHWYQQKPGQPPKLLITYASNLESGVPAR FSGSGSGTDFTLDIHPVEEDDSSTYYCQHSWEIPFTFGSGTKLEIKGGGGSGGGGSGGGGSQVQL QQSGPDLVKPGASVKMSCKASGHTFTDCVIIWVKQRAGQGLEWIGQIYPGTGRSYYNEIFKGKAT LTADKSSNTVHIQLSSLTSEDSAVYFCALSTLIHGTWFSYWGQGTLVTVSSGGGGSEVQLLESGG GLVQPGGSLKLSCAASGFTFNVYAMNWVRQAPGKGLEWVARIRSKYNNYATYYADSVKKRFTISR DDSKNTAYLQMNNLKTEDTAVYYCVRHGNFGNSYISWWAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 308 | EGF-R 21-63 VL-VH x F7O VH-VL-P | artificial | nt | GACATTGTGCTGACACAGTCTCCTGCTTCCTTACCTGTGTCTCTGGGGCAGAGGGCCACCATCTC ATGCAGGGCCAGCCAAAGTGTCAGTTCATCTACTTATAGTTATATACACTGGTACCAACAGAAAC CAGGACAGCCACCCAAACTCCTCATCACGTATGCATCCAACCTAGAATCTGGGGTCCCTGCCAGG TTCAGTGGCAGTGGGTCTGGGACAGACTTCACCCTCGACATCCATCCTGTGGAGGAGGATGATTC TTCAACATATTACTGTCAGCACAGTTGGGAGATTCCATTTACGTTCGGCTCGGGGACAAAGTTGG AAATAAAAGGTGGTGGTGGTTCTGGCGGCGGCGGCTCCGGTGGTGGTGGTTCTCAGGTTCAGCTG CAGCAGTCTGGACCTGATCTGGTGAAGCCTGGGGCCTCAGTGAAGATGTCCTGCAAGGCTTCTGG ACACACTTTCACTGACTGTGTTATAATCTGGGTGAAACAGAGAGCTGGACAGGGCCTTGAGTGGA TTGGACAGATTTATCCAGGGACTGGTCGTTCTTACTACAATGAGATTTTCAAGGGCAAGGCCACA CTGACTGCAGACAAATCCTCCAACACAGTCCACATTCAACTCAGCAGCCTGACATCTGAGGACTC TGCGGTCTATTTCTGTGCCCTATCTACTCTTATTCACGGGACCTGGTTTTCTTATTGGGGCCAAG GGACTCTGGTCACTGTCTCTTCCGGAGGTGGTGGCTCCGAGGTGCAGCTGCTCGAGTCTGGAGGA GGATTGGTGCAGCCTGGAGGGTCATTGAAACTCTCATGTGCAGCCTCTGGATTCACCTTCAATGT GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAAAGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTGGTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 309 | MCSP-G4 VH-VL x H2C VH-VL | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG |

| | | | | GSDIVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLVESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SQTVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
|---|---|---|---|---|
| 310 | MCSP-G4 VH-VL x H2C VH-VL | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGATATCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGACAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 311 | MCSP-G4 VH-VL x F12Q VH-VL | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGGG GSDIVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLVESGG GLVQPGGSLKLSCAASGFTFNSYAMNWVRQAPGKGLEWVARIRSKYNNYATYYADSVKGRFTISR DDSKNTAYLQMNNLKTEDTAVYYCVRHGNFGNSYVSWWAYWGQGTLVTVSSGGGGSGGGGSGGGG |

EP 2 155 788 B1

| | | | | |
|---|---|---|---|---|
| | | | | SQTVVTQEPSLTVSPGGTVTLTCGSSTGAVTSGNYPNWVQQKPGGQAPRGLIGGTKFLAPGTPARF SGSLLGGKAALTLSGVQPEDEAEYYCVLWYSNRWVFGGGTKLTVL |
| 312 | MCSP-G4 VH-VL x F12Q VH-VL | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGATATCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGCAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACGTTTCCTGGTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGTTCTATGGTACAGCAACCGCTGGGTGTTCGGTGGAGGAACCAAACTGACTG TCCTA |
| 313 | MCSP-G4 VH-VL x I2C VH-VL | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGGG GSDIVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLVESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SQTVVTQEPSLTVSPGGTVTLTCGSSTGAVTSGNYPNWVQQKPGGQAPRGLIGGTKFLAPGTPARF SGSLLGGKAALTLSGVQPEDEAEYYCVLWYSNRWVFGGGTKLTVL |
| 314 | MCSP-G4 VH-VL | artificial | nt | CAGGTGCAGCTGGTCCAGTCTGGGGCTGAGGTGAAGAGGCCTGGGGCCTCAATGAAGGTCTCCTG |

| SEQ ID NO | Name | Organism | Type | Sequence |
|---|---|---|---|---|
|  | x I2C VH-VL |  |  | CAAGGCTTCTGGGTACACCTTCACCAACTACTATATACACTGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGACACGGCCGTGTATTACTGTGCGAAATCCTGGGTCTTGTTGCTCCGGTGGTGGTGGTAAGGAACCTTGGTCACCGTCTCCTCAGGTGGTGGTGGTTCTGGCGGCGGCGGCTCCGGAGGGGCCACGGTTCTGATATCGTGATGACCCAGTCTCCAGCTCCCCTGTCTGTGTCTCCGGGAGAGAGAGTCAGTCATCAACTGCAAGTCCAGCCAGAGTGTTTTAAACAGCTCCAACAATAAGAACTACTTAGCTTGGTACCAGCAGAAACCAGGACAGCCTCCTAAGCTGCTCATTTACTGGGCATCTCCACCATCAGTGGGGGTCCCTGACCGATTCAGTGGCAGCGGGTCTGGCAGTGGCATCTCTGGGACAGATTTCACTCTCACGGCTGAAGATGTGGCAGTTTATTACTGCCAGCAGTATTATAGTACTCCATTCACTTTTGGCCCTGGGACCAAAGTGGATATCAAATCCGGAGGTGGTGGATCTGGAGGTGGTGGTTCTGGAGGTGGTGGAGGTACGGCCATGACTGGGTCTCCGCCAGCTCCAGGGAAAGGTTTGGAATGGGTTGCTCGCATAAGAGTAAATATAATAATTATGCAACATATTATGCCGATTCAGTGAAAGACAGGTTCACCATCTCCAGAGATGATGAAAAAACACTGCCTATCTACAAATGAACAACTGAAAAATGAACAACTGCTACTGTGTGAGACATGGGAACTTCGGTAATAGTGGGACTAAGTTCCTCCGCCCCGGGGGTACAGGGACTCTGGTCACCGTCTCCTCAGGAGGATCAGGAGAACTGGTCATGACTCAGTCTCCAGATTCTCTGGCTGTGTCTGACTGGGCTGTGTTACATAGTGGGACTAAGTTCCTCCGCCCCGGGGGTACAGGGACTCTGGTCACCGTCTCCTCAGGAGGATCAGGAGAACTGGTCATGACTCAGTCTCCAGATTCTCTGGCTGTGTCTCTGGGCGAGAGGGCCACCATCAACTGCAAGTCCAGCCAGAGTGTTCTATATAGTAGTAACAATAAGAACTACTTAGCTTGGTACCAGCAGAAACCAGGACAGCCTCCTAAGCTGCTCATTTACTGGGCATCTCCACCATCAGTGGGGGTCCCTGCTCGGTTCTGACTGGGTCTGGGACTGATTTCACTCTCACCATCTGTGTGTGGTTGACTGGGTCTGTTACATAGTGGGACTAAGTTCGGACTAAGTTCCTCCGCCCCGGGGGTACAGGGACTCTGGTCACCGTCTCCTCAGGAGGATCAGGAGAACTGGTCATGACTCAGTCTCCAGATTCTCCTA |
| 315 | MCSP-G4 VH-VL-P x F6A VH-VL-P | artificial | aa | QVQLVQSGAEVKRPGASMKVSCKASGYTFTNYYIHWVRQAPGQGLEWMGWINPNSGATNYAQKFQGRVTMTRDTSTSTVYMELSSLRSEDTAVYYCAKSWVSWFASWGQGTLVTVSSGGGGSGGGGSGSELVMTQSPDSLAVSLGERATINCKSSQSVLNSSNNRNYLAWYQQKPGQPPKLLIYWASTRESGVPDRFSGSGSGTDFTLTISGLQAEDVAVYYCQQHYSTPFTFGPGTKVDIKSGGGGSEVQLLESGGGLVQPGGSLKLSCAASGFTFNIYAMNWVRQAPGKGLEWVARIRSKYNNYATYYADSVKSRFTISRDDSKNTAYLQMNNLKTEDTAVYYCVRHGNFGNSYVSFFAYWGQGTLVTVSSGYYPNWVQQKPGQASELVVTQEPSLTVSPGGTVTLTCGSSTGAVTSGYYPNWVQQKPGQAPRGLIGGTKFLAPGTPARFSGSLLGGKAALTLSGVQPEDEAEYYCALWYSNRWVFGGGTKLTVL |
| 316 | MCSP-G4 VH-VL-P x F6A VH-VL-P | artificial | nt | CAGGTGCAGCTGGTCCAGTCTGGGGCTGAGGTGAAGAGGCCTGAGGTGAAGAAGCCTGGGGCCTCAGTGAAGGTCTCCTGCAAGGCTTCTGGGTACACCTTCACCAACTACTATATACACTGGTGCGACAGGCCCCTGGACAAGGTCTTGAGTGGATGGGTTGGATCAACCCTAACAGTGGTGCCACAAACTATGCACAGAAGTTCCAGGGCAGAGTCACCATGACCAGGGACACGTCCACGAGCACAGTCTACATGGAGCTGAGCAGCCTGAGATCTGAGGAGGACACGGCCGTGTATTACTGTGCGAGAGTCCA |

EP 2 155 788 B1

| | | | | |
|---|---|---|---|---|
| | | | | AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGCTCGAGTCTGGAGGA<br>GGATTGGTGCAGCCTGGAGGGTCATTGAAACTCTCATGTGCAGCCTCTGGATTCACCTTCAATAT<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAAGCAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACGTATCCTTCTTCGCTTACTGGGGCCAAG<br>GGACTCTGGTCACCGTCTCCTCAGGTGGTGGTGGTTCTGGCGGCGGCGGCTCCGGTGGTGGTGGT<br>TCTGAGCTCGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 317 | MCSP-G4 VH-VL-P x H2C VH-VL-P | artificial | aa | QVQLVQSGAEVKRPGASMKVSCKASGYTFTNYYIHWVRQAPGQGLEWMGWINPNSGATNYAQKFQ<br>GRVTMTRDTSTSTVYMELSSLRSEDTAVYYCAKSWVSWFASWGQGTLVTVSSGGGGSGGGGSGGGG<br>GSELVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLLESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SELVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 318 | MCSP-G4 VH-VL-P x H2C VH-VL-P | artificial | nt | CAGGTGCAGCTGGTCCAGTCTGGGGCTGAGGTGAAGAGGCCTGGGGCCTCAATGAAGGTCTCCTG<br>CAAGGCTTCTGGGTACACCTTCACCAACTACTATATACACTGGGTGCGACAGGCCCCTGGACAAG<br>GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG |

| | | | | |
|---|---|---|---|---|
| | | | | GTCCCTGACCGACCGATTCAGTGGCAGCGGGTCTGGGACAGATTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGTTGCTCGCATAAGAA GTAAATATAATAATTATGCAACATATTATGCCGATTCAGTGAAGAACAGTTCACCATCTCCAGA GATGATTCAAAAAACACTGCCTATCTACAAAATGAACAACTTGAAAATCCTACTGGGCGTA CTACTGTGTGAGACATGGGAACTTCGGTAATAGCTACATATCCTACTGGGCTTACTGGGGCCAAG GGACTCTGGTCACCGTCTCCTCAGGTGGTGGTGGTTCACCGGCTATCACCGTGTGGAACAGTCACACTCAC TCTGAGCTCGTTGTGACTCAGGAACCTTCACTCACCGTATCACCGTCCAAACTGGGTCCAAACAAAACCAG TTGTGGCTCCTCCGACTGGGCTGTCTAATAGTGGACTAAGTTCCTCGCCCCCGTACTCCTGCCAGATTC GTCAGGCACCCGTGGTCTTGGAGGCAAGGCTGCCCTCCACCCTCCAGGGTACAGCCAGGATGAGGC TCAGGCTCCCTGCTTGGAGGCAAGGCTGCCCTCCACCCTCCAGGGTACAGCCAGGATGAGGC AGAATATTACTGTGCTCTATGGTACAGCAACCGCTGGGTGTTCGGTGGAGGAACCAAACTGACTG TCCTA |
| 319 | MCSP-G4 VH-VL-P x H1E VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQTLVTVSSGGGGSGGGGSGGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLLESGG GLEQPGGSLKLSCAASGFTENSYAMNWVRQAPGKGLEWVARIRSKYNNYATYYADSVKGRFTISR DDSKNTAYLQMNNLKTEDTAVYYCVRHGNFGNSYLSFWAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 320 | MCSP-G4 VH-VL-P x H1E VH-VL-P | artificial | nt | CAGGTGCAGCTGGTCCAGTCTGGGGCTGAGGTGAAGAAGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGTACAGTCTACATGGAGCTGAGCAGCCTGAG ATCTGAGGACACGGCCGTGTATTACTGTGCGAAATCCTGGGTTTCTTGGTTTGCTTCCTGGGGTC AAGGAACCTTGGTCACCGTCTCCTCTGGCTGTGTCTCTGGGCGCGGAGAGGGCCAC GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGTCGCTCATTACTGGGCATCTCACCATCATTCACTTTGGT ACCAGCAGAAACCAGGACAGCCTCCTAAGCTGCTCATTACTGGGCATCTCACCATCATTCACTTTGGT GTCCCTGACCGACCGATTCAGTGGCAGTGGCGGGTCTGCGGGACAGATTTATTACTGTCAGCAACTA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATTC |

EP 2 155 788 B1

| | | | | |
|---|---|---|---|---|
| | | | | GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACCTATCCTTCTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 321 | MCSP-G4 VH-VL-P x G4H VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLLESGG GLVQPGGSLKLSCAASGFTFNRYAMNWVRQAPGKGLEWVARIRSKYNNYATYYADSVKGRFTISR DDSKNTAYLQMNNLKTEDTAVYYCVRHGNFGNSYLSYFAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 322 | MCSP-G4 VH-VL-P x G4H VH-VL-P | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATCG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACTTATCCTACTTCGCTTACTGGGGCCAAG |

| | | | | |
|---|---|---|---|---|
| | | | | GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 323 | MCSP-G4 VH-VL-P x A2J VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLLESGG GLVQPGGSLKLSCAASGFTFNVYAMNWVRQAPGKGLEWVARIRSKYNNYATYYADSVKKRFTISR DDSKNTAYLQMNNLKTEDTAVYYCVRHGNFGNSYLSWWAYWGQGTLVTVSSGGGGSGGGGSGGGG SELVVTQEPSLTVSPGGTVTLTCRSSTGAVTSGYYPNWVQQKPGQAPRGLIGATDMRPSGTPARF SGSLLGGKAALTLSGVQPEDEAEYYCALWYSNRWVFGGGTKLTVL |
| 324 | MCSP-G4 VH-VL-P x A2J VH-VL-P | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATGT CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAAAGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACTTATCCTGGTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTCGCTCCTCGACTGGGGCTGTTACATCTGGCTACTACCCAAACTGGGTCCAACAAAAACCAG GTCAGGCACCCCGTGGTCTAATAGGTGCCACTGACATGAGGCCCTCTGGTACTCCTGCCAGATTC |

| | | | | |
|---|---|---|---|---|
| | | | | TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 325 | MCSP-G4 VH-VL-P x E1L VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLLESGG GLVQPGGSLKLSCAASGFTFNKYAMNWVRQAPGKGLEWVARIRSKYNNYATYYADSVKSRFTISR DDSKNTAYLQMNNLKTEDTAVYYCVRHGNFGNSYTSYYAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 326 | MCSP-G4 VH-VL-P x E1L VH-VL-P | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATAA GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAATCGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACACATCCTACTACGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |

EP 2 155 788 B1

106

| 327 | MCSP-G4 VH-VL-P x E2M VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLLESGG GLVQPGGSLKLSCAASGFTFNGYAMNWVRQAPGKGLEWVARIRSKYNNYATYYADSVKERFTISR DDSKNTAYLQMNNLKTEDTAVYYCVRHRNFGNSYLSWFAYWGQGTLVTVSSGGGGSGGGGSGGGG SELVVTQEPSLTVSPGGTVTLTCRSSTGAVTSGYYPNWVQQKPGQAPRGLIGATDMRPSGTPARF SGSLLGGKAALTLSGVQPEDEAEYYCALWYSNRWVFGGGTKLTVL |
| 328 | MCSP-G4 VH-VL-P x E2M VH-VL-P | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATGG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGAGAGGTTCACCATCTCCAGA GATGATTCAAAAAACACTGCCTATCTACAAATGAACAACTTGAAAACTGAGGACACTGCCGTGTA CTACTGTGTGAGACATAGGAACTTCGGTAATAGCTACTTATCCTGGTTCGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTCGCTCCTCGACTGGGGCTGTTACATCTGGCTACTACCCAAACTGGGTCCAACAAAAACCAG GTCAGGCACCCCGTGGTCTAATAGGTGCCACTGACATGAGGCCCTCTGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 329 | MCSP-G4 VH-VL-P x F7O VH-VL-P | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSELVMTQSPDSLAVSLGERATINCKSSQSVLNSSNNRNYLAWYQQKPGQPPKLLIYWASTRESG |

EP 2 155 788 B1

| | | | | VPDRFSGSGSGTDFTLTISGLQAEDVAVYYCQQHYSTPFTFGPGTKVDIKSGGGGSEVQLLESGG GLVQPGGSLKLSCAASGFTFNVYAMNWVRQAPGKGLEWVARIRSKYNNYATYYADSVKKRFTISR DDSKNTAYLQMNNLKTEDTAVYYCVRHGNFGNSYISWWAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
|---|---|---|---|---|
| 330 | MCSP-G4 VH-VL-P x F7O VH-VL-P | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATGT GTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAAAGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACATATCCTGGTGGGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 331 | MCSP-G4 VH-VL-P x F12Q VH-VL | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLVESGG GLVQPGGSLKLSCAASGFTFNSYAMNWVRQAPGKGLEWVARIRSKYNNYATYYADSVKGRFTISR DDSKNTAYLQMNNLKTEDTAVYYCVRHGNFGNSYVSWWAYWGQGTLVTVSSGGGGSGGGGSGGGG SQTVVTQEPSLTVSPGGTVTLTCGSSTGAVTSGNYPNWVQQKPGQAPRGLIGGTKFLAPGTPARF |

| | | | | SGSLLGGKAALTLSGVQPEDEAEYYCVLWYSNRWVFGGGTKLTVL |
|---|---|---|---|---|
| 332 | MCSP-G4 VH-VL-P x F12Q VH-VL | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG GTCTTGAGTGGATGGGTTGGATCAACCCTAACAGTGGTGCCACAAACTATGCACAGAAGTTCCAG GGCAGAGTCACCATGACCAGGGACACGTCCACGAGCACAGTCTACATGGAGCTGAGCAGCCTGAG ATCTGAGGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACCCAGTCTCCAGACTCCCTGGCTGTGTCTCTGGGCGAGAGGGCCAC CATCAACTGCAAGTCCAGCCAGAGTGTTTTAAACAGCTCCAACAATAGGAACTACTTAGCTTGGT ACCAGCAGAAACCAGGACAGCCTCCTAAGCTGCTCATTTACTGGGCATCTACCCGGGAATCCGGG GTCCCTGACCGATTCAGTGGCAGCGGGTCTGGGACAGATTTCACTCTCACCATCAGTGGCCTGCA GGCTGAAGATGTGGCAGTTTATTACTGTCAGCAACATTATAGTACTCCATTCACTTTTGGCCCTG GGACCAAAGTGGATATCAAATCCGGAGGTGGTGGATCCGAGGTGCAGCTGGTCGAGTCTGGAGGA GGATTGGTGCAGCCTGGAGGGTCATTGAAACTCTCATGTGCAGCCTCTGGATTCACCTTCAATAG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGCAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACGTTTCCTGGTGGGCTTACTGGGGCCAAG GGACTCTGGTCACCGTCTCCTCAGGTGGTGGTGGTTCTGGCGGCGGCGGCTCCGGTGGTGGTGGT TCTCAGACTGTTGTGACTCAGGAACCTTCACTCACCGTATCACCTGGTGGAACAGTCACACTCAC TTGTGGCTCCTCGACTGGGGCTGTTACATCTGGCAACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGTTCTATGGTACAGCAACCGCTGGGTGTTCGGTGGAGGAACCAAACTGACTG TCCTA |
| 333 | MCSP-G4 VH-VL-P x I2C VH-VL | artificial | aa | QVQLVQSGAEVKRPGASMKVSCKASGYTFTNYYIHWVRQAPGQGLEWMGWINPNSGATNYAQKFQ GRVTMTRDTSTSTVYMELSSLRSEDTAVYYCAKSWVSWFASWGQGTLVTVSSGGGGSGGGGSGGG GSELVMTQSPDSLAVSLGERATINCKSSQSVLNSSNNRNYLAWYQQKPGQPPKLLIYWASTRESG VPDRFSGSGSGTDFTLTISGLQAEDVAVYYCQQHYSTPFTFGPGTKVDIKSGGGGSEVQLVESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SQTVVTQEPSLTVSPGGTVTLTCGSSTGAVTSGNYPNWVQQKPGQAPRGLIGGTKFLAPGTPARF SGSLLGGKAALTLSGVQPEDEAEYYCVLWYSNRWVFGGGTKLTVL |
| 334 | MCSP-G4 VH-VL-P x I2C VH- | artificial | nt | CAGGTGCAGCTGGTCCAGTCTGGGGCTGAGGTGAAGAGGCCTGGGGCCTCAATGAAGGTCTCCTG CAAGGCTTCTGGGTACACCTTCACCAACTACTATATACACTGGGTGCGACAGGCCCCTGGACAAG |

EP 2 155 788 B1

| | VL | | | GTCTTGAGTGGATGGGTTGGATCAACCCTAACAGTGGTGCCACAAACTATGCACAGAAGTTCCAG<br>GGCAGAGTCACCATGACCAGGGACACGTCCACGAGCACAGTCTACATGGAGCTGAGCAGCCTGAG<br>ATCTGAGGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGAGCTCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 335 | MCSP-D2 VH-VL<br>x H2C VH-VL | artificial | aa | QVQLVQSGAEVKKPGASVKVSCKASGYTFTGYYMHWVRQAPGQGLEWMGWINPNSGGTSYAQKFQ<br>GRVTMTRDTSTSTVYMELSNLRSDDTAVYYCAKSWVSWFASWGQGTLVTVSSGGGGSGGGGSGGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGYYPNWVQQKPGQAPRGLIGGTKFLAPGTPARF<br>SGSLLGGKAALTLSGVQPEDEAEYYCALWYSNRWVFGGGTKLTVL |
| 336 | MCSP-D2 VH-VL<br>x H2C VH-VL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACACCTTCACCGGCTACTATATGCACTGGGTGCGACAGGCCCCTGGACAAG<br>GGCTTGAGTGGATGGGATGGATCAACCCTAACAGTGGTGGCACAAGCTACGCACAGAAGTTCCAG<br>GGCAGAGTCACCATGACTAGGGACACGTCCACGAGCACAGTCTACATGGAGCTGAGCAACCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT |

| | | | | |
|---|---|---|---|---|
| | | | | GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGACAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACATATCCTACTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCTACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGCTCTATGGTACAGCAACCGCTGGGTGTTCGGTGGAGGAACCAAACTGACTG<br>TCCTA |
| 337 | MCSP-D2 VH-VL x F12Q VH-VL | artificial | aa | QVQLVQSGAEVKKPGASVKVSCKASGYTFTGYYMHWVRQAPGQGLEWMGWINPNSGGTSYAQKFQ<br>GRVTMTRDTSTSTVYMELSNLRSDDTAVYYCAKSWVSWFASWGQGTLVTVSSGGGGSGGGGSGGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNSYAMNWVRQAPGKGLEWVARIRSKYNNYATYYADSVKGRFTISR<br>DDSKNTAYLQMNNLKTEDTAVYYCVRHGNFGNSYVSWWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 338 | MCSP-D2 VH-VL x F12Q VH-VL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACACCTTCACCGGCTACTATATGCACTGGGTGCGACAGGCCCCTGGACAAG<br>GGCTTGAGTGGATGGGATGGATCAACCCTAACAGTGGTGGCACAAGCTACGCACAGAAGTTCCAG<br>GGCAGAGTCACCATGACTAGGGACACGTCCACGAGCACAGTCTACATGGAGCTGAGCAACCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA |

| | | | | |
|---|---|---|---|---|
| | | | | GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAG<br>CTACGCCATGAACTGGGTCCGCCAGGCTCCAGGAAAGGGTTTGGAATGGGTTGCTCGCATAAGAA<br>GTAAATATAATAATTATGCAACATATTATGCCGATTCAGTGAAAGGCAGGTTCACCATCTCCAGA<br>GATGATTCAAAAAACACTGCCTATCTACAAATGAACAACTTGAAAACTGAGGACACTGCCGTGTA<br>CTACTGTGTGAGACATGGGAACTTCGGTAATAGCTACGTTTCCTGGTGGGCTTACTGGGGCCAAG<br>GGACTCTGGTCACCGTCTCCTCAGGTGGTGGTGGTTCTGGCGGCGGCGGCTCCGGTGGTGGTGGT<br>TCTCAGACTGTTGTGACTCAGGAACCTTCACTCACCGTATCACCTGGTGGAACAGTCACACTCAC<br>TTGTGGCTCCTCGACTGGGGCTGTTACATCTGGCAACTACCCAAACTGGGTCCAACAAAAACCAG<br>GTCAGGCACCCCGTGGTCTAATAGGTGGGACTAAGTTCCTCGCCCCCGGTACTCCTGCCAGATTC<br>TCAGGCTCCCTGCTTGGAGGCAAGGCTGCCCTCACCCTCTCAGGGGTACAGCCAGAGGATGAGGC<br>AGAATATTACTGTGTTCTATGGTACAGCAACCGCTGGGTGTTCGGTGGAGGAACCAAACTGACTG<br>TCCTA |
| 339 | MCSP-D2 VH-VL<br>x I2C VH-VL | artificial | aa | QVQLVQSGAEVKKPGASVKVSCKASGYTFTGYYMHWVRQAPGQGLEWMGWINPNSGGTSYAQKFQ<br>GRVTMTRDTSTSTVYMELSNLRSDDTAVYYCAKSWVSWFASWGQGTLVTVSSGGGGSGGGGSGGG<br>GSDIVMTQSPDSLAVSLGERATINCKSSQSVLNSSNNRNYLAWYQQKPGQPPKLLIYWASTRESG<br>VPDRFSGSGSGTDFTLTISGLQAEDVAVYYCQQHYSTPFTFGPGTKVDIKSGGGGSEVQLVESGG<br>GLVQPGGSLKLSCAASGFTFNKYAMNWVRQAPGKGLEWVARIRSKYNNYATYYADSVKDRFTISR<br>DDSKNTAYLQMNNLKTEDTAVYYCVRHGNFGNSYISYWAYWGQGTLVTVSSGGGGSGGGGSGGGG<br>SQTVVTQEPSLTVSPGGTVTLTCGSSTGAVTSGNYPNWVQQKPGQAPRGLIGGTKFLAPGTPARF<br>SGSLLGGKAALTLSGVQPEDEAEYYCVLWYSNRWVFGGGTKLTVL |
| 340 | MCSP-D2 VH-VL<br>x I2C VH-VL | artificial | nt | CAGGTGCAGCTGGTCCAGTCTGGGGCTGAGGTGAAGAAGCCTGGGGCCTCAGTGAAGGTCTCCTG<br>CAAGGCTTCTGGATACACCTTCACCGGCTACTATATGCACTGGGTGCGACAGGCCCCTGGACAAG<br>GGCTTGAGTGGATGGGATGGATCAACCCTAACAGTGGTGGCACAAGCTACGCACAGAAGTTCCAG<br>GGCAGAGTCACCATGACTAGGGACACGTCCACGAGCACAGTCTACATGGAGCTGAGCAACCTGAG<br>ATCTGACGACACGGCCGTGTATTACTGTGCGAAATCCTGGGTCTCCTGGTTTGCTTCCTGGGGTC<br>AAGGAACCTTGGTCACCGTCTCCTCAGGTGGTGGTGGTTCTGGCGGCGGCGGCTCCGGTGGTGGT<br>GGTTCTGATATCGTGATGACCCAGTCTCCAGACTCCCTGGCTGTGTCTCTGGGCGAGAGGGCCAC<br>CATCAACTGCAAGTCCAGCCAGAGTGTTTTAAACAGCTCCAACAATAGGAACTACTTAGCTTGGT<br>ACCAGCAGAAACCAGGACAGCCTCCTAAGCTGCTCATTTACTGGGCATCTACCCGGGAATCCGGG<br>GTCCCTGACCGATTCAGTGGCAGCGGGTCTGGGACAGATTTCACTCTCACCATCAGTGGCCTGCA<br>GGCTGAAGATGTGGCAGTTTATTACTGTCAGCAACATTATAGTACTCCATTCACTTTTGGCCCTG<br>GGACCAAAGTGGATATCAAATCCGGAGGTGGTGGATCCGAGGTGCAGCTGGTCGAGTCTGGAGGA<br>GGATTGGTGCAGCCTGGAGGGTCATTGAAACTCTCATGTGCAGCCTCTGGATTCACCTTCAATAA<br>GTACGCCATGAACTGGGTCCGCCAGGCTCCAGGAAAGGGTTTGGAATGGGTTGCTCGCATAAGAA |

| SEQ ID | Name | Organism | Type | Sequence |
|---|---|---|---|---|
|  |  |  |  | GTAAATATAATAATTATGCAACACATTATGCCGATTCAGTGAAAGACAGGTTCACCATCTCCAGAGATGATTCAAAAAACACTGCCTATCTACAAATGAACAACTTGAAAACTGAGGACACTGCCGTGTACTACTGTGTGAGACATGGGAACATTCGGTACGTGGTGGTTCAACTACTACATATCCTGGCGGCGGCCAAGGGACTCTGGTCACCGTCTCCTCAGGAGAACCTTCACTCACGGTGTGTGTGACTCAGCCTATCACCCAGCACTCTCAGACTGTTGTGTGACTCAGGGGGTCGTTACATCTGGCAACTACCACCAAACTGGTCAACAAAAACCAGTTGTGGCTCCTCGACCCCGTGGTCGTTAATAGGTGGGACTAAGTTCCTGCCCCTCACCCTCACCATCTCCGGGACTAAGTTCCTGCCAGATTCGTCAGGCACCCGTGGTCGTTGAGGCAAGGCTAAGTTCCTGCCCCTCACCCTCACCATCTCCGGGAGAATATTACTGTGTTCTATGTGACAGCAACCGCTGGGTGTTCGGTGGAGGAACCAAACTGACTGTCCTA |
| 341 | MCSP-D2 VH-VL-P x H2C VH-VL-P | artificial | aa | QVQLVQSGAEVKKPGASVKVSCKASGYTFTGYYMHWVRQAPGQGLEWMGWINPNSGGTSYAQKFQGRVTMTRDTSTSTVYMELSNLRSDDTAVYYCAKSWVSWFASWGQGTLVTVSSGGGGSGGGGSGGGGSGSELVMTQSPDSLAVSLGERATINCKSSQSVLNSSNNRNYLAWYQQKPGQPPKLLIYWASTRESGVPDRFSGSGSGTDFTLTISGLQAEDVAVYYCQQHYSTPFTFGPGTKVDIKSGGGGSEVQLLESGGGLVQPGGSLKLSCAASGFTNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSELVVTQEPSLTVSPGGTVTLTCGSSTGAVTSGYYPNWVQQKPGQAPRGLIGGTKFLAPGTPARFSGSLLGGKAALTLSGVPEDEAEYYCALWYSNRWVFGGGTKLTVL |
| 342 | MCSP-D2 VH-VL-P x H2C VH-VL-P | artificial | nt | CAGGTGCAGCTGGTCCAGTCTGGGGCTGAGGTGAAGAAGCCTGGGGCCTCAGTGAAGGTCTCCTGCAAGGCTTCTGGATACACCTTCACCGGCTACTATATGCACTGGGTGCGACAGGCCCCTGGACAAGGGCTTGAGTGGATGGGATGGATCAACCCTAACAGTGGTGGCACAGTTACGCACAGAAGTTCCAGGGCAGAGTCACCATGACCAGGGACACGTCCACGAGCACAGTCTACATGGAGCTGAGCAACCTGAGATCTGACGACACGGCCGTGTATTACTGTGCAAAGTCCTGGGTCTCGTGGTTGCTTCCTGGGGTCAAGGAACCTTGGTCACCGTCTCCTCAGGTGGTGGTGGTTCTGGCGGCGGCGGCTCCGGTGGTGGTGGTTCTGAGCTCGTGATGACCCAGAGTCCTCCAGCTGTTTAAACAGCTGCTCATTTGCTGGGGTCCATCAACTGCAAGTCCAGAGACAGCCTCCAGTCTCCAACATTACTGGGCATCTCACCAGTAGTCTGGGGTCCCTGACCGATTCAGTGGCAGCGGGTCTGGGACAGATTCACTCTCACCATTCACTTTTGGCCCTGGGACCAAAGTGGATATCAAATCCGGAGGTGGTGGATCTCTGAGGTGGTGGATCTGGAGGAGGATTGGTGCAGCCTGGAGGGTCATTGAAACTCTCTGATTCAGCCTCTGGATTCACCTTCAATAAGTATGCCATGAACTGGGTCCGCCAGGCTCCAGGGAAAGGGTTTGGAATGGGTTGCTCGCATAAGAAGTAAATATAATTATGCAACATTATGCCGATTCAGTGAAAGACAGGTTGCTCACCATCTCCAGAGATGATTCAAAAAACACTGCCTATCTACAAATGAACAACTTGAAAACTGAGGACACTGCCGTGTACTACTGTGTGAGACATGGGAACATTCGGTACGTGGTGGTTCTGGCGGCGGCGGCTCCGGTGGTGGTGGT |

113

| | | | | |
|---|---|---|---|---|
| | | | | TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 343 | MCSP-F9 VH-VL x H2C VH-VL | artificial | aa | QVQLQESGPGLVKPSETLSLTCVVSGGSISSSNWWSWVRQPPGKGLEWLGTIYYNGNTYYNPSLK SRVTISVDTSKNQFSLRLSSVTAADTAVYYCAKSWVSWFASWGQGTLVTVSSGGGGSGGGGSGGG GSDIVMTQSPDSLAVSLGERATINCKSSQSVLSSSNNKNYLNWYQQKPGQPPKLLIYWASTRESG VPDRFSGSGSGTDFTLTISSLQAEDVAVYYCQQHYSVPFTFGPGTKVDIKGGGGSEVQLVESGGG LVQPGGSLKLSCAASGFTFNKYAMNWVRQAPGKGLEWVARIRSKYNNYATYYADSVKDRFTISRD DSKNTAYLQMNNLKTEDTAVYYCVRHGNFGNSYISYWAYWGQGTLVTVSSGGGGSGGGGSGGGGS QTVVTQEPSLTVSPGGTVTLTCGSSTGAVTSGYYPNWVQQKPGQAPRGLIGGTKFLAPGTPARFS GSLLGGKAALTLSGVQPEDEAEYYCALWYSNRWVFGGGTKLTVL |
| 344 | MCSP-F9 VH-VL x H2C VH-VL | artificial | nt | CAGGTGCAGCTGCAAGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGAGGCTGAGCTCTGTGAC CGCCGCAGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGATATCGTGATGACACAGTCTCCAGACTCCCTGGCTGTGTCTCTGGGCGAGAGGGCCAC CATCAACTGCAAGTCCAGCCAGAGTGTCTTATCCAGCTCCAACAATAAGAACTACTTAAATTGGT ACCAGCAGAAACCAGGACAGCCTCCTAAGTTGCTCATTTACTGGGCATCTACCCGGGAATCCGGG GTCCCTGACCGATTCAGTGGCAGCGGGTCTGGGACAGATTTCACTCTCACCATCAGCAGCCTGCA GGCTGAAGATGTGGCAGTTTATTACTGTCAGCAACATTATAGTGTTCCATTCACTTTCGGCCCTG GGACCAAAGTGGATATCAAAGGAGGTGGTGGCTCCGAGGTGCAGCTGGTCGAGTCTGGAGGAGGA TTGGTGCAGCCTGGAGGGTCATTGAAACTCTCATGTGCAGCCTCTGGATTCACCTTCAATAAGTA CGCCATGAACTGGGTCCGCCAGGCTCCAGGAAAGGGTTTGGAATGGGTTGCTCGCATAAGAAGTA AATATAATAATTATGCAACATATTATGCCGATTCAGTGAAAGACAGGTTCACCATCTCCAGAGAT GATTCAAAAAACACTGCCTATCTACAAATGAACAACTTGAAAACTGAGGACACTGCCGTGTACTA CTGTGTGAGACATGGGAACTTCGGTAATAGCTACATATCCTACTGGGCTTACTGGGGCCAAGGGA CTCTGGTCACCGTCTCCTCAGGTGGTGGTGGTTCTGGCGGCGGCGGCTCCGGTGGTGGTGGTTCT CAGACTGTTGTGACTCAGGAACCTTCACTCACCGTATCACCTGGTGGAACAGTCACACTCACTTG TGGCTCCTCGACTGGGGCTGTTACATCTGGCTACTACCCAAACTGGGTCCAACAAAAACCAGGTC AGGCACCCCGTGGTCTAATAGGTGGGACTAAGTTCCTCGCCCCCGGTACTCCTGCCAGATTCTCA GGCTCCCTGCTTGGAGGCAAGGCTGCCCTCACCCTCTCAGGGGTACAGCCAGAGGATGAGGCAGA |

| | | | | |
|---|---|---|---|---|
| 345 | MCSP-F9 VH-VL-P x H2C VH-VL-P | artificial | aa | EVQLQESGPGLVKPSETLSLTCVVSGGSISSSNWWSWVRQPPGKGLEWLGTIYYNGNTYYNPSLK SRVTISVDTSKNQFSLRLSSVTAADTAVYYCAKSWVSWFASWGQGTLVTVSSGGGGSGGGGSGGGG GSELVMTQSPDSLAVSLGERATINCKSSQSVLSSSNNKNYLNWYQQKPGQPPKLLIYWASTRESG VPDRFSGSGSGTDFTLTISSLQAEDVAVYYCQQHYSVPFTFGPGTKVDIKSGGGGSEVQLLESGG GLVQPGGSLKLSCAASGFTFNKYAMNWVRQAPGKGLEWVARIRSKYNNYATYYADSVKDRFTISR DDSKNTAYLQMNNLKTEDTAVYYCVRHGNFGNSYISYWAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 346 | MCSP-F9 VH-VL-P x H2C VH-VL-P | artificial | nt | GAGGTGCAGCTGCAAGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGAGCTGTTGCTCTGTGAC CGCCGCAGACACGGCCGTGTATTACTGTGCGAAATCCTGGGTCTGGTTCGCGGCGGCCTCCTGGGTC AAGGAACCTTGGTCTGTCGGTGATGACAGTCCCAGACTCCCTGGTCCCTGGTGTCTCTGGGCGAGGGGCCAC GGTTCTGAGCTCCGTGATGACAGTCCCAGACTCCCTGGTCCCTGGTGTCTCTGGGCGAGGGGCCAC CATCAACTGCAAGTCCAGCAGAGTGTCTTATCCAGCTCCAAGTTGCTCTCAGTGGCATCTCACCTCCACCATCAGCAGCCTGCA GTCCCTGACCGATTCAGTGGCAGCGGGTCTGGCAGCGATTCCATTCACTCTCCACCATCAGCAGCCTGCA GGCTGAAGATGTGGCAGTTTATTACTGTCAGCAACATTATAGTGTTCCATTCACTTTCGGCCCTG GGACCAAAGTGGATATCAAATCCGGAGGTGGTGGATCTGAGGTGCAGCTGCTGGAGGA GGATTGGTGCAGCCTGGAGGGTCCGCCAGCCTCTGAATGGGTTGCTCGCCATCTCCAGA GTACCGCCATGAACTGGGTCCGCCAGGCTCCAGGAAAGGGTTTCAGTGAACAGAGTTCACCATCTCCAGA GTAAATATAATAATGCCTATCTACACAAATGAACAACTGAAAAACTGAGGACATGCGGTA CTACTGTGTGAGACATGGCAGACTTCGGTAATAGCTACATATCCTACTGGGGCCAAG GGACTCTGGTCACCGTCTCCTCCAGGTGGTTCTGGCTTACTGGGTTCTGGCTTACTGGGTGGTGGT TCTGAGCTCGTTGTGACTCAGGAACCTTCACTCTGCTATCACCGTATCACCGTATCACTCAC TTGTGGCTCCTCCGACTGGGCGTGTTACATCTGGCTACTACCCAAACTGGTCCAACAAAAACCAG GTCAGGCACCCGTGGTCAAGGCTGCCCTCACCCTCTCAGGGGTACAGCCAGGGATGAGGC TCAGGTCCTGGAGGCAAGGCTCCATGTGCTCTATGGTACAGCAACGCTGGTCGGTGTTCGGTGAGGAACTGACTG AGAATATTACTGTGCTCTATGGTACAGCAACCGCTGGTCGGTGTTCGGTGGAGGAACTGACTG TCCTA |
| 347 | MCSP-F9 VH-VL- | artificial | aa | EVQLQESGPGLVKPSETLSLTCVVSGGSISSSNWWSWVRQPPGKGLEWLGTIYYNGNTYYNPSLK |

EP 2 155 788 B1

| | | | | |
|---|---|---|---|---|
| | P x G4H VH-VL-P | | | SRVTISVDTSKNQFSLRLSSVTAADTAVYYCAKSWVSWFASWGQGTLVTVSSGGGGSGGGGSGGG GSELVMTQSPDSLAVSLGERATINCKSSQSVLSSSNNKNYLNWYQQKPGQPPKLLIYWASTRESG VPDRFSGSGSGTDFTLTISSLQAEDVAVYYCQQHYSVPFTFGPGTKVDIKSGGGGSEVQLLESGG GLVQPGGSLKLSCAASGFTFNRYAMNWVRQAPGKGLEWVARIRSKYNNYATYYADSVKGRFTISR DDSKNTAYLQMNNLKTEDTAVYYCVRHGNFGNSYLSYFAYWGQGTLVTVSSGGGGSGGGGSGGGG SELVVTQEPSLTVSPGGTVTLTCGSSTGAVTSGYYPNWVQQKPGQAPRGLIGGTKFLAPGTPARF SGSLLGGKAALTLSGVQPEDEAEYYCALWYSNRWVFGGGTKLTVL |
| 348 | MCSP-F9 VH-VL-P x G4H VH-VL-P | artificial | nt | GAGGTGCAGCTGCAAGAGTCTGGCCCAGGACTGGTGAAGCCTTCGGAGACCCTGTCCCTCACCTG CGTTGTCTCTGGTGGCTCCATCAGCAGTAGTAACTGGTGGAGCTGGGTCCGCCAGCCCCCAGGGA AGGGACTGGAGTGGCTTGGGACTATCTATTATAATGGGAATACCTACTACAACCCGTCCCTCAAG AGTCGAGTCACCATCTCCGTAGACACGTCCAAGAACCAGTTCTCCCTGAGGCTGAGCTCTGTGAC CGCCGCAGACACGGCCGTGTATTACTGTGCGAAATCCTGGGTCTCCTGGTTTGCTTCCTGGGGTC AAGGAACCTTGGTCACCGTCTCCTCAGGTGGTGGTGGTTCTGGCGGCGGCGGCTCCGGTGGTGGT GGTTCTGAGCTCGTGATGACACAGTCTCCAGACTCCCTGGCTGTGTCTCTGGGCGAGAGGGCCAC CATCAACTGCAAGTCCAGCCAGAGTGTCTTATCCAGCTCCAACAATAAGAACTACTTAAATTGGT ACCAGCAGAAACCAGGACAGCCTCCTAAGTTGCTCATTTACTGGGCATCTACCCGGGAATCCGGG GTCCCTGACCGATTCAGTGGCAGCGGGTCTGGGACAGATTTCACTCTCACCATCAGCAGCCTGCA GGCTGAAGATGTGGCAGTTTATTACTGTCAGCAACATTATAGTGTTCCATTCACTTTCGGCCCTG GGACCAAAGTGGATATCAAATCCGGAGGTGGTGGATCCGAGGTGCAGCTGCTCGAGTCTGGAGGA GGATTGGTGCAGCCTGGAGGGTCATTGAAACTCTCATGTGCAGCCTCTGGATTCACCTTCAATCG CTACGCCATGAACTGGGTCCGCCAGGCTCCAGGAAAGGGTTTGGAATGGGTTGCTCGCATAAGAA GTAAATATAATAATTATGCAACATATTATGCCGATTCAGTGAAAGGGAGGTTCACCATCTCCAGA GATGATTCAAAAAACACTGCCTATCTACAAATGAACAACTTGAAAACTGAGGACACTGCCGTGTA CTACTGTGTGAGACATGGGAACTTCGGTAATAGCTACTTATCCTACTTCGCTTACTGGGGCCAAG GGACTCTGGTCACCGTCTCCTCAGGTGGTGGTGGTTCTGGCGGCGGCGGCTCCGGTGGTGGTGGT TCTGAGCTCGTTGTGACTCAGGAACCTTCACTCACCGTATCACCTGGTGGAACAGTCACACTCAC TTGTGGCTCCTCGACTGGGGCTGTTACATCTGGCTACTACCCAAACTGGGTCCAACAAAAACCAG GTCAGGCACCCCGTGGTCTAATAGGTGGGACTAAGTTCCTCGCCCCCGGTACTCCTGCCAGATTC TCAGGCTCCCTGCTTGGAGGCAAGGCTGCCCTCACCCTCTCAGGGGTACAGCCAGAGGATGAGGC AGAATATTACTGTGCTCTATGGTACAGCAACCGCTGGGTGTTCGGTGGAGGAACCAAACTGACTG TCCTA |
| 349 | 1-27 CD3ε-Fc | artificial | aa | QDGNEEMGGITQTPYKVSISGTTVILTSGEPKSCDKTHTCPPCPAPELLGGPSVFLFPPKPKDTL MISRTPEVTCVVVDVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEW ESNGQPENNYKTTPPVLDSDGSFFLYSKLTVDKSRWQQGNVFSCSVMHEALHNHYTQKSLSLSPG |

| | | | | KHHHHHH |
|---|---|---|---|---|
| 350 | 1-27 CD3ε-Fc | artificial | cDNA | atgggatggagctgtatcatcctcttcttggtagcaacagctacaggtgtacactcccaagatgg taatgaagaaatgggtggtattacacagacaccatataaagtctccatctctggaaccacagtaa tattgacatccggagagcccaaatcttgtgacaaaactcacacatgcccaccgtgcccagcacct gaactcctggggggaccgtcagtcttcctcttccccccaaaacccaaggacaccctcatgatctc ccggacccctgaggtcacatgcgtggtggtggacgtgagccacgaagaccctgaggtcaagttca actggtacgtggacggcgtggaggtgcataatgccaagacaaagccgcgggaggagcagtacaac agcacgtaccgtgtggtcagcgtcctcaccgtcctgcaccaggactggctgaatggcaaggagta caagtgcaaggtctccaacaaagccctcccagcccccatcgagaaaaccatctccaaagccaaag ggcagccccgagaaccacaggtgtacaccctgcccccatcccgggaggagatgaccaagaaccag gtcagcctgacctgcctggtcaaaggcttctatcccagcgacatcgccgtggagtgggagagcaa tgggcagccggagaacaactacaagaccacgcctcccgtgctggactccgacggctccttcttcc tctatagcaagctcaccgtggacaagagcaggtggcagcagggggaacgtcttctcatgctccgtg atgcatgaggctctgcacaaccactacacgcagaagagcctctccctgtccccgggtaaacatca tcaccatcatcat |
| 351 | human 1-27 CD3 ε -EpCAM | artificial | aa | QDGNEEMGGITQTPYKVSISGTTVILTDYKDDDDKTASFAAAQKECVCENYKLAVNCFLNDNGQC QCTSIGAQNTVLCSKLAAKCLVMKAEMNGSKLGRRAKPEGALQNNDGLYDPDCDESGLFKAKQCN GTSTCWCVNTAGVRRTDKDTEITCSERVRTYWIIIELKHKAREKPYDVQSLRTALEEAIKTRYQL DPKFITNILYEDNVITIDLVQNSSQKTQNDVDIADVAYYFEKDVKGESLFHSKKMDLRVNGEQLD LDPGQTLIYYVDEKAPEFSMQGLKAGVIAVIVVVVIAIVAGIVVLVISRKKRMAKYEKAEIKEMG EMHRELNA |
| 352 | human 1-27 CD3 ε -EpCAM | artificial | cDNA | atgggatggagctgtatcatcctcttcttggtagcaacagctacaggtgtacactcccaagatgg taatgaagaaatgggtggtattacacagacaccatataaagtctccatctctggaaccacagtaa tattgacagattacaaggacgacgatgacaagactgcgagttttgccgcagctcagaaagaatgt gtctgtgaaaactacaagctggccgtaaactgctttttgaatgacaatggtcaatgccagtgtac ttcgattggtgcacaaaatactgtcctttgctcaaagctggctgccaaatgtttggtgatgaagg cagaaatgaacggctcaaaacttgggagaagagcgaaacctgaagggggctctccagaacaatgat ggcctttacgatcctgactgcgatgagagcgggctctttaaggccaagcagtgcaacggcacctc cacgtgctggtgtgtgaacactgctggggtcagaagaactgacaaggacactgaaataacctgct ctgagcgagtgagaacctactggatcatcattgaattaaaacacaaagcaagagaaaaaccttat gatgttcaaagtttgcggactgcacttgaggaggcgatcaaaacgcgttatcaactggatccaaa atttatcacaaatatttttgtatgaggataatgttatcactattgatctggttcaaaattcttctc agaaaactcagaatgatgtggacatagctgatgtggcttattattttgaaaaagatgttaaaggt gaatccttgtttcattctaagaaaatggacctgagagtaaatggggaacaactggatctggatcc tggtcaaacttttaatttattatgtcgatgaaaaagcacctgaattctcaatgcagggtctaaaag |

EP 2 155 788 B1

| | | | | |
|---|---|---|---|---|
| | | | | ctggtgttattgctgttattgtggttgtggtgatagcaattgttgctggaattgttgtgctggtt atttccagaaagaagagaatggcaaagtatgagaaggctgagataaaggagatgggtgagatgca tagggaactcaatgca |
| 353 | marmoset 1-27 CD3 ε -EpCAM | artificial | aa | QDGNEEMGDTTQNPYKVSISGTTVTLTDYKDDDDKTASFAAAQKECVCENYKLAVNCFLNDNGQC QCTSIGAQNTVLCSKLAAKCLVMKAEMNGSKLGRRAKPEGALQNNDGLYDPDCDESGLFKAKQCN GTSTCWCVNTAGVRRTDKDTEITCSERVRTYWIIIELKHKAREKPYDVQSLRTALEEAIKTRYQL DPKFITNILYEDNVITIDLVQNSSQKTQNDVDIADVAYYFEKDVKGESLFHSKKMDLRVNGEQLD LDPGQTLIYYVDEKAPEFSMQGLKAGVIAVIVVVVIAIVAGIVVLVISRKKRMAKYEKAEIKEMG EMHRELNA |
| 354 | marmoset 1-27 CD3 ε -EpCAM | artificial | cDNA | atgggatggagctgtatcatcctcttcttggtagcaacagctacaggtgtacactcccaggacgg taatgaagaaatgggtgatactacacagaacccatataaagtttccatctcaggaaccacagtaa cactgacagattacaaggacgacgatgacaagactgcgagttttgccgcagctcagaaagaatgt gtctgtgaaaactacaagctggccgtaaactgctttttgaatgacaatggtcaatgccagtgtac ttcgattggtgcacaaaatactgtcctttgctcaaagctggctgccaaatgtttggtgatgaagg cagaaatgaacggctcaaaacttgggagaagagcgaaacctgaggggctctccagaacaatgat ggcctttacgatcctgactgcgatgagagcgggctctttaaggccaagcagtgcaacggcacctc cacgtgctggtgtgtgaacactgctggggtcagaagaactgacaaggacactgaaataacctgct ctgagcgagtgagaacctactggatcatcattgaattaaaacacaaagcaagagaaaaaccttat gatgttcaaagtttgcggactgcacttgaggaggcgatcaaaacgcgttatcaactggatccaaa atttatcacaaatattttgtatgaggataatgttatcactattgatctggttcaaaattcttctc agaaaactcagaatgatgtggacatagctgatgtggcttattattttgaaaaagatgttaaaggt gaatccttgtttcattctaagaaaatggacctgagagtaaatggggaacaactggatctggatcc tggtcaaactttaatttattatgtcgatgaaaaagcacctgaattctcaatgcagggtctaaaag ctggtgttattgctgttattgtggttgtggtgatagcaattgttgctggaattgttgtgctggtt atttccagaaagaagagaatggcaaagtatgagaaggctgagataaaggagatgggtgagatgca tagggaactcaatgca |
| 355 | tamarin 1-27 CD3 ε -EpCAM | artificial | aa | QDGNEEMGDTTQNPYKVSISGTTVTLTDYKDDDDKTASFAAAQKECVCENYKLAVNCFLNDNGQC QCTSIGAQNTVLCSKLAAKCLVMKAEMNGSKLGRRAKPEGALQNNDGLYDPDCDESGLFKAKQCN GTSTCWCVNTAGVRRTDKDTEITCSERVRTYWIIIELKHKAREKPYDVQSLRTALEEAIKTRYQL DPKFITNILYEDNVITIDLVQNSSQKTQNDVDIADVAYYFEKDVKGESLFHSKKMDLRVNGEQLD LDPGQTLIYYVDEKAPEFSMQGLKAGVIAVIVVVVIAIVAGIVVLVISRKKRMAKYEKAEIKEMG EMHRELNA |
| 356 | tamarin 1-27 CD3 ε -EpCAM | artificial | cDNA | atgggatggagctgtatcatcctcttcttggtagcaacagctacaggtgtacactcccaggacgg taatgaagaaatgggtgatactacacagaacccatataaagtttccatctcaggaaccacagtaa |

EP 2 155 788 B1

| SEQ ID | Name | Organism | Type | Sequence |
|---|---|---|---|---|
| | | | | cactgacagattacacaaggacgacgatgacaaactgcgagttctgtccgccagtcagaaagaatgt gtctgtgaaaactacaagctggccgtaaactgctttttgaatgacaatggtcaatgccagtgtac ttcgattggtgcacaaatactgtccttggagaagagagcggctcttgctcaaagctgctgccaagg cagaaatgaacggctctcttcgactgcgcagcactgctgggtcaggtcagaagaactgacaagcagtgat cacgtgctggtgtgaacactgctgactgcgctgggtcattgaggagcgatcatcattgaataatacaacaaagcgttatcaactgatccaaa atttatcacaaatatttgtatggagacatagctgatgtggcttattctgatgtgttcaaaaatgttaaaggt agaaaactcagaatgatgttcattctaagaaaatgtcgatgaaaagcaccctgaattctcaatgcagctaaaag gaatcctttgttcaaacttttaatttattatgtcgatgaagaaaaagcacctgaattctcaatgcagctaaaag tggtcaaacttttaatttattatgtcgatgaagaaaaagcacctgaattgttgctggaattgttgtgtctggtt ctggtgttattgctgttgattgtgtctggttgatagaaggctaaagataaggagatgagatgca taggaactcaatgca |
| 357 | squirrel monkey 1-27 CD3 ε - EpCAM | artificial | aa | atggatggagctgtatcatcctctcttggtagcaacagctacactcccaggacgg taatgaagagagattggtgatactaccccagaaccacagagttccatctccaggaaccacagtaa cactgacagattacacaaggacgacgatgacaaactgcgagttctgtccgccagtcagaaagaatgt gtctgtgaaaactacaagctggccgtaaactgctttttgaatgacaatggtcaatgccagtgtac ttcgattggtgcacaaatactgtccttggagaagagagcgaaacctaaggccaagcagtgaagg cagaaatgaacggctctcttcgactgcgcagcactgctgggtcaggtcagaagaactgacaagcagtgat ggccttacgatcctgactgcgctggtcattgaataacacaagcagtgaaataacctgct cacgtgctggtgtgaacactgctgactgcgctgggtcattgaggagcgatcatcattgaataatacaacaaagcgttatcaactgct ctgagcgagtgagaactgctactgactgcgctgggtcattgaggagcgatcatcattgaataatacaacaaagcgttatcaactgct gatgttcaaagtttgctggtgacaatagctgatgtggcttattctgatgtgttgctgttgctgttgct atttatcacaaatatttgtatggagacatagctgatgtggcttattctgatgtgttcaaaaatgttcaa agaaaactcagaatgatgttcattctaagaaatgtcgatgaaatgggaacaacctgatct tggtcaaacttttaatttattatgtcgatgaagaaaaagcacctgaattgttgctggaattgttgtgtctggtt ctggtgttattgctgttgattgtgtctggttgatagaaggctaaagataaggagatgagatgca atttccagaagaagaagaatatgagagtatgcaaagctgcaaagtatgagataaagaggagatgggtgagatgca taggaactcaatgca |
| 358 | squirrel monkey 1-27 CD3 ε - EpCAM | artificial | cDNA | QDGNEEIGDTTQNPYKVSISGTTVTLTDYKDDDDKTASFAAAQKECVCENYKLAVNCFLNDNGQC QCTSIGAQNTVLCSKLAAKCLVMKAEMNGSKLGRRAKPEGALQNNDGLYDPDCDESGLFKAKQCN GTSTCWCVNTAGVRRTDKDTEITCSERVRTYWIIELKHKAREKPYDVQSLRTALEEAIKTRYQL DPKFITNILYEDNVITIDLVQNSSQKTQNDVDIADVAYYFEKDVKGESLFHSKKMDLRVNGEQLD |

| | | | | |
|---|---|---|---|---|
| | | | | LDPGQTLIYYVDEKAPEFSMQGLKAGVIAVIVVVVIAIVAGIVVLVISRKKRMAKYEKAEIKEMG EMHRELNA |
| 359 | swine 1-27 CD3 ε -EpCAM | artificial | aa | QEDIERPDEDTQKTFKVSISGDKVELTDYKDDDDKTASFAAAQKECVCENYKLAVNCFLNDNGQC QCTSIGAQNTVLCSKLAAKCLVMKAEMNGSKLGRRAKPEGALQNNDGLYDPDCDESGLFKAKQCN GTSTCWCVNTAGVRRTDKDTEITCSERVRTYWIIIELKHKAREKPYDVQSLRTALEEAIKTRYQL DPKFITNILYEDNVITIDLVQNSSQKTQNDVDIADVAYYFEKDVKGESLFHSKKMDLRVNGEQLD LDPGQTLIYYVDEKAPEFSMQGLKAGVIAVIVVVVIAIVAGIVVLVISRKKRMAKYEKAEIKEMG EMHRELNA |
| 360 | swine 1-27 CD3 ε -EpCAM | artificial | cDNA | atgggatggagctgtatcatcctcttcttggtagcaacagctacaggtgtacactcccaagaaga cattgaaagaccagatgaagatacacagaaaacatttaaagtctccatctctggagacaaagtag agctgacagattacaaggacgacgatgacaagactgcgagttttgccgcagctcagaaagaatgt gtctgtgaaaactacaagctggccgtaaactgctttttgaatgacaatggtcaatgccagtgtac ttcgattggtgcacaaaatactgtcctttgctcaaagctggctgccaaatgtttggtgatgaagg cagaaatgaacggctcaaaacttgggagaagagcgaaacctgaaggggctctccagaacaatgat ggcctttacgatcctgactgcgatgagagcgggctctttaaggccaagcagtgcaacggcacctc cacgtgctggtgtgtgaacactgctggggtcagaagaactgacaaggacactgaaataacctgct ctgagcgagtgagaacctactggatcatcattgaattaaaacacaaagcaagagaaaaaccttat gatgttcaaagtttgcggactgcacttgaggaggcgatcaaaacgcgttatcaactggatccaaa atttatcacaaatattttgtatgaggataatgttatcactattgatctggttcaaaattcttctc agaaaactcagaatgatgtggacatagctgatgtggcttattattttgaaaaagatgttaaaggt gaatccttgtttcattctaagaaaatggacctgagagtaaatggggaacaactggatctggatcc tggtcaaactttaatttattatgtcgatgaaaaagcacctgaattctcaatgcagggtctaaaag ctggtgttattctgttattgtggttgtggtgatagcaattgttgctggaattgttgtgctggtt atttccagaaagaagagaatggcaaagtatgagaaggctgagataaaggagatgggtgagatgca tagggaactcaatgca |
| 361 | human CD3 epsilon chain | artificial | aa | QDGNEEMGGITQTPYKVSISGTTVILTCPQYPGSEILWQHNDKNIGGDEDDKNIGSDEDHLSLKE FSELEQSGYYVCYPRGSKPEDANFYLYLRARVCENCMEMDVMSVATIVIVDICITGGLLLLVYYW SKNRKAKAKPVTRGAGAGGRQRGQNKERPPPVPNPDYEPIRKGQRDLYSGLNQRRI |
| 362 | human CD3 epsilon chain | artificial | cDNA | atgcagtcgggcactcactggagagttctgggcctctgcctcttatcagttggcgtttgggggca agatggtaatgaagaaatgggtggtattacacagacaccatataaagtctccatctctggaacca cagtaatattgacatgccctcagtatcctggatctgaaatactatggcaacacaatgataaaaac ataggcggtgatgaggatgataaaaacataggcagtgatgaggatcacctgtcactgaaggaatt ttcagaattggagcaaagtggttattatgtctgctaccccagaggaagcaaaccagaagatgcga actttatctctacctgagggcacgcgtgtgtgagaactgcatggagatggatgtgatgtcggtg |

EP 2 155 788 B1

| | | | | |
|---|---|---|---|---|
| | | | | gccacaattgtcatagtggacatctgcatcactgggggcttgctgctgctggtttactactggag caagaatagaaaggccaaggccaagcctgtgacacgaggagcgggtgctggcggcaggcaaaggg gacaaaacaaggagaggccaccacctgttcccaacccagactatgagcccatccggaaaggccag cgggacctgtattctggcctgaatcagagacgcatc |
| 363 | 19 amino acid immunoglobulin leader peptide | artificial | aa | MGWSCIILFLVATATGVHS |
| 364 | 19 amino acid immunoglobulin leader peptide | artificial | cDNA | atgggatggagctgtatcatcctcttcttggtagcaacagctacaggtgtacactcc |
| 365 | murine IgG1 heavy chain constant region | murine | aa | AKTTPPSVYPLAPGSAAQTNSMVTLGCLVKGYFPEPVTVTWNSGSLSSGVHTFPAVLQSDLYTLS SSVTVPSSTWPSETVTCNVAHPASSTKVDKKIVPRDCGCKPCICTVPEVSSVFIFPPKPKDVLTI TLTPKVTCVVVDISKDDPEVQFSWFVDDVEVHTAQTQPREEQFNSTFRSVSELPIMHQDWLNGKE FKCRVNSAAFPAPIEKTISKTKGRPKAPQVYTIPPPKEQMAKDKVSLTCMITDFFPEDITVEWQW NGQPAENYKNTQPIMDTDGSYFVYSKLNVQKSNWEAGNTFTCSVLHEGLHNHHTEKSLSHSPGK |
| 366 | murine IgG1 heavy chain constant region | murine | cDNA | gccaaaacgacacccccatctgtctatccactggcccctggatctgctgcccaaactaactccat ggtgaccctgggatgcctggtcaagggctatttccctgagccagtgacagtgacctggaactctg gatccctgtccagcggtgtgcacaccttccagctgtcctgcagtctgacctctacactctgagc agctcagtgactgtcccctccagcacctggcccagcgagaccgtcacctgcaacgttgcccaccc ggccagcagcaccaaggtggacaagaaaattgtgcccagggattgtggttgtaagccttgcatat gtacagtcccagaagtatcatctgtcttcatcttccccccaaagcccaaggatgtgctcaccatt actctgactcctaaggtcacgtgtgttgtggtagacatcagcaaggatgatcccgaggtccagtt cagctggtttgtagatgatgtggaggtgcacacagctcagacgcaacccggggaggagcagttca acagcactttccgctcagtcagtgaacttcccatcatgcaccaggactggctcaatggcaaggag ttcaaatgcagggtcaacagtgcagcttttccctgcccccatcgagaaaaccatctccaaaaccaa aggcagaccgaaggctccacaggtgtacaccattccacctcccaaggagcagatggccaaggata aagtcagtctgacctgcatgataacagacttcttccctgaagacattactgtggagtggcagtgg aatgggcagccagcggagaactacaagaacactcagcccatcatggacacagatggctcttactt cgtctacagcaagctcaatgtgcagaagagcaactgggaggcaggaaatactttcacctgctctg tgttacatgagggcctgcacaaccaccatactgagaagagcctctcccactctcctggtaaa |
| 367 | human lambda light chain constant region | human | aa | GQPKAAPSVTLFPPSSEELQANKATLVCLISDFYPGAVTVAWKADSSPVKAGVETTTPSKQSNNK YAASSYLSLTPEQWKSHKSYSCQVTHEGSTVEKTVAPTECS |

| 368 | human lambda light chain constant region | human | cDNA | ggtcagcccaaggctgccccctcggtcactctgttcccaccctcctctgaggagcttcaagccaa caaggccacactggtgtgtctcataagtgacttctacccgggagccgtgacagtggcctggaagg cagatagcagccccgtcaaggcgggagtggagaccaccacaccctccaaacaaagcaacaacaag tacgcggccagcagctacctgagcctgacgcctgagcagtggaagtcccacaaaagctacagctg ccaggtcacgcatgaagggagcaccgtggagaagacagtggcccctacagaatgttca |
|-----|-----|-----|-----|-----|
| 369 | human EGFR | human | aa | LEEKKVCQGTSNKLTQLGTFEDHFLSLQRMFNNCEVVLGNLEITYVQRNYDLSFLKTIQEVAGYV LIALNTVERIPLENLQIIRGNMYYENSYALAVLSNYDANKTGLKELPMRNLQEILHGAVRFSNNP ALCNVESIQWRDIVSSDFLSNMSMDFQNHLGSCQKCDPSCPNGSCWGAGEENCQKLTKIICAQQC SGRCRGKSPSDCCHNQCAAGCTGPRESDCLVCRKFRDEATCKDTCPPLMLYNPTTYQMDVNPEGK YSFGATCVKKCPRNYVVTDHGSCVRACGADSYEMEEDGVRKCKKCEGPCRKVCNGIGIGEFKDSL SINATNIKHFKNCTSISGDLHILPVAFRGDSFTHTPPLDPQELDILKTVKEITGFLLIQAWPENR TDLHAFENLEIIRGRTKQHGQFSLAVVSLNITSLGLRSLKEISDGDVIISGNKNLCYANTINWKK LFGTSGQKTKIISNRGENSCKATGQVCHALCSPEGCWGPEPRDCVSCRNVSRGRECVDKCNLLEG EPREFVENSECIQCHPECLPQAMNITCTGRGPDNCIQCAHYIDGPHCVKTCPAGVMGENNTLVWK YADAGHVCHLCHPNCTYGCTGPGLEGCPTNGPKIPSIATGMVGALLLLLVVALGIGLFMRRRHIV RKRTLRRLLQERELVEPLTPSGEAPNQALLRILKETEFKKIKVLGSGAFGTVYKGLWIPEGEKVK IPVAIKELREATSPKANKEILDEAYVMASVDNPHVCRLLGICLTSTVQLITQLMPFGCLLDYVRE HKDNIGSQYLLNWCVQIAKGMNYLEDRRLVHRDLAARNVLVKTPQHVKITDFGLAKLLGAEEKEY HAEGGKVPIKWMALESILHRIYTHQSDVWSYGVTVWELMTFGSKPYDGIPASEISSILEKGERLP QPPICTIDVYMIMVKCWMIDADSRPKFRELIIEFSKMARDPQRYLVIQGDERMHLPSPTDSNFYR ALMDEEDMDDVVDADEYLIPQQGFFSSPSTSRTPLLSSLSATSNNSTVACIDRNGLQSCPIKEDS FLQRYSSDPTGALTEDSIDDTFLPVPEYINQSVPKRPAGSVQNPVYHNQPLNPAPSRDPHYQDPH STAVGNPEYLNTVQPTCVNSTFDSPAHWAQKGSHQISLDNPDYQQDFFPKEAKPNGIFKGSTAEN AEYLRVAPQSSEFIGA |
| 370 | human EGFR | human | cDNA | atgcgaccctccgggacggccgggggcagcgctcctggcgctgctggctgcgctctgcccggcgag tcgggctctggaggaaaagaaagtttgccaaggcacgagtaacaagctcacgcagttgggcactt ttgaagatcattttctcagcctccagaggatgttcaataactgtgaggtggtccttgggaatttg gaaattacctatgtgcagaggaattatgatctttccttcttaaagaccatccaggaggtggctgg ttatgtcctcattgccctcaacacagtggagcgaattcctttggaaaacctgcagatcatcagag gaaatatgtactacgaaaattcctatgccttagcagtcttatctaactatgatgcaaataaaacc ggactgaaggagctgcccatgagaaatttacaggaaatcctgcatggcgccgtgcggttcagcaa caaccctgccctgtgcaacgtggagagcatccagtggcgggacatagtcagcagtgactttctca gcaacatgtcgatggacttccagaaccacctgggcagctgccaaaagtgtgatccaagctgtccc aatgggagctgctggggtgcaggagaggagaactgccagaaactgaccaaaatcatctgtgccca gcagtgctccgggcgctgccgtggcaagtcccccagtgactgctgccacaaccagtgtgctgcag |

gctgcacaggcccccgggagagcgactgcctggtctgccgcaaattccgagacgaagccacgtgc
aaggacacctgcccccactcatgctctacaaccccaccacgtaccagatggatgtgaaccccga
gggcaaatacagctttggtgccacctgcgtgaagaagtgtccccgtaattatgtggtgacagatc
acggctcgtgcgtccgagcctgtggggccgacagctatgagatggaggaagacggcgtccgcaag
tgtaagaagtgcgaagggccttgccgcaaagtgtgtaacggaataggtattggtgaatttaaaga
ctcactctccataaatgctacgaatattaaacacttcaaaaactgcacctccatcagtggcgatc
tccacatcctgccggtggcatttaggggtgactccttcacacatactcctcctctggatccacag
gaactggatattctgaaaaccgtaaaggaaatcacagggttttgctgattcaggcttggcctga
aaacaggacggacctccatgcctttgagaacctagaaatcatacgcggcaggaccaagcaacatg
gtcagttttctcttgcagtcgtcagcctgaacataacatccttgggattacgctccctcaaggag
ataagtgatggagatgtgataatttcaggaaacaaaaatttgtgctatgcaaatacaataaactg
gaaaaaactgtttgggacctccggtcagaaaaccaaaattataagcaacagaggtgaaaacagct
gcaaggccacaggccaggtctgccatgccttgtgctcccccgagggctgctggggccgggagccc
agggactgcgtctcttgccggaatgtcagccgaggcagggaatgcgtggacaagtgcaaccttct
ggagggtgagccaagggagtttgtggagaactctgagtgcatacagtgccacccagagtgcctgc
ctcaggccatgaacatcacctgcacaggacggggaccagacaactgtatccagtgtgcccactac
attgacggcccccactgcgtcaagacctgcccggcaggagtcatgggagaaaacaacaccctggt
ctggaagtacgcagacgccggccatgtgtgccacctgtgccatccaaactgcacctacggatgca
ctgggccaggtcttgaaggctgtccaacgaatgggcctaagatcccgtccatcgccactgggatg
gtgggggccctcctcttgctgctggtggtggccctggggatcggcctcttcatgcgaaggcgcca
catcgttcggaagcgcacgctgcggaggctgctgcaggagagggagcttgtggagcctcttacac
ccagtggagaagctcccaaccaagctctcttgaggatcttgaaggaaactgaattcaaaaagatc
aaagtgctgggctccggtgcgttcggcacggtgtataagggactctggatcccagaaggtgagaa
agttaaaattcccgtcgctatcaaggaattaagagaagcaacatctccgaaagccaacaaggaaa
tcctcgatgaagcctacgtgatggccagcgtggacaacccccacgtgtgccgcctgctgggcatc
tgcctcacctccaccgtgcagctcatcacgcagctcatgcccttcggctgcctcctggactatgt
ccgggaacacaaagacaatattggctcccagtacctgctcaactggtgtgtgcagatcgcaaagg
gcatgaactacttggaggaccgtcgcttggtgcaccgcgacctggcagccaggaacgtactggtg
aaaacaccgcagcatgtcaagatcacagattttgggctggccaaactgctgggtgcggaagagaa
agaataccatgcagaaggaggcaaagtgcctatcaagtggatggcattggaatcaattttacaca
gaatctatacccaccagagtgatgtctggagctacggggtgaccgtttgggagttgatgaccttt
ggatccaagccatatgacggaatccctgccagcgagatctcctccatcctggagaaaggagaacg
cctccctcagccacccatatgtaccatcgatgtctacatgatcatggtcaagtgctggatgatag
acgcagatagtcgcccaaagttccgtgagttgatcatcgaattctccaaaatggcccgagacccc
cagcgctaccttgtcattcaggggggatgaaagaatgcatttgccaagtcctacagactccaactt
ctaccgtgccctgatggatgaagaagacatggacgacgtggtggatgccgacgagtacctcatcc
cacagcagggcttcttcagcagccccctccacgtcacggactcccctcctgagctctctgagtgca

EP 2 155 788 B1

| 371 | artificial | cynomolgus EGF-R extracellular domain with human EGF-R transmembrane and intracellular domain | aa | LEEKKVCQGTSNKLTQLGTFEDHFLSLQRMFNNCEVVLGNLEITYVQRNYDLSFLKTIQEVAGYVLIALNTVERIPLENLQIIRGNMYYENSYALAVLSNYDANKTGLKELPMRNLQEILHGAVRFSNNPALCNVESIQWRDIVSSEFLSNMSMDFQNHLGSCQKCDPSCPNGSCWGAGEENCQKLTKIICAQQCSGRCRGKSPSDCCHNQCAAGCTGPRESDCLVCRKFRDEATCKDTCPPLMLYNPTTYQMDVNPEGKYSFGATCVKKCPRNYVVTDHGSCVRACGADSYEMEEDGVRKCKKCEGPCRKVCNGIGIGEFKDTLSINATNIKHFKNCTSISGDLHILPVAFRGDSFTHTPPLDPQELDILKTVKEITGFLLIQAWPENRTDLHAFENLEIIRGRTKQHGQFSLAVVSLNITSLGLRSLKEISDGDVIISGNKNLCYANTINWKKLFGTSSQKTKIISNRGENSCKATGQVCHALCSPEGCWGPEPRDCVSCQNVSRGRECVDKCNILEGEPREFVENSECIQCHPECLPQVMNITCTGRGPDNCIQCAHYIDGPHCVKTCPAGVMGENNTLVWKYADAGHVCHLCHPNCTYGCTGPGLEGCARNGPKIPSIATGMLGALLLLVVALGIGLFMRRRHIVRKRTLRRLLQERELVEPLTPSGEAPNQALLRILKETEFKKIKVLGSGAFGTVYKGLWIPEGEKVKIPVAIKELREATSPKANKEILDEAYVMASVDNPHVCRLLGICLTSTVQLITQLMPFGCLLDYVREHKDNIGSQYLLNWCVQIAKGMNYLEDRRLVHRDLAARNVLVKTPQHVKITDFGLAKLLGAEEKEYHAEGGKVPIKWMALESILHRIYTHQSDVWSYGVTVWELMTFGSKPYDGIPASEISSILEKGERLPQPPICTIDVYMIMVKCWMIDADSRPKFRELIIEFSKMARDPQRYLVIQGDERMHLPSPTDSNFYRALMDEEDMDDVVDADEYLIPQQGFFSSPSTSRTPLLSSLSATSNNSTVACIDRNGLQSCPIKEDSFLQRYSSDPTGALTEDSIDDTFLPVPEYINQSVKRPAGSVQNPVYHNQPLNPAPSRDPHYQDPHSTAVGNPEYLNTVQPTCVNSTFDSPAHWAQKGSHQISLDNPDYQQDFFPKEAKPNGIFKGSTAENAEYLRVAPQSSEFIGA |
| 372 | artificial | cynomolgus EGF-R extracellular domain with human EGF-R transmembrane and intracellular domain | cDNA | (cDNA sequence) |

124

```
aatgggagctgctgggtgcaggaggagaactgccagaaactgaccaaaatcatctgtgccca
gcagtgctccggcggcgccggcgccgg
gctgcacggccccccgggagagcgactgcctgtctctgccaaattccgagacgaagccacgtgc
aaggacacctgcccccactcatgctctacaacccaccacacagatggatgtgtgaacccccga
ggcaaatacagctttggtgccacctgcgcgggccgacagctatgagatgaggaagacggcgtccgcaag
acggctcgtgcgtccgagcctgcgggccaaagtgtaatgaatagtattggtgaatttaaaga
tgtaagaagtgcgaaggccttgccgacaaatattaaacacttcaaaaaactgcacactctccacacactcct
cacactctccataaatgctacaaatattaaaaactgcacactctccgcctctggatccacag
tccacatcctgccggtgcatttagggtgactcctttcacacactccgcctctcaggcttggcctga
gaactggatattctnaaaaccgtaaaggaaatcacaggtttttgctgattcaggcttggcctga
aaacaggacgacctccatgctccatgctttgagaacctgacataacgtggcaggaccaagcaacacg
gtcagtttctcttgcggtcgtcagcctgaacataacatccttgggattacgctccctcaaggag
ataagcgatggagatgtgataattcaggaacaaaacaaaattgtgctatgcaaatacaataaaactg
gaaaaaactgtttggacctccagtctgtgccatgcctttgtgctctccccgagggctgctgggccccnagccc
gcaaggccacgggccaggtctgccagaatgctctgcggtgcatataaagaggtgaaaaacagct
aggactgcgtctcctgccagaatctcgagtgcacagagaatgctgtgtggacaagtgcaacatcct
ggaggcgagccaagggagtttgtggagaactctgagtcgatacagatgtgccgcacctac
cccagtcatgaaccccactgcgctcaagaacctgccagatctccagaaaacaacaccactctggt
attgacggccccccactgctcaagaacctgccagagacgagccaggagtcatggagaaaaacaacacacctggt
ctggaagtacgcagacgccggcacgtgtcaaggacggaacggcctaagatccatccatcgccactgcatg
ctggccagtcttgaaggctgtgtgcaagctgtgctcgggaatcggcctcttcatgcgaaggcgcatg
ctggggcctcctcttgctcgtgctgtggttggcggaggctgctgcaggacgctgtttgtggagccgcgcca
catcgttcggaagcgcacgctgccaaccagctctcttgaggatcttgaaggaaactgaattcaaaaagatc
ccagtgagaagctccaaccaagctgcttcggcacgtgtgctgccgccgcagaggcttgtggagcctcttacac
aaagtgctggctccgtggctgttcggcacgtgtataaagaagactctgatccagaaggtgagaa
agttaaaattccgtctgctatcaaggaattaagagaagcaacatctccgaaagccaacaaggaaa
tcctcgatgaagctacgtgcagtgccagctcatcacgcagctcatgctcttcggcctgtgctgggcatc
tgcctcacctcaccgtgcagctcttcatcacgcagctacctgctcaactggtgtgtcagatcgggcatatgt
ccggaacacaaagacaataattggctccaagtacctgtggctgtggttgtcagccaggacgcgcca
gcatgaactacttgaaggactttggggtctgctgcggggtacgtacggggtcggggatcgcggagaaagg
aaaacaccgcagcatgtcagaggaggcaaagtgccatgcgacacgtacgggtcggggtcggaagaa
agataccagcatatgacagacagatatcacacaaggcattgtatacctgtggttggaatcaatttacaca
gaatctatacaccaccagagtgtgctggagctacgggggtacgttgtggagttgatgatgacctttt
ggatccagccatcacgtgcagatcccgtccagcgagatctccacatcatgtctctctggcggcatc
tgcctcacctcaccgtgatgacgtcagctcatcacgtacctgcagcgtctctacatgatgctcctggactatgt
ccggaacacaaagacaattattggctcccagtacctgtggctgtgttgtcagccaggacgcgcca
gcatgaactacttgaagactttgggtctgctatcaagtgcctatcaagtacgggtcggggatcgcggagaaagg
aaaacaccgcagcatgtcagaggaggcaaagtgccatgcgacacgtacgggtcggggtcggaagaa
agataccagcatatgacagacagatatcacacaaggcattgtatacctgtggttggaatcaatttacaca
gaatctatacaccaccagagtgtgctggagctacgggggtacgttgtggagttgatgatgacctttt
ggatccagccatcacgtgcagatcccgtccagcgagatctccacatcatgtctctctggcggcatc
cctccctcagccagatagtgacgaatcccatgtctaccatcatgtactacatggtctcaagtgctggatgatag
acgcagatagtcgccaccataatggccccaaagttcctgtgagttgatcatcgaattctccaaaaatgctggatatag
cagcgctaccttgtgtcattcaggggggatgaaagaatgcatttgccaagtcctacagactccaactt
```

| | | | Sequence |
|---|---|---|---|
| | | | ctaccgtgccctgatgatgaagaagacatggacgacgacgtggtggatgccgacgagtacctcatcc cacagcaggcggcttcttcaccacgtggctcttgcattgcattgatagaaatggctgcaaagctgtcccatcaagga accagcaacaattccaccgtgcctgcctgcagctcagacacagaatacataaaccagtccgttcccaaaaggccgctgacatagacg acaccttcctcccagtgctctatcacaatcagcctctgaacccccgccgccaacactgtccagcagacaccacctacccagga ccccacagcacctgcagtggcaaccccgagtatctccaacactgttccagccagctgtcaaca gcacattcgacagccgcctgccactggcccaggaagcagccaccaaattagcctggacaaccct gactaccagcaggacttcttcccaaggaagtcgcgccacaaagcagtgaattttattggagca tgaaaatgcagaataccctaaggtcgcgccacaaagcagtgaattttattggagca |
| 373 | c-terminal domain construct of human MCSP | aa | DYKDDDDKSRTRSGSQLDGGLVLFSHRGTLDGGFRFRLSDGEHTSPGHFFFRVTAQKQVLLSLKGS QTLTVCPGSVQPLSSQTLRASSSAGTDPQLLYRVVRGPQLGRLFHAQQDSTGEALVNFTQAEVY AGNILYEHEMPPEPFWEAHDTLELQLSSPPARDVAATLAVAVSFEAACPQRPSHLWKNKGLWVPE GQRARITVAALDASNLLASVPSPQRSEHDVLFQVTQFPSRGQLLVSEEPLHAGQPHFLQSQLAAG QLVYAHGGGTQQDGFHFRAHLQGPAGASVAGPQTSEAFAITVRDVNERPPQPQASVPLRLTRGS RAPISRAQLSVVDPDSAPGEIEYEVQRAPHNGFLSLVGGLGPVTRFTQADVDSGRLAFVANGSS VAGIFQLSMSDGASPPLPMSLAVDILPSAIEVQLRAPLEVPQALGRSSLSQQQLRVVSDREEPEA AYRLIQGPQYGHLLVGGRPTSAFSQFQIDQGEVVFAFTNSSSSHDHFRVLALARGVNASAVVNVT VRALLHVWAGGPWPQGATLRLDPTVLDAGELANRTDSVPRFRLLEGPRHGRVVRVPRARTEPGGS QLVEQFTQQDLEDGRLGLEVGRPEGRAPGAGDSLTLELWAQGVPPAVASLDFATEPYNAARPYS VALLSVPEAARTEAGKPESSTPTGEPGPMASSPEPAVAKGGFLSFLEANMFSVIIPMCLVLLLLA LILPLLFYLRKRNKTGKHDVQVLTAKPRNGLAGDTETFRKVEPGQAIPLTAVPGQGPPPGGQPDP ELLQFCRTPNPALKNGQYWV |
| 374 | c-terminal domain construct of human MCSP | cDNA | atgggatggagctgtatcatcctctcttcttggtagcaacagctacacactccgactacaa agacgatgacgacaagtccgtacgagatctggatcccgcttccgcctctccgacgcgagcacacttcccccc cacacagagaaccctgatggaggcttccgcctcgctcctctccgctgcgaaggggccagacact ggacactgtctgccaggtcggtcgtctcgctctaccgtggtggtggcgggggccagctccagcgcag gactgtctgccaggtcggtcgtctcgctctaccgtggtggtggcgggggccagctccagcgcag gccagccccagccaggcagcaggggaggccctgttgaacttcactcaggcagcaggtctacgctggaa tattctgtatgagcacatgagatgcccgcggctgcccagctgcgttgtctgtctttgaggct gctgtcctcgcccagcgccgcccagctgcctctgatgtctctgccagcgtctcatcaccccagc ggccaggatcaccgtgctgctctgctccttccagctgcctcaggccagctcagcccagcagctgtgtgtcc gctcagagagctgatgtgctcttccagtcacagaggttcccagcgcggccagctgttgtgtgtcc gaggagcccccactcctgggcagcccagtgctgctggctgggggccagctgtcccagttgtgtgtcc gaggagcccccactcctgggcagcccagtgctgctggctgggggccagctgtcccagttgtgtgtcc gaggagcccccactcctgggcagcccagtgctgctggctgggggccagctgtcccagctggtgtcc gaggagcccccactcctgggcagcccagtgctgctggctgggggccagctgtcccagctggtgtcc gaggagcccccactcctgggcagcccagtgctgctggctgggggccagctgtcccagctggtgtcc gaggagcccccactcctgggcagcccagtgctgctggctgggggccagctgtcccagctggtgtcc gaggagcccccactcctgggcagcccagtgctgctggctgggggccagctgtcccagctggtgtcagctagt |

| 375 | partial sequence of cynomolgus MCSP | cynomolgus | aa | |
|---|---|---|---|---|
| | | | | gtatgccccacgcggcggtggggcaccccagcaggatggcttccacttctgtgccacctccaggggc<br>cagcagggcctccgtggctggctgaccacagcctcagagcctttgccatcacggtgaggggatgta<br>aatgagcggccccctggccccagccccgagtctccactccgagctctgtgcccc<br>catctcccggcggcacccacccagctgagtcgtggtggttcctcagcgactcagctcctgggggagattgagtacgagg<br>tccagcggcaagccgatgtggattcaggcggcgcttcgtggtggttcgtggtggccaacgggagcagcgtggcagg<br>ttcacgcaagccgatgtggattcaggcggcgcttcgtgtggttcgtggtggccaaccctgagagtcgggacccgtgacccgc<br>catcttccagctgagcatgtctgatgggccagccacccctgagtcctccaagtccctgtgtggaca<br>tcctaccatccgccatcgaggtcgagctgcagcgcggtggttcgtggtggccaaccctgagagtgcccccaccg<br>tcctcactactgccaggaccagcagctccggtggtgtttcagatcgggcggcggcaccctcagcc<br>gttgatccaggaggaccccagtatggcatctcctggtggcggccaactcctcctctcatgaccac<br>aattccagatagaccaggcgagggcggtgtcttgccttcaatgcatcagccgtagtgaacgtcactgtgagggc<br>ttcagagtcctggcactggcctgctgggtcaggtgcagtggccagcgtgccacctgcctgcctccaccg<br>tctgctgcatgtgtgggcaggtgggccatgtgcccccaggtgccacctgcgcctgcctccaccg<br>tcctagagtctgggcgagctggccaaccgtggccagacagtgtgccgcgcttccgcgctcctgaggga<br>ccccggcatgccgccgtggtccgcgtgccaggacgggagctgggggctgaggtgggcaggccagag<br>ggaggcagttcactcaggacctttgagacagtcactctgagcctacactgctggccagggctcccgcct<br>gctgtgccctcccctgactttgccactgagcctacaatgctgcccggccctacagcgtggccct<br>gctcagtgtcccgagccgccgagcctgagcctgtgccaaggagccgctgcctgagcttttcta<br>gaggccaacatgttcagcgtcatcatccgaaaacgcaacaagacaccgagaccttcaggaggcaagtcctgactg<br>gccccctgtctcttacctcctccggcaaccggctgctgctgccggccaggcaggtccaggtcctgactg<br>ccaagccccaacctcacagctgtgccggctgctgccggccaggggccccctaagaatggccagtactgggtg<br>gcagttctgccgacaccccaacctgccgacaccctaagaatggccagtactgggtg | PSNGRVVLRAAPGTEVRSFTQAQLDGGLVLFSHRGTLDGGFRFGLSDGEHTSSGHFFRVTAQKQV<br>LLSLEGSRTLTVCPGSVQPLSSQTLRASSSAGTDPQLLLYRVVRGPQLGRLFHAQQDSTGEALVN<br>FTQAEVYAGNILYEHEMPTEPFWEAHDTLELQLSSPPARDVAATLAVAVSFEAACPQRPSHLWKN<br>KGLWVPEGQRAKITMAALDASNLLASVPSSQRLEHDVLFQVTQFPSRGQLLVSEEPLHAGQPHFL<br>QSQLAAGQLVYAHGGGTQQDGFHFRAHLQGPAGATVAGPQTSEAFAITVRDVNERPPQPQASVP<br>LRITRGSRAPISRAQLSVVDPDSAPGEIEYEVQRAPHNGFLSLVGGGPVNRFTQADVDSGRLA<br>FVANGSSVAGVFQLSMSDGASPPLPMSLAVDILPSAIEVQLQAPLEVPQALGRSSLSQQQLRVVS<br>DREEPEAAYRLIQGPKYGHLLVGGQPASAFSQLQIDQGEVVFAFTNFSSSHDHFRVLALARGVNA<br>SAVVNITVRALLHVWAGGPWPQGATLRLDPTILDAGELANRTGSVPRFRLLEGPRHGRVVRVPRA<br>RMEPGGSQLVEQFTQQDLEDGRLGLEVGRPEGRAPSPTGDSLTLELWAQGVPPAVASLDFATEPY<br>NAARPYSVALLSVPEATRTEAGKPESSTPTGEPGPMASSPVPAVAKGGFLGFLEANMFSVIIPXC |

| 376 | partial sequence of cynomolgus MCSP | cynomolgus | cDNA | LVLLLLALILPLLFYLRKRNKTGKHDVQVLTAKPRNGLAGDTETFRKVEPGQAIPLTAVPGQGPP PGGQPDPELLQFCRTPNPALKNGQYWV |
|---|---|---|---|---|

cccagcaacgacgggtagtgctgctgctgggcggccggcaccgaggtgcgcagcttcacgcgcaggc
ccagctggatggccggactgctgctgttctcacacagagacccctggatggaggcttccgcttcg
gcctctccgatggcgagcacacttccctggacacttcttcgccaggtgacggcccagaagcaagtg
ctcctctcgctggagggcagccgacactgactgtctgcccaggtccgtcgctcctaccgtgtggtgc
tcagaccctcagagctcagctcctgttccatgccagcagaccagcagcaggggaggccctgtgaac
gggccccagctaggccggctgttccagatatctggaatattctgtatgagcatgagatgcccagcctt
ttcactcaggcagaggtctatgctggagctccagctgtcctcacccagcgcccacccagctggaagaac
ctggaggcccatgataccctagagctccagctgtcctgtgtccctgtgcctgcctgcca
cccttgctgctgctgtctctttgaggctgcagcggccagcggccaagatcaccatggctgccctgatgctcctccaa
aaagtctctggccagcgttccatcatccagcgcctagagcaccatgatgtgtcttccagtcacgcagt
cctcttgccagccgggccagctattggtgtctgaggagcccctccacggcgtgggggtaccccacaaacctcctg
tccccagccgggccagctgctgcaggcagctagtgtatgcccagcaggggccaccgtgctggacccccaaacctcag
cttccactttcgtgccacctcgcagggccagcggtaaatgagcggccacaactctcccca
agctttgccatcacggtgcgggatgtaaatgagcggccccatctcccgcgggcaccagcctgacccaga
ctccggatcacccgagctctcgagccccatctcccgcgggcacccaacggcttcctcagcctgg
ctcagctcctgggagattgagtatgaggtcagaccgcttcagccaagccgatgtgattcggggcggctggcc
tgggtggtggccaacggggccgtgaaccgcttcacgcaagccgttcttcaccggcgtgtggattcggggcggctggcc
ttcgtggccaacggagcagcgtagcaggcgtcttccagctgacatgtctgagcatgtctgatgggccagccc
accgctgccacatgtccctggccctgctggacatcctaccatccgcctgagcagccatccagcaggcac
ccctggaggtgcccccaagctttgggccgctctcagctcatccaggaccagaaagtacggccatctcctggt
gggtggggcagcccgcctcggccttcagctccctctcatgaccacacttcagagtcctggcactggctaggggtgtcaacgca
tcaccaacttctcctctcatgaccacactttcagagtcctgcacgttgctctgctgccatgacccca
tcagccgtagtgaacatcactgtgagccctggccctgctggacatcctagatgctggcgagctggccatgacccca
gggtgctaccctgcgcctccgcctcctgagggacccaccatctctagatgctggcgagctggcacaaccgcacaggca
gtgtgccccgcgcttccgctcctctgagggaccccggcatgccgccgtcctgtgtccgtgcccgagcc
aggatggagcctgggggcagccagtcggtggagcagttcactcagcaggcagttgaggatgggag
gctgggcggctgtgagagcagcacccccacagggccagcggtgcctgcctcagtgtcccactc
tggagctgtgtggcacaggccgtgccccacaggcgtcccacctgctggcttccttgccactgagccttac
aatgctctgcccctgctgaacatgttcctggccaacatgttcagtgtcatccccagccagagcca
gaagccagagcagcagcaccccacaggagccccgagccaccctagccctgtgcctgtctg
tggccaagggaggcttcctctgctgcgctcatcttgccctgagcctcatcatcccccrtgtgc
ctggtcctctgctcctgccgctcattctgcccctgctccttctctacttcctcacctccgaaaacgcaacaagac

| | | | | |
|---|---|---|---|---|
| | | | | gggcaagcatgacgtccaggtcctgactgccaagccccgcaatggtctggctggtgacactgaga<br>cctttcgcaaggtggagccaggccaggccatcccgctcacagctgtgcctggccaggggccccct<br>ccgggaggccagcctgacccagagctgctgcagttctgccggacacccaaccctgcccttaagaa<br>tggccagtactgggtg |
| 377 | PCR primer for CD3ε chain - forward primer | artificial | DNA | AGAGTTCTGGGCCTCTGC |
| 378 | PCR primer for CD3ε chain - reverse primer | artificial | DNA | CGGATGGGCTCATAGTCTG |
| 379 | His6-human CD3ε | artificial | aa | HHHHHHQDGNEEMGGITQTPYKVSISGTTVILTCPQYPGSEILWQHNDKNIGGDEDDKNIGSDED<br>HLSLKEFSELEQSGYYVCYPRGSKPEDANFYLYLRARVCENCMEMDVMSVATIVIVDICITGGLL<br>LLVYYWSKNRKAKAKPVTRGAGAGGRQRGQNKERPPPVPNPDYEPIRKGQRDLYSGLNQRRI |
| 380 | His6-human CD3ε | artificial | cDNA | atgggatggagctgtatcatcctcttcttggtagcaacagctacaggtgtacactcccatcatca<br>ccatcatcatcaagatggtaatgaagaaatgggtggtattacacagacaccatataaagtctcca<br>tctctggaaccacagtaatattgacatgccctcagtatcctggatctgaaatactatggcaacac<br>aatgataaaaacataggcggtgatgaggatgataaaaacataggcagtgatgaggatcacctgtc<br>actgaaggaattttcagaattggagcaaagtggttattatgtctgctaccccagaggaagcaaac<br>cagaagatgcgaacttttatctctacctgagggcacgcgtgtgtgagaactgcatggagatggat<br>gtgatgtcggtggccacaattgtcatagtggacatctgcatcactggggggcttgctgctgctggt<br>ttactactggagcaagaatagaaaggccaaggccaagcctgtgacacgaggagcgggtgctggcg<br>gcaggcaaaggggacaaaacaaggagaggccaccacctgttcccaacccagactatgagcccatc<br>cggaaaggccagcgggacctgtattctggcctgaatcagagacgcatc |
| 381 | VH of EGFR | artificial | aa | QVQLQESGPGLVKPSETLSLTCTVSGGSVSSGDYYWTWIRQSPGKGLEWIGHIYYSGNTNYNPSL<br>KSRLTISIDTSKTQFSLKLSSVTAADTAIYYCVRDRVTGAFDIWGQGTMVTVSS |
| 382 | VH of EGFR | artificial | nt | CAGGTGCAGCTGCAGGAGTCGGGCCCAGGACTGGTGAAGCCTTCGGAGACCCTGTCCCTCACCTG<br>CACTGTCTCTGGTGGCTCCGTCAGCAGTGGTGATTACTACTGGACCTGGATCCGGCAGTCCCCAG<br>GGAAGGGACTGGAGTGGATTGGACACATCTATTACAGTGGGAACACCAATTATAACCCCTCCCTC<br>AAGAGCCGACTCACCATATCAATTGACACGTCCAAGACTCAGTTCTCCCTGAAGCTGAGTTCTGT<br>GACCGCTGCGGACACGGCCATTTATTACTGTGTGCGAGATCGAGTGACTGGTGCTTTTGATATCT<br>GGGGCCAAGGGACAATGGTCACCGTCTCTTCC |
| 383 | VL of EGFR | artificial | aa | DIQMTQSPSSLSASVGDRVTITCQASQDISNYLNWYQQKPGKAPKLLIYDASNLETGVPSRFSGS |

EP 2 155 788 B1

| | | | | |
|---|---|---|---|---|
| | | | | GSGTDFTFTISSLQPEDIATYFCQHFDHLPLAFGGGTKVEIK |
| 384 | VL of EGFR | artificial | nt | GACATCCAGATGACCCAGTCTCCATCCTCCCTGTCTGCATCTGTCGGAGACAGAGTCACCATCAC TTGCCAGGCGAGTCAGGACATCAGCAACTATTTAAATTGGTATCAGCAGAAACCAGGGAAAGCCC CTAAACTCCTGATCTACGATGCATCCAATTTGGAAACAGGGGTCCCATCAAGGTTCAGTGGAAGT GGATCTGGGACAGATTTTACTTTCACCATCAGCAGCCTGCAGCCTGAAGATATTGCAACATATTT CTGTCAACACTTTGATCATCTCCCGCTCGCTTTCGGCGGAGGGACCAAGGTGGAGATCAAA |
| 385 | VH-VL of EGFR | artificial | aa | QVQLQESGPGLVKPSETLSLTCTVSGGSVSSGDYYWTWIRQSPGKGLEWIGHIYYSGNTNYNPSL KSRLTISIDTSKTQFSLKLSSVTAADTAIYYCVRDRVTGAFDIWGQGTMVTVSSGGGGSGGGGSG GGGSDIQMTQSPSSLSASVGDRVTITCQASQDISNYLNWYQQKPGKAPKLLIYDASNLETGVPSR FSGSGSGTDFTFTISSLQPEDIATYFCQHFDHLPLAFGGGTKVEIK |
| 386 | VH-VL of EGFR | artificial | nt | CAGGTGCAGCTGCAGGAGTCGGGCCCAGGACTGGTGAAGCCTTCGGAGACCCTGTCCCTCACCTG CACTGTCTCTGGTGGCTCCGTCAGCAGTGGTGATTACTACTGGACCTGGATCCGGCAGTCCCCAG GGAAGGGACTGGAGTGGATTGGACACATCTATTACAGTGGGAACACCAATTATAACCCCTCCCTC AAGAGCCGACTCACCATATCAATTGACACGTCCAAGACTCAGTTCTCCCTGAAGCTGAGTTCTGT GACCGCTGCGGACACGGCCATTTATTACTGTGTGCGAGATCGAGTGACTGGTGCTTTTGATATCT GGGGCCAAGGGACAATGGTCACCGTCTCTTCCGGTGGTGGTGGTTCTGGCGGCGGCGGCTCCGGT GGTGGTGGTTCTGACATCCAGATGACCCAGTCTCCATCCTCCCTGTCTGCATCTGTCGGAGACAG AGTCACCATCACTTGCCAGGCGAGTCAGGACATCAGCAACTATTTAAATTGGTATCAGCAGAAAC CAGGGAAAGCCCCTAAACTCCTGATCTACGATGCATCCAATTTGGAAACAGGGGTCCCATCAAGG TTCAGTGGAAGTGGATCTGGGACAGATTTTACTTTCACCATCAGCAGCCTGCAGCCTGAAGATAT TGCAACATATTTCTGTCAACACTTTGATCATCTCCCGCTCGCTTTCGGCGGAGGGACCAAGGTGG AGATCAAA |
| 387 | VL-VH of EGFR | artificial | aa | DIQMTQSPSSLSASVGDRVTITCQASQDISNYLNWYQQKPGKAPKLLIYDASNLETGVPSRFSGS GSGTDFTFTISSLQPEDIATYFCQHFDHLPLAFGGGTKVEIKGGGGSGGGGSGGGGSQVQLQESG PGLVKPSETLSLTCTVSGGSVSSGDYYWTWIRQSPGKGLEWIGHIYYSGNTNYNPSLKSRLTISI DTSKTQFSLKLSSVTAADTAIYYCVRDRVTGAFDIWGQGTMVTVSS |
| 388 | VL-VH of EGFR | artificial | nt | GACATCCAGATGACCCAGTCTCCATCCTCCCTGTCTGCATCTGTCGGAGACAGAGTCACCATCAC TTGCCAGGCGAGTCAGGACATCAGCAACTATTTAAATTGGTATCAGCAGAAACCAGGGAAAGCCC CTAAACTCCTGATCTACGATGCATCCAATTTGGAAACAGGGGTCCCATCAAGGTTCAGTGGAAGT GGATCTGGGACAGATTTTACTTTCACCATCAGCAGCCTGCAGCCTGAAGATATTGCAACATATTT CTGTCAACACTTTGATCATCTCCCGCTCGCTTTCGGCGGAGGGACCAAGGTGGAGATCAAAGGTG GTGGTGGTTCTGGCGGCGGCGGCTCCGGTGGTGGTGGTTCTCAGGTGCAGCTGCAGGAGTCGGGC CCAGGACTGGTGAAGCCTTCGGAGACCCTGTCCCTCACCTGCACTGTCTCTGGTGGCTCCGTCAG CAGTGGTGATTACTACTGGACCTGGATCCGGCAGTCCCCAGGGAAGGGACTGGAGTGGATTGGAC |

| | | | | |
|---|---|---|---|---|
| | | | | ACATCTATTACAGTGGGAACACCAATTATAACCCTCCCTCAAGAGCCGACTCACCATATCAATTGACACGTCTCCAAGACTCAGTTCTCCTGAAGCTGAGTTCTGTGACCGCTGCGGACACGGCCATTTATTACTGTGCGAGATCGAGTCGAGTGCTTTGATATCTGGGGCCAAGGGACAATGGTCACCGTCTCTTCC |
| 389 | EGFR VH-VL x I2C VH VL | artificial | aa | QVQLQESGPGLVKPSETLSLTCTVSGGSVSSGDYYWTWIRQSPGKGLEWIGHIYYSGNTNYNPSLKSRLTISIDTSKTQFSLKLSSVTAADTAIYYCVRDRVTGAFDIWGQGTMVTVSSGGGGSGGGGSGGGSDIQMTQSPSSLSASVGDRVTITCQASQDISNYLNWYQQKPGKAPKLLIYDASNLETGVPSRFSGSGSGTDFTFTISSLQPEDIATYFCQHFDHLPLAFGGGTKVEIKGGGSEVQLVESGGGLVQPGGSLKLSCAASGFTFNKYAMNWVRQAPGKGLEWVARIRSKYNNYATYYADSVKDRFTISRDDSKNTAYLQMNNLKTEDTAVYYCVRHGNFGNSYISYWAYWGQGTLVTVSSGGGGSGGGGSGGGGSQTVVTQEPSLTVSPGGTVTLTCGSSTGAVTSGNYPNWVQQKPGQAPRGLIGGTKFLAPGTPARFSGSLLGGKAALTLSGVQPEDEAEYYCVLWYSNRWVFGGGTKLTVL |
| 390 | EGFR VH-VL x I2C VH VL | artificial | nt | CAGGTGCAGCTGCAGGAGTCGGGCCCAGGACTGGTGGAGCCTTCGGAGACCCTGTCCCTCACCTGCACTGTCTCTGGTGGCTCCGTCAGCAGTGGTGATTACTACTGGACCTGGATCCGGCAGTCCCCAGGGAAGGGACTGGAGTGGATTGGACACATCTATTACAGTGGGAACACCAATTATAACCCTCCCTCAAGAGCCGACTCAGTTCTCCCTGAAGCTGAGTTCTGTGACCGCTGCGGACACGGCCGTTTATTACTGTGTGCGAGATCGAGTCGAGTGGGTTCTGGCGGCGTCCGGTGGGCCAAGGGACAATGGTCACCGTCTCTTCCGGTGGTGGTGGTTCTGGCGGCGGCGGATCTGGTGGTGGTGGTTCTGGCGGCGGCGGATCCGGAGGCAGTCAGGACATCAGCGAGTCCTGATCTACGATGCATCCAATTTAAATTGGTATCAGCAGAAACCAGGGAAAGCCCCTAAACTCCTGATCTACGATGCATCCAATTTGGAAACAGGGGTCCCATCAAGGTTCAGTGGAAGTGGATCTGGGACAGATTTTACTTTCACCATCAGCAGCCTGCAGCCTGAAGATATTGCAACATATTTCTGTCAACACTTTGATCATCTCCCGCTCGCTTTCGGCGGAGGGACCAAGGTGGAGATCAAAGGAGGTGGTGGATCTGGAGGTGGTGGTTCAGGCGGAGGTGGCTCAGGTGGAGGTGGATCTGAGGTTCAATTGGTGGAGTCTGGGGGAGGCTTGGTCCAGCCTGGAGGGTCCCTGAAACTCTCCTGTGCAGCCTCTGGATTCACTTTCAATAAGTACGCCATGAACTGGGTCCGCCAGGCTCCAGGGAAGGGGTTGGAATGGGTTGCTCGCATAAGAAGTAAATATAATAATTATGCAACATATTATGCCGATTCAGTGAAGGACAGATTCACCATCTCCAGAGATGATTCAAAAAACACTGCCTATCTCCAACAAATGAACAACCTGAAAGACTGAGGACACTGCCGTGTACTACTGTGTGAGACATGGGAACTTCGGTAATAGCTACATATCCTACTGGGCGTGGGCCTCCGGGGCTGGGCCTCGGTTCTGGGCTGTGGTGTTCTCAGGGTTGGTGGTGGTTCTCAGACTGTGTGACTCAGGAACCTTCACTCTGTGGCTCCTCGACTGGGGCTGTTACATCTGGCAACTCTGGGCTGCAACTACCCAAACTGGTCCAACAAACCAGGTCAGGCTCCACGGGGTCTAATAGGTGGGACTAAGTTCCTCGCCCCTCACCCCTCTCAGGGTGGTACAGCCGCTGGGTGTTCGGTGGTGGTTCTCAGACTGTGTGTCTATGTGGTACAGCCAACCGCTGGGTGTTCGGTGGTGGGACTAAGCTCACCGTCCTA |

| 391 | EGFR VL-VH x I2C VH VL | artificial | aa | DIQMTQSPSSLSASVGDRVTITCQASQDISNYLNWYQQKPGKAPKLLIYDASNLETGVPSRFSGS GSGTDFTFTISSLQPEDIATYFCQHFDHLPLAFGGGTKVEIKGGGGSGGGGSGGGGSQVQLQESG PGLVKPSETLSLTCTVSGGSVSSGDYYWTWIRQSPGKGLEWIGHIYYSGNTNYNPSLKSRLTISI DTSKTQFSLKLSSVTAADTAIYYCVRDRVTGAFDIWGQGTMVTVSSGGGGSEVQLVESGGGLVQP GGSLKLSCAASGFTFNKYAMNWVRQAPGKGLEWVARIRSKYNNYATYYADSVKDRFTISRDDSKN TAYLQMNNLKTEDTAVYYCVRHGNFGNSYISYWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |
| 392 | EGFR VL-VH x I2C VH VL | artificial | nt | GACATCCAGATGACCCAGTCTCCATCCTCCCTGTCTGCATCTGTCGGAGACAGAGTCACCATCAC TTGCCAGGCGAGTCAGGACATCAGCAACTATTTAAATTGGTATCAGCAGAAACCAGGGAAAGCCC CTAAACTCCTGATCTACGATGCATCCAATTTGGAAACAGGGGTCCCATCAAGGTTCAGTGGAAGT GGATCTGGGACAGATTTTACTTTCACCATCAGCAGCCTGCAGCCTGAAGATATTGCAACATATTT CTGTCAACACTTTGATCATCTCCCGCTCGCTTTCGGCGGAGGGACCAAGGTGGAGATCAAAGGTG GTGGTGGTTCTGGCGGCGGCGGCTCCGGTGGTGGTGGTTCTCAGGTGCAGCTGCAGGAGTCGGGC CCAGGACTGGTGAAGCCTTCGGAGACCCTGTCCCTCACCTGCACTGTCTCTGGTGGCTCCGTCAG CAGTGGTGATTACTACTGGACCTGGATCCGGCAGTCCCCAGGGAAGGGACTGGAGTGGATTGGAC ACATCTATTACAGTGGGAACACCAATTATAACCCCTCCCTCAAGAGCCGACTCACCATATCAATT GACACGTCCAAGACTCAGTTCTCCCTGAAGCTGAGTTCTGTGACCGCTGCGGACACGGCCATTTA TTACTGTGTGCGAGATCGAGTGACTGGTGCTTTTGATATCTGGGGCCAAGGGACAATGGTCACCG TCTCTTCCGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAAGTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGACAGGTTCACCATCTCCAGAGATGATTCAAAAAAC ACTGCCTATCTACAAATGAACAACTTGAAGACTGAGGACACTGCCGTGTACTACTGTGTGAGACA TGGGAACTTCGGTAATAGCTACATATCCTACTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCAACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGT TCTATGGTACAGCAACCGCTGGGTGTTCGGTGGAGGAACCAAACTGACTGTCCTA |
| 393 | EGFR VH-VL x F12Q VH VL | artificial | aa | QVQLQESGPGLVKPSETLSLTCTVSGGSVSSGDYYWTWIRQSPGKGLEWIGHIYYSGNTNYNPSL KSRLTISIDTSKTQFSLKLSSVTAADTAIYYCVRDRVTGAFDIWGQGTMVTVSSGGGGSGGGGSG GGGSDIQMTQSPSSLSASVGDRVTITCQASQDISNYLNWYQQKPGKAPKLLIYDASNLETGVPSR FSGSGSGTDFTFTISSLQPEDIATYFCQHFDHLPLAFGGGTKVEIKGGGGSEVQLVESGGGLVQP |

EP 2 155 788 B1

| | | | | |
|---|---|---|---|---|
| | | | | GGSLKLSCAASGFTFNSYAMNWVRQAPGKGLEWVARIRSKYNNYATYYADSVKGRFTISRDDSKN TAYLQMNNLKTEDTAVYYCVRHGNFGNSYVSWWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |
| 394 | EGFR VH-VL x F12Q VH VL | artificial | nt | CAGGTGCAGCTGCAGGAGTCGGGCCCAGGACTGGTGAAGCCTTCGGAGACCCTGTCCCTCACCTG CACTGTCTCTGGTGGCTCCGTCAGCAGTGGTGATTACTACTGGACCTGGATCCGGCAGTCCCCAG GGAAGGGACTGGAGTGGATTGGACACATCTATTACAGTGGGAACACCAATTATAACCCCTCCCTC AAGAGCCGACTCACCATATCAATTGACACGTCCAAGACTCAGTTCTCCCTGAAGCTGAGTTCTGT GACCGCTGCGGACACGGCCATTTATTACTGTGTGCGAGATCGAGTGACTGGTGCTTTTGATATCT GGGGCCAAGGGACAATGGTCACCGTCTCTTCCGGTGGTGGTGGTTCTGGCGGCGGCGGCTCCGGT GGTGGTGGTTCTGACATCCAGATGACCCAGTCTCCATCCTCCCTGTCTGCATCTGTCGGAGACAG AGTCACCATCACTTGCCAGGCGAGTCAGGACATCAGCAACTATTTAAATTGGTATCAGCAGAAAC CAGGGAAAGCCCCTAAACTCCTGATCTACGATGCATCCAATTTGGAAACAGGGGTCCCATCAAGG TTCAGTGGAAGTGGATCTGGGACAGATTTTACTTTCACCATCAGCAGCCTGCAGCCTGAAGATAT TGCAACATATTTCTGTCAACACTTTGATCATCTCCCGCTCGCTTTCGGCGGAGGGACCAAGGTGG AGATCAAAGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAGCTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGGCAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACGTTTCCTGGTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCAACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGT TCTATGGTACAGCAACCGCTGGGTGTTCGGTGGAGGAACCAAACTGACTGTCCTA |
| 395 | EGFR VL-VH x F12Q VH VL | artificial | aa | DIQMTQSPSSLSASVGDRVTITCQASQDISNYLNWYQQKPGKAPKLLIYDASNLETGVPSRFSGS GSGTDFTFTISSLQPEDIATYFCQHFDHLPLAFGGGTKVEIKGGGGSGGGGSGGGGSQVQLQESG PGLVKPSETLSLTCTVSGGSVSSGDYYWTWIRQSPGKGLEWIGHIYYSGNTNYNPSLKSRLTISI DTSKTQFSLKLSSVTAADTAIYYCVRDRVTGAFDIWGQGTMVTVSSGGGGSEVQLVESGGGLVQP GGSLKLSCAASGFTFNSYAMNWVRQAPGKGLEWVARIRSKYNNYATYYADSVKGRFTISRDDSKN TAYLQMNNLKTEDTAVYYCVRHGNFGNSYVSWWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |

EP 2 155 788 B1

| 396 | EGFR VL-VH x F12Q VH VL | artificial | nt | GACATCCAGATGACCCAGTCTCCATCCTCCCTGTCTGCATCTGTCGGAGACAGAGTCACCATCAC TTGCCAGGCGAGTCAGGACATCAGCAACTATTTAAATTGGTATCAGCAGAAACCAGGGAAAGCCC CTAAACTCCTGATCTACGATGCATCCAATTTGGAAACAGGGGTCCCATCAAGGTTCAGTGGAAGT GGATCTGGGACAGATTTTACTTTCACCATCAGCAGCCTGCAGCCTGAAGATATTGCAACATATTT CTGTCAACACTTTGATCATCTCCCGCTCGCTTTCGGCGGAGGGACCAAGGTGGAGATCAAAGGTG GTGGTGGTTCTGGCGGCGGCGGCTCCGGTGGTGGTGGTTCTCAGGTGCAGCTGCAGGAGTCGGGC CCAGGACTGGTGAAGCCTTCGGAGACCCTGTCCCTCACCTGCACTGTCTCTGGTGGCTCCGTCAG CAGTGGTGATTACTACTGGACCTGGATCCGGCAGTCCCCAGGGAAGGGACTGGAGTGGATTGGAC ACATCTATTACAGTGGGAACACCAATTATAACCCCTCCCTCAAGAGCCGACTCACCATATCAATT GACACGTCCAAGACTCAGTTCTCCCTGAAGCTGAGTTCTGTGACCGCTGCGGACACGGCCATTTA TTACTGTGTGCGAGATCGAGTGACTGGTGCTTTTGATATCTGGGGCCAAGGGACAATGGTCACCG TCTCTTCCGGAGGTGGTGGATCCGAGGTGCAGCTGGTCGAGTCTGGAGGAGGATⓉGGTGCAGCCT GGAGGGTCATTGAAACTCTCATGTGCAGCCTCTGGATTCACCTTCAATAGCTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGGCAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACGTTTCCTGGTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCAACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGᴀTGAGGCAGAATATTACTGTGT TCTATGGTACAGCAACCGCTGGGTGTTCGGTGGAGGAACCAAACTGACTGTCCTA |
| 397 | EGFR VH-VL x H2C VH VL | artificial | aa | QVQLQESGPGLVKPSETLSLTCTVSGGSVSSGDYYWTWIRQSPGKGLEWIGHIYYSGNTNYNPSL KSRLTISIDTSKTQFSLKLSSVTAADTAIYYCVRDRVTGAFDIWGQGTMVTVSSGGGGSGGGGSG GGGSDIQMTQSPSSLSASVGDRVTITCQASQDISNYLNWYQQKPGKΑPKLLIYDASNLETGVPSR FSGSGSGTDFTFTISSLQPEDIATYFCQHFDHLPLAFGGGTKVEIKGGGGSEVQLVESGGGLVQP GGSLKLSCAASGFTFNKYAMNWVRQAPGKGLEWVARIRSKYNNYATYYADSVKDRFTISRDDSKN TAYLQMNNLKTEDTAVYYCVRHGNFGNSYISYWAYWGQGTLVTVSSGGGGSGGGGSGGGGSQTVV TQEPSLTVSPGGTVTLTCGSSTGAVTSGYYPNWVQQKPGQAPRGLIGGTKFLAPGTPARFSGSLL GGKAALTLSGVQPEDEAEYYCALWYSNRWVFGGGTKLTVL |
| 398 | EGFR VH-VL x H2C VH VL | artificial | nt | CAGGTGCAGCTGCAGGAGTCGGGCCCAGGACTGGTGAAGCCTTCGGAGACCCTGTCCCTCACCTG CACTGTCTCTGGTGGCTCCGTCAGCAGTGGTGATTACTACTGGACCTGGATCCGGCAGTCCCCAG GGAAGGGACTGGAGTGGATTGGACACATCTATTACAGTGGGAACACCAATTATAACCCCTCCCTC AAGAGCCGACTCACCATATCAATTGACACGTCCAAGACTCAGTTCTCCCTGAAGCTGAGTTCTGT |

| | | | | GACCGCTGCGGACACGGCCATTTATTACTGTGTGCGAGATCGAGTGACTGGTGCTTTTGATATCT<br>GGGGCCAAGGGACAATGGTCACCGTCTCTTCCGGTGGTGGTGGTTCTGGCGGCGGCGGCTCCGGT<br>GGTGGTGGTTCTGACATCCAGATGACCCAGTCTCCATCCTCCCTGTCTGCATCTGTCGGAGACAG<br>AGTCACCATCACTTGCCAGGCGAGTCAGGACATCAGCAACTATTTAAATTGGTATCAGCAGAAAC<br>CAGGGAAAGCCCCTAAACTCCTGATCTACGATGCATCCAATTTGGAAACAGGGGTCCCATCAAGG<br>TTCAGTGGAAGTGGATCTGGGACAGATTTTACTTTCACCATCAGCAGCCTGCAGCCTGAAGATAT<br>TGCAACATATTTCTGTCAACACTTTGATCATCTCCCGCTCGCTTTCGGCGGAGGGACCAAGGTGG<br>AGATCAAAGGAGGTGGTGGATCCGAGGTGCAGCTGGTCGAGTCTGGAGGAGGATTGGTGCAGCCT<br>GGAGGGTCATTGAAACTCTCATGTGCAGCCTCTGGATTCACCTTCAATAAGTACGCCATGAACTG<br>GGTCCGCCAGGCTCCAGGAAAGGGTTTGGAATGGGTTGCTCGCATAAGAAGTAAATATAATAATT<br>ATGCAACATATTATGCCGATTCAGTGAAAGACAGGTTCACCATCTCCAGAGATGATTCAAAAAAC<br>ACTGCCTATCTACAAATGAACAACTTGAAAACTGAGGACACTGCCGTGTACTACTGTGTGAGACA<br>TGGGAACTTCGGTAATAGCTACATATCCTACTGGGCTTACTGGGGCCAAGGGACTCTGGTCACCG<br>TCTCCTCAGGTGGTGGTGGTTCTGGCGGCGGCGGCTCCGGTGGTGGTGGTTCTCAGACTGTTGTG<br>ACTCAGGAACCTTCACTCACCGTATCACCTGGTGGAACAGTCACACTCACTTGTGGCTCCTCGAC<br>TGGGGCTGTTACATCTGGCTACTACCCAAACTGGGTCCAACAAAAACCAGGTCAGGCACCCCGTG<br>GTCTAATAGGTGGGACTAAGTTCCTCGCCCCCGGTACTCCTGCCAGATTCTCAGGCTCCCTGCTT<br>GGAGGCAAGGCTGCCCTCACCCTCTCAGGGGTACAGCCAGAGGATGAGGCAGAATATTACTGTGC<br>TCTATGGTACAGCAACCGCTGGGTGTTCGGTGGAGGAACCAAACTGACTGTCCTA |
| 399 | EGFR VL-VH x<br>H2C VH VL | artificial | aa | DIQMTQSPSSLSASVGDRVTITCQASQDISNYLNWYQQKPGKAPKLLIYDASNLETGVPSRFSGS<br>GSGTDFTFTISSLQPEDIATYFCQHFDHLPLAFGGGTKVEIKGGGGSGGGGSGGGGSQVQLQESG<br>PGLVKPSETLSLTCTVSGGSVSSGDYYWTWIRQSPGKGLEWIGHIYYSGNTNYNPSLKSRLTISI<br>DTSKTQFSLKLSSVTAADTAIYYCVRDRVTGAFDIWGQGTMVTVSSGGGGSEVQLVESGGGLVQP<br>GGSLKLSCAASGFTFNKYAMNWVRQAPGKGLEWVARIRSKYNNYATYYADSVKDRFTISRDDSKN<br>TAYLQMNNLKTEDTAVYYCVRHGNFGNSYISYWAYWGQGTLVTVSSGGGGSGGGGSGGGGSQTVV<br>TQEPSLTVSPGGTVTLTCGSSTGAVTSGYYPNWVQQKPGQAPRGLIGGTKFLAPGTPARFSGSLL<br>GGKAALTLSGVQPEDEAEYYCALWYSNRWVFGGGTKLTVL |
| 400 | EGFR VL-VH x<br>H2C VH VL | artificial | nt | GACATCCAGATGACCCAGTCTCCATCCTCCCTGTCTGCATCTGTCGGAGACAGAGTCACCATCAC<br>TTGCCAGGCGAGTCAGGACATCAGCAACTATTTAAATTGGTATCAGCAGAAACCAGGGAAAGCCC<br>CTAAACTCCTGATCTACGATGCATCCAATTTGGAAACAGGGGTCCCATCAAGGTTCAGTGGAAGT<br>GGATCTGGGACAGATTTTACTTTCACCATCAGCAGCCTGCAGCCTGAAGATATTGCAACATATTT<br>CTGTCAACACTTTGATCATCTCCCGCTCGCTTTCGGCGGAGGGACCAAGGTGGAGATCAAAGGTG<br>GTGGTGGTTCTGGCGGCGGCGGCTCCGGTGGTGGTGGTTCTCAGGTGCAGCTGCAGGAGTCGGGC<br>CCAGGACTGGTGAAGCCTTCGGAGACCCTGTCCCTCACCTGCACTGTCTCTGGTGGCTCCGTCAG<br>CAGTGGTGATTACTACTGGACCTGGATCCGGCAGTCCCCAGGGAAGGGACTGGAGTGGATTGGAC<br>ACATCTATTACAGTGGGAACACCAATTATAACCCCTCCCTCAAGAGCCGACTCACCATATCAATT |

| | | | | |
|---|---|---|---|---|
| | | | | GACACGTCCAAGACTCAGTTCTCCCTGAAGCTGAGTTCTGTGACCGCTGCGGACACGGCCATTTA TTACTGTGTGCGAGATCGAGTGACTGGTGCTTTTGATATCTGGGGCCAAGGGACAATGGTCACCG TCTCTTCCGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAAGTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGACAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACATATCCTACTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCTACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGC TCTATGGTACAGCAACCGCTGGGTGTTCGGTGGAGGAACCAAACTGACTGTCCTA |
| 401 | VH of EGFR | artificial | aa | QVQLKQSGPGLVQPSQSLSITCTVSGFSLTNYGVHWVRQSPGKGLEWLGVIWSGGNTDYNTPFTS RLSINKDNSKSQVFFKMNSLQSNDTAIYYCARALTYYDYEFAYWGQGTLVTVSS |
| 402 | VH of EGFR | artificial | nt | CAGGTGCAGCTGAAGCAGTCAGGACCTGGCCTAGTGCAGCCCTCACAGAGCCTGTCCATCACCTG CACAGTCTCTGGTTTCTCATTAACTAACTATGGAGTACACTGGGTTCGCCAGTCTCCAGGAAAGG GTCTGGAGTGGCTGGGAGTGATATGGAGTGGTGGAAACACAGACTATAATACACCTTTCACATCC AGACTGAGCATCAACAAGGACAATTCCAAGAGCCAAGTTTTCTTTAAAATGAACAGTCTGCAATC TAATGACACAGCCATATATTACTGTGCCAGAGCCCTGACCTATTATGACTACGAGTTCGCCTATT GGGGTCAGGGAACCCTGGTTACCGTGTCTTCC |
| 403 | VL of EGFR | artificial | aa | DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELK |
| 404 | VL of EGFR | artificial | nt | GACATCTTGCTGACTCAGTCTCCAGTCATCCTGTCTGTGAGTCCAGGAGAAAGAGTCAGTTTCTC CTGCAGGGCCAGTCAGAGCATTGGCACAAACATACACTGGTATCAGCAAAGAACAAATGGTTCTC CAAGGCTTCTCATAAAGTATGCTTCTGAGTCTATCTCTGGGATCCCTTCCAGGTTTAGTGGCAGT GGATCAGGGACAGATTTTACTCTTAGCATCAACAGTGTGGAGTCTGAAGATATTGCAGATTATTA CTGTCAACAAAATAATAACTGGCCAACCACATTTGGTGCAGGAACAAAGCTGGAACTGAAA |
| 405 | VH-VL of EGFR | artificial | aa | QVQLKQSGPGLVQPSQSLSITCTVSGFSLTNYGVHWVRQSPGKGLEWLGVIWSGGNTDYNTPFTS RLSINKDNSKSQVFFKMNSLQSNDTAIYYCARALTYYDYEFAYWGQGTLVTVSSGGGGSGGGGSG GGGSDILLTQSPVILSVSPGERVSFSCRASQSIGTNIHWYQQRTNGSPRLLIKYASESISGIPSR FSGSGSGTDFTLSINSVESEDIADYYCQQNNNWPTTFGAGTKLELK |

| 406 | VH-VL of EGFR | artificial | nt | CAGGTGCAGCTGAAGCAGTCAGGACCTGGCCTAGTGCAGCCCTCACAGAGCCTGTCCATCACCTG CACAGTCTCTGGTTTCTCATTAACTAACTATGGAGTACACTGGGTTCGCCAGTCTCCAGGAAAGG GTCTGGAGTGGCTGGGAGTGATATGGAGTGGTGGAAACACAGACTATAATACACCTTTCACATCC AGACTGAGCATCAACAAGGACAATTCCAAGAGCCAAGTTTTCTTTAAAATGAACAGTCTGCAATC TAATGACACAGCCATATATTACTGTGCCAGAGCCCTGACCTATTATGACTACGAGTTCGCCTATT GGGGTCAGGGAACCCTGGTTACCGTGTCTTCCGGTGGTGGTGGTTCTGGCGGCGGCGGCTCCGGT GGTGGTGGTTCTGACATCTTGCTGACTCAGTCTCCAGTCATCCTGTCTGTGAGTCCAGGAGAAAG AGTCAGTTTCTCCTGCAGGGCCAGTCAGAGCATTGGCACAAACATACACTGGTATCAGCAAAGAA CAAATGGTTCTCCAAGGCTTCTCATAAAGTATGCTTCTGAGTCTATCTCTGGGATCCCTTCCAGG TTTAGTGGCAGTGGATCAGGGACAGATTTTACTCTTAGCATCAACAGTGTGGAGTCTGAAGATAT TGCAGATTATTACTGTCAACAAAATAATAACTGGCCAACCACATTTGGTGCAGGAACAAAGCTGG AACTGAAA |
| 407 | VL-VH of EGFR | artificial | aa | DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELKGGGGSGGGGSGGGGSQVQLKQSG PGLVQPSQSLSITCTVSGFSLTNYGVHWVRQSPGKGLEWLGVIWSGGNTDYNTPFTSRLSINKDN SKSQVFFKMNSLQSNDTAIYYCARALTYYDYEFAYWGQGTLVTVSS |
| 408 | VL-VH of EGFR | artificial | nt | GACATCTTGCTGACTCAGTCTCCAGTCATCCTGTCTGTGAGTCCAGGAGAAAGAGTCAGTTTCTC CTGCAGGGCCAGTCAGAGCATTGGCACAAACATACACTGGTATCAGCAAAGAACAAATGGTTCTC CAAGGCTTCTCATAAAGTATGCTTCTGAGTCTATCTCTGGGATCCCTTCCAGGTTTAGTGGCAGT GGATCAGGGACAGATTTTACTCTTAGCATCAACAGTGTGGAGTCTGAAGATATTGCAGATTATTA CTGTCAACAAAATAATAACTGGCCAACCACATTTGGTGCAGGAACAAAGCTGGAACTGAAAGGTG GTGGTGGTTCTGGCGGCGGCGGCTCCGGTGGTGGTGGTTCTCAGGTGCAGCTGAAGCAGTCAGGA CCTGGCCTAGTGCAGCCCTCACAGAGCCTGTCCATCACCTGCACAGTCTCTGGTTTCTCATTAAC TAACTATGGAGTACACTGGGTTCGCCAGTCTCCAGGAAAGGGTCTGGAGTGGCTGGGAGTGATAT GGAGTGGTGGAAACACAGACTATAATACACCTTTCACATCCAGACTGAGCATCAACAAGGACAAT TCCAAGAGCCAAGTTTTCTTTAAAATGAACAGTCTGCAATCTAATGACACAGCCATATATTACTG TGCCAGAGCCCTGACCTATTATGACTACGAGTTCGCCTATTGGGGTCAGGGAACCCTGGTTACCG TGTCTTCC |
| 409 | EGFR VH-VL x I2C VH VL | artificial | aa | QVQLKQSGPGLVQPSQSLSITCTVSGFSLTNYGVHWVRQSPGKGLEWLGVIWSGGNTDYNTPFTS RLSINKDNSKSQVFFKMNSLQSNDTAIYYCARALTYYDYEFAYWGQGTLVTVSSGGGGSGGGGSG GGGSDILLTQSPVILSVSPGERVSFSCRASQSIGTNIHWYQQRTNGSPRLLIKYASESISGIPSR FSGSGSGTDFTLSINSVESEDIADYYCQQNNNWPTTFGAGTKLELKGGGGSEVQLVESGGGLVQP GGSLKLSCAASGFTFNKYAMNWVRQAPGKGLEWVARIRSKYNNYATYYADSVKDRFTISRDDSKN TAYLQMNNLKTEDTAVYYCVRHGNFGNSYISYWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPARFSGSLL |

| | | | | GGKAALTLSGVQPEDEAEYYCVLWYSNRWVFGGGTKLTVL |
|---|---|---|---|---|
| 410 | EGFR VH-VL x I2C VH VL | artificial | nt | CAGGTGCAGCTGAAGCAGTCAGGACCTGGCCTAGTGCAGCCCTCACAGAGCCTGTCCATCACCTG CACAGTCTCTGGTTTCTCATTAACTAACTATGGAGTACACTGGGTTCGCCAGTCTCCAGGAAAGG GTCTGGAGTGGCTGGGAGTGATATGGAGTGGTGGAAACACAGACTATAATACACCTTTCACATCC AGACTGAGCATCAACAAGGACAATTCCAAGAGCCAAGTTTTCTTTAAAATGAACAGTCTGCAATC TAATGACACAGCCATATATTACTGTGCCAGAGCCCTGACCTATTATGACTACGAGTTCGCCTATT GGGGTCAGGGAACCCTGGTTACCGTGTCTTCCGGTGGTGGTGGTTCTGGCGGCGGCGGCTCCGGT GGTGGTGGTTCTGACATCTTGCTGACTCAGTCTCCAGTCATCCTGTCTGTGAGTCCAGGAGAAAG AGTCAGTTTCTCCTGCAGGGCCAGTCAGAGCATTGGCACAAACATACACTGGTATCAGCAAAGAA CAAATGGTTCTCCAAGGCTTCTCATAAAGTATGCTTCTGAGTCTATCTCTGGGATCCCTTCCAGG TTTAGTGGCAGTGGATCAGGGACAGATTTTACTCTTAGCATCAACAGTGTGGAGTCTGAAGATAT TGCAGATTATTACTGTCAACAAAATAATAACTGGCCAACCACATTTGGTGCAGGAACAAAGCTGG AACTGAAAGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAAGTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGACAGGTTCACCATCTCCAGAGATGATTCAAAAAAC ACTGCCTATCTACAAATGAACAACTTGAAGACTGAGGACACTGCCGTGTACTACTGTGTGAGACA TGGGAACTTCGGTAATAGCTACATATCCTACTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCAACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGT TCTATGGTACAGCAACCGCTGGGTGTTCGGTGGAGGAACCAAACTGACTGTCCTA |
| 411 | EGFR VL-VH x I2C VH VL | artificial | aa | DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELKGGGGSGGGGSGGGGSQVQLKQSG PGLVQPSQSLSITCTVSGFSLTNYGVHWVRQSPGKGLEWLGVIWSGGNTDYNTPFTSRLSINKDN SKSQVFFKMNSLQSNDTAIYYCARALTYYDYEFAYWGQGTLVTVSSGGGGSEVQLVESGGGLVQP GGSLKLSCAASGFTFNKYAMNWVRQAPGKGLEWVARIRSKYNNYATYYADSVKDRFTISRDDSKN TAYLQMNNLKTEDTAVYYCVRHGNFGNSYISYWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |
| 412 | EGFR VL-VH x I2C VH VL | artificial | nt | GACATCTTGCTGACTCAGTCTCCAGTCATCCTGTCTGTGAGTCCAGGAGAAAGAGTCAGTTTCTC CTGCAGGGCCAGTCAGAGCATTGGCACAAACATACACTGGTATCAGCAAAGAACAAATGGTTCTC CAAGGCTTCTCATAAAGTATGCTTCTGAGTCTATCTCTGGGATCCCTTCCAGGTTTAGTGGCAGT |

EP 2 155 788 B1

| | Name | Organism | Type | Sequence |
|---|---|---|---|---|
| | | | | GGATCAGGAGGACAGATTTTACTCTTAGCATCAACAGTGTGGAGTCTGAAGATATTGCAGATTATTA<br>CTGTCAACAAATAATAATAACTGGCCAACCACATTTGGTGCAGGAACCAAGCTGGAACTGAAAGGTG<br>GTGGTGGTTCTGGCGGCGGCGGCTCCGGTGGTGGTGGATCCATCCAGAGACCTGGCCCTCAGAGGA<br>CCTGGCCCTCAGTGCAGCCCTCACAGAGCCTGTCCAGTCTCACCTGCACAGTCTCTGGTTTCTCATTAAC<br>TAACTATGGAGTACACAGACTATAATACACCTTCACATCCAGACTGAGCATCAACAAGGACAAT<br>GGAGTGGTGGAAACACAGACTATATAATGAACAGTCTGCAATCTAATGACACAGCCATATATTACTG<br>TCCAAGAGCCAAGTTTCTTTAAAATGAACAGTCTGCCTATTGGGGTCAGGAGAGTTCGCAGTTACCG<br>TGCCAGAGCCCTGACCTATTATGACTACGAGTTCGCAGCTGGTGCCAGTCTGGTGCAGCCT<br>TGTCTTCCGGAGGTGGTGGATCCGAGTCGCAGCCTCTGGATTCAACTTCACCTTCAATAGTTATGCCATGAACTG<br>GGAGGGTCATTGAAACTCTCATGTGCAGCCTCCGGTTGGAATGGGTTGCTCGCAATAAGAAGTAAATATAATAATT<br>GGTCCGCCAGGCTCCAGGAAAGGGTTGGAATGGGTTGCTCGCAATAAGAAGTAAATATAATAATT<br>ATGCAACACATATTATGCCGATTCAGTGAAAGACAGCAGTTGAAGACTGAGGACACTGTGTGAGACA<br>ACTGCCTATCTACAAATGAACAACTTGAAGACTGAGGACACTGTACTGTGTGAGACA<br>TGGGAACTTCGGTAATAGCTACATATCCTACTGGGCGGCGCTCCGGTGGTGGTGGATCCAACAAAAAC<br>TCTCCTCAGGTGGTGGTTCTGGCGGCGCTATCACCTGGAACAGTCCAACAAACTGGGTCCAACAAAAAC<br>ACTCAGGAACTTCACTCTGCCAACTACCCAAACTGGGTCCAACAAACTGGGTCCCCCGGTCACCG<br>TGGGGCGTTACATCTGGGGACTAAGTTCCTGCCCCCTCAGGGTACCAGCAGGACTCTGGTCCTCGAC<br>GTCTAATAGGTGGGACTAAGTTCCTGCCCCCTCAGGGTACCAGCAGGACTCTGGTCCTCGAC<br>GGAGGCAAGGCTGCCCTCACCCTGTCCGGTGTTCGGTGGAGCAACCAAACTGACTGTCCTA<br>TCTATGGTACAGCAACCGCTGGGTTCGGTGGAGCAACCAAACTGACTGTCCTA |
| 413 | EGFR VH-VL x F12Q VH VL | artificial | aa | QVQLKQSGPGLVQPSQSLSITCTVSGFSLTNYGVHWVRQSPGKGLEWLGVIWSGGNTPFTS<br>RLSINKDNSKSQVFFKMNSLQSNDTAIYYCARALTYYDYEFAYWGQTLVTVSSGGGGSGGGGSG<br>GGGSDILLTQSPVILSVSPGERVSFSCRASQSIGTNIHWYQQRTNGSPRLLIKYASESISGIPSR<br>FSGSGSGTDFTLSINSVESEDIADYCYCQQNNNWPTTFGAGTKLELKGGGGSEVQLVESGGGLVQP<br>GGSLKLSCAASGFTFNSYAMNWVRQAPGKGLEWVARIRSKYNNYATYADSVKGRFTISRDDSKN<br>TAYLQMNNLKTEDTAVYYCVRHGNFGNSYVSWWAYWGQGTLVTVSSGGGGSGGGGSGGGGSQTVV<br>TQEPSLTVSPGGTVTLTCGSSTGAVTSGNYPNWVQQKPGQAPRGLIGGTKFLAPGTPARFSGSLL<br>GGKAALTLSGVQPEDEAEYYCVLWYSNRWVFGGGTKLTVL |
| 414 | EGFR VH-VL x F12Q VH VL | artificial | nt | CAGGTGCAGCTGAAGCAGTCAGGACCTGGCCTAGTGCAGCCCTCACAGAGCCTGTCCATCACCTG<br>CACAGTCTCTGGTTTCTCATTAACTAACTATGGAGTACACTGGGTTCGCCAGTCTCCAGGAAAGG<br>GTCTGGAGTGGCTGGGAGTGATATGGAGTGGTGGAAACACTATAATACACCTTTCACATCC<br>AGACTGAGACTCAAGAAGGACATCAACAAGGACAATTCCAAGAGCCAAGTTTTCTTTAAAATGAAC<br>TAATGACACAGCCATATATTACTGTGCCAGAGCCCTGACCTATTATGACTACGAGTTCGCCTATT<br>GGGGTCAGGGAACCCTGGTTACCGTCTCCAGTCAGGGTGGTGGTTCTGGCGGCGGCGGCTCCGGT<br>GGTGGTGGTTCTGGACATCTTGCTGACTCAGTCTCCTGTGTCTGTGTGTCCAGGAGAAAG<br>AGTCAGTTTCTCCTGCAGAGGGCCAGTCAGAGCATTGCACAAACATACACTGGTATCAGCAAAGAA |

139

| | | | | |
|---|---|---|---|---|
| | | | | CAAATGGTTCTCCAAGGCTTCTCATAAAGTATGCTTCTGAGTCTATCTCTGGGATCCCTTCCAGG TTTAGTGGCAGTGGATCAGGGACAGATTTTACTCTTAGCATCAACAGTGTGGAGTCTGAAGATAT TGCAGATTATTACTGTCAACAAAATAATAACTGGCCAACCACATTTGGTGCAGGAACAAAGCTGG AACTGAAAGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAGCTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGGCAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACGTTTCCTGGTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCAACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGT TCTATGGTACAGCAACCGCTGGGTGTTCGGTGGAGGAACCAAACTGACTGTCCTA |
| 415 | EGFR VL-VH x F12Q VH VL | artificial | aa | DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELKGGGGSGGGGSGGGGSQVQLKQSG PGLVQPSQSLSITCTVSGFSLTNYGVHWVRQSPGKGLEWLGVIWSGGNTDYNTPFTSRLSINKDN SKSQVFFKMNSLQSNDTAIYYCARALTYYDYEFAYWGQGTLVTVSSGGGGSEVQLVESGGGLVQP GGSLKLSCAASGFTFNSYAMNWVRQAPGKGLEWVARIRSKYNNYATYYADSVKGRFTISRDDSKN TAYLQMNNLKTEDTAVYYCVRHGNFGNSYVSWWAYWGQGTLVTVSSGGGGSGGGGSGGGGSQTVV TQEPSLTVSPGGTVTLTCGSSTGAVTSGNYPNWVQQKPGQAPRGLIGGTKFLAPGTPARFSGSLL GGKAALTLSGVQPEDEAEYYCVLWYSNRWVFGGGTKLTVL |
| 416 | EGFR VL-VH x F12Q VH VL | artificial | nt | GACATCTTGCTGACTCAGTCTCCAGTCATCCTGTCTGTGAGTCCAGGAGAAAGAGTCAGTTTCTC CTGCAGGGCCAGTCAGAGCATTGGCACAAACATACACTGGTATCAGCAAAGAACAAATGGTTCTC CAAGGCTTCTCATAAAGTATGCTTCTGAGTCTATCTCTGGGATCCCTTCCAGGTTTAGTGGCAGT GGATCAGGGACAGATTTTACTCTTAGCATCAACAGTGTGGAGTCTGAAGATATTGCAGATTATTA CTGTCAACAAAATAATAACTGGCCAACCACATTTGGTGCAGGAACAAAGCTGGAACTGAAAGGTG GTGGTGGTTCTGGCGGCGGCGGCTCCGGTGGTGGTGGTTCTCAGGTGCAGCTGAAGCAGTCAGGA CCTGGCCTAGTGCAGCCCTCACAGAGCCTGTCCATCACCTGCACAGTCTCTGGTTTCTCATTAAC TAACTATGGAGTACACTGGGTTCGCCAGTCTCCAGGAAAGGGTCTGGAGTGGCTGGGAGTGATAT GGAGTGGTGGAAACACAGACTATAATACACCTTTCACATCCAGACTGAGCATCAACAAGGACAAT TCCAAGAGCCAAGTTTTCTTTAAAATGAACAGTCTGCAATCTAATGACACAGCCATATATTACTG TGCCAGAGCCCTGACCTATTATGACTACGAGTTCGCCTATTGGGGTCAGGGAACCCTGGTTACCG TGTCTTCCGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAGCTACGCCATGAACTG |

140

EP 2 155 788 B1

| | | | | |
|---|---|---|---|---|
| | | | | GGTCCGCCAGGCTCCAGGAAAGGGTTTGGAATGGGTTGCTCGCATAAGAAGTAAATATAATAATT ATGCAACATATTATGCCGATTCAGTGAAAGGCAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACGTTTCCTGGTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCAACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGT TCTATGGTACAGCAACCGCTGGGTGTTCGGTGGAGGAACCAAACTGACTGTCCTA |
| 417 | EGFR VH-VL x H2C VH VL | artificial | aa | QVQLKQSGPGLVQPSQSLSITCTVSGFSLTNYGVHWVRQSPGKGLEWLGVIWSGGNTDYNTPFTS RLSINKDNSKSQVFFKMNSLQSNDTAIYYCARALTYYDYEFAYWGQGTLVTVSSGGGGSGGGGSG GGGSDILLTQSPVILSVSPGERVSFSCRASQSIGTNIHWYQQRTNGSPRLLIKYASESISGIPSR FSGSGSGTDFTLSINSVESEDIADYYCQQNNNWPTTFGAGTKLELKGGGGSEVQLVESGGGLVQP GGSLKLSCAASGFTFNKYAMNWVRQAPGKGLEWVARIRSKYNNYATYYADSVKDRFTISRDDSKN TAYLQMNNLKTEDTAVYYCVRHGNFGNSYISYWAYWGQGTLVTVSSGGGGSGGGGSGGGGSQTVV TQEPSLTVSPGGTVTLTCGSSTGAVTSGYYPNWVQQKPGQAPRGLIGGTKFLAPGTPARFSGSLL GGKAALTLSGVQPEDEAEYYCALWYSNRWVFGGGTKLTVL |
| 418 | EGFR VH-VL x H2C VH VL | artificial | nt | CAGGTGCAGCTGAAGCAGTCAGGACCTGGCCTAGTGCAGCCCTCACAGAGCCTGTCCATCACCTG CACAGTCTCTGGTTTCTCATTAACTAACTATGGAGTACACTGGGTTCGCCAGTCTCCAGGAAAGG GTCTGGAGTGGCTGGGAGTGATATGGAGTGGTGGAAACACAGACTATAATACACCTTTCACATCC AGACTGAGCATCAACAAGGACAATTCCAAGAGCCAAGTTTTCTTTAAAATGAACAGTCTGCAATC TAATGACACAGCCATATATTACTGTGCCAGAGCCCTGACCTATTATGACTACGAGTTCGCCTATT GGGGTCAGGGAACCCTGGTTACCGTGTCTTCCGGTGGTGGTGGTTCTGGCGGCGGCGGCTCCGGT GGTGGTGGTTCTGACATCTTGCTGACTCAGTCTCCAGTCATCCTGTCTGTGAGTCCAGGAGAAAG AGTCAGTTTCTCCTGCAGGGCCAGTCAGAGCATTGGCACAAACATACACTGGTATCAGCAAAGAA CAAATGGTTCTCCAAGGCTTCTCATAAAGTATGCTTCTGAGTCTATCTCTGGGATCCCTTCCAGG TTTAGTGGCAGTGGATCAGGGACAGATTTTACTCTTAGCATCAACAGTGTGGAGTCTGAAGATAT TGCAGATTATTACTGTCAACAAAATAATAACTGGCCAACCACATTTGGTGCAGGAACAAAGCTGG AACTGAAAGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAAGTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGACAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACATATCCTACTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG |

EP 2 155 788 B1

| | | | | |
|---|---|---|---|---|
| | | | | ACTCAGGAACCTTCACTCACCGTATCACCTGGTGGAACAGTCACACTCACTTGTGGCTCCTCGAC TGGGGCTGTTACATCTGGCTACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGC TCTATGGTACAGCAACCGCTGGGTGTTCGGTGGAGGAACCAAACTGACTGTCCTA |
| 419 | EGFR VL-VH x H2C VH VL | artificial | aa | DILLTQSPVILSVSPGERVSFSCRASQSIGTNIHWYQQRTNGSPRLLIKYASESISGIPSRFSGS GSGTDFTLSINSVESEDIADYYCQQNNNWPTTFGAGTKLELKGGGGSGGGGSGGGGSQVQLKQSG PGLVQPSQSLSITCTVSGFSLTNYGVHWVRQSPGKGLEWLGVIWSGGNTDYNTPFTSRLSINKDN SKSQVFFKMNSLQSNDTAIYYCARALTYYDYEFAYWGQGTLVTVSSGGGGSEVQLVESGGGLVQP GGSLKLSCAASGFTFNKYAMNWVRQAPGKGLEWVARIRSKYNNYATYYADSVKDRFTISRDDSKN TAYLQMNNLKTEDTAVYYCVRHGNFGNSYISYWAYWGQGTLVTVSSGGGGSGGGGSGGGGSQTVV TQEPSLTVSPGGTVTLTCGSSTGAVTSGYYPNWVQQKPGQAPRGLIGGTKFLAPGTPARFSGSLL GGKAALTLSGVQPEDEAEYYCALWYSNRWVFGGGTKLTVL |
| 420 | EGFR VL-VH x H2C VH VL | artificial | nt | GACATCTTGCTGACTCAGTCTCCAGTCATCCTGTCTGTGAGTCCAGGAGAAAGAGTCAGTTTCTC CTGCAGGGCCAGTCAGAGCATTGGCACAAACATACACTGGTATCAGCAAAGAACAAATGGTTCTC CAAGGCTTCTCATAAAGTATGCTTCTGAGTCTATCTCTGGGATCCCTTCCAGGTTTAGTGGCAGT GGATCAGGGACAGATTTTACTCTTAGCATCAACAGTGTGGAGTCTGAAGATATTGCAGATTATTA CTGTCAACAAAATAATAACTGGCCAACCACATTTGGTGCAGGAACAAAGCTGGAACTGAAAGGTG GTGGTGGTTCTGGCGGCGGCGGCTCCGGTGGTGGTGGTTCTCAGGTGCAGCTGAAGCAGTCAGGA CCTGGCCTAGTGCAGCCCTCACAGAGCCTGTCCATCACCTGCACAGTCTCTGGTTTCTCATTAAC TAACTATGGAGTACACTGGGTTCGCCAGTCTCCAGGAAAGGGTCTGGAGTGGCTGGGAGTGATAT GGAGTGGTGGAAACACAGACTATAATACACCTTTCACATCCAGACTGAGCATCAACAAGGACAAT TCCAAGAGCCAAGTTTTCTTTAAAATGAACAGTCTGCAATCTAATGACACAGCCATATATTACTG TGCCAGAGCCCTGACCTATTATGACTACGAGTTCGCCTATTGGGGTCAGGGAACCCTGGTTACCG TGTCTTCCGGAGGTGGTGGATCCGAGGTGCAGCTGGTCGAGTCTGGAGGAGGATTGGTGCAGCCT GGAGGGTCATTGAAACTCTCATGTGCAGCCTCTGGATTCACCTTCAATAAGTACGCCATGAACTG GGTCCGCCAGGCTCCAGGAAAGGGTTTGGAATGGGTTGCTCGCATAAGAAGTAAATATAATAATT ATGCAACATATTATGCCGATTCAGTGAAAGACAGGTTCACCATCTCCAGAGATGATTCAAAAAAC ACTGCCTATCTACAAATGAACAACTTGAAAACTGAGGACACTGCCGTGTACTACTGTGTGAGACA TGGGAACTTCGGTAATAGCTACATATCCTACTGGGCTTACTGGGGCCAAGGGACTCTGGTCACCG TCTCCTCAGGTGGTGGTGGTTCTGGCGGCGGCGGCTCCGGTGGTGGTGGTTCTCAGACTGTTGTG ACTCAGGAACCTTCACTCACCGTATCACCTGGTGGAACAGTCACACTCACTTGTGGCTCCTCGAC TGGGGCTGTTACATCTGGCTACTACCCAAACTGGGTCCAACAAAAACCAGGTCAGGCACCCCGTG GTCTAATAGGTGGGACTAAGTTCCTCGCCCCCGGTACTCCTGCCAGATTCTCAGGCTCCCTGCTT GGAGGCAAGGCTGCCCTCACCCTCTCAGGGGTACAGCCAGAGGATGAGGCAGAATATTACTGTGC |

| | | | | TCTATGGTACAGCAACCGCTGGGTGTTCGGTGGAGGAACCAAACTGACTGTCCTA |
|---|---|---|---|---|
| 421 | VH of HER2/neu | artificial | aa | EVQLVESGGGLVQPGGSLRLSCAASGFNIKDTYIHWVRQAPGKGLEWVARIYPTNGYTRYADSVK GRFTISADTSKNTAYLQMNSLRAEDTAVYYCSRWGGDGFYAMDYWGQGTLVTVSS |
| 422 | VH of Her2/neu | artificial | nt | GAGGTTCAGCTGGTGGAGTCTGGCGGTGGCCTGGTGCAGCCAGGGGGCTCACTCCGTTTGTCCTG TGCAGCTTCTGGCTTCAACATTAAAGACACCTATATACACTGGGTGCGTCAGGCCCCGGGTAAGG GCCTGGAATGGGTTGCAAGGATTTATCCTACGAATGGTTATACTAGATATGCCGATAGCGTCAAG GGCCGTTTCACTATAAGCGCAGACACATCCAAAAACACAGCCTACCTGCAGATGAACAGCCTGCG TGCTGAGGACACTGCCGTCTATTATTGTTCTAGGTGGGGAGGGGACGGCTTCTATGCTATGGACT ATTGGGGTCAAGGAACCCTGGTCACTGTCTCCTCC |
| 423 | VL of Her2/neu | artificial | aa | DIQMTQSPSSLSASVGDRVTITCRASQDVNTAVAWYQQKPGKAPKLLIYSASFLYSGVPSRFSGS RSGTDFTLTISSLQPEDFATYYCQQHYTTPPTFGQGTKVEIK |
| 424 | VL of Her2/neu | artificial | nt | GATATCCAGATGACCCAGTCCCCGAGCTCCCTGTCCGCCTCTGTGGGCGATAGGGTCACCATCAC CTGCCGTGCCAGTCAGGATGTGAATACTGCTGTAGCCTGGTATCAACAGAAACCAGGAAAAGCTC CGAAACTACTGATTTACTCGGCATCCTTCCTCTACTCTGGAGTCCCTTCTCGCTTCTCTGGATCC AGATCTGGGACGGATTTCACTCTGACCATCAGCAGTCTGCAGCCGGAAGACTTCGCAACTTATTA CTGTCAGCAACATTATACTACTCCTCCCACGTTCGGACAGGGTACCAAGGTGGAGATCAAA |
| 425 | VH-VL of Her2/neu | artificial | aa | EVQLVESGGGLVQPGGSLRLSCAASGFNIKDTYIHWVRQAPGKGLEWVARIYPTNGYTRYADSVK GRFTISADTSKNTAYLQMNSLRAEDTAVYYCSRWGGDGFYAMDYWGQGTLVTVSSGGGGSGGGGS GGGGSDIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIK |
| 426 | VH-VL of Her2/neu | artificial | nt | GAGGTTCAGCTGGTGGAGTCTGGCGGTGGCCTGGTGCAGCCAGGGGGCTCACTCCGTTTGTCCTG TGCAGCTTCTGGCTTCAACATTAAAGACACCTATATACACTGGGTGCGTCAGGCCCCGGGTAAGG GCCTGGAATGGGTTGCAAGGATTTATCCTACGAATGGTTATACTAGATATGCCGATAGCGTCAAG GGCCGTTTCACTATAAGCGCAGACACATCCAAAAACACAGCCTACCTGCAGATGAACAGCCTGCG TGCTGAGGACACTGCCGTCTATTATTGTTCTAGGTGGGGAGGGGACGGCTTCTATGCTATGGACT ATTGGGGTCAAGGAACCCTGGTCACTGTCTCCTCCGGTGGTGGTGGTTCTGGCGGCGGCGGCTCC GGTGGTGGTGGTTCTGATATCCAGATGACCCAGTCCCCGAGCTCCCTGTCCGCCTCTGTGGGCGA TAGGGTCACCATCACCTGCCGTGCCAGTCAGGATGTGAATACTGCTGTAGCCTGGTATCAACAGA AACCAGGAAAAGCTCCGAAACTACTGATTTACTCGGCATCCTTCCTCTACTCTGGAGTCCCTTCT CGCTTCTCTGGATCCAGATCTGGGACGGATTTCACTCTGACCATCAGCAGTCTGCAGCCGGAAGA CTTCGCAACTTATTACTGTCAGCAACATTATACTACTCCTCCCACGTTCGGACAGGGTACCAAGG TGGAGATCAAA |

EP 2 155 788 B1

| 427 | VL-VH of Her2/neu | artificial | aa | DIQMTQSPSSLSASVGDRVTITCRASQDVNTAVAWYQQKPGKAPKLLIYSASFLYSGVPSRFSGS RSGTDFTLTISSLQPEDFATYYCQQHYTTPPTFGQGTKVEIKGGGGSGGGGSGGGGSEVQLVESG GGLVQPGGSLRLSCAASGFNIKDTYIHWVRQAPGKGLEWVARIYPTNGYTRYADSVKGRFTISAD TSKNTAYLQMNSLRAEDTAVYYCSRWGGDGFYAMDYWGQGTLVTVSS |
| 428 | VL-VH of Her2/neu | artificial | nt | GATATCCAGATGACCCAGTCCCCGAGCTCCCTGTCCGCCTCTGTGGGCGATAGGGTCACCATCAC CTGCCGTGCCAGTCAGGATGTGAATACTGCTGTAGCCTGGTATCAACAGAAACCAGGAAAAGCTC CGAAACTACTGATTTACTCGGCATCCTTCCTCTACTCTGGAGTCCCTTCTCGCTTCTCTGGATCC AGATCTGGGACGGATTTCACTCTGACCATCAGCAGTCTGCAGCCGGAAGACTTCGCAACTTATTA CTGTCAGCAACATTATACTACTCCTCCCACGTTCGGACAGGGTACCAAGGTGGAGATCAAAGGTG GTGGTGGTTCTGGCGGCGGCGGCTCCGGTGGTGGTGGTTCTGAGGTTCAGCTGGTGGAGTCTGGC GGTGGCCTGGTGCAGCCAGGGGGCTCACTCCGTTTGTCCTGTGCAGCTTCTGGCTTCAACATTAA AGACACCTATATACACTGGGTGCGTCAGGCCCCGGGTAAGGGCCTGGAATGGGTTGCAAGGATTT ATCCTACGAATGGTTATACTAGATATGCCGATAGCGTCAAGGGCCGTTTCACTATAAGCGCAGAC ACATCCAAAAACACAGCCTACCTGCAGATGAACAGCCTGCGTGCTGAGGACACTGCCGTCTATTA TTGTTCTAGGTGGGGAGGGGACGGCTTCTATGCTATGGACTATTGGGGTCAAGGAACCCTGGTCA CTGTCTCCTCC |
| 429 | Her2/neu VH-VL x I2C VH VL | artificial | aa | EVQLVESGGGLVQPGGSLRLSCAASGFNIKDTYIHWVRQAPGKGLEWVARIYPTNGYTRYADSVK GRFTISADTSKNTAYLQMNSLRAEDTAVYYCSRWGGDGFYAMDYWGQGTLVTVSSGGGGSGGGGS GGGGSDIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKGGGGSEVQLVESGGGLVQ PGGSLKLSCAASGFTFNKYAMNWVRQAPGKGLEWVARIRSKYNNYATYYADSVKDRFTISRDDSK NTAYLQMNNLKTEDTAVYYCVRHGNFGNSYISYWAYWGQGTLVTVSSGGGGSGGGGSGGGGSQTV VTQEPSLTVSPGGTVTLTCGSSTGAVTSGNYPNWVQQKPGQAPRGLIGGTKFLAPGTPARFSGSL LGGKAALTLSGVQPEDEAEYYCVLWYSNRWVFGGGTKLTVL |
| 430 | Her2/neu VH-VL x I2C VH VL | artificial | nt | GAGGTTCAGCTGGTGGAGTCTGGCGGTGGCCTGGTGCAGCCAGGGGGCTCACTCCGTTTGTCCTG TGCAGCTTCTGGCTTCAACATTAAAGACACCTATATACACTGGGTGCGTCAGGCCCCGGGTAAGG GCCTGGAATGGGTTGCAAGGATTTATCCTACGAATGGTTATACTAGATATGCCGATAGCGTCAAG GGCCGTTTCACTATAAGCGCAGACACATCCAAAAACACAGCCTACCTGCAGATGAACAGCCTGCG TGCTGAGGACACTGCCGTCTATTATTGTTCTAGGTGGGGAGGGGACGGCTTCTATGCTATGGACT ATTGGGGTCAAGGAACCCTGGTCACTGTCTCCTCCGGTGGTGGTGGTTCTGGCGGCGGCGGCTCC GGTGGTGGTGGTTCTGATATCCAGATGACCCAGTCCCCGAGCTCCCTGTCCGCCTCTGTGGGCGA TAGGGTCACCATCACCTGCCGTGCCAGTCAGGATGTGAATACTGCTGTAGCCTGGTATCAACAGA AACCAGGAAAAGCTCCGAAACTACTGATTTACTCGGCATCCTTCCTCTACTCTGGAGTCCCTTCT CGCTTCTCTGGATCCAGATCTGGGACGGATTTCACTCTGACCATCAGCAGTCTGCAGCCGGAAGA CTTCGCAACTTATTACTGTCAGCAACATTATACTACTCCTCCCACGTTCGGACAGGGTACCAAGG |

| | | | | |
|---|---|---|---|---|
| | | | | TGGAGATCAAAGGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAAGTACGCCATGAA CTGGGTCCGCCAGGCTCCAGGAAAGGGTTTGGAATGGGTTGCTCGCATAAGAAGTAAATATAATA ATTATGCAACATATTATGCCGATTCAGTGAAAGACAGGTTCACCATCTCCAGAGATGATTCAAAA AACACTGCCTATCTACAAATGAACAACTTGAAGACTGAGGACACTGCCGTGTACTACTGTGTGAG ACATGGGAACTTCGGTAATAGCTACATATCCTACTGGGCTTACTGGGGCCAAGGGACTCTGGTCA CCGTCTCCTCAGGTGGTGGTGGTTCTGGCGGCGGCGGCTCCGGTGGTGGTGGTTCTCAGACTGTT GTGACTCAGGAACCTTCACTCACCGTATCACCTGGTGGAACAGTCACACTCACTTGTGGCTCCTC GACTGGGGCTGTTACATCTGGCAACTACCCAAACTGGGTCCAACAAAAACCAGGTCAGGCACCCC GTGGTCTAATAGGTGGGACTAAGTTCCTCGCCCCCGGTACTCCTGCCAGATTCTCAGGCTCCCTG CTTGGAGGCAAGGCTGCCCTCACCCTCTCAGGGGTACAGCCAGAGGATGAGGCAGAATATTACTG TGTTCTATGGTACAGCAACCGCTGGGTGTTCGGTGGAGGAACCAAACTGACTGTCCTA |
| 431 | Her2/neu VL-VH x I2C VH VL | artificial | aa | DIQMTQSPSSLSASVGDRVTITCRASQDVNTAVAWYQQKPGKAPKLLIYSASFLYSGVPSRFSGS RSGTDFTLTISSLQPEDFATYYCQQHYTTPPTFGQGTKVEIKGGGGSGGGGSGGGGSEVQLVESG GGLVQPGGSLRLSCAASGFNIKDTYIHWVRQAPGKGLEWVARIYPTNGYTRYADSVKGRFTISAD TSKNTAYLQMNSLRAEDTAVYYCSRWGGDGFYAMDYWGQGTLVTVSSGGGGSEVQLVESGGGLVQ PGGSLKLSCAASGFTFNKYAMNWVRQAPGKGLEWVARIRSKYNNYATYYADSVKDRFTISRDDSK NTAYLQMNNLKTEDTAVYYCVRHGNFGNSYISYWAYWGQGTLVTVSSGGGGSGGGGSGGGGSQTV VTQEPSLTVSPGGTVTLTCGSSTGAVTSGNYPNWVQQKPGQAPRGLIGGTKFLAPGTPARFSGSL LGGKAALTLSGVQPEDEAEYYCVLWYSNRWVFGGGTKLTVL |
| 432 | Her2/neu VL-VH x I2C VH VL | artificial | nt | GATATCCAGATGACCCAGTCCCCGAGCTCCCTGTCCGCCTCTGTGGGCGATAGGGTCACCATCAC CTGCCGTGCCAGTCAGGATGTGAATACTGCTGTAGCCTGGTATCAACAGAAACCAGGAAAAGCTC CGAAACTACTGATTTACTCGGCATCCTTCCTCTACTCTGGAGTCCCTTCTCGCTTCTCTGGATCC AGATCTGGGACGGATTTCACTCTGACCATCAGCAGTCTGCAGCCGGAAGACTTCGCAACTTATTA CTGTCAGCAACATTATACTACTCCTCCCACGTTCGGACAGGGTACCAAGGTGGAGATCAAAGGTG GTGGTGGTTCTGGCGGCGGCGGCTCCGGTGGTGGTGGTTCTGAGGTTCAGCTGGTGGAGTCTGGC GGTGGCCTGGTGCAGCCAGGGGGCTCACTCCGTTTGTCCTGTGCAGCTTCTGGCTTCAACATTAA AGACACCTATATACACTGGGTGCGTCAGGCCCCGGGTAAGGGCCTGGAATGGGTTGCAAGGATTT ATCCTACGAATGGTTATACTAGATATGCCGATAGCGTCAAGGGCCGTTTCACTATAAGCGCAGAC ACATCCAAAAACACAGCCTACCTGCAGATGAACAGCCTGCGTGCTGAGGACACTGCCGTCTATTA TTGTTCTAGGTGGGGAGGGGACGGCTTCTATGCTATGGACTATTGGGGTCAAGGAACCCTGGTCA CTGTCTCCTCCGGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAAGTACGCCATGAA CTGGGTCCGCCAGGCTCCAGGAAAGGGTTTGGAATGGGTTGCTCGCATAAGAAGTAAATATAATA ATTATGCAACATATTATGCCGATTCAGTGAAAGACAGGTTCACCATCTCCAGAGATGATTCAAAA AACACTGCCTATCTACAAATGAACAACTTGAAGACTGAGGACACTGCCGTGTACTACTGTGTGAG |

| | | | | |
|---|---|---|---|---|
| | | | | ACATGGGAACTTCGGTAATAGCTACATATCCTACTGGGCTTACTGGGGCCAAGGGACTCTGGTCA CCGTCTCCTCAGGTGGTGGTGGTTCTGGCGGCGGCGGCTCCGGTGGTGGTGGTTCTCAGACTGTT GTGACTCAGGAACCTTCACTCACCGTATCACCTGGTGGAACAGTCACACTCACTTGTGGCTCCTC GACTGGGGCTGTTACATCTGGCAACTACCCAAACTGGGTCCAACAAAAACCAGGTCAGGCACCCC GTGGTCTAATAGGTGGGACTAAGTTCCTCGCCCCCGGTACTCCTGCCAGATTCTCAGGCTCCCTG CTTGGAGGCAAGGCTGCCCTCACCCTCTCAGGGGTACAGCCAGAGGATGAGGCAGAATATTACTG TGTTCTATGGTACAGCAACCGCTGGGTGTTCGGTGGAGGAACCAAACTGACTGTCCTA |
| 433 | Her2/neu VH-VL x F12Q VH VL | artificial | aa | EVQLVESGGGLVQPGGSLRLSCAASGFNIKDTYIHWVRQAPGKGLEWVARIYPTNGYTRYADSVK GRFTISADTSKNTAYLQMNSLRAEDTAVYYCSRWGGDGFYAMDYWGQGTLVTVSSGGGGSGGGGS GGGGSDIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKGGGGSEVQLVESGGGLVQ PGGSLKLSCAASGFTFNSYAMNWVRQAPGKGLEWVARIRSKYNNYATYYADSVKGRFTISRDDSK NTAYLQMNNLKTEDTAVYYCVRHGNFGNSYVSWWAYWGQGTLVTVSSGGGGSGGGGSGGGGSQTV VTQEPSLTVSPGGTVTLTCGSSTGAVTSGNYPNWVQQKPGQAPRGLIGGTKFLAPGTPARFSGSL LGGKAALTLSGVQPEDEAEYYCVLWYSNRWVFGGGTKLTVL |
| 434 | Her2/neu VH-VL x F12Q VH VL | artificial | nt | GAGGTTCAGCTGGTGGAGTCTGGCGGTGGCCTGGTGCAGCCAGGGGGCTCACTCCGTTTGTCCTG TGCAGCTTCTGGCTTCAACATTAAAGACACCTATATACACTGGGTGCGTCAGGCCCCGGGTAAGG GCCTGGAATGGGTTGCAAGGATTTATCCTACGAATGGTTATACTAGATATGCCGATAGCGTCAAG GGCCGTTTCACTATAAGCGCAGACACATCCAAAAACACAGCCTACCTGCAGATGAACAGCCTGCG TGCTGAGGACACTGCCGTCTATTATTGTTCTAGGTGGGGAGGGGACGGCTTCTATGCTATGGACT ATTGGGGTCAAGGAACCCTGGTCACTGTCTCCTCCGGTGGTGGTGGTTCTGGCGGCGGCGGCTCC GGTGGTGGTGGTTCTGATATCCAGATGACCCAGTCCCCGAGCTCCCTGTCCGCCTCTGTGGGCGA TAGGGTCACCATCACCTGCCGTGCCAGTCAGGATGTGAATACTGCTGTAGCCTGGTATCAACAGA AACCAGGAAAGCTCCGAAACTACTGATTTACTCGGCATCCTTCCTCTACTCTGGAGTCCCTTCT CGCTTCTCTGGATCCAGATCTGGGACGGATTTCACTCTGACCATCAGCAGTCTGCAGCCGGAAGA CTTCGCAACTTATTACTGTCAGCAACATTATACTACTCCTCCCACGTTCGGACAGGGTACCAAGG TGGAGATCAAAGGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAGCTACGCCATGAA CTGGGTCCGCCAGGCTCCAGGAAAGGGTTTGGAATGGGTTGCTCGCATAAGAAGTAAATATAATA ATTATGCAACATATTATGCCGATTCAGTGAAAGGCAGGTTCACCATCTCCAGAGATGATTCAAAA AACACTGCCTATCTACAAATGAACAACTTGAAAACTGAGGACACTGCCGTGTACTACTGTGTGAG ACATGGGAACTTCGGTAATAGCTACGTTTCCTGGTGGGCTTACTGGGGCCAAGGGACTCTGGTCA CCGTCTCCTCAGGTGGTGGTGGTTCTGGCGGCGGCGGCTCCGGTGGTGGTGGTTCTCAGACTGTT GTGACTCAGGAACCTTCACTCACCGTATCACCTGGTGGAACAGTCACACTCACTTGTGGCTCCTC GACTGGGGCTGTTACATCTGGCAACTACCCAAACTGGGTCCAACAAAAACCAGGTCAGGCACCCC GTGGTCTAATAGGTGGGACTAAGTTCCTCGCCCCCGGTACTCCTGCCAGATTCTCAGGCTCCCTG |

| | Name | source | type | Sequence |
|---|---|---|---|---|
| | | | | CTTGGAGGCAAGGCTGCCCTCACCCTCTCAGGGGTACAGCAGCCAGAGGATGAGGAGGCAGAATATTACTG<br>TGTTCTATGGTACAGCAACCGCTGGGTGTTCGGTGGAGGAACCAAACTGACTGTCCTA |
| 435 | Her2/neu VL-VH x F12Q VH VL | artificial | aa | DIQMTQSPSSLSASVGDRVTITCRASQDVNTAVAWYQQKPGKAPKLLIYSASFLYSGVPSRFSGS<br>RSGTDFTLTISSLQPEDFATYYCQQHYTTPPTFGQGTKVEIKGGGSGGGGSGGGGSEVQLVESG<br>GGLVQPGGSLRLSCAASGFNIKDTYIHWVRQAPGKGLEWVARIYPTNGYTRYADSVKGRFTISAD<br>TSKNTAYLQMNSLRAEDTAVYYCSRWGGDGFYAMDYWGQGTLVTVSSGGGGSEVQLVESGGGLVQ<br>PGGSLKLSCAASGFTFNSYAMNWVRQAPGKGLEWVARIRSKYNNYATYYADSVKGRFTISRDDSK<br>NTAYLQMNNLKTEDTAVYYCVRHGNFGNSYVSWWAYWGQGTLVTVSSGGGGSGGGGSGGGGSQTV<br>VTQEPSLTVSPGGTVTLTCGSSTGAVTSGNYPNWVQQKPGQAPRGLIGGTKFLAPGTPARFSGSL<br>LGGKAALTLSGVQPEDEAEYYCVLWYSNRWVFGGGTKLTVL |
| 436 | Her2/neu VL-VH x F12Q VH VL | artificial | nt | GATATCCAGATGACCCAGTCCCCGAGCTCCCTGTCCGCCTCTGTGGGCGATAGGGTCACCATCAC<br>CTGCCGTGCCAGTGAGATGTGAATACTGCTGTAGCCTGGTATCAACAGAAACCAGGAAAAGCTC<br>CGAAACTACTGATTTACTGGACGGATTCACTCTGACCATCAGCAGTCTGCAGCCGGAAGACTTCGCAACTTATTA<br>AGATCTCAGCAACATTATACTACTCCTCCCACGTTCGGACAGGGTACCAAGGTGGAGATCAAAGGTG<br>CTGTCAGCAGGTTCTGGTGGTGGTGGTTCAGCTGGTTCAGCTTCTGCTGAGTGTGGC<br>GTGGTGGTTCTGGCGGCGGCGGCCAGGGGCTCACTCGTTGTCCGTTGTGAGGTTCAGCTTCTGGC<br>GGTGGCCTGGTGCAGCCAGGGGGCTCACTGGTGGTGCGTCAGGCGCCCCCGATAGCGTCAAGGGC<br>AGACACCTATATACACTGGTATACTAGATATGGCCAGATGCCGATGAACAGCTCAGCCTGGTTTCACTTTGCAAGGATTT<br>ATCCTACGAATGGTTATACTAGATATGGCCAGATGCCGATGAACAGCTCAATAAGAAGTAAATATAATA<br>ACATCCAAAAACACAGCCTACCTGCAGATGAACAGCAGCCTATTGGGTCAAGGAACCCTGGTCA<br>TTGTTCTAGGTGGGGAGGGGACGGCTTCTATGCTATGGACTATTGGGGTCAAGGAACCCTGGTCA<br>CTGTCTCCCGGAGGTGGTGGATCCGAGGTGCAGCTGGTGGAGTCTGGAGGAGGATTGGTGCAG<br>CCTGGAGGGTCATTGAAACTCTCATGTGCAGCCTCTGGATTCACCTTCAATAGCTACGCCATGAA<br>CTGGGTCCGCCAGGCTCCAGGAAAGGGTTTGGAATGGGTTGCTCTCGCATAAGAAGTAAATATAATA<br>ATTATGCAACATATTATGCCGATAGTGTGAAAGGCAGGTTCAGTGAAAGATGATTCAAAA<br>AACACTGCCTATCTACAAATGAACAACTTGAAAACTGAGGACACTGCCGTGTACTACTGTGTGAG<br>ACATGGGAACTCGGTAATAGCTACGTTTCCTGGTGGGCCGGCGCCCAGGGACTCTGGTCA<br>CCGTCTCCTCAGGAGAACCTTCACCTGTGGTGGTTCTGGCGGCGGCGGCAGTGGTGGTTCTGGTGGTTCTCAGACTGTT<br>GTGACTCAGGAACCTTCACTCACCGTATCACCTGGTGAACAGTCACACTGCCTGCTCCTC<br>GACTGGGCGTGTTACATCTGGCAACTACCCAAACTGGGTCCAACAAAAACCAGGTCAGGCCACCCC<br>GTGGTCTAATAGGTGGGACTAAGTTCCTCGCCCCCGGCTACAGCGCCAGCGCCCAGGCTCCCTG<br>CTTGGAGGCAAGGCTGCCCTCACCCTCTCAGGGGTACAGCGAGAGGATGAGGAGGCAGAATATTACTG<br>TGTTCTATGGTACAGCAACCGCTGGGTGTTCGGTGGAGGAACCAAACTGACTGTCCTA |
| 437 | Her2/neu VH-VL x H2C VH VL | artificial | aa | EVQLVESGGGLVQPGGSLRLSCAASGFNIKDTYIHWVRQAPGKGLEWVARIYPTNGYTRYADSVK<br>GRFTISADTSKNTAYLQMNSLRAEDTAVYYCSRWGGDGFYAMDYWGQGTLVTVSSGGGGSGGGGGS |

EP 2 155 788 B1

| | | | | |
|---|---|---|---|---|
| | | | | GGGGSDIQMTQSPSSLSASVGDRVTITCRASQDVNTAVAWYQQKPGKAPKLLIYSASFLYSGVPS<br>RFSGSRSGTDFTLTISSLQPEDFATYYCQQHYTTPPTFGQGTKVEIKGGGGSEVQLVESGGGLVQ<br>PGGSLKLSCAASGFTFNKYAMNWVRQAPGKGLEWVARIRSKYNNYATYYADSVKDRFTISRDDSK<br>NTAYLQMNNLKTEDTAVYYCVRHGNFGNSYISYWAYWGQGTLVTVSSGGGGSGGGGSGGGGSQTV<br>VTQEPSLTVSPGGTVTLTCGSSTGAVTSGYYPNWVQQKPGQAPRGLIGGTKFLAPGTPARFSGSL<br>LGGKAALTLSGVQPEDEAEYYCALWYSNRWVFGGGTKLTVL |
| 438 | Her2/neu VH-VL x H2C VH VL | artificial | nt | GAGGTTCAGCTGGTGGAGTCTGGCGGTGGCCTGGTGCAGCCAGGGGGGCTCACTCCGTTTGTCCTG<br>TGCAGCTTCTGGCTTCAACATTAAAGACACCTATATACACTGGGTGCGTCAGGCCCCGGGTAAGG<br>GCCTGGAATGGGTTGCAAGGATTTATCCTACGAATGGTTATACTAGATATGCCGATAGCGTCAAG<br>GGCCGTTTCACTATAAGCGCAGACACATCCAAAAACACAGCCTACCTGCAGATGAACAGCCTGCG<br>TGCTGAGGACACTGCCGTCTATTATTGTTCTAGGTGGGGAGGGGACGGCTTCTATGCTATGGACT<br>ATTGGGGTCAAGGAACCCTGGTCACTGTCTCCTCCGGTGGTGGTGGTTCTGGCGGCGGCGGCTCC<br>GGTGGTGGTGGTTCTGATATCCAGATGACCCAGTCCCCGAGCTCCCTGTCCGCCTCTGTGGGCGA<br>TAGGGTCACCATCACCTGCCGTGCCAGTCAGGATGTGAATACTGCTGTAGCCTGGTATCAACAGA<br>AACCAGGAAAAGCTCCGAAACTACTGATTTACTCGGCATCCTTCCTCTACTCTGGAGTCCCTTCT<br>CGCTTCTCTGGATCCAGATCTGGGACGGATTTCACTCTGACCATCAGCAGTCTGCAGCCGGAAGA<br>CTTCGCAACTTATTACTGTCAGCAACATTATACTACTCCTCCCACGTTCGGACAGGGTACCAAGG<br>TGGAGATCAAAGGAGGTGGTGGATCCGAGGTGCAGCTGGTCGAGTCTGGAGGAGGATTGGTGCAG<br>CCTGGAGGGTCATTGAAACTCTCATGTGCAGCCTCTGGATTCACCTTCAATAAGTACGCCATGAA<br>CTGGGTCCGCCAGGCTCCAGGAAAGGGTTTGGAATGGGTTGCTCGCATAAGAAGTAAATATAATA<br>ATTATGCAACATATTATGCCGATTCAGTGAAAGACAGGTTCACCATCTCCAGAGATGATTCAAAA<br>AACACTGCCTATCTACAAATGAACAACTTGAAAACTGAGGACACTGCCGTGTACTACTGTGTGAG<br>ACATGGGAACTTCGGTAATAGCTACATATCCTACTGGGCTTACTGGGGCCAAGGGACTCTGGTCA<br>CCGTCTCCTCAGGTGGTGGTGGTTCTGGCGGCGGCGGCTCCGGTGGTGGTGGTTCTCAGACTGTT<br>GTGACTCAGGAACCTTCACTCACCGTATCACCTGGTGGAACAGTCACACTCACTTGTGGCTCCTC<br>GACTGGGGCTGTTACATCTGGCTACTACCCAAACTGGGTCCAACAAAAACCAGGTCAGGCACCCC<br>GTGGTCTAATAGGTGGGACTAAGTTCCTCGCCCCCGGTACTCCTGCCAGATTCTCAGGCTCCCTG<br>CTTGGAGGCAAGGCTGCCCTCACCCTCTCAGGGGTACAGCCAGAGGATGAGGCAGAATATTACTG<br>TGCTCTATGGTACAGCAACCGCTGGGTGTTCGGTGGAGGAACCAAACTGACTGTCCTA |
| 439 | Her2/neu VL-VH x H2C VH VL | artificial | aa | DIQMTQSPSSLSASVGDRVTITCRASQDVNTAVAWYQQKPGKAPKLLIYSASFLYSGVPSRFSGS<br>RSGTDFTLTISSLQPEDFATYYCQQHYTTPPTFGQGTKVEIKGGGGSGGGGSGGGGSEVQLVESG<br>GGLVQPGGSLRLSCAASGFNIKDTYIHWVRQAPGKGLEWVARIYPTNGYTRYADSVKGRFTISAD<br>TSKNTAYLQMNSLRAEDTAVYYCSRWGGDGFYAMDYWGQGTLVTVSSGGGGSEVQLVESGGGLVQ<br>PGGSLKLSCAASGFTFNKYAMNWVRQAPGKGLEWVARIRSKYNNYATYYADSVKDRFTISRDDSK<br>NTAYLQMNNLKTEDTAVYYCVRHGNFGNSYISYWAYWGQGTLVTVSSGGGGSGGGGSGGGGSQTV<br>VTQEPSLTVSPGGTVTLTCGSSTGAVTSGYYPNWVQQKPGQAPRGLIGGTKFLAPGTPARFSGSL |

148

EP 2 155 788 B1

| | | | | LGGKAALTLSGVQPEDEAEYYCALWYSNRWVFGGGTKLTVL |
|---|---|---|---|---|
| 440 | Her2/neu VL-VH x H2C VH VL | artificial | nt | GATATCCAGATGACCCAGTCCCCGAGCTCCCTGTCCGCCTCTGTGGGCGATAGGGTCACCATCAC CTGCCGTGCCAGTCAGGATGTGAATACTGCTGTAGCCTGGTATCAACAGAAACCAGGAAAAGCTC CGAAACTACTGATTTACTCGGCATCCTTCCTCTACTCTGGAGTCCCTTCTCGCTTCTCTGGATCC AGATCTGGGACGGATTTCACTCTGACCATCAGCAGTCTGCAGCCGGAAGACTTCGCAACTTATTA CTGTCAGCAACATTATACTACTCCTCCCACGTTCGGACAGGGTACCAAGGTGGAGATCAAAGGTG GTGGTGGTTCTGGCGGCGGCGGCTCCGGTGGTGGTGGTTCTGAGGTTCAGCTGGTGGAGTCTGGC GGTGGCCTGGTGCAGCCAGGGGGCTCACTCCGTTTGTCCTGTGCAGCTTCTGGCTTCAACATTAA AGACACCTATATACACTGGGTGCGTCAGGCCCCGGGTAAGGGCCTGGAATGGGTTGCAAGGATTT ATCCTACGAATGGTTATACTAGATATGCCGATAGCGTCAAGGGCCGTTTCACTATAAGCGCAGAC ACATCCAAAAACACAGCCTACCTGCAGATGAACAGCCTGCGTGCTGAGGACACTGCCGTCTATTA TTGTTCTAGGTGGGGAGGGGGACGGCTTCTATGCTATGGACTATTGGGGTCAAGGAACCCTGGTCA CTGTCTCCTCCGGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAAGTACGCCATGAA CTGGGTCCGCCAGGCTCCAGGAAAGGGTTTGGAATGGGTTGCTCGCATAAGAAGTAAATATAATA ATTATGCAACATATTATGCCGATTCAGTGAAAGACAGGTTCACCATCTCCAGAGATGATTCAAAA AACACTGCCTATCTACAAATGAACAACTTGAAAACTGAGGACACTGCCGTGTACTACTGTGTGAG ACATGGGAACTTCGGTAATAGCTACATATCCTACTGGGCTTACTGGGGCCAAGGGACTCTGGTCA CCGTCTCCTCAGGTGGTGGTGGTTCTGGCGGCGGCGGCTCCGGTGGTGGTGGTTCTCAGACTGTT GTGACTCAGGAACCTTCACTCACCGTATCACCTGGTGGAACAGTCACACTCACTTGTGGCTCCTC GACTGGGGCTGTTACATCTGGCTACTACCCAAACTGGGTCCAACAAAAACCAGGTCAGGCACCCC GTGGTCTAATAGGTGGGACTAAGTTCCTCGCCCCCGGTACTCCTGCCAGATTCTCAGGCTCCCTG CTTGGAGGCAAGGCTGCCCTCACCCTCTCAGGGGTACAGCCAGAGGATGAGGCAGAATATTACTG TGCTCTATGGTACAGCAACCGCTGGGTGTTCGGTGGAGGAACCAAACTGACTGTCCTA |
| 441 | HCDR1 of EGFR | artificial | aa | SGDYYWT |
| 442 | HCDR2 of EGFR | artificial | aa | HIYYSGNTNYNPSLKS |
| 443 | HCDR3 of EGFR | artificial | aa | DRVTGAFDI |
| 444 | LCDR1 of EGFR | artificial | aa | QASQDISNYLN |
| 445 | LCDR2 of EGFR | artificial | aa | DASNLET |
| 446 | LCDR3 of EGFR | artificial | aa | QHFDHLPLA |
| 447 | EGFR HL x H2C | artificial | aa | QVQLQESGPGLVKPSETLSLTCTVSGGSVSSGDYYWTWIRQSPGKGLEWIGHIYYSGNTNYNPSL |

| | HL | | | KSRLTISIDTSKTQFSLKLSSVTAADTAIYYCVRDRVTGAFDIWGQGTMVTVSSGGGGSGGGGSG<br>GGGSDIQMTQSPSSLSASVGDRVTITCQASQDISNYLNWYQQKPGKAPKLLIYDASNLETGVPSR<br>FSGSGSGTDFTFTISSLQPEDIATYFCQHFDHLPLAFGGGTKVEIKSGGGGSEVQLVESGGGLVQ<br>PGGSLKLSCAASGFTFNKYAMNWVRQAPGKGLEWVARIRSKYNNYATYYADSVKDRFTISRDDSK<br>NTAYLQMNNLKTEDTAVYYCVRHGNFGNSYISYWAYWGQGTLVTVSSGGGGSGGGGSGGGGSQTV<br>VTQEPSLTVSPGGTVTLTCGSSTGAVTSGYYPNWVQQKPGQAPRGLIGGTKFLAPGTPARFSGSL<br>LGGKAALTLSGVQPEDEAEYYCALWYSNRWVFGGGTKLTVL |
| 448 | EGFR HL x H2C HL | artificial | nt | CAGGTGCAGCTGCAGGAGTCGGGCCCAGGACTGGTGAAGCCTTCGGAGACCCTGTCCCTCACCTG<br>CACTGTCTCTGGTGGCTCCGTCAGCAGTGGTGATTACTACTGGACCTGGATCCGGCAGTCCCCAG<br>GGAAGGGACTGGAGTGGATTGGACACATCTATTACAGTGGGAACACCAATTATAACCCCTCCCTC<br>AAGAGCCGACTCACCATATCAATTGACACGTCCAAGACTCAGTTCTCCCTGAAGCTGAGTTCTGT<br>GACCGCTGCGGACACGGCCATTTATTACTGTGTGCGAGATCGAGTGACTGGTGCTTTTGATATCT<br>GGGGCCAAGGGACAATGGTCACCGTCTCTTCCGGTGGTGGTGGTTCTGGCGGCGGCGGCTCCGGT<br>GGTGGTGGTTCTGACATCCAGATGACCCAGTCTCCATCCTCCCTGTCTGCATCTGTCGGAGACAG<br>AGTCACCATCACTTGCCAGGCGAGTCAGGACATCAGCAACTATTTAAATTGGTATCAGCAGAAAC<br>CAGGGAAAGCCCCTAAACTCCTGATCTACGATGCATCCAATTTGGAAACAGGGGTCCCATCAAGG<br>TTCAGTGGAAGTGGATCTGGGACAGATTTTACTTTCACCATCAGCAGCCTGCAGCCTGAAGATAT<br>TGCAACATATTTCTGTCAACACTTTGATCATCTCCCGCTCGCTTTCGGCGGAGGGACCAAGGTGG<br>AGATCAAATCCGGAGGTGGTGGATCCGAGGTGCAGCTGGTCGAGTCTGGAGGAGGATTGGTGCAG<br>CCTGGAGGGTCATTGAAACTCTCATGTGCAGCCTCTGGATTCACCTTCAATAAGTACGCCATGAA<br>CTGGGTCCGCCAGGCTCCAGGAAAGGGTTTGGAATGGGTTGCTCGCATAAGAAGTAAATATAATA<br>ATTATGCAACATATTATGCCGATTCAGTGAAAGACAGGTTCACCATCTCCAGAGATGATTCAAAA<br>AACACTGCCTATCTACAAATGAACAACTTGAAAACTGAGGACACTGCCGTGTACTACTGTGTGAG<br>ACATGGGAACTTCGGTAATAGCTACATATCCTACTGGGCTTACTGGGGCCAAGGGACTCTGGTCA<br>CCGTCTCCTCAGGTGGTGGTGGTTCTGGCGGCGGCGGCTCCGGTGGTGGTGGTTCTCAGACTGTT<br>GTGACTCAGGAACCTTCACTCACCGTATCACCTGGTGGAACAGTCACACTCACTTGTGGCTCCTC<br>GACTGGGGCTGTTACATCTGGCTACTACCCAAACTGGGTCCAACAAAAACCAGGTCAGGCACCCC<br>GTGGTCTAATAGGTGGGACTAAGTTCCTCGCCCCCGGTACTCCTGCCAGATTCTCAGGCTCCCTG<br>CTTGGAGGCAAGGCTGCCCTCACCCTCTCAGGGGTACAGCCAGAGGATGAGGCAGAATATTACTG<br>TGCTCTATGGTACAGCAACCGCTGGGTGTTCGGTGGAGGAACCAAACTGACTGTCCTA |
| 449 | EGFR HL x F12Q LH | artificial | aa | QVQLQESGPGLVKPSETLSLTCTVSGGSVSSGDYYWTWIRQSPGKGLEWIGHIYYSGNTNYNPSL<br>KSRLTISIDTSKTQFSLKLSSVTAADTAIYYCVRDRVTGAFDIWGQGTMVTVSSggggsgggggs<br>ggggsDIQMTQSPSSLSASVGDRVTITCQASQDISNYLNWYQQKPGKAPKLLIYDASNLETGVP<br>SRFSGSGSGTDFTFTISSLQPEDIATYFCQHFDHLPLAFGGGTKVEIKSGGGGSEVQLVESGGGL<br>VQPGGSLKLSCAASGFTFNSYAMNWVRQAPGKGLEWVARIRSKYNNYATYYADSVKGRFTISRDD |

EP 2 155 788 B1

| | | | | |
|---|---|---|---|---|
| | | | | SKNTAYLQMNNLKTEDTAVYYCVRHGNFGNSYVSWWAYWGQGTLVTVSSGGGGSGGGGSGGGGSQ TVVTQEPSLTVSPGGTVTLTCGSSTGAVTSGNYPNWVQQKPGQAPRGLIGGTKFLAPGTPARFSG SLLGGKAALTLSGVQPEDEAEYYCVLWYSNRWVFGGGTKLTVL |
| 450 | EGFR HL x F12Q LH | artificial | nt | CAGGTGCAGCTGCAGGAGTCGGGCCCAGGACTGGTGAAGCCTTCGGAGACCCTGTCCCTCACCTG CACTGTCTCTGGTGGCTCCGTCAGCAGTGGTGATTACTACTGGACCTGGATCCGGCAGTCCCCAG GGAAGGGACTGGAGTGGATTGGACACATCTATTACAGTGGGAACACCAATTATAACCCCTCCCTC AAGAGCCGACTCACCATATCAATTGACACGTCCAAGACTCAGTTCTCCCTGAAGCTGAGTTCTGT GACCGCTGCGGACACGGCCATTTATTACTGTGTGCGAGATCGAGTGACTGGTGCTTTTGATATCT GGGGCCAAGGGACAATGGTCACCGTCTCTTCCGGTGGTGGTGGTTCTGGCGGCGGCGGCTCCGGT GGTGGTGGTTCTGACATCCAGATGACCCAGTCTCCATCCTCCCTGTCTGCATCTGTCGGAGACAG AGTCACCATCACTTGCCAGGCGAGTCAGGACATCAGCAACTATTTAAATTGGTATCAGCAGAAAC CAGGGAAAGCCCCTAAACTCCTGATCTACGATGCATCCAATTTGGAAACAGGGGTCCCATCAAGG TTCAGTGGAAGTGGATCTGGGACAGATTTTACTTTCACCATCAGCAGCCTGCAGCCTGAAGATAT TGCAACATATTTCTGTCAACACTTTGATCATCTCCCGCTCGCTTTCGGCGGAGGGACCAAGGTGG AGATCAAATCCGGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAGCTACGCCATGAA CTGGGTCCGCCAGGCTCCAGGAAAGGGTTTGGAATGGGTTGCTCGCATAAGAAGTAAATATAATA ATTATGCAACATATTATGCCGATTCAGTGAAAGGCAGGTTCACCATCTCCAGAGATGATTCAAAA AACACTGCCTATCTACAAATGAACAACTTGAAAACTGAGGACACTGCCGTGTACTACTGTGTGAG ACATGGGAACTTCGGTAATAGCTACGTTTCCTGGTGGGCTTACTGGGGCCAAGGGACTCTGGTCA CCGTCTCCTCAGGTGGTGGTGGTTCTGGCGGCGGCGGCTCCGGTGGTGGTGGTTCTCAGACTGTT GTGACTCAGGAACCTTCACTCACCGTATCACCTGGTGGAACAGTCACACTCACTTGTGGCTCCTC GACTGGGGCTGTTACATCTGGCAACTACCCAAACTGGGTCCAACAAAAACCAGGTCAGGCACCCC GTGGTCTAATAGGTGGGACTAAGTTCCTCGCCCCCGGTACTCCTGCCAGATTCTCAGGCTCCCTG CTTGGAGGCAAGGCTGCCCTCACCCTCTCAGGGGTACAGCCAGAGGATGAGGCAGAATATTACTG TGTTCTATGGTACAGCAACCGCTGGGTGTTCGGTGGAGGAACCAAACTGACTGTCCTA |
| 451 | EGFR HL x I2C HL | artificial | aa | QVQLQESGPGLVKPSETLSLTCTVSGGSVSSGDYYWTWIRQSPGKGLEWIGHIYYSGNTNYNPSL KSRLTISIDTSKTQFSLKLSSVTAADTAIYYCVRDRVTGAFDIWGQGTMVTVSSggggsggggs ggggsDIQMTQSPSSLSASVGDRVTITCQASQDISNYLNWYQQKPGKAPKLLIYDASNLETGVP SRFSGSGSGTDFTFTISSLQPEDIATYFCQHFDHLPLAFGGGTKVEIKSGGGGSEVQLVESGGGL VQPGGSLKLSCAASGFTFNKYAMNWVRQAPGKGLEWVARIRSKYNNYATYYADSVKDRFTISRDD SKNTAYLQMNNLKTEDTAVYYCVRHGNFGNSYISYWAYWGQGTLVTVSSGGGGSGGGGSGGGGSQ TVVTQEPSLTVSPGGTVTLTCGSSTGAVTSGNYPNWVQQKPGQAPRGLIGGTKFLAPGTPARFSG SLLGGKAALTLSGVQPEDEAEYYCVLWYSNRWVFGGGTKLTVL |

| 452 | EGFR HL x I2C HL | artificial | nt | CAGGTGCAGCTGCAGGAGTCGGGCCCAGGACTGGTGAAGCCTTCGGAGACCCTGTCCCTCACCTG CACTGTCTCTGGTGGCTCCGTCAGCAGTGGTGATTACTACTGGACCTGGATCCGGCAGTCCCCAG GGAAGGGACTGGAGTGGATTGGACACATCTATTACAGTGGGAACACCAATTATAACCCCTCCCTC AAGAGCCGACTCACCATATCAATTGACACGTCCAAGACTCAGTTCTCCCTGAAGCTGAGTTCTGT GACCGCTGCGGACACGGCCATTTATTACTGTGTGCGAGATCGAGTGACTGGTGCTTTTGATATCT GGGGCCAAGGGACAATGGTCACCGTCTCTTCCGGTGGTGGTGGTTCTGGCGGCGGCGGCTCCGGT GGTGGTGGTTCTGACATCCAGATGACCCAGTCTCCATCCTCCCTGTCTGCATCTGTCGGAGACAG AGTCACCATCACTTGCCAGGCGAGTCAGGACATCAGCAACTATTTAAATTGGTATCAGCAGAAAC CAGGGAAAGCCCCTAAACTCCTGATCTACGATGCATCCAATTTGGAAACAGGGGTCCCATCAAGG TTCAGTGGAAGTGGATCTGGGACAGATTTTACTTTCACCATCAGCAGCCTGCAGCCTGAAGATAT TGCAACATATTTCTGTCAACACTTTGATCATCTCCCGCTCGCTTTCGGCGGAGGGACCAAGGTGG AGATCAAATCCGGAGGTGGTGGATCCGAGGTGCAGCTGGTCGAGTCTGGAGGAGGATTGGTGCAG CCTGGAGGGTCATTGAAACTCTCATGTGCAGCCTCTGGATTCACCTTCAATAAGTACGCCATGAA CTGGGTCCGCCAGGCTCCAGGAAAGGGTTTGGAATGGGTTGCTCGCATAAGAAGTAAATATAATA ATTATGCAACATATTATGCCGATTCAGTGAAAGACAGGTTCACCATCTCCAGAGATGATTCAAAA AACACTGCCTATCTACAAATGAACAACTTGAAAACTGAGGACACTGCCGTGTACTACTGTGTGAG ACATGGGAACTTCGGTAATAGCTACATATCCTACTGGGCTTACTGGGGCCAAGGGACTCTGGTCA CCGTCTCCTCAGGTGGTGGTGGTTCTGGCGGCGGCGGCTCCGGTGGTGGTGGTTCTCAGACTGTT GTGACTCAGGAACCTTCACTCACCGTATCACCTGGTGGAACAGTCACACTCACTTGTGGCTCCTC GACTGGGGCTGTTACATCTGGCAACTACCCAAACTGGGTCCAACAAAAACCAGGTCAGGCACCCC GTGGTCTAATAGGTGGGACTAAGTTCCTCGCCCCCGGTACTCCTGCCAGATTCTCAGGCTCCCTG CTTGGAGGCAAGGCTGCCCTCACCCTCTCAGGGGTACAGCCAGAGGATGAGGCAGAATATTACTG TGTTCTATGGTACAGCAACCGCTGGGTGTTCGGTGGAGGAACCAAACTGACTGTCCTA |
| 453 | HCDR1 of EGFR | artificial | aa | NYGVH |
| 454 | HCDR2 of EGFR | artificial | aa | VIWSGGNTDYNTPFTS |
| 455 | HCDR3 of EGFR | artificial | aa | ALTYYDYEFAY |
| 456 | LCDR1 of EGFR | artificial | aa | RASQSIGTNIH |
| 457 | LCDR2 of EGFR | artificial | aa | YASESIS |
| 458 | LCDR3 of EGFR | artificial | aa | QQNNNWPTT |
| 459 | human HER2 protein | human | nt | ATGGAGCTGGCGGCCTTGTGCCGCTGGGGGCTCCTCCTCGCCCTCTTGCCCCCCGGAGCCGCGAG CACCCAAGTGTGCACCGGCACAGACATGAAGCTGCGGCTCCCTGCCAGTCCCGAGACCCACCTGG ACATGCTCCGCCACCTCTACCAGGGCTGCCAGGTGGTGCAGGGAAACCTGGAACTCACCTACCTG |

EP 2 155 788 B1

```
CCCACCAATGCCAGCCTGTCCTTCCTGCAGGATATCCAGGAGGTGCAGGGCTACGTGCTCATCGC
TCACAACCAAGTGAGGCAGGTCCCACTGCGCCGTGCTAGACAATGGAGACCCGCTGAACATACCACCCCTGTCTTTG
AGGACAACTATGCCCTGGCCGTGCTAGAGCTGCAGCTTCGAGCCTCGAACGATCTCACAGAGATCTTGAAAGGAGG
GGGGCCTCCCCAGGAGGCCTGCGGGAGCTGCAGCTTCCAGGACACCAACCGCTCTCGGGCCTGCCACCCCTGT
GGTCTTGATCCAGCGGAACCCCAGCTCTCCACACTGATAGACACCAACCGCTCTCGGGCCTGCCACCCCTGT
ACAAGAACAACCAGCTGGCTCTCCACTGATAGACAGCACCAACCGCTCTCGGGCCTGCCACCCCTGT
TCTCCGATGTGTAAGGGCTGGCTGTGCCCGCTGCCCGTGCTGCCCTGCCCAGGGGCCACTGCCCACTGCTCGCCATGAGC
CACTGTCTGTGCCGGTGGCTGCACGGGCCTGCCAGCCTGGCTCACTACAACACAGACACGTTGAGTCCAT
AGTGTGCTGCGGCCTGCACTGCCAGTTTGAGCTCACTGCTACAACACAGACACGTTGAGTCCAT
AGTGGCATCTGTGAGCTGCACTGCCGTATACATTCGGCGCGTCGTCCCCTGCACAACCAAGAGGTGACAGCAGAG
GCCCAATCCCGAGGGGGCCGGTATACATTCGGCGCGTCGTCCCCTGCACAACCAAGAGGTGACAGCAGAG
TTTCTACGGACGTGGGATCCTGCACCCTCGTCTGCCCCCTGCACAACCAAGAGGTGACAGCAGAG
GATGGAACACAGCCGGTGTGAGAAGTGCAGCAAGACCCCTGTGCCCAGTGTGCTATGGTCTGGGCAT
GGAGCCACTTGCTGAGGTGAGGGCAGTTACCAGTGCCAATATCCAGGAGTTGCTGGCTGCAAGA
AGATCTTTGGGAGCCTGGCATTTCTGCCGGAAAGCTTTGATGGGGACCCAGCCTCCAACACTGCC
CCGCTCCAGCCAGAGCAGCTCCAAGTGTTTGAACTCTGGAAGAGATCACAGGTTACCTATACAT
CTCAGCCATGGCCGGACAGCCTGCCTACTGCTCTGACCCTGCTGCCAGGGTCGCATCTGGGGCTG
GAATTCTGCACAATGGCGCCTACTGCTGGACTGGCCCTCATCCACCATAACACCCACCTGTCTTCGT
CGCTCACTGAGGGAACTGGGCAGTGGCCAGTGACTGGCCCTCATCTGCTCCACACTGCCAACC
GCACACGGTGCCCTGGGACCAGCTCTTTCGGAACCGCCTGCCTGCGCGCCCGAGGGCCACTGC
GGCCAGAGGACGAGTGTGGGCGAGGGCCTGCACTGCAGCTGTCAACTGCAGCCAGTTCCTTCGGGGCCCAGGAGTGCGTGGA
TGGGGTCCAGGGCCCACCCAGTGTCAACTGCCCCAGGGAGTATGTGAATGGGGCCAGCCACTGTTGCCGTGCC
GGAATGCCGAGTACTGCAGGGGGCTCCCCAGAATGGCTCAGTGACCTGTTTGGACCGGCCCGTGCCCGCTGGTGTG
ACCCTGAGTGTGCCCACTATAAGGACCCTCCCTTCTGCGTGCCCGCTGTGACCAGCGTGTGAAACCTGA
GCCTGTGCCACTACTGCCCTATGCCCCATCCTGGAAGTTCCAGATGACGAGGAGGGCTGCCCGCCTTGCCCCATCA
CCTCTCCTACATGCCCACCTCCTGTGTGACCTGGATGACTGGATGACAAGAGGGCTGCCCGAGAGCAGCCCCT
ACTGCACCCACCTCCTGTGTGACCTGGATGGTTGGCATTCTGCTGTTCGTGTTCGGGGTCTTTGG
CTGAGCGTCCATCATCTCTGCGGTGGTTGGCATTCTGCTGTTCGTGTTCGGGGTCTTTGG
GATCCTTCATCAAGCGACGGCAGCAGAGATCCGGAGCAGAAGTACACGATGCGGAGACTGCTGCAGGAAA
CGGAGCTGGTGGAGCGCTGGTACTGCCCGCTGACCTGGGAGCGCGATGCCCAACCAGCCGCAGATGCGGATCCTG
AAAGAGACGGAGGCTGAGGAAGGTGAAGCTGGATCTGGATCTGGATCTTTGGCGCTTTTGGCGAGAATGCGGAGAAA
CATCTGGATCCCTGATGGGGAGGAATGTGAAAATTCCAGTCAGTGCCATCAAAGTGTTGAGGGAAAACA
CATCCCCCAAAGCCAACAAAGAAATCTTAGACGAAGCATACGTGATGGCTGGTGTGTGGGCTCCCCA
TATGTCTCCCGCCTTCGGGCATCTGCCTGACATCCACGGTGACATCCACGGTGACACAGCTTATGCC
CTATGGCTGCCTCTTAGACATGTCCGGGAAAAACCGCGGACGCCTCCAGGACCTCCGAGGATGTCGCGGCTCCTACACAGGGAC
ACTGGTTATGCAGATTGCCAGGGGATGAGCTACCTGGAGGATGTGCGGCTGCCAACCGACACTTCGGGGCTGGC
TTGGCCGCTGCTGGAACGTGCTGGTGCTGCTGCAAGAGTGCCAACCATGTCAAAATTACAGACTTCGGGGCTGGC
```

EP 2 155 788 B1

|  |  |  |  | TCGGCTGCTGGACATTGACGAGACAGAGTACCATGCAGATGGGGGCAAGGTGCCCATCAAGTGGA<br>TGGCGCTGGAGTCCATTCTCCGCCGGCGGTTCACCCACCAGAGTGATGTGTGGAGTTATGGTGTG<br>ACTGTGTGGGAGCTGATGACTTTTGGGGCCAAACCTTACGATGGGATCCCAGCCCGGGAGATCCC<br>TGACCTGCTGGAAAAGGGGGAGCGGCTGCCCCAGCCCCCCATCTGCACCATTGATGTCTACATGA<br>TCATGGTCAAATGTTGGATGATTGACTCTGAATGTCGGCCAAGATTCCGGGAGTTGGTGTCTGAA<br>TTCTCCCGCATGGCCAGGGACCCCCAGCGCTTTGTGGTCATCCAGAATGAGGACTTGGGCCCAGC<br>CAGTCCCTTGGACAGCACCTTCTACCGCTCACTGCTGGAGGACGATGACATGGGGGACCTGGTGG<br>ATGCTGAGGAGTATCTGGTACCCCAGCAGGGCTTCTTCTGTCCAGACCCTGCCCCGGGCGCTGGG<br>GGCATGGTCCACCACAGGCACCGCAGCTCATCTACCAGGAGTGGCGGTGGGGACCTGACACTAGG<br>GCTGGAGCCCTCTGAAGAGGAGGCCCCCAGGTCTCCACTGGCACCCTCCGAAGGGGCTGGCTCCG<br>ATGTATTTGATGGTGACCTGGGAATGGGGGCAGCCAAGGGGCTGCAAAGCCTCCCCACACATGAC<br>CCCAGCCCTCTACAGCGGTACAGTGAGGACCCCACAGTACCCCTGCCCTCTGAGACTGATGGCTA<br>CGTTGCCCCCCTGACCTGCAGCCCCCAGCCTGAATATGTGAACCAGCCAGATGTTCGGCCCCAGC<br>CCCCTTCGCCCCGAGAGGGCCCTCTGCCTGCTGCCCGACCTGCTGGTGCCACTCTGGAAAGGCCC<br>AAGACTCTCTCCCCAGGGAAGAATGGGGTCGTCAAAGACGTTTTTGCCTTTGGGGGTGCCGTGGA<br>GAACCCCGAGTACTTGACACCCCAGGGAGGAGCTGCCCCTCAGCCCCACCCTCCTCCTGCCTTCA<br>GCCCAGCCTTCGACAACCTCTATTACTGGGACCAGGACCCACCAGAGCGGGGGGGCTCCACCCAGC<br>ACCTTCAAAGGGACACCTACGGCAGAGAACCCAGAGTACCTGGGTCTGGACGTGCCAGTGTGA |
| 460 | human HER2 protein | human | aa | MELAALCRWGLLLALLPPGAASTQVCTGTDMKLRLPASPETHLDMLRHLYQGCQVVQGNLELTYL<br>PTNASLSFLQDIQEVQGYVLIAHNQVRQVPLQRLRIVRGTQLFEDNYALAVLDNGDPLNNTTPVT<br>GASPGGLRELQLRSLTEILKGGVLIQRNPQLCYQDTILWKDIFHKNNQLALTLIDTNRSRACHPC<br>SPMCKGSRCWGESSEDCQSLTRTVCAGGCARCKGPLPTDCCHEQCAAGCTGPKHSDCLACLHFNH<br>SGICELHCPALVTYNTDTFESMPNPEGRYTFGASCVTACPYNYLSTDVGSCTLVCPLHNQEVTAE<br>DGTQRCEKCSKPCARVCYGLGMEHLREVRAVTSANIQEFAGCKKIFGSLAFLPESFDGDPASNTA<br>PLQPEQLQVFETLEEITGYLYISAWPDSLPDLSVFQNLQVIRGRILHNGAYSLTLQGLGISWLGL<br>RSLRELGSGLALIHHNTHLCFVHTVPWDQLFRNPHQALLHTANRPEDECVGEGLACHQLCARGHC<br>WGPGPTQCVNCSQFLRGQECVEECRVLQGLPREYVNARHCLPCHPECQPQNGSVTCFGPEADQCV<br>ACAHYKDPPFCVARCPSGVKPDLSYMPIWKFPDEEGACQPCPINCTHSCVDLDDKGCPAEQRASP<br>LTSIISAVVGILLVVVLGVVFGILIKRRQQKIRKYTMRRLLQETELVEPLTPSGAMPNQAQMRIL<br>KETELRKVKVLGSGAFGTVYKGIWIPDGENVKIPVAIKVLRENTSPKANKEILDEAYVMAGVGSP<br>YVSRLLGICLTSTVQLVTQLMPYGCLLDHVRENRGRLGSQDLLNWCMQIAKGMSYLEDVRLVHRD<br>LAARNVLVKSPNHVKITDFGLARLLDIDETEYHADGGKVPIKWMALESILRRRFTHQSDVWSYGV<br>TVWELMTFGAKPYDGIPAREIPDLLEKGERLPQPPICTIDVYMIMVKCWMIDSECRPRFRELVSE<br>FSRMARDPQRFVVIQNEDLGPASPLDSTFYRSLLEDDDMGDLVDAEEYLVPQQGFFCPDPAPGAG<br>GMVHHRHRSSSTRSGGGDLTLGLEPSEEEAPRSPLAPSEGAGSDVFDGDLGMGAAKGLQSLPTHD<br>PSPLQRYSEDPTVPLPSETDGYVAPLTCSPQPEYVNQPDVRPQPPSPREGPLPAARPAGATLERP |

EP 2 155 788 B1

| | | | | |
|---|---|---|---|---|
| | | | | KTLSPGKNGVVKDVFAFGGAVENPEYLTPQGGAAPQPHPPPAFSPAFDNLYYWDQDPPERGAPPS TFKGTPTAENPEYLGLDVPV |
| 461 | chimeric human/ macaque HER2 protein | artificial | nt | ATGGAGCTGGCGGCCTTGTGCCGCTGGGGGCTCCTCCTCGCCCTCTTGCCCCCCGGAGCCGCGAG CACCCAAGTGTGCACCGGCACAGACATGAAGCTGCGGCTCCCTGCCAGTCCCGAGACCCACCTGG ACATGCTCCGCCACCTCTACCAGGGCTGCCAGGTGGTGCAGGGAAACCTGGAACTCACCTACCTG CCCACCAATGCCAGCCTGTCCTTCCTGCAGGATATCCAGGAGGTGCAGGGCTACGTGCTCATCGC TCACAACCAAGTGAGGCAGGTCCCACTGCAGAGGCTGCGGATTGTGCGAGGCACCCAGCTCTTTG AGGACAACTATGCCCTGGCCGTGCTAGACAATGGAGACCCGCTGAACAATACCACCCCTGTCACA GGGGCCTCCCCAGGAGGCCTGCGGGAGCTGCAGCTTCGAAGCCTCACAGAGATCTTGAAAGGAGG GGTCTTGATCCAGCGGAACCCCCAGCTCTGCTACCAGGACACGATTTTGTGGAAGGACATCTTCC ACAAGAACAACCAGCTGGCTCTCACACTGATAGACACCAACCGCTCTCGGGCCTGCCACCCCTGT TCTCCAGTGTGTAAGGGCTCCCGCTGCTGGGGAGAGAGTTCTGAGGATTGTCAGAGCCTGACGCG CACTGTCTGTGCCGGTGGCTGTGCCCGCTGCAAGGGGCCACTGCCCACTGACTGCTGCCATGAGC AGTGTGCTGCCGGCTGCACGGGCCCCAAGCACTCTGACTGCCTGGCCTGCCTCCACTTCAACCAC AGTGGCATCTGTGAGCTGCACTGCCCAGCCCTGGTCACCTACAACACAGACACGTTTGAGTCCAT GCCCAATCCCGAGGGCCGGTATACATTCGGCGCCAGCTGTGTGACTGCCTGTCCCTACAACTACC TTTCTACGGACGTGGGATCCTGCACCCTCGTCTGCCCCCTGCACAACCAAGAGGTGACAGCAGAG GATGGAACACAGCGGTGTGAGAAGTGCAGCAAGCCCTGTGCCCGAGTGTGCTATGGTCTGGGCAT GGAGCACTTGCGAGAGGTGAGGGCAGTTACCAGTGCCAATATCCAGGAGTTTGCTGGCTGCAAGA AGATCTTTGGGAGCCTGGCATTTCTGCCGGAAAGCTTTGATGGGGACCCAGCCTCCAACACTGCC CCGCTTCAGCCGGAGCAGCTCCGAGTGTTTGAGACTCTGGAAGAGATCACAGGTTACCTATACAT CTCAGCATGGCCAGACAGCCTGCCTGACCTTAGCGTCCTCCAGAACCTGCAAGTAATCCGGGGAC GAATTCTGCACAATGGCGCCTACTCGCTGACCCTGCAAGGGCTGGGCATCAGCTGGCTGGGGCTG CGCTCACTGAGGGAACTGGGCAGTGGACTGGCCCTCATCCACCATAACACCCGCCTCTGCTTTGT GCACACGGTGCCCTGGGACCAGCTCTTTCGGAACCCGCACCAAGCTCTGCTCCACACTGCCAACC GGCCAGAGGACGAGTGTGTGGGCGAGGGCCTGGCCTGCCACCAGCTGTGCGCCCGAGGGCACTGC TGGGGTCCAGGGCCCACCCAGTGTGTCAACTGCAGCCAGTTCCTTCGGGGCCAGGAGTGCGTGGA GGAATGCCGAGTACTGCAGGGGCTCCCCAGGGAGTATGTGAATGCCAGGCACTGTTTGCCGTGCC ACCCTGAGTGTCAGCCCCAGAATGGCTCAGTGACCTGTTTTGGACCGGAGGCTGACCAGTGTGTG GCCTGTGCCCACTATAAGGACCCTCCCTTCTGCGTGGCCCGCTGCCCCAGCGGTGTGAAACCTGA CCTCTCCTACATGCCCATCTGGAAGTTTCCAGATGAGGAGGGCACGTGCCAGTCTTGCCCCATCA ACTGCACCCACTCCTGTGTGGACCTGGATGACAAGGGCTGCCCCGCCGAGCAGAGAGCCAGCCCT CTGACTAGTATCATCTCTGCGGTGGTTGGCATTCTGCTGGTCGTGGTCTTGGGGGTGGTCTTTGG GATCCTCATCAAGCGACGGCAGCAGAAGATCCGGAAGTACACGATGCGGAGACTGCTGCAGGAAA CGGAGCTGGTGGAGCCGCTGACACCTAGCGGAGCGATGCCCAACCAGGCGCAGATGCGGATCCTG AAAGAGACGGAGCTGAGGAAGGTGAAGGTGCTTGGATCTGGCGCTTTTGGCACAGTCTACAAGGG |

| 462 | chimeric human/ macaque HER2 protein | artificial | aa | CATCTGGATCCCTGATGGGGAGAATGTGAAAATTCCAGTGCCATCAAAGTGTTGAGGAGGAAAACA<br>CATCCCCCCAAAGCCAACAAAGAAATCTTAGACGAAGCATACGTGATGGCTGGTGTGACAGCTCCCCA<br>TATGTCTCCGCCTTCTGGCATCTGCCTGACATCCACGGTCCGGGAAAACCGGCGGACGCCTGGGCTCCCAGGACCTGCTGA<br>CTATGGCTGCCTCTTAGACATGTCCAGGGGATGAGCTACCTGGAGGATGTGCGGCTCGTACAGGAC<br>ACTGGTGTATGCCAAGGGGATTGCCAAGGGGATGAGCTACCTGGAGGATGTGCGGCTCGTACAGGAC<br>TTGGCCGCTCGGAACGTGCTGGTCAAGAGTCCAACCATGTCAAAATTACAGACTTCGGCTGGC<br>TCGGCTGCTGGACATTGACGAGACAGAGTACCATGCAGATGGGGCAAGGTGCCATCAAGTGGA<br>TGGCGGCTGGAGTCCATTCTCCGGCGGTTCACCCGCCGGTTCCCAAACCTTACGATGGGGATCCC<br>ACTGTGTGGGAGCTGATGACTTTTGGGGGCCGCTGCGCCTGCCCCAGAGTCTGCTACGGGCTGGG<br>TGACCTGCTGGAAAAGGGGAGCGGCGGCTGTGCCTTTGACTCTGAATGTCGGCCCAAGATTCCGGAGTTGGTGTCTGAA<br>TCATGGCCAATGTGGATGATTGACTCTCCAGCGCGCTTTGTGGTCATCCGAGGACGATGACACCCTGCTGTGGC<br>TTCTCCCGCATGGCCAGGACCACTTCTACCCGCTCCTCACTGCTGGAGGACGATGACACCCTGCGGGGCTGGTGG<br>CAGTCCCTTGGACAGCACACTTCTACCCCAGCAGGGCTTCTCTGTCCAGGAGTGGCGGCACCCTCCGAAAGGGCGCTGGG<br>ATGCTGAGGAGTATCTGGTACCCCGGTACAGTGAGGACCCCCAGCAGGGCTTCTCTGTCCAGGAGACTGTTCGGCCCCAGC<br>GGCATGGTCCACCACAGGCACCGCAGCTCATCTACACCAGGGGCGGCACCCTCCGAAAGGGCGCTGGG<br>GCTGGAGCCCTCTGAAGAGGAGCCCTGAGGACCCCCAGGTCTCCACTGCTGGAGGGCGCTGCACTAGG<br>ATGTATTTGATGGTGACCTGGGAATGGGGGTACAGTGAGGACCCTCCCACACATGAC<br>CCAGCCCCTCTACAGCGGTACAGTGAGGACCCCCAGCAGTACCCCTGCCCTCTGAGACTGATGGCTA<br>CGTTGCCCCCCTGACCTGCGAGCCCCCTGCCTGCTGCTGCCACCAGCCAGATGTTCGGCCCCAGC<br>CCCCTTCGCCCCGAGAGGGCCCTGCTGTGCCGACCTGCTGGTGCCCACTCTGGAAAGGCCC<br>AAGACTCTCTCCCCAGGAAGAATGGGGTCGTCAAAGACGTTTTGCCTTTGGGGGTGCCGTGGA<br>GAACCCCGAGTACTTGACAACCTCTATTACTGGACCAGGAGGAGCCCCCAGAGCGGGGGCTCCACCCAGC<br>ACCTTCAAAGGACGACACCTGGTCTGGACGTCCCAGTGTGA |

MELAALCRWGLLLALLPPGAASTQVCTGTDMKLRLPASPETHLDMLRHLYQGCQVVQGNLELTYL PTNASLSFLQDIQEVQGYVLIAHNQVRQVPLQRLRIVRGTQLFEDNYALAVLDNGDPLNNTTPVT GASPGGLRELQLRSLTEILKGGVLIQRNPQLCYQDTILWKDIFHKNNQLALTLIDTNRSRACHPC SPVCKGSRCWGESSEDCQSLTRTVCAGGCARCKGPLPTDCCHEQCAAGCTGPKHSDCLACLHFNH SGICELHCPALVTYNTDTFESMPNPEGRYTFGASCVTACPYNYLSTDVGSCTLVCPLHNQEVTAE DGTQRCEKCSKPCARVCYGLGMEHLREVRAVTSANIQEFAGCKKIFGSLAFLPESFDGDPASNTA PLQPEQLRVFETLEEITGYLYISAWPDSLPDLSVLQNLQVIRGRILHNGAYSLTLQGLGISWLGL RSLRELGSGLALIHHNTRLCFVHTVPWDQLFRNPHQALLHTANRPEDECVGEGLACHQLCARGHC WGPGPTQCVNCSQFLRGQECVEECRVLQGLPREYVNARHCLPCHPECQPQNGSVTCFGPEADQCV ACAHYKDPPFCVARCPSGVKPDLSYMPIWKFPDEEGTCQSCPINCTHSCVDLDDKGCPAEQRASP LTSIISAVVGILLVVVLGVVFGILIKRRQQKIRKYTMRRLLQETELVEPLTPSGAMPNQAQMRIL KETELRKVKVLGSGAFGTVYKGIWIPDGENVKIPVAIKVLRENTSPKANKEILDEAYVMAGVGSP

156

| | | | | |
|---|---|---|---|---|
| | | | | YVSRLLGICLTSTVQLVTQLMPYGCLLDHVRENRGRLGSQDLLNWCMQIAKGMSYLEDVRLVHRD LAARNVLVKSPNHVKITDFGLARLLDIDETEYHADGGKVPIKWMALESILRRRFTHQSDVWSYGV TVWELMTFGAKPYDGIPAREIPDLLEKGERLPQPPICTIDVYMIMVKCWMIDSECRPRFRELVSE FSRMARDPQRFVVIQNEDLGPASPLDSTFYRSLLEDDDMGDLVDAEEYLVPQQGFFCPDPAPGAG GMVHHRHRSSSTRSGGGDLTLGLEPSEEEAPRSPLAPSEGAGSDVFDGDLGMGAAKGLQSLPTHD PSPLQRYSEDPTVPLPSETDGYVAPLTCSPQPEYVNQPDVRPQPPSPREGPLPAARPAGATLERP KTLSPGKNGVVKDVFAFGGAVENPEYLTPQGGAAPQPHPPPAFSPAFDNLYYWDQDPPERGAPPS TFKGTPTAENPEYLGLDVPV |
| 463 | HCDR1 of Her2/neu | artificial | aa | DTYIH |
| 464 | HCDR2 of Her2/neu | artificial | aa | RIYPTNGYTRYADSVKG |
| 465 | HCDR3 of Her2/neu | artificial | aa | WGGDGFYAMDY |
| 466 | LCDR1 of Her2/neu | artificial | aa | RASQDVNTAVA |
| 467 | LCDR2 of Her2/neu | artificial | aa | SASFLYS |
| 468 | LCDR3 of Her2/neu | artificial | aa | QQHYTTPPT |
| 469 | Her2/neu HL x H2C HL | artificial | aa | EVQLVESGGGLVQPGGSLRLSCAASGFNIKDTYIHWVRQAPGKGLEWVARIYPTNGYTRYADSVK GRFTISADTSKNTAYLQMNSLRAEDTAVYYCSRWGGDGFYAMDYWGQGTLVTVSSGGGGSGGGGS GGGGSDIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKSGGGGSEVQLVESGGGLV QPGGSLKLSCAASGFTFNKYAMNWVRQAPGKGLEWVARIRSKYNNYATYYADSVKDRFTISRDDS KNTAYLQMNNLKTEDTAVYYCVRHGNFGNSYISYWAYWGQGTLVTVSSGGGGSGGGGSGGGGSQT VVTQEPSLTVSPGGTVTLTCGSSTGAVTSGYYPNWVQQKPGQAPRGLIGGTKFLAPGTPARFSGS LLGGKAALTLSGVQPEDEAEYYCALWYSNRWVFGGGTKLTVL |
| 470 | Her2/neu HL x H2C HL | artificial | nt | GAGGTTCAGCTGGTGGAGTCTGGCGGTGGCCTGGTGCAGCCAGGGGGGCTCACTCCGTTTGTCCTG TGCAGCTTCTGGCTTCAACATTAAAGACACCTATATACACTGGGTGCGTCAGGCCCCGGGTAAGG GCCTGGAATGGGTTGCAAGGATTTATCCTACGAATGGTTATACTAGATATGCCGATAGCGTCAAG |

| | | | | |
|---|---|---|---|---|
| | | | | GGCCGTTTCACTATAAGCGCAGACACATCCAAAAACACAGCCTACCTGCAGATGAACAGCCTGCG TGCTGAGGACACTGCCGTCTATTATTGTTCTAGGTGGGGAGGGGACGGCTTCTATGCTATGGACT ATTGGGGTCAAGGAACCCTGGTCACTGTCTCCTCCGGTGGTGGTGGTTCTGGCGGCGGCGGCTCC GGTGGTGGTGGTTCTGACATCCAGATGACCCAGTCCCCTAGCTCCCTGTCCGCCTCTGTGGGCGA TAGGGTCACCATCACCTGCCGTGCCAGTCAGGATGTGAATACTGCTGTAGCCTGGTATCAACAGA AACCAGGAAAAGCTCCGAAACTACTGATTTACTCGGCATCCTTCCTCTACTCTGGAGTCCCTTCT CGCTTCTCTGGATCCAGATCTGGGACGGATTTCACTCTGACCATCAGCAGTCTGCAGCCGGAAGA CTTCGCAACTTATTACTGTCAGCAACATTATACTACTCCTCCCACGTTCGGACAGGGTACCAAGG TGGAGATCAAATCCGGAGGTGGTGGATCCGAGGTGCAGCTGGTCGAGTCTGGAGGAGGATTGGTG CAGCCTGGAGGGTCATTGAAACTCTCATGTGCAGCCTCTGGATTCACCTTCAATAAGTACGCCAT GAACTGGGTCCGCCAGGCTCCAGGAAAGGGTTTGGAATGGGTTGCTCGCATAAGAAGTAAATATA ATAATTATGCAACATATTATGCCGATTCAGTGAAAGACAGGTTCACCATCTCCAGAGATGATTCA AAAAACACTGCCTATCTACAAATGAACAACTTGAAAACTGAGGACACTGCCGTGTACTACTGTGT GAGACATGGGAACTTCGGTAATAGCTACATATCCTACTGGGCTTACTGGGGCCAAGGGACTCTGG TCACCGTCTCCTCAGGTGGTGGTGGTTCTGGCGGCGGCGGCTCCGGTGGTGGTGGTTCTCAGACT GTTGTGACTCAGGAACCTTCACTCACCGTATCACCTGGTGGAACAGTCACACTCACTTGTGGCTC CTCGACTGGGGCTGTTACATCTGGCTACTACCCAAACTGGGTCCAACAAAAACCAGGTCAGGCAC CCCGTGGTCTAATAGGTGGGACTAAGTTCCTCGCCCCCGGTACTCCTGCCAGATTCTCAGGCTCC CTGCTTGGAGGCAAGGCTGCCCTCACCCTCTCAGGGGTACAGCCAGAGGATGAGGCAGAATATTA CTGTGCTCTATGGTACAGCAACCGCTGGGTGTTCGGTGGAGGAACCAAACTGACTGTCCTA |
| 471 | Her2/neu HL x F12Q HL | artificial | aa | EVQLVESGGGLVQPGGSLRLSCAASGFNIKDTYIHWVRQAPGKGLEWVARIYPTNGYTRYADSVK GRFTISADTSKNTAYLQMNSLRAEDTAVYYCSRWGGDGFYAMDYWGQGTLVTVSSGGGGSGGGGS GGGGSDIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKSGGGGSEVQLVESGGGLV QPGGSLKLSCAASGFTFNSYAMNWVRQAPGKGLEWVARIRSKYNNYATYYADSVKGRFTISRDDS KNTAYLQMNNLKTEDTAVYYCVRHGNFGNSYVSWWAYWGQGTLVTVSSGGGGSGGGGSGGGGSQT VVTQEPSLTVSPGGTVTLTCGSSTGAVTSGNYPNWVQQKPGQAPRGLIGGTKFLAPGTPARFSGS LLGGKAALTLSGVQPEDEAEYYCVLWYSNRWVFGGGTKLTVL |
| 472 | Her2/neu HL x F12Q HL | artificial | nt | GAGGTTCAGCTGGTGGAGTCTGGCGGTGGCCTGGTGCAGCCAGGGGGCTCACTCCGTTTGTCCTG TGCAGCTTCTGGCTTCAACATTAAAGACACCTATATACACTGGGTGCGTCAGGCCCCGGGTAAGG GCCTGGAATGGGTTGCAAGGATTTATCCTACGAATGGTTATACTAGATATGCCGATAGCGTCAAG GGCCGTTTCACTATAAGCGCAGACACATCCAAAAACACAGCCTACCTGCAGATGAACAGCCTGCG TGCTGAGGACACTGCCGTCTATTATTGTTCTAGGTGGGGAGGGGACGGCTTCTATGCTATGGACT ATTGGGGTCAAGGAACCCTGGTCACTGTCTCCTCCGGTGGTGGTGGTTCTGGCGGCGGCGGCTCC GGTGGTGGTGGTTCTGACATCCAGATGACCCAGTCCCCTAGCTCCCTGTCCGCCTCTGTGGGCGA TAGGGTCACCATCACCTGCCGTGCCAGTCAGGATGTGAATACTGCTGTAGCCTGGTATCAACAGA |

| | | | | |
|---|---|---|---|---|
| | | | | AACCAGGAAAAGCTCCGAAACTACTGATTTACTCGGCATCCTTCCTCTACTCTGGAGTCCCTTCT<br>CGCTTCTCTGGATCCAGATCGGGACGGATTTCACTCTGACCATCAGCAGTCTGCAGCCGGAAGA<br>CTTCGCAACTTATTACTGTGCAGCAACATTATATACTCCTCCCACGTTCGGAGGTCTGGGACT<br>TGGAGATCAAATCCGGAGGGTGTGGATCCGAGGTGCAGCTGGTGGAGTCTGGGAGGAGGATTGGTG<br>CAGCCTGGAGGGTCATTGAAACTCTGAGCTGCAGCCTCTGGATTCACCTTCAGTAGCTACGCCAT<br>GAACTGGGTCCGCCAGGCTCCAGGAAAGGGTTTGGAATGGGTTGCTCGCATAAGAAGTAAATATA<br>ATAATTATGCAACATATTATGCCGATTCAGTGAAAGGCAGGTTCACCATCTCCAGAGATGATTCA<br>AAAAACACTGCCTATCTACAAATGAACAACTTGAAAACTGAGGACACTGCÇGTGTACTACTGTGT<br>GAGACATGGGAACTTCGGTAATAGCTACGTTCCTGGTGGTTCCGGCGGCGGCCAAGGGACTCTGG<br>TCACCGTCTCCTCAGGTGGTGGTGGTTCTGGCGGCGGCGGCTCCGGTGGTGGTGGTTCTCAGACT<br>GTTGTGACTCAGGAACCTTCACCGTATCACCACCCAAACTGGTGGAACAGTCACACTTGTGGCTC<br>CTCGACTGGGCGTGTTACATCTGCAACTAGTCCTGCGACTAGTTCCTGCGCCAGATTCTCAGGCTCC<br>CCCGTGGTCTAATAGTGGGACTAGGTGGGAATAGGTCCTCACCCCTCGCCCTCTCAGGCTCC<br>CTGCTTGGAGGCAAGGCTGCCCTCACCCTCTCAGGGGTACAGCCCCAGGATGAGGCCGAATATTA<br>CTGTGTTCTATGGTACAGCAACCGCTGGGTGTTCGGTGGAGGAACCAAACTGACTGTCCTA |
| 473 | Her2/neu HL x I2C HL | artificial | aa | EVQLVESGGGLVQPGGSLRLSCAASGFNIKDTYIHWVRQAPGKGLEWVARIYPTNGYTRYADSVK<br>GRFTISADTSKNTAYLQMNSLRAEDTAVYYCSRWGGDGFYAMDYWGQGTLVTVSSGGGGSGGGGS<br>GGGGSDIQMTQSPSSLSASVGDRVTITCRASQDVNTAVAWYQQKPGKAPKLLIYSASFLYSGVPS<br>RFSGSRSGTDFTLTISSLQPEDFATYYCQQHYTTPPTFGQGTKVEIKSGGGGSEVQLVESGGGLV<br>QPGGSLKLSCAASGFTFNKYAMNWVRQAPGKGLEWVARIRSKYNNYATYYADSVKDRFTISRDDS<br>KNTAYLQMNNLKTEDTAVYYCVRHGNFGNSYISYWAYWGQGTLVTVSSGGGGSGGGGSGGGGSQT<br>VVTQEPSLTVSPGGTVTLTCGSSTGAVTSGNYPNWVQQKPGQAPRGLIGGTKFLAPGTPARFSGS<br>LLGGKAALTLSGVQPEDEAEYYCVLWYSNRWVFGGGTKLTVL |
| 474 | Her2/neu HL x I2C HL | artificial | nt | GAGGTTCAGCTGGTGGAGTCTGGCGGTGGCCTGGTGCAGCCAGGGGGCTCACTCCGTTTGTCCTG<br>TGCAGCTTCTGGCTTCAACATTAAAGACACCTATATACACTGGGTGCGTCAGGCCCCGGGTAAGG<br>GCCTGGAATGGGTTGCAAGAATTTATCCTACGAATGGTTATACTAGATATGCCGATAGCGTCAAG<br>GGCCGTTCACTATAAGCGGAGACACATCCAAAAACACAGCCTACCTGCAGATGAACAGCCTGCG<br>TGCTGAGGACACTGCCGTCTATTATTGTTCTAGGTGGGGAGGGGACGGCTTCTATGCTATGGACT<br>ATTGGGGTCAAGGAACCCTGGTCACTGTCTCCTCAGGTGGTGGTGGTTCTGGCGGCGGCGGCTCC<br>GGTGGTGGTGGTTCTGACATCCAGATGACCCAGTCTCCTTCCTCCCTGTCTGCGTCTGTGGGCGA<br>TAGGGTCACCATCACCTGCCGTGCCAGTCAGGATGTGAATACTGTTGCTAGCTGGTATCAACAGA<br>AACCAGGAAAAGCTCCGAAACTACTGATTTACTCGGCATCCTTCCTCTACTCTGGAGTCCCTTCT<br>CGCTTCTCTGGATCCAGATCGGGACGGATTTCACTCTGACCATCAGCAGTCTGCAGCCGGAAGA<br>CTTCGCAACTTATTACTGTCAGCAACATTATACTCCTCCCACGTTCGGAGGTCTGGGACT<br>TGGAGATCAAATCCGGAGGGTGTGGATCCGAGGTGCAGCTGGTGGAGTCTGGGAGGAGGATTGGTG<br>CAGCCTGGAGGGTCATTGAAACTCTGAGCTGCAGCCTCTGGATTCACCTTCAATAGTACGCCAT |

EP 2 155 788 B1

| | | | | |
|---|---|---|---|---|
| | | | | GAACTGGGTCCGCCAGGCTCCAGGAAAGGGTTTGGAATGGGTTGCTCGCATAAGAAGTAAATATA ATAATTATGCAACATATTATGCCGATTCAGTGAAAGACAGGTTCACCATCTCCAGAGATGATTCA AAAAACACTGCCTATCTACAAATGAACAACTTGAAAACTGAGGACACTGCCGTGTACTACTGTGT GAGACATGGGAACTTCGGTAATAGCTACATATCCTACTGGGCTTACTGGGGCCAAGGGACTCTGG TCACCGTCTCCTCAGGTGGTGGTGGTTCTGGCGGCGGCGGCTCCGGTGGTGGTGGTTCTCAGACT GTTGTGACTCAGGAACCTTCACTCACCGTATCACCTGGTGGAACAGTCACACTCACTTGTGGCTC CTCGACTGGGGCTGTTACATCTGGCAACTACCCAAACTGGGTCCAACAAAAACCAGGTCAGGCAC CCCGTGGTCTAATAGGTGGGACTAAGTTCCTCGCCCCCGGTACTCCTGCCAGATTCTCAGGCTCC CTGCTTGGAGGCAAGGCTGCCCTCACCCTCTCAGGGGTACAGCCAGAGGATGAGGCAGAATATTA CTGTGTTCTATGGTACAGCAACCGCTGGGTGTTCGGTGGAGGAACCAAACTGACTGTCCTA |
| 475 | H2C HL x Her2/neu LH | artificial | aa | EVQLVESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSQTVVTQEPSLTVSPGGTVTLTCGSSTGAVTSGYYPNWVQQKPGQAPRGLIGGTKF LAPGTPARFSGSLLGGKAALTLSGVQPEDEAEYYCALWYSNRWVFGGGTKLTVLSGGGGSDIQMT QSPSSLSASVGDRVTITCRASQDVNTAVAWYQQKPGKAPKLLIYSASFLYSGVPSRFSGSRSGTD FTLTISSLQPEDFATYYCQQHYTTPPTFGQGTKVEIKGGGGSGGGGSGGGGSEVQLVESGGGLVQ PGGSLRLSCAASGFNIKDTYIHWVRQAPGKGLEWVARIYPTNGYTRYADSVKGRFTISADTSKNT AYLQMNSLRAEDTAVYYCSRWGGDGFYAMDYWGQGTLVTVSS |
| 476 | H2C HL x Her2/neu LH | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCTACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGCTCTATGGTACAGCAACCGCTGGG TGTTCGGTGGAGGAACCAAACTGACTGTCCTATCCGGAGGTGGTGGATCCGACATCCAGATGACC CAGTCCCCTAGCTCCCTGTCCGCCTCTGTGGGCGATAGGGTCACCATCACCTGCCGTGCCAGTCA GGATGTGAATACTGCTGTAGCCTGGTATCAACAGAAACCAGGAAAAGCTCCGAAACTACTGATTT ACTCGGCATCCTTCCTCTACTCTGGAGTCCCTTCTCGCTTCTCTGGATCCAGATCTGGGACGGAT TTCACTCTGACCATCAGCAGTCTGCAGCCGGAAGACTTCGCAACTTATTACTGTCAGCAACATTA TACTACTCCTCCCACGTTCGGACAGGGTACCAAGGTGGAGATCAAAGGTGGTGGTGGTTCTGGCG GCGGCGGCTCCGGTGGTGGTGGTTCTGAGGTTCAGCTGGTGGAGTCTGGCGGTGGCCTGGTGCAG |

| SEQ ID NO | Name | Organism | Type | Sequence |
|---|---|---|---|---|
| 477 | F12Q HL x Her2/neu LH | artificial | aa | EVQLVESGGGLVQPGGSLKLSCAASGFTFNSYAMNWVRQAPGKGLEWVARI<br>SRDDSKNTAYLQMNNLKTEDTAVYYCVRHGNFGNSYVSWWAYWGQGTLVTVSSGGGS<br>GGGSGGGGSQTVVTQEPSLTVSPGGTVTLTCGSSTGAVTSGNYPNWVQQKPGQAPRGLIGGTKF<br>LAPGTPARFSGSLLGGKAALTLSGVQPEDEAEYYCVLWYSNRWVFGGGTKLTVLSGGGSDIQMT<br>QSPSSLSASVGDRVTITCRASQDVNTAVAWYQQKPGKAPKLLIYSASFLYSGVPSRFSGSRSGTD<br>FTLTISSLQPEDFATYYCQQHYTTPPTFGQGTKVEIKGGGGSGGGGSGGGGSEVQLVESGGGLVQ<br>PGGSLRLSCAASGFNIKDTYIHWVRQAPGKGLEWVARIYPTNGYTRYADSVKGRFTISADTSKNT<br>AYLQMNSLRAEDTAVYYCSRWGGDGFYAMDYWGQGTLVTVSS |
| 478 | F12Q HL x Her2/neu LH | artificial | nt | GAGGTGCAGCTGGTCGAGTCTGGAGGAGGATTGGTGCAGCCTGGAGGGTCATTGAAACTCTCATG<br>TGCAGCCTCTGGATTCACCTTCAGTTACTACGCCATGAACTGGGTCCGCCAGGCTCCAGGAAAGG<br>GTTTGGAATGGGTTGCTCGCATAAGAAGTAAATATAATTATGCAACATATATGCCGATTCA<br>GTGAAAGGCAGGTTCACCATCTCCAGAGATGATTCAAAAAACACTGCCTATCTACAAATGAACAA<br>CTTGAAAACTGAGGACACTGCCGTGTACTACTGTGTGAGACATGGCAATGGGAACTTCGGTAATAGCTACG<br>TTTCCTGGTGGGGCTTACTGGGGCCAAGGGACTCTGGTCACCGTCTCCTCAGGTGGTGGTGGTTCT<br>GGCGGCGGCGGCTCCGGTGGTGGTGGTTCTCAGACTGTTGTGACTCAGGAACCTTCACTCACCGT<br>ATCACCTGGTGGAACAGTCACACTCACTTGTGGCTCCTCAGGCTGTTACATCTGGCAACT<br>ACCCAAACTGGGTCCAACAAAAACCAGGTCAGGCACCCCGTGGTCTAATAGGTGGGACTAAGTTC<br>CTCGCCCCCGTACTCCTGCCAGATTCTCAGGCTCCCTGCCTGGAGGCAAGGCTGCCCTCACCCT<br>CTCAGGGGTACAGCCAGAGGATGAGGCAGAATATTACTGTGTTCTATGGTACAGCAACCGCTGGG<br>TGTTCGGTGGAGGAACCAAACTGACTGTCCTATCCGGCGATGACC<br>CAGTCCCCTAGCTCCCTGTCCGCCGCTAGTGTCGGCGATATCAACAGAGAAAGCTCCGAAACTACTGATTT<br>GGATGTGAATACTGCTGTAGCCTGGTATCAACAGAAACCAGGAAAAGCTCCGAAACTACTGATTT<br>ACTCGGCCATCCTTCCTCTACTCGAGTCCCTTCTCGGATCCAGATCTGGGACGGAT<br>TTCACTCTGACCATCAGCAGTCTGCAGCCGGAAGACTTCGCAACTTATTACTGTCAGCAACATTA<br>TACTACTCCTCCCACGTTCGGACAGGGTACCAAGGTGGTGGTGGCTCTGGCG<br>GCGGCGGCTCCGGTGGTGGTGGTTCTGGCGGCTTCTGGTGCTCTGGCAG<br>CCAGGGGGCTCACTCCGTTGTTCTGTGTGCAGCTTCTGTGGCTTCAACATTAAAGACACCTATATACA<br>CTGGGTGCGTCAGGCCCCGGGTAAGGGCCTGGAATGGGTTGCAAGGATTTATCCTACGAATGGTT<br>ATACTAGATATGCCGATAGCGTCAAGGGCCGTTCACTATAAGCGCAGACACATCCAAAAACACA<br>GCCTACCTGCAGATGAACAGCCTGCGTGCTGAGGACACTGCCGTCTATTATTGTGCTGTCTCCTCC<br>AGGGGACGGCGCTTCTATGCTATGGACTATTGGGGTCAAGGAACCCTGGTCACTGTCTCCTCC |

| | | | | AGGGGACGGCTTCTATGCTATGGACTATTGGGGTCAAGGAACCCTGGTCACTGTCTCCTCC |
|---|---|---|---|---|
| 479 | I2C HL x Her2/neu LH | artificial | aa | EVQLVESGGGLVQPGGSLKLSCAASGFTFNKYAMNWVRQAPGKGLEWVARIRSKYNNYATYYADS VKDRFTISRDDSKNTAYLQMNNLKTEDTAVYYCVRHGNFGNSYISYWAYWGQGTLVTVSSGGGGS GGGGSGGGGSQTVVTQEPSLTVSPGGTVTLTCGSSTGAVTSGNYPNWVQQKPGQAPRGLIGGTKF LAPGTPARFSGSLLGGKAALTLSGVQPEDEAEYYCVLWYSNRWVFGGGTKLTVLSGGGGSDIQMT QSPSSLSASVGDRVTITCRASQDVNTAVAWYQQKPGKAPKLLIYSASFLYSGVPSRFSGSRSGTD FTLTISSLQPEDFATYYCQQHYTTPPTFGQGTKVEIKGGGGSGGGGSGGGGSEVQLVESGGGLVQ PGGSLRLSCAASGFNIKDTYIHWVRQAPGKGLEWVARIYPTNGYTRYADSVKGRFTISADTSKNT AYLQMNSLRAEDTAVYYCSRWGGDGFYAMDYWGQGTLVTVSS |
| 480 | I2C HL x Her2/neu LH | artificial | nt | GAGGTGCAGCTGGTCGAGTCTGGAGGAGGATTGGTGCAGCCTGGAGGGTCATTGAAACTCTCATG TGCAGCCTCTGGATTCACCTTCAATAAGTACGCCATGAACTGGGTCCGCCAGGCTCCAGGAAAGG GTTTGGAATGGGTTGCTCGCATAAGAAGTAAATATAATAATTATGCAACATATTATGCCGATTCA GTGAAAGACAGGTTCACCATCTCCAGAGATGATTCAAAAAACACTGCCTATCTACAAATGAACAA CTTGAAAACTGAGGACACTGCCGTGTACTACTGTGTGAGACATGGGAACTTCGGTAATAGCTACA TATCCTACTGGGCTTACTGGGGCCAAGGGACTCTGGTCACCGTCTCCTCAGGTGGTGGTGGTTCT GGCGGCGGCGGCTCCGGTGGTGGTGGTTCTCAGACTGTTGTGACTCAGGAACCTTCACTCACCGT ATCACCTGGTGGAACAGTCACACTCACTTGTGGCTCCTCGACTGGGGCTGTTACATCTGGCAACT ACCCAAACTGGGTCCAACAAAAACCAGGTCAGGCACCCCGTGGTCTAATAGGTGGGACTAAGTTC CTCGCCCCCGGTACTCCTGCCAGATTCTCAGGCTCCCTGCTTGGAGGCAAGGCTGCCCTCACCCT CTCAGGGGTACAGCCAGAGGATGAGGCAGAATATTACTGTGTTCTATGGTACAGCAACCGCTGGG TGTTCGGTGGAGGAACCAAACTGACTGTCCTATCCGGAGGTGGTGGATCCGACATCCAGATGACC CAGTCCCCTAGCTCCCTGTCCGCCTCTGTGGGCGATAGGGTCACCATCACCTGCCGTGCCAGTCA GGATGTGAATACTGCTGTAGCCTGGTATCAACAGAAACCAGGAAAAGCTCCGAAACTACTGATTT ACTCGGCATCCTTCCTCTACTCTGGAGTCCCTTCTCGCTTCTCTGGATCCAGATCTGGGACGGAT TTCACTCTGACCATCAGCAGTCTGCAGCCGGAAGACTTCGCAACTTATTACTGTCAGCAACATTA TACTACTCCTCCCACGTTCGGACAGGGTACCAAGGTGGAGATCAAAGGTGGTGGTGGTTCTGGCG GCGGCGGCTCCGGTGGTGGTGGTTCTGAGGTTCAGCTGGTGGAGTCTGGCGGTGGCCTGGTGCAG CCAGGGGGCTCACTCCGTTTGTCCTGTGCAGCTTCTGGCTTCAACATTAAAGACACCTATATACA CTGGGTGCGTCAGGCCCCGGGTAAGGGCCTGGAATGGGTTGCAAGGATTTATCCTACGAATGGTT ATACTAGATATGCCGATAGCGTCAAGGGCCGTTTCACTATAAGCGCAGACACATCCAAAAACACA GCCTACCTGCAGATGAACAGCCTGCGTGCTGAGGACACTGCCGTCTATTATTGTTCTAGGTGGGG AGGGGACGGCTTCTATGCTATGGACTATTGGGGTCAAGGAACCCTGGTCACTGTCTCCTCC |
| 481 | HCDR1 of IgE | artificial | aa | SGYSWN |
| 482 | HCDR2 of IgE | artificial | aa | SITYDGSTNYADSVKG |

| | | | | |
|---|---|---|---|---|
| 483 | HCDR3 of IgE | artificial | aa | GSHYFGHWHFAV |
| 484 | LCDR1 of IgE | artificial | aa | RASQSVDYDGDSYMN |
| 485 | LCDR2 of IgE | artificial | aa | AASYLES |
| 486 | LCDR3 of IgE | artificial | aa | QQSHEDPYT |
| 487 | VH of IgE | artificial | aa | EVQLVESGGGLVQPGGSLRLSCAVSGYSITSGYSWNWIRQAPGKGLEWVASITYDGSTNYADSVKGRFTISRDDSKNTFYLQMNSLRAEDTAVYYCARGSHYFGHWHFAVWGQGTLVTVSS |
| 488 | VH of IgE | artificial | nt | GAGGTGCAGCTGGTGGAGTCTGGGGGAGGCTTGGTCCAGCCTGGGGGGTCCCTGAGACTCTCCTGTGCAGTCTCTGGATACTCCATCACCAGCGGCTACAGTTGGAACTGGATCCGCCAGGCTCCAGGGAAGGGGCTGGAGTGGGTCGCAAGTATTACGTATGATGGGAGTACAAACGGCAGATTCACCATCTCCAGAGACGACTCCAAGAACACGTTCTATCTTCAAATGAACAGCCTGAGAGCCGAGGACACGGCTGTGTATTACTGTGCGAGAGGTTCCCATTATTTCGGCCATTGGCACTTCGCCGTGTGGGGCCAGGGAACGCTGGTCACAGTTAGCTCC |
| 489 | VL of IgE | artificial | aa | DIQLTQSPSSLSASVGDRVTITCRASQSVDYDGDSYMNWYQQKPGKAPKLLIYAASYLESGVPSRFSGSGSGTDFTLTISSLQPEDFATYYCQQSHEDPYTFGQGTKVEIK |
| 490 | VL of IgE | artificial | nt | GACATCCAGCTGACCCAGTCTCCATCCTCCCTGTCTGCATCTGTTGGAGACAGAGTCACCATCACTTGCCGGGCAAGTCAGAGCGTTGACTATGATGGGGACAGCTATATGAATTGGTATCAGCAGAAACCAGGGAAAGCCCCTAAGCTCCTGATCTATGCTGCATCCTACTTGGAAAGTGGGGTCCCATCAAGGTTCAGTGGCAGTGGATCTGGGACAGATTCTCACTCTCACCATCAGCAGTCTCATGAAGATCCATATACCTTTGGCCAGGGGACCACTAAAGTCGAAATAAAG |
| 491 | HL of IgE | artificial | aa | EVQLVESGGGLVQPGGSLRLSCAVSGYSITSGYSWNWIRQAPGKGLEWVASITYDGSTNYADSVKGRFTISRDDSKNTFYLQMNSLRAEDTAVYYCARGSHYFGHWHFAVWGQGTLVTVSSggggsgggggsDIQLTQSPSSLSASVGDRVTITCRASQSVDYDGDSYMNWYQQKPGKAPKLLIYAASYLESGVPSRFSGSGSGTDFTLTISSLQPEDFATYYCQQSHEDPYTFGQGTKVEIK |
| 492 | HL of IgE | artificial | nt | GAGGTGCAGCTGGTGGAGTCTGGGGGAGGCTTGGTCCAGCCTGGGGGGTCCCTGAGACTCTCCTGTGCAGTCTCTGGATACTCCATCACCAGCGGCTACAGTTGGAACTGGATCCGCCAGGCTCCAGGGAAGGGGCTGGAGTGGGTCGCAAGTATTACGTATGATGGGAGTACAAAGGGCAGATTCACCATCTCCAGAGACGACTCCAAGAACACGTTCTATCTTCAAATGAACAGCCTGAGAGCCGAGGACACGGCTGTGTATTACTGTGCGAGAGGTTCCCATTATTTCGGCCATTGGCACTTCGCCGTGTGGGGCTTGGTGGTTCTGGCGGCGGC |

163

| | | | | |
|---|---|---|---|---|
| | | | | TCCGGTGGTGGTGGTTCTGACATCCAGCTGACCCAGTCTCCATCCTCCCTGTCTGCATCTGTTGG AGACAGAGTCACCATCACTTGCCGGGCAAGTCAGAGCGTTGACTATGATGGGGACAGCTATATGA ATTGGTATCAGCAGAAACCAGGGAAAGCCCCTAAGCTCCTGATCTATGCTGCATCCTACTTGGAA AGTGGGGTCCCATCAAGGTTCAGTGGCAGTGGATCTGGGACAGATTTCACTCTCACCATCAGCAG TCTGCAACCTGAAGATTTTGCAACTTACTACTGTCAACAGTCTCATGAAGATCCATATACCTTTG GACAGGGCACTAAAGTCGAAATAAAG |
| 493 | LH of IgE | artificial | aa | DIQLTQSPSSLSASVGDRVTITCRASQSVDYDGDSYMNWYQQKPGKAPKLLIYAASYLESGVPSR FSGSGSGTDFTLTISSLQPEDFATYYCQQSHEDPYTFGQGTKVEIKggggsggggsggggsEV QLVESGGGLVQPGGSLRLSCAVSGYSITSGYSWNWIRQAPGKGLEWVASITYDGSTNYADSVKGR FTISRDDSKNTFYLQMNSLRAEDTAVYYCARGSHYFGHWHFAVWGQGTLVTVSS |
| 494 | LH of IgE | artificial | nt | GACATCCAGCTGACCCAGTCTCCATCCTCCCTGTCTGCATCTGTTGGAGACAGAGTCACCATCAC TTGCCGGGCAAGTCAGAGCGTTGACTATGATGGGGACAGCTATATGAATTGGTATCAGCAGAAAC CAGGGAAAGCCCCTAAGCTCCTGATCTATGCTGCATCCTACTTGGAAAGTGGGGTCCCATCAAGG TTCAGTGGCAGTGGATCTGGGACAGATTTCACTCTCACCATCAGCAGTCTGCAACCTGAAGATTT TGCAACTTACTACTGTCAACAGTCTCATGAAGATCCATATACCTTTGGACAGGGCACTAAAGTCG AAATAAAGGGTGGTGGTGGTTCTGGCGGCGGCGGCTCCGGTGGTGGTGGTTCTGAGGTGCAGCTG GTGGAGTCTGGGGGAGGCTTGGTCCAGCCTGGGGGGTCCCTGAGACTCTCCTGTGCAGTCTCTGG ATACTCCATCACCAGCGGCTACAGTTGGAACTGGATCCGCCAGGCTCCAGGGAAGGGGCTGGAGT GGGTCGCAAGTATTACGTACGACGGTAGCACAAACTACGCAGACTCCGTGAAGGGCAGATTCACC ATCTCCAGAGACGACTCCAAGAACACGTTCTATCTTCAAATGAACAGCCTGAGAGCCGAGGACAC GGCTGTGTATTACTGTGCGAGAGGTTCCCATTATTTCGGCCATTGGCACTTCGCCGTGTGGGGCC AGGGAACGCTTGTCACAGTTAGCTCC |
| 495 | IgE HL x H2C HL | artificial | aa | EVQLVESGGGLVQPGGSLRLSCAVSGYSITSGYSWNWIRQAPGKGLEWVASITYDGSTNYADSVK GRFTISRDDSKNTFYLQMNSLRAEDTAVYYCARGSHYFGHWHFAVWGQGTLVTVSSggggsggg gsggggsDIQLTQSPSSLSASVGDRVTITCRASQSVDYDGDSYMNWYQQKPGKAPKLLIYAASY LESGVPSRFSGSGSGTDFTLTISSLQPEDFATYYCQQSHEDPYTFGQGTKVEIKSGGGGSEVQLV ESGGGLVQPGGSLKLSCAASGFTFNKYAMNWVRQAPGKGLEWVARIRSKYNNYATYYADSVKDRF TISRDDSKNTAYLQMNNLKTEDTAVYYCVRHGNFGNSYISYWAYWGQGTLVTVSSGGGGSGGGGS GGGGSQTVVTQEPSLTVSPGGTVTLTCGSSTGAVTSGYYPNWVQQKPGQAPRGLIGGTKFLAPGT PARFSGSLLGGKAALTLSGVQPEDEAEYYCALWYSNRWVFGGGTKLTVL |
| 496 | IgE HL x H2C HL | artificial | nt | GAGGTGCAGCTGGTGGAGTCTGGGGGAGGCTTGGTCCAGCCTGGGGGGTCCCTGAGACTCTCCTG TGCAGTCTCTGGATACTCCATCACCAGCGGCTACAGTTGGAACTGGATCCGCCAGGCTCCAGGGA AGGGGCTGGAGTGGGTCGCAAGTATTACGTACGACGGTAGCACAAACTACGCAGACTCCGTGAAG GGCAGATTCACCATCTCCAGAGACGACTCCAAGAACACGTTCTATCTTCAAATGAACAGCCTGAG |

EP 2 155 788 B1

| | | | | Sequence |
|---|---|---|---|---|
| | | | | AGCCGAGGACACGGCTGTGTATTACTGTGCGAGAGAGGTTCCGAGAGTTCG CCGTGTGGGGCCAGGGAACCCTGGTCACCGTCTCCTCGGTTCTGGCGGCGGC TCCGGTGGTGGTGGTTCTGACATCCAGCTCCATCCCCGTGTCTGCATCTGTTGG AGACAGAGTCACCATCACTTGCCGGCAAGTCAGAGCGTTGACTATGATGGGGACAGCTATATGA ATTGGTATCAGCAGAAACCAGGGAAAGCCCCTAAGCTCCTGATCTATGCTGCATCCTACTTGGAA AGTGGGGTCCCATCAAGGTTCAGTGGCAGTGGATCTGGGACAGATTTCACTCTCACCATCAGCAG TCTGCAACCTGAAGATTTTGCAACTTACTACTGTCAACAGTCTCATGAAGATCCATATACCTTTG GACAGGGCACTAAAGTCGAAATAAAGTCCGGAGGTGGTGGATCGGGAGGTGGTGGTCGAGTCT GGAGGAGGATTGGTGCAGCCTGGAGGGTCCCTGCGCCTCTCCTGTGAAGCTTCACCTT CAATAAGTACGCCCATGAACTGGGTTCGCCAGGCTCCAGGAAAGGGTTTGGAATGGGTTGCTCGCA TAAGAAGTAAATATAATATTATTATGCCAACATATTATGCTGATTCAGTGAAAGACAGGTTCACCATC TCCAGAGATGATTCAAAAAACACTGCCTATCTACAAATGAACAGCCTGAGAGCTGAGGACACTGC CGTGTACTACTGTGTGAGACATGGGAACATGGGAACTTCGGTAATAGCTACATATCCTACTGGGCTTACTGGG GCCAAGGGACTCTGGTCACCGTCTCCTCAGGTGGTGGTTCTCACCGTCTAC ACTCACTTGTGGCTCCTGACTGGGTCTGTTACTCTGTGTTACATCTGTACTACCACCAAACTGGTCCAACAAA AACCAGGTCAGGCCACCACCCCGTGGTCTAATAGGTGGACTAAGTTCCTGCCCCGGTACTCCTGCC AGATTCTCAGGCTCCCTGCTTGGAGGCAAGGCTCACCCTCACCCTCTCAGGGGTACAGCCAGGAGGA TGAGGCAGAATATTACTGTGCTCTATGGTACAGCAACCGCTGGGTGTTCGGTGGAGGAACCAAAAC TGACTGTCCTA |
| 497 | IgE HL x F12Q HL | artificial | aa | EVQLVESGGGLVQPGGSLRLSCAVSGYSITSGYSWNWIRQAPGKGLEWVASITYDGSTNYADSVK GRFTISRDDSKNTFYLQMNSLRAEDTAVYYCARGSHYFGHWHFAVWGQGTLVTVSSggggsggg gsgggsDIQLTQSPSSLSASVGDRVTITCRASQSVDYDGDSYMNWYQQKPGKAPKLLIYAASY LESGVPSRFSGSGSGTDFTLTISSLQPEDFATYYCQQSHEDPYTFGQGTKVEIKSGGGGSEVQLV ESGGGLVQPGGSLKLSCAASGFTFNSYAMNWVRQAPGKGLEWVARIRSKYNNYATYYADSVKGRF TISRDDSKNTAYLQMNNLKTEDTAVYYCVRHGNFGNSYVSWWAYWGQGTLVTVSSGGGGSGGGGS GGGGSQTVVTQEPSLTVSPGGTVTLTCGSSTGAVTSGNYPNWVQQKPGQAPRGLIGGTKFLAPGT PARFSGSLLGGKAALTLSGVQPEDEAEYYCVLWYSNRWVFGGGTKLTVL |
| 498 | IgE HL x F12Q HL | artificial | nt | GAGGTGCAGCTGGTGGAGTCTGGGGGAGGCTTGGTCCAGCCTGGGGGGTCCCTGAGACTCTCCTG TGCAGTCTCTGGATACTCCATCACCAGCGGCTACAGTTGGAACTGGATCCGCCAGGCTCCAGGGA AGGGGCTGGAGTGGGTCGCAAGTATTACGTATGATGGAAGTACCAACTACGCAGACTCCGTGAAG GGCAGATTCACCATCTCCAGAGACGACTCCAAGAACACGTTCTATCTTCAAATGAACAGCCTGAG AGCCGAGGACACGGCTGTGTATTACTGTGCCAGAGGCTCCCATTATTTCGGCCACTGGCACTTCG CCGTGTGGGGCCAGGGAACCCTGGTCACCGTCTCCTCGGTTCTGGCGGCGGCGGCTCGTTGG |

| | | | | |
|---|---|---|---|---|
| | | | | AGACAGAGTCACCATCACTTGCCGGGCAAGTCAGAGCGTTGACTATGATGGGGACAGCTATATGA<br>ATTGGTATCAGCAGAAACCAGGGAAAGCCCCTAAGCTCCTGATCTATGCTGCATCCTACTTGGAA<br>AGTGGGGTCCCATCAAGGTTCAGTGGCAGTGGATCTGGGACAGATTTCACTCTCACCATCAGCAG<br>TCTGCAACCTGAAGATTTTGCAACTTACTACTGTCAACAGTCTCATGAAGATCCATATACCTTTG<br>GACAGGGCACTAAAGTCGAAATAAAGTCCGGAGGTGGTGGATCCGAGGTGCAGCTGGTCGAGTCT<br>GGAGGAGGATTGGTGCAGCCTGGAGGGTCATTGAAACTCTCATGTGCAGCCTCTGGATTCACCTT<br>CAATAGCTACGCCATGAACTGGGTCCGCCAGGCTCCAGGAAAGGGTTTGGAATGGGTTGCTCGCA<br>TAAGAAGTAAATATAATAATTATGCAACATATTATGCCGATTCAGTGAAAGGCAGGTTCACCATC<br>TCCAGAGATGATTCAAAAAACACTGCCTATCTACAAATGAACAACTTGAAAACTGAGGACACTGC<br>CGTGTACTACTGTGTGAGACATGGGAACTTCGGTAATAGCTACGTTTCCTGGTGGGCTTACTGGG<br>GCCAAGGGACTCTGGTCACCGTCTCCTCAGGTGGTGGTGGTTCTGGCGGCGGCGGCTCCGGTGGT<br>GGTGGTTCTCAGACTGTTGTGACTCAGGAACCTTCACTCACCGTATCACCTGGTGGAACAGTCAC<br>ACTCACTTGTGGCTCCTCGACTGGGGCTGTTACATCTGGCAACTACCCAAACTGGGTCCAACAAA<br>AACCAGGTCAGGCACCCCGTGGTCTAATAGGTGGGACTAAGTTCCTCGCCCCCGGTACTCCTGCC<br>AGATTCTCAGGCTCCCTGCTTGGAGGCAAGGCTGCCCTCACCCTCTCAGGGGTACAGCCAGAGGA<br>TGAGGCAGAATATTACTGTGTTCTATGGTACAGCAACCGCTGGGTGTTCGGTGGAGGAACCAAAC<br>TGACTGTCCTA |
| 499 | IgE HL x I2C HL | artificial | aa | EVQLVESGGGLVQPGGSLRLSCAVSGYSITSGYSWNWIRQAPGKGLEWVASITYDGSTNYADSVK<br>GRFTISRDDSKNTFYLQMNSLRAEDTAVYYCARGSHYFGHWHFAVWGQGTLVTVSSggggsggg<br>gsggggsDIQLTQSPSSLSASVGDRVTITCRASQSVDYDGDSYMNWYQQKPGKAPKLLIYAASY<br>LESGVPSRFSGSGSGTDFTLTISSLQPEDFATYYCQQSHEDPYTFGQGTKVEIKSGGGGSEVQLV<br>ESGGGLVQPGGSLKLSCAASGFTFNKYAMNWVRQAPGKGLEWVARIRSKYNNYATYYADSVKDRF<br>TISRDDSKNTAYLQMNNLKTEDTAVYYCVRHGNFGNSYISYWAYWGQGTLVTVSSGGGGSGGGGS<br>GGGGSQTVVTQEPSLTVSPGGTVTLTCGSSTGAVTSGNYPNWVQQKPGQAPRGLIGGTKFLAPGT<br>PARFSGSLLGGKAALTLSGVQPEDEAEYYCVLWYSNRWVFGGGTKLTVL |
| 500 | IgE HL x I2C HL | artificial | nt | GAGGTGCAGCTGGTGGAGTCTGGGGGAGGCTTGGTCCAGCCTGGGGGGTCCCTGAGACTCTCCTG<br>TGCAGTCTCTGGATACTCCATCACCAGCGGCTACAGTTGGAACTGGATCCGCCAGGCTCCAGGGA<br>AGGGGCTGGAGTGGGTCGCAAGTATTACGTACGACGGTAGCACAAACTACGCAGACTCCGTGAAG<br>GGCAGATTCACCATCTCCAGAGACGACTCCAAGAACACGTTCTATCTTCAAATGAACAGCCTGAG<br>AGCCGAGGACACGGCTGTGTATTACTGTGCGAGAGGTTCCCATTATTTCGGCCATTGGCACTTCG<br>CCGTGTGGGGCCAGGGAACGCTTGTCACAGTTAGCTCCGGTGGTGGTGGTTCTGGCGGCGGCGGC<br>TCCGGTGGTGGTGGTTCTGACATCCAGCTGACCCAGTCTCCATCCTCCCTGTCTGCATCTGTTGG<br>AGACAGAGTCACCATCACTTGCCGGGCAAGTCAGAGCGTTGACTATGATGGGGACAGCTATATGA<br>ATTGGTATCAGCAGAAACCAGGGAAAGCCCCTAAGCTCCTGATCTATGCTGCATCCTACTTGGAA<br>AGTGGGGTCCCATCAAGGTTCAGTGGCAGTGGATCTGGGACAGATTTCACTCTCACCATCAGCAG |

| | | | | |
|---|---|---|---|---|
| | | | | TCTGCAACCTGAAGATTTTGCAACTTACTACTGTCAACAGTCTCATGAAGATCCATATACCTTTG GACAGGGCACTAAAGTCGAAATAAAGTCCGGAGGTGGTGGATCCGAGGTGCAGCTGGTCGAGTCT GGAGGAGGATTGGTGCAGCCTGGAGGGTCATTGAAACTCTCATGTGCAGCCTCTGGATTCACCTT CAATAAGTACGCCATGAACTGGGTCCGCCAGGCTCCAGGAAAGGGTTTGGAATGGGTTGCTCGCA TAAGAAGTAAATATAATAATTATGCAACATATTATGCCGATTCAGTGAAAGACAGGTTCACCATC TCCAGAGATGATTCAAAAAACACTGCCTATCTACAAATGAACAACTTGAAAACTGAGGACACTGC CGTGTACTACTGTGTGAGACATGGGAACTTCGGTAATAGCTACATATCCTACTGGGCTTACTGGG GCCAAGGGACTCTGGTCACCGTCTCCTCAGGTGGTGGTGGTTCTGGCGGCGGCGGCTCCGGTGGT GGTGGTTCTCAGACTGTTGTGACTCAGGAACCTTCACTCACCGTATCACCTGGTGGAACAGTCAC ACTCACTTGTGGCTCCTCGACTGGGGCTGTTACATCTGGCAACTACCCAAACTGGGTCCAACAAA AACCAGGTCAGGCACCCCGTGGTCTAATAGGTGGGACTAAGTTCCTCGCCCCCGGTACTCCTGCC AGATTCTCAGGCTCCCTGCTTGGAGGCAAGGCTGCCCTCACCCTCTCAGGGGTACAGCCAGAGGA TGAGGCAGAATATTACTGTGTTCTATGGTACAGCAACCGCTGGGTGTTCGGTGGAGGAACCAAAC TGACTGTCCTA |
| 501 | IgE LH x H2C HL | artificial | aa | DIQLTQSPSSLSASVGDRVTITCRASQSVDYDGDSYMNWYQQKPGKAPKLLIYAASYLESGVPSR FSGSGSGTDFTLTISSLQPEDFATYYCQQSHEDPYTFGQGTKVEIKgggggsggggsgggggsEV QLVESGGGLVQPGGSLRLSCAVSGYSITSGYSWNWIRQAPGKGLEWVASITYDGSTNYADSVKGR FTISRDDSKNTFYLQMNSLRAEDTAVYYCARGSHYFGHWHFAVWGQGTLVTVSSGGGGSEVQLVE SGGGLVQPGGSLKLSCAASGFTFNKYAMNWVRQAPGKGLEWVARIRSKYNNYATYYADSVKDRFT ISRDDSKNTAYLQMNNLKTEDTAVYYCVRHGNFGNSYISYWAYWGQGTLVTVSSGGGGSGGGGSG GGGSQTVVTQEPSLTVSPGGTVTLTCGSSTGAVTSGYYPNWVQQKPGQAPRGLIGGTKFLAPGTP ARFSGSLLGGKAALTLSGVQPEDEAEYYCALWYSNRWVFGGGTKLTVL |
| 502 | IgE LH x H2C HL | artificial | nt | GACATCCAGCTGACCCAGTCTCCATCCTCCCTGTCTGCATCTGTTGGAGACAGAGTCACCATCAC TTGCCGGGCAAGTCAGAGCGTTGACTATGATGGGGACAGCTATATGAATTGGTATCAGCAGAAAC CAGGGAAAGCCCCTAAGCTCCTGATCTATGCTGCATCCTACTTGGAAAGTGGGGTCCCATCAAGG TTCAGTGGCAGTGGATCTGGGACAGATTTCACTCTCACCATCAGCAGTCTGCAACCTGAAGATTT TGCAACTTACTACTGTCAACAGTCTCATGAAGATCCATATACCTTTGGACAGGGCACTAAAGTCG AAATAAAGGGTGGTGGTGGTTCTGGCGGCGGCGGCTCCGGTGGTGGTGGTTCTGAGGTGCAGCTG GTGGAGTCTGGGGGAGGCTTGGTCCAGCCTGGGGGGTCCCTGAGACTCTCCTGTGCAGTCTCTGG ATACTCCATCACCAGCGGCTACAGTTGGAACTGGATCCGCCAGGCTCCAGGGAAGGGGCTGGAGT GGGTCGCAAGTATTACGTACGACGGTAGCACAAACTACGCAGACTCCGTGAAGGGCAGATTCACC ATCTCCAGAGACGACTCCAAGAACACGTTCTATCTTCAAATGAACAGCCTGAGAGCCGAGGACAC GGCTGTGTATTACTGTGCGAGAGGTTCCCATTATTTCGGCCATTGGCACTTCGCCGTGTGGGGCC AGGGAACGCTTGTCACAGTTAGCTCCGGAGGTGGTGGATCCGAGGTGCAGCTGGTCGAGTCTGGA GGAGGATTGGTGCAGCCTGGAGGGTCATTGAAACTCTCATGTGCAGCCTCTGGATTCACCTTCAA |

| | | | | |
|---|---|---|---|---|
| | | | | TAAGTACGCCATGAACTGGGTCCGCCAGGCTCCAGGAAAGGGTTTGGAATGGGTTGCTCGCATAA GAAGTAAATATAATAATTATGCAACATATTATGCCGATTCAGTGAAAGACAGGTTCACCATCTCC AGAGATGATTCAAAAAACACTGCCTATCTACAAATGAACAACTTGAAAACTGAGGACACTGCCGT GTACTACTGTGTGAGACATGGGAACTTCGGTAATAGCTACATATCCTACTGGGCTTACTGGGGCC AAGGGACTCTGGTCACCGTCTCCTCAGGTGGTGGTGGTTCTGGCGGCGGCGGCTCCGGTGGTGGT GGTTCTCAGACTGTTGTGACTCAGGAACCTTCACTCACCGTATCACCTGGTGGAACAGTCACACT CACTTGTGGCTCCTCGACTGGGGCTGTTACATCTGGCTACTACCCAAACTGGGTCCAACAAAAAC CAGGTCAGGCACCCCGTGGTCTAATAGGTGGGACTAAGTTCCTCGCCCCCGGTACTCCTGCCAGA TTCTCAGGCTCCCTGCTTGGAGGCAAGGCTGCCCTCACCCTCTCAGGGGTACAGCCAGAGGATGA GGCAGAATATTACTGTGCTCTATGGTACAGCAACCGCTGGGTGTTCGGTGGAGGAACCAAACTGA CTGTCCTA |
| 503 | IgE LH x F12Q HL | artificial | aa | DIQLTQSPSSLSASVGDRVTITCRASQSVDYDGDSYMNWYQQKPGKAPKLLIYAASYLESGVPSR FSGSGSGTDFTLTISSLQPEDFATYYCQQSHEDPYTFGQGTKVEIKggggsggggsggggsEV QLVESGGGLVQPGGSLRLSCAVSGYSITSGYSWNWIRQAPGKGLEWVASITYDGSTNYADSVKGR FTISRDDSKNTFYLQMNSLRAEDTAVYYCARGSHYFGHWHFAVWGQGTLVTVSSGGGGSEVQLVE SGGGLVQPGGSLKLSCAASGFTFNSYAMNWVRQAPGKGLEWVARIRSKYNNYATYYADSVKGRFT ISRDDSKNTAYLQMNNLKTEDTAVYYCVRHGNFGNSYVSWWAYWGQGTLVTVSSGGGGSGGGGSG GGGSQTVVTQEPSLTVSPGGTVTLTCGSSTGAVTSGNYPNWVQQKPGQAPRGLIGGTKFLAPGTP ARFSGSLLGGKAALTLSGVQPEDEAEYYCVLWYSNRWVFGGGTKLTVL |
| 504 | IgE LH x F12Q HL | artificial | nt | GACATCCAGCTGACCCAGTCTCCATCCTCCCTGTCTGCATCTGTTGGAGACAGAGTCACCATCAC TTGCCGGGCAAGTCAGAGCGTTGACTATGATGGGGACAGCTATATGAATTGGTATCAGCAGAAAC CAGGGAAAGCCCCTAAGCTCCTGATCTATGCTGCATCCTACTTGGAAAGTGGGGTCCCATCAAGG TTCAGTGGCAGTGGATCTGGGACAGATTTCACTCTCACCATCAGCAGTCTGCAACCTGAAGATTT TGCAACTTACTACTGTCAACAGTCTCATGAAGATCCATATACCTTTGGACAGGGCACTAAAGTCG AAATAAAGGGTGGTGGTGGTTCTGGCGGCGGCGGCTCCGGTGGTGGTGGTTCTGAGGTGCAGCTG GTGGAGTCTGGGGGAGGCTTGGTCCAGCCTGGGGGGTCCCTGAGACTCTCCTGTGCAGTCTCTGG ATACTCCATCACCAGCGGCTACAGTTGGAACTGGATCCGCCAGGCTCCAGGGAAGGGGCTGGAGT GGGTCGCAAGTATTACGTACGACGGTAGCACAAACTACGCAGACTCCGTGAAGGGCAGATTCACC ATCTCCAGAGACGACTCCAAGAACACGTTCTATCTTCAAATGAACAGCCTGAGAGCCGAGGACAC GGCTGTGTATTACTGTGCGAGAGGTTCCCATTATTTCGGCCATTGGCACTTCGCCGTGTGGGGCC AGGGAACGCTTGTCACAGTTAGCTCCGGAGGTGGTGGATCCGAGGTGCAGCTGGTCGAGTCTGGA GGAGGATTGGTGCAGCCTGGAGGGTCATTGAAACTCTCATGTGCAGCCTCTGGATTCACCTTCAA TAGCTACGCCATGAACTGGGTCCGCCAGGCTCCAGGAAAGGGTTTGGAATGGGTTGCTCGCATAA GAAGTAAATATAATAATTATGCAACATATTATGCCGATTCAGTGAAAGGCAGGTTCACCATCTCC AGAGATGATTCAAAAAACACTGCCTATCTACAAATGAACAACTTGAAAACTGAGGACACTGCCGT |

| | | | | |
|---|---|---|---|---|
| | | | | GTACTACTGTGTGAGACATGGGAACTTCGGTAATAGCTACGTTTCCTGGTGGGCTTACTGGGGCC<br>AAGGGACTCTGGTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAACAGTCACACT<br>CACTTGTGGCTCCTCGACTGGGGCTGTTACATCTGGCAACTACCCAAACTGGGTCCAACAAAAAC<br>CAGGTCAGGCACCCCGTGGTCTAATAGGTGGGACTAAGTTCCTCGCCCCCGGTACTCCTGCCAGA<br>TTCTCAGGCTCCCTGCTTGGAGGCAAGGCTGCCCTCACCCTCTCAGGGGTACAGCCAGAGGATGA<br>GGCAGAATATTACTGTGTTCTATGGTACAGCAACCGCTGGGTGTTCGGTGGAGGAACCAAACTGA<br>CTGTCCTA |
| 505 | IgE LH x I2C HL | artificial | aa | DIQLTQSPSSLSASVGDRVTITCRASQSVDYDGDSYMNWYQQKPGKAPKLLIYAASYLESGVPSR<br>FSGSGSGTDFTLTISSLQPEDFATYYCQQSHEDPYTFGQGTKVEIKgggggsggggsgggggsEV<br>QLVESGGGLVQPGGSLRLSCAVSGYSITSGYSWNWIRQAPGKGLEWVASITYDGSTNYADSVKGR<br>FTISRDDSKNTFYLQMNSLRAEDTAVYYCARGSHYFGHWHFAVWGQGTLVTVSSGGGGSEVQLVE<br>SGGGLVQPGGSLKLSCAASGFTFNKYAMNWVRQAPGKGLEWVARIRSKYNNYATYYADSVKDRFT<br>ISRDDSKNTAYLQMNNLKTEDTAVYYCVRHGNFGNSYISYWAYWGQGTLVTVSSGGGGSGGGGSG<br>GGGSQTVVTQEPSLTVSPGGTVTLTCGSSTGAVTSGNYPNWVQQKPGQAPRGLIGGTKFLAPGTP<br>ARFSGSLLGGKAALTLSGVQPEDEAEYYCVLWYSNRWVFGGGTKLTVL |
| 506 | IgE LH x I2C HL | artificial | nt | GACATCCAGCTGACCCAGTCTCCATCCTCCCTGTCTGCATCTGTTGGAGACAGAGTCACCATCAC<br>TTGCCGGGCAAGTCAGAGCGTTGACTATGATGGGGACAGCTATATGAATTGGTATCAGCAGAAAC<br>CAGGGAAAGCCCCTAAGCTCCTGATCTATGCTGCATCCTACTTGGAAAGTGGGGTCCCATCAAGG<br>TTCAGTGGCAGTGGATCTGGGACAGATTTCACTCTCACCATCAGCAGTCTGCAACCTGAAGATTT<br>TGCAACTTACTACTGTCAACAGTCTCATGAAGATCCATATACCTTTGGACAGGGCACTAAAGTCG<br>AAATAAAGGGTGGTGGTGGTTCTGGCGGCGGCGGCTCCGGTGGTGGTGGTTCTGAGGTGCAGCTG<br>GTGGAGTCTGGGGGAGGCTTGGTCCAGCCTGGGGGGTCCCTGAGACTCTCCTGTGCAGTCTCTGG<br>ATACTCCATCACCAGCGGCTACAGTTGGAACTGGATCCGCCAGGCTCCAGGGAAGGGGCTGGAGT<br>GGGTCGCAAGTATTACGTACGACGGTAGCACAAACTACGCAGACTCCGTGAAGGGCAGATTCACC<br>ATCTCCAGAGACGACTCCAAGAACACGTTCTATCTTCAAATGAACAGCCTGAGAGCCGAGGACAC<br>GGCTGTGTATTACTGTGCGAGAGGTTCCCATTATTTCGGCCATTGGCACTTCGCCGTGTGGGGCC<br>AGGGAACGCTTGTCACAGTTAGCTCCGGAGGTGGTGGATCCGAGGTGCAGCTGGTCGAGTCTGGA<br>GGAGGATTGGTGCAGCCTGGAGGGTCATTGAAACTCTCATGTGCAGCCTCTGGATTCACCTTCAA<br>TAAGTACGCCATGAACTGGGTCCGCCAGGCTCCAGGAAAGGGTTTGGAATGGGTTGCTCGCATAA<br>GAAGTAAATATAATAATTATGCAACATATTATGCCGATTCAGTGAAAGACAGGTTCACCATCTCC<br>AGAGATGATTCAAAAAACACTGCCTATCTACAAATGAACAACTTGAAAACTGAGGACACTGCCGT<br>GTACTACTGTGTGAGACATGGGAACTTCGGTAATAGCTACATATCCTACTGGGCTTACTGGGGCC<br>AAGGGACTCTGGTCACCGTCTCCTCAGGTGGTGGTGGTTCTGGCGGCGGCGGCTCCGGTGGTGGT<br>GGTTCTCAGACTGTTGTGACTCAGGAACCTTCACTCACCGTATCACCTGGTGGAACAGTCACACT |

CACTTGTGGCTCCTCGACTGGGGCTGTTACATCTGGCAACTACCCAAACTGGTCCAACAAAAAC
CAGGTCAGGCACCCCGTGGTCTAATAGTGGGACTAAGTTCCTGCCCCCGTACTCCTGCCAGA
TTCTCAGGCTCCCCTGGAGGCAAGGCTGCCCTCACCCTCCAGGGTACAGCCAGAGGATGA
GGCAGAATATTACTGTGTTCTATGGTACAGCAACCGCTGGGTGTTCGGTGGAGGAACCAAACTGA
CTGTCCTA

| 507 | IgE Ag | rhesus | nt | GAATTCCACCATGGGATGGAGCTGTATCATCCTCTTCTTGGTAGCAACAGCTACAGGTAAGGGGC TCACAGTAGCAGGCTTGAGGTCTGGACATATATGGGTGACAATGACATCCACTTTGCCTTTCT CTCCTTAGGTGTACACTCCCAGGTCCAACTGCACCAGCCTGAGCTTGTGAAGCCTGGG GCTTCAGTGAAGCTGTCCTGCAAGGCTTCTGGCTACACCTTCACCAGCTACTGCACTGGGT GAAGCAGAGGCCTGGACGAGGCCTTGAGTGGATGGAAGGATTGATCCTAATAGTGGTGGTACTA AGTACAATGAGAAGTTCAAGAGCAAGGCCACACTGACTGTAGACAAACCCTCCAGCACAGCCTAC ATGCAGCTCAGCAGCCTGACATCTGAGGACTCTGCGGTCTATTATTGTGCAAGATACGATTACTA CGGTAGTAGCTACTTTGACTACTGGGGCCAAGGCACCACTCTCACAGTCTCCTCAGGTGAGTCCT TACAACCTCTCTCTTCTATTCAGCTTAAATAGATTTTACTGCATTTGTTGGGGGGAAATGTGTG TATCTGAATTTCAGTCAGGTCATGAAGACTAGGGACACCTTGGGAGTCAGAAAGGGTCATTGGGAGCC CTGGCTGATGCAGACAGACATCCTCAGCTCCCAGACTTCATGGCCCAGAGATTTATAGGGATCCAC TAGTTCTAGATGTCCCACACCGCGGTCACCCTCTTGCAGCCTCCATCACAGAGCC CATTCGTCTTCTTCCCCCTTGATCCCCTGCTGCAAACACCATTGCCTCCAATGCCACCTCCCGTCCGACTCTG GGCTGCCTGGCCACGGGCTACTTCCCGAGCCACCTTCACCTGGTGACGCAGGCTCCCTCAA CCGGTCAACTATGACCTTACCAGCCACACCTTCTGGGTCACTATGCCACCATCAGCT TGTGACCGTCCGGGTGCGTGGGCGTGGGCCAAGGAGACTTTCACCTGCCATGTGGTACACACTCCATCG TCCGCAGACAAGGAGGTTAACAAAACCTTCGGCGTGCTGCTCCAGGAACTTCACCCCGCCCACCGT GAAGATCTTACAGTCGTCCTGCGACGATGACGGGCACTTCCCCCGACCATCCAGCTCCTGTGCC TCATCTCTGGGTACACCCCAGGGGCTATCAACGCGCAGGAGGGTGAGCTGCAGGTCATGAAA GTGAACTCGCCCACCCCCGCCCTGCGCAACGGCAGTGGCCTCCACAACAAGCGAGTTTAC CCTCGCTCAGAAGAGCAGCAGTGGCTGACAGAAGTGTGCAGTACACCCGAGATTCCAACCGAGGCCTCAAGTCACCTATCAAGGTACCA CCTATAACGACACAGCACCAAGAAGTGTGCAGATTCCAACCCGAGGGGGTCACCGTGCTGGTGGTGGACCT CGGCCCCAGCCCAGGCAAGGACCGTCGGACCGTTGAACCTGACCTGTGCCCCCACA GGCACCCCAGCAGGGAGAAGAGCCGGAGACCTGAACCTGTAACGTCACGTCACGGCAGGTCATGAAA TCCCCACAACGACTGGATCGAGGGGGAGAGATCGAGAGCCCAATGGCACGTTAACCTGACCTGACCTGGAAGCCTGTCCATCTCGCCGGGTAGTC ACCCAAGACTGGATCGAGGGGGGAGAACCAGACCTACCAGGTGCAGCCTGGGACTTCGGCAGCC CCTCGTGCGCCATGATCCATGACCATGTGGCTGCTTCTTCAGCCTGTCTTCAGGTCTATGTTTTGCGA CGCCGGAGAGAGCTGGAGAGCTGGAGCCGGACAGCCGGACAGACCACCACCGGAAGTCTTATGTTTGCGA GCGCCCCGCCAAGACCAGAAAGATGAGTTCATCTGCCGTGCAGTCAGTGCAGAGGCAGCAGCCAAGGCCG AATGGGAGCAGACAGAAAGATGAGTTCATCTGCCGTGCAGTCAGTGCAGAGGCAGCAGCCCCTCATGGATC |

| | | | | GTCCAGCAAGCGGTGTCTGTAAATCCCGAGCTGGACGTGTGCGTGGAGGAGGCCGAGGGCGAGGC GCCGTGGACGTGGACCGGCCTCTGCATCTTCGCCGCACTCTTCCTGCTCAGCGTGAGCTACAGCG CCGCCCTCACGCTCCTCATGGTGCAGCGGTTCCTCTCAGCCACGCGGCAGGGGAGGCCCCAGACC TCCCTCGACTACACCAACGTCCTCCAGCCCCACGCCTAGTCTAGAGTCGAC |
|---|---|---|---|---|
| 508 | IgE Ag | human | nt | GAATTCCACCATGGGATGGAGCTGTATCATCCTCTTCTTGGTAGCAACAGCTACAGGTAAGGGGC TCACAGTAGCAGGCTTGAGGTCTGGACATATATATGGGTGACAATGACATCCACTTTGCCTTTCT CTCCTTAGGGTGTACACTCCCAGGTCCAACTGCACCAGCCTGGGGCTGAGCTTGTGAAGCCTGGG GCTTCAGTGAAGCTGTCCTGCAAGGCTTCTGGCTACACCTTCACCAGCTACTGGATGCACTGGGT GAAGCAGAGGCCTGGACGAGGCCTTGAGTGGATTGGAAGGATTGATCCTAATAGTGGTGGTACTA AGTACAATGAGAAGTTCAAGAGCAAGGCCACACTGACTGTAGACAAACCCTCCAGCACAGCCTAC ATGCAGCTCAGCAGCCTGACATCTGAGGACTCTGCGGTCTATTATTGTGCAAGATACGATTACTA CGGTAGTAGCTACTTTGACTACTGGGGCCAAGGCACCACTCTCACAGTCTCCTCAGGTGAGTCCT TACAACCTCTCTCTTCTATTCAGCTTAAATAGATTTTACTGCATTTGTTGGGGGGGAAATGTGTG TATCTGAATTTCAGGTCATGAAGGACTAGGGACACCTTGGGAGTCAGAAAGGGTCATTGGGAGCC CTGGCTGATGCAGACAGACATCCTCAGCTCCCAGACTTCATGGCCAGAGATTTATAGGGGATCCC TGCCACGGGGTCCCCAGCTCCCCCATCCAGGCCCCCCAGGCTGATGGGCGCTGGCCTGAGGCTGG CACTGACTAGGTTCTGTCCTCACAGCCTCCACACAGAGCCCATCCGTCTTCCCCTTGACCCGCTG CTGCAAAAACATTCCCTCCAATGCCACCTCCGTGACTCTGGGCTGCCTGGCCACGGGCTACTTCC CGGAGCCGGTGATGGTGACCTGGGACACAGGCTCCCTCAACGGGACAACTATGACCTTACCAGCC ACCACCCTCACGCTCTCTGGTCACTATGCCACCATCAGCTTGCTGACCGTCTCGGGTGCGTGGGC CAAGCAGATGTTCACCTGCCGTGTGGCACACACTCCATCGTCCACAGACTGGGTCGACAACAAAA CCTTCAGCGGTAAGAGAGGGCCAAGCTCAGAGACCACAGTTCCCAGGAGTGCCAGGCTGAGGGCT GGCAGAGTGGGCAGGGGTTGAGGGGGTGGGTGGGCTCAAACGTGGGAACACCCAGCATGCCTGGG GACCCGGGCCAGGACGTGGGGGGCAAGAGGAGGGCACACAGAGCTCAGAGAGGCCAACAACCCTCA TGACCACCAGCTCTCCCCCAGTCTGCTCCAGGGACTTCACCCCGCCCACCGTGAAGATCTTACAG TCGTCCTGCGACGGCGGCGGGCACTTCCCCCCGACCATCCAGCTCCTGTGCCTCGTCTCTGGGTA CACCCCAGGGACTATCAACATCACCTGGCTGGAGGACGGGCAGGTCATGGACGTGGACTTGTCCA CCGCCTCTACCACGCAGGAGGGTGAGCTGGCCTCCACACAAAGCGAGCTCACCCTCAGCCAGAAG CACTGGCTGTCAGACCGCACCTACACCTGCCAGGTCACCTATCAAGGTCACACCTTTGAGGACAG CACCAAGAAGTGTGCAGGTACGTTCCCACCTGCCCTGGTGGCCGCCACGGAGGCCAGAGAAGAGG GGCGGGTGGGCCTCACACAGCCCTCCGGTGTACCACAGATTCCAACCCGAGAGGGGTGAGCGCCT ACCTAAGCCGGCCCAGCCCGTTCGACCTGTTCATCCGCAAGTCGCCCACGATCACCTGTCTGGTG GTGGACCTGGCACCCAGCAAGGGGACCGTGAACCTGACCTGGTCCCGGGCCAGTGGGAAGCCTGT GAACCACTCCACCAGAAAGGAGGAGAAGCAGCGCAATGGCACGTTAACCGTCACGTCCACCCTGC CGGTGGGCACCCGAGACTGGATCGAGGGGGAGACCTACCAGTGCAGGGTGACCCACCCCCACCTG CCCAGGGCCCTCATGCGGTCCACGACCAAGACCAGCGGTGAGCCATGGGCAGGCCGGGGTCGTGG |

```
GGGAAGGGAGGAGCGAGTGAGCGGGGCCCGGGGCTGACCCCACGTCTGGCCACAGGCCCGCGTGC
TGCCCCGGAAGTCTATGCGTTTGCGACGCCGAGTGGCCGGGGAGCCGGGACAAGCGCACCCTCG
CCTGCCTGATCCAGAACTTCATGCCTGAGGACATCTCGGTGCAGTGGCTGCACAACGAGGTGCAG
CTCCCGGACGCCCGGCACAGCACGACCCCGCAAGACCAAGGGCTCCGGCTTCTTCGTCTT
CAGCCGCCTGGAGGTGACCAGGGCCGAATGGGAGCAGAAAGATGAGTTCATCTGCCGTGCAGTCC
ATGAGGCAGCGAGCCCCTCACAGACCGTCCAGCGAGCGGTGTCTGTAAATCCCGAGCTGGACGTG
TGCGTGGAGGAGGCCGGAGGCGGAGGCGCCGGGCCGTGGACGTGGACCGGCCTCCTCACGCTCTCATGGTGCAGCGGTTCCTCTCAG
CTTCCTGCTCAGCGTGAGCTACAGCGCGCGCCCCGGCACGCTACAGCCTCCCTGACTACACCACCAACGTCCTCCCCAGCCCCACGCGCCTAG
CCACGCGGCAGGGGAGGGGAGGCCCCCAGACCCTCCCTCGACTACACCACCAACGTCCTCCCCAGCCCCACGCGCCTAG
TCTAGAGTCGAC
```

**Claims**

1. A polypeptide comprising a first binding domain which is an antibody capable of binding to an epitope of human and Callithrix jacchus, Saguinus oedipus or Saimiri sciureus CD3ε chain, wherein the epitope is part of an amino acid sequence comprised in the group consisting of SEQ ID NO:2, 4, 6, or 8 and comprises at least the amino acid sequence Gln-Asp-Gly-Asn-Glu, and a second binding domain capable of binding to EGFR, Her2/neu or IgE of a human and/or a non-chimpanzee primate.

2. The polypeptide as defined in claim 1, wherein the epitope is part of an amino acid sequence comprised in the group consisting of SEQ ID NOs:2, 4, 6, and 8 and comprises at least the amino acid sequence Gln-Asp-Gly-Asn-Glu.

3. The polypeptide according to any one of claims 1 or 2, wherein the first binding domain comprises a VL region comprising CDR-L1, CDR-L2 and CDR-L3 selected from:

   (a) CDR-L1 as depicted in SEQ ID NO:27, CDR-L2 as depicted in SEQ ID NO:28 and CDR-L3 as depicted in SEQ ID NO:29;
   (b) CDR-L1 as depicted in SEQ ID NO:117, CDR-L2 as depicted in SEQ ID NO:118 and CDR-L3 as depicted in SEQ ID NO:119; and
   (c) CDR-L1 as depicted in SEQ ID NO:153, CDR-L2 as depicted in SEQ ID NO:154 and CDR-L3 as depicted in SEQ ID NO:155.

4. The polypeptide according to any one of claims 1 or 2, wherein the first binding domain comprises a VH region comprising CDR-HL, CDR-H2 and CDR-H3 selected from:

   (a) CDR-H1 as depicted in SEQ ID NO:12, CDR-H2 as depicted in SEQ ID NO:13 and CDR-H3 as depicted in SEQ ID NO:14;
   (b) CDR-H1 as depicted in SEQ ID NO:30, CDR-H2 as depicted in SEQ ID NO:31 and CDR-H3 as depicted in SEQ ID NO:32;
   (c) CDR-H1 as depicted in SEQ ID NO:48, CDR-H2 as depicted in SEQ ID NO:49 and CDR-H3 as depicted in SEQ ID NO:50;
   (d) CDR-H1 as depicted in SEQ ID NO:66, CDR-H2 as depicted in SEQ ID NO:67 and CDR-H3 as depicted in SEQ ID NO:68;
   (e) CDR-H1 as depicted in SEQ ID NO:84, CDR-H2 as depicted in SEQ ID NO:85 and CDR-H3 as depicted in SEQ ID NO:86;
   (f) CDR-H1 as depicted in SEQ ID NO:102, CDR-H2 as depicted in SEQ ID NO:103 and CDR-H3 as depicted in SEQ ID NO:104;
   (g) CDR-H1 as depicted in SEQ ID NO:120, CDR-H2 as depicted in SEQ ID NO:121 and CDR-H3 as depicted in SEQ ID NO:122;
   (h) CDR-H1 as depicted in SEQ ID NO:138, CDR-H2 as depicted in SEQ ID NO:139 and CDR-H3 as depicted in SEQ ID NO:140;
   (i) CDR-H1 as depicted in SEQ ID NO:156, CDR-H2 as depicted in SEQ ID NO:157 and CDR-H3 as depicted in SEQ ID NO:158; and
   (j) CDR-H1 as depicted in SEQ ID NO:174, CDR-H2 as depicted in SEQ ID NO:175 and CDR-H3 as depicted in SEQ ID NO:176.

5. The polypeptide according to any one of claims 1 to 3, wherein the first binding domain comprises a VL region selected from the group consisting of a VL region as depicted in SEQ ID NO:35, 39, 125, 129, 161 or 165.

6. The polypeptide according to any one of claims 1 to 4, wherein the first binding domain comprises a VH region selected from the group consisting of a VH region as depicted in SEQ ID NO:15, 19, 33, 37, 51, 55, 69, 73, 87, 91, 105, 109, 123, 127, 141, 145, 159, 163, 177 or 181.

7. The polypeptide according to any one of claims 1 to 6, wherein the first binding domain comprises a VL region and a VH region selected from the group consisting of:

   (a) a VL region as depicted in SEQ ID NO:17 or 21 and a VH region as depicted in SEQ I D NO:15 or 19;
   (b) a VL region as depicted in SEQ ID NO:35 or 39 and a VH region as depicted in SEQ ID NO:33 or 37;
   (c) a VL region as depicted in SEQ ID NO:53 or 57 and a VH region as depicted in SEQ ID NO:51 or 55;

(d) a VL region as depicted in SEQ ID NO:71 or 75 and a VH region as depicted in SEQ ID NO:69 or 73;
(e) a VL region as depicted in SEQ ID NO:89 or 93 and a VH region as depicted in SEPA ID NO:87 or 91 ;
(f) a VL region as depicted in SEQ ID NO:107 or 111 and a VH region as depicted in SEQ ID NO:105 or 109;
(g) a VL region as depicted in SEQ ID NO:125 or 129 and a VH region as depicted in SEQ ID NO:123 or 127;
(h) a VL region as depicted in SEQ ID NO:143 or 147 and a VH region as depicted in SEQ ID NO:141 or 145;
(i) a VL region as depicted in SEQ ID NO:161 or 165 and a VH region as depicted in SEQ ID NO:159 or 163; and
(j) a VL region as depicted in SEQ ID NO:179 or 183 and a VH region as depicted in SEQ ID NO:177 or 181.

8. The polypeptide according to claim 7, wherein the first binding domain comprises an amino acid sequence selected from the group consisting of SEQ ID NO:23, 25, 41, 43, 59, 61, 77, 79, 95, 97, 113, 115, 131, 133, 149, 151, 167, 169, 185 or 187.

9. The polypeptide of any one of claims 1 to 8, wherein said polypeptide is a bispecific single chain antibody molecule.

10. The polypeptide according to claim 9, wherein the bispecific single chain antibody molecule comprises a group of the following sequences as CDR H1, CDR H2, CDR H3, CDR L1, CDR L2 and CDR L3 in the second binding domain selected from SEQ ID NOs:441 - 446, SEQ ID NOs:453 - 458, SEQ ID NOs:463 - 468, SEQ ID NOs:481 - 486.

11. The polypeptide according to claim 9, wherein the bispecific single chain antibody molecule comprises a sequence selected from:

(a) an amino acid sequence as depicted in any of SEQ ID NOs:389, 391, 393, 395, 397, 399, 409, 411, 413, 415, 417, 419, 429, 431, 433, 435, 437, 439, 447, 449, 451, 469, 471, 473, 475, 477, 479, 495, 497, 499, 501, 503 and 505; and
(b) an amino acid sequence encoded by a nucleic acid sequence as depicted in any of SEQ ID NOs:390, 392, 394, 396, 398, 400, 410, 412, 414, 416, 418, 420, 430, 432, 434, 436, 438, 440, 448, 450, 452, 470, 472, 474, 476, 478, 480, 496, 498, 500, 502, 504 and 506.

12. A nucleic acid sequence encoding a polypeptide as defined in any of claims 1 to 11.

13. A vector, which comprises a nucleic acid sequence as defined in claim 12.

14. A host cell transformed or transfected with a vector defined in claim 13.

15. A process for the production of a polypeptide according to any of claims 1 to 11, said process comprising culturing a host cell defined in claim 14 under conditions allowing the expression of the polypeptide as defined in any of claims 1 to 11 and recovering the produced polypeptide from the culture.

16. A pharmaceutical composition comprising a polypeptide according to any one of claims 1 to 11, or produced according to the process of claim 15.

17. The polypeptide according to any one of claims 1 to 11, or produced according to the process of claim 15 for use in the prevention, treatment or amelioration of a disease selected from a proliferative disease, a tumorous disease, or an immunological disorder.

18. A kit comprising a polypeptide as defined in any of claims 1 to 11, a nucleic acid molecule as defined in claim 12, a vector as defined in claim 13, or a host cell as defined in claim 14.


**Patentansprüche**

1. Polypeptid, umfassend eine erste Bindedomäne, die ein Antikörper ist, der in der Lage ist an ein Epitop einer menschlichen CD3ε-Kette und einer CD3ε-Kette von Callithrix jacchus, Saguinus oedipus oder Saimiri sciureus zu binden, wobei das Epitop Teil einer Aminosäuresequenz ist, umfasst von der Gruppe bestehend aus SEQ ID NO: 2, 4, 6, oder 8 und zumindest die Aminosäuresequenz Gln-Asp-Gly-Asn-Glu umfasst, und eine zweite Bindedomäne, die in der Lage ist, an EGFR, Her2/neu oder IgE eines Menschen und/oder eines Primaten, der kein Schimpanse ist, zu binden.

**2.** Polypeptid wie in Anspruch 1 definiert, wobei das Epitop Teil einer Aminosäuresequenz ist, umfasst von der Gruppe bestehend aus SEQ ID NO:2, 4, 6, und 8 und wobei es zumindest die Aminosäuresequenz Gln-Asp-Gly-Asn-Glu umfasst.

**3.** Polypeptid gemäß Anspruch 1 oder 2, wobei die erste Bindedomäne eine VL Region umfasst, umfassend CDR-L1, CDR-L2 und CDR-L3 ausgewählt aus:

(a) CDR-L1 wie dargestellt in SEQ ID NO:27, CDR-L2 wie dargestellt in SEQ ID NO:28 und CDR-L3 wie dargestellt in SEQ ID NO:29;
(b) CDR-L1 wie dargestellt in SEQ ID NO:117, CDR-L2 wie dargestellt in SEQ ID NO:118 und CDR-L3 wie dargestellt in SEQ ID NO:119; und
(c) CDR-L1 wie dargestellt in SEQ ID NO:153, CDR-L2 wie dargestellt in SEQ ID NO:154 und CDR-L3 wie dargestellt in SEQ ID NO:155.

**4.** Polypeptid gemäß Anspruch 1 oder 2, wobei die erste Bindedomäne eine VH Region umfasst, umfassend CDR-H1, CDR-H2 und CDR-H3 ausgewählt aus:

(a) CDR-H1 wie dargestellt in SEQ ID NO:12, CDR-H2 wie dargestellt in SEQ ID NO:13 und CDR-H3 wie dargestellt in SEQ ID NO:14;
(b) CDR-H1 wie dargestellt in SEQ ID NO:30, CDR-H2 wie dargestellt in SEQ ID NO:31 und CDR-H3 wie dargestellt in SEQ ID NO:32;
(c) CDR-H1 wie dargestellt in SEQ ID NO:48, CDR-H2 wie dargestellt in SEQ ID NO:49 und CDR-H3 wie dargestellt in SEQ ID NO:50;
(d) CDR-H1 wie dargestellt in SEQ ID NO:66, CDR-H2 wie dargestellt in SEQ ID NO:67 und CDR-H3 wie dargestellt in SEQ ID NO:68;
(e) CDR-H1 wie dargestellt in SEQ ID NO:84, CDR-H2 wie dargestellt in SEQ ID NO:85 und CDR-H3 wie dargestellt in SEQ ID NO:86;
(f) CDR-H1 wie dargestellt in SEQ ID NO:102, CDR-H2 wie dargestellt in SEQ ID NO:103 und CDR-H3 wie dargestellt in SEQ ID NO:104;
(g) CDR-H1 wie dargestellt in SEQ ID NO:120, CDR-H2 wie dargestellt in SEQ ID NO:121 und CDR-H3 wie dargestellt in SEQ ID NO:122;
(h) CDR-H1 wie dargestellt in SEQ ID NO:138, CDR-H2 wie dargestellt in SEQ ID NO:139 und CDR-H3 wie dargestellt in SEQ ID NO:140;
(i) CDR-H1 wie dargestellt in SEQ ID NO:156, CDR-H2 wie dargestellt in SEQ ID NO:157 und CDR-H3 wie dargestellt in SEQ ID NO:158; und
(j) CDR-H1 wie dargestellt in SEQ ID NO:174, CDR-H2 wie dargestellt in SEQ ID NO:175 und CDR-H3 wie dargestellt in SEQ ID NO:176.

**5.** Polypeptid gemäß einem der Ansprüche 1 bis 3, wobei die erste Bindedomäne eine VL Region umfasst, ausgewählt aus der Gruppe bestehend aus einer VL Region wie dargestellt in SEQ ID NO:35, 39, 125, 129, 161 oder 165.

**6.** Polypeptid gemäß einem der Ansprüche 1 bis 4, wobei die erste Bindedomäne eine VH Region umfasst, ausgewählt aus der Gruppe bestehend aus einer VH Region wie dargestellt in SEQ ID NO:15, 19, 33, 37, 51, 55, 69, 73, 87, 91, 105, 109, 123, 127, 141, 145, 159, 163, 177 oder 181.

**7.** Polypeptid gemäß einem der Ansprüche 1 bis 6, wobei die erste Bindedomäne eine VL Region und eine VH Region umfasst, ausgewählt aus der Gruppe bestehend aus:

(a) einer VL Region wie dargestellt in SEQ ID NO:17 oder 21 und einer VH Region wie dargestellt in SEQ ID NO:15 oder 19;
(b) einer VL Region wie dargestellt in SEQ ID NO:35 oder 39 und einer VH Region wie dargestellt in SEQ ID NO:33 oder 37;
(c) einer VL Region wie dargestellt in SEQ ID NO:53 oder 57 und einer VH Region wie dargestellt in SEQ ID NO:51 oder 55;
(d) einer VL Region wie dargestellt in SEQ ID NO:71 oder 75 und einer VH Region wie dargestellt in SEQ ID NO:69 oder 73;
(e) einer VL Region wie dargestellt in SEQ ID NO:89 oder 93 und einer VH Region wie dargestellt in SEQ ID NO:87 oder 91 ;

(f) einer VL Region wie dargestellt in SEQ ID NO:107 oder 111 und einer VH Region wie dargestellt in SEQ ID NO:105 oder 109;

(g) einer VL Region wie dargestellt in SEQ ID NO:125 oder 129 und einer VH Region wie dargestellt in SEQ ID NO:123 oder 127;

(h) einer VL Region wie dargestellt in SEQ ID NO:143 oder 147 und einer VH Region wie dargestellt in SEQ ID NO:141 oder 145;

(i) einer VL Region wie dargestellt in SEQ ID NO:161 oder 165 und einer VH Region wie dargestellt in SEQ ID NO:159 oder 163; und

(j) einer VL Region wie dargestellt in SEQ ID NO:179 oder 183 und einer VH Region wie dargestellt in SEQ ID NO:177 oder 181.

8. Polypeptid gemäß Anspruch 7, wobei die erste Bindedomäne eine Aminosäuresequenz umfasst, ausgewählt aus der Gruppe bestehend aus SEQ ID NO:23, 25, 41, 43, 59, 61, 77, 79, 95, 97, 113, 115, 131, 133, 149, 151, 167, 169, 185 oder 187.

9. Polypeptid gemäß einem der Ansprüche 1 bis 8, wobei das Polypeptid ein bispezifisches Einzelketten-Antikörpermolekül ist.

10. Polypeptid gemäß Anspruch 9, wobei das bispezifische Einzelketten-Antikörpermolekül eine Gruppe der folgenden Sequenzen als CDR H1, CDR H2, CDR H3, CDR L1, CDR L2 und CDR L3 in der zweiten Bindedomäne umfasst ausgewählt aus SEQ ID NOs:441 - 446, SEQ ID NOs:453 - 458, SEQ ID NOs:463 - 468, SEQ ID NOs:481 - 486.

11. Polypeptid gemäß Anspruch 9, wobei das bispezifische Einzelketten-Antikörpermolekül eine zweite Sequenz umfasst, ausgewählt aus:

(a) einer Aminosäuresequenz wie dargestellt in einer der Sequenzen SEQ ID NOs:389, 391, 393, 395, 397, 399, 409, 411, 413, 415, 417, 419, 429, 431, 433, 435, 437, 439, 447, 449, 451, 469, 471, 473, 475, 477, 479, 495, 497, 499, 501, 503 und 505; und

(b) einer Aminosäuresequenz kodiert von einer Nukleinsäuresequenz wie dargestellt in einer der Sequenzen SEQ ID NOs:390, 392, 394, 396, 398, 400, 410, 412, 414, 416, 418, 420, 430, 432, 434, 436, 438, 440, 448, 450, 452, 470, 472, 474, 476, 478, 480, 496, 498, 500, 502, 504 und 506.

12. Nukleinsäuresequenz, kodierend ein wie in einem der Ansprüche 1 bis 11 definiertes Polypeptid.

13. Vektor, umfassend eine Nukleinsäuresequenz gemäß Anspruch 12.

14. Wirtszelle, die mit einem wie in Anspruch 13 definierten Vektor transformiert oder transfiziert wurde.

15. Verfahren zur Herstellung eines Polypeptids gemäß einem der Ansprüche 1 bis 11, wobei das Verfahren die Schritte umfasst: Kultivieren einer wie in Anspruch 14 definierten Wirtszelle unter Bedingungen, die die Expression des wie in einem der Ansprüche 1 bis 11 definierten Polypeptids erlauben, und Gewinnen der hergestellten Polypeptide aus der Kultur.

16. Pharmazeutische Zusammensetzung, umfassend ein Polypeptid gemäß einem der Ansprüche 1 bis 11, oder hergestellt gemäß dem Verfahren nach Anspruch 15.

17. Polypeptid gemäß einem der Ansprüche 1 bis 11, oder hergestellt gemäß dem Verfahren nach Anspruch 15 zur Verwendung bei der Prävention, Behandlung oder Ablinderung einer Krankheit ausgewählt aus einer proliferativen Krankheit, einer tumorösen Krankheit oder einer immunologischen Störung.

18. Kit, umfassend ein wie in einem der Ansprüche 1 bis 11 definiertes Polypeptid, eine wie in Anspruch 12 definierte Nukleinsäure, einen wie in Anspruch 13 definierten Vektor oder eine wie in Anspruch 14 definierte Wirtszelle.

**Revendications**

1. Polypeptide comprenant un premier domaine de liaison qui est un anticorps capable de se fixer à un déterminant antigénique de la chaîne CDR ε humaine et de Callithrix jacchus, Saguinus oedipus ou Saimiri sciureus, dans lequel le déterminant antigénique fait partie d'une séquence d'acides aminés comprise dans le groupe constitué de SEQ ID N° : 2, 4, 6 ou 8 et comprend au moins la séquence d'acides aminés Gln-Asp-Gly-Asn-Glu, et un second domaine de liaison capable de se fixer à EGFR, Her2/neu ou IgE d'un homme et/ou d'un primate différent du chimpanzé.

2. Polypeptide selon la revendication 1, dans lequel le déterminant antigénique fait partie d'une séquence d'acides aminés comprise dans le groupe constitué de SEQ ID N° : 2, 4, 6 et 8 et comprend au moins la séquence d'acides aminés Gln-Asp-Gly-Asn-Glu.

3. Polypeptide selon l'une quelconque des revendications 1 ou 2, dans lequel le premier domaine de liaison comprend une région VL comprenant CDR-L1, CDR-L2 et CDR-L3 sélectionnée parmi :

（a) CDR-L1 telle que représentée dans SEQ ID N° : 27, CDR-L2 telle que représentée dans SEQ ID N° : 28 et CDR-L3 telle que représentée dans SEQ ID N° : 29 ;
(b) CDR-L1 telle que représentée dans SEQ ID N° : 117, CDR-L2 telle que représentée dans SEQ ID N° : 118 et CDR-L3 telle que représentée dans SEQ ID N° : 119 ; et
(c) CDR-L1 telle que représentée dans SEQ ID N° : 153, CDR-L2 telle que représentée dans SEQ ID N° : 154 et CDR-L3 telle que représentée dans SEQ ID N° : 155.

4. Polypeptide selon l'une quelconque des revendications 1 ou 2, dans lequel le premier domaine de liaison comprend une région VH comprenant CDR-H1, CDR-H2 et CDR-H3 sélectionnée parmi :

(a) CDR-H1 telle que représentée dans SEQ ID N° : 12, CDR-H2 telle que représentée dans SEQ ID N° : 13 et CDR-H3 telle que représentée dans SEQ ID N° : 14 ;
(b) CDR-H1 telle que représentée dans SEQ ID N° : 30, CDR-H2 telle que représentée dans SEQ ID N° : 31 et CDR-H3 telle que représentée dans SEQ ID N° : 32 ;
(c) CDR-H1 telle que représentée dans SEQ ID N° : 48, CDR-H2 telle que représentée dans SEQ ID N° : 49 et CDR-H3 telle que représentée dans SEQ ID N° : 50 ;
(d) CDR-H1 telle que représentée dans SEQ ID N° : 66, CDR-H2 telle que représentée dans SEQ ID N° : 67 et CDR-H3 telle que représentée dans SEQ ID N° : 68 ;
(e) CDR-H1 telle que représentée dans SEQ ID N° : 84, CDR-H2 telle que représentée dans SEQ ID N° : 85 et CDR-H3 telle que représentée dans SEQ ID N° : 86 ;
(f) CDR-H1 telle que représentée dans SEQ ID N° : 102, CDR-H2 telle que représentée dans SEQ ID N° : 103 et CDR-H3 telle que représentée dans SEQ ID N° : 104 ;
(g) CDR-H1 telle que représentée dans SEQ ID N° : 120, CDR-H2 telle que représentée dans SEQ ID N° : 121 et CDR-H3 telle que représentée dans SEQ ID N° : 122 ;
(h) CDR-H1 telle que représentée dans SEQ ID N° : 138, CDR-H2 telle que représentée dans SEQ ID N° : 139 et CDR-H3 telle que représentée dans SEQ ID N° : 140 ;
(i) CDR-H1 telle que représentée dans SEQ ID N° : 156, CDR-H2 telle que représentée dans SEQ ID N° : 157 et CDR-H3 telle que représentée dans SEQ ID N° : 158 ; et
(j) CDR-H1 telle que représentée dans SEQ ID N° : 174, CDR-H2 telle que représentée dans SEQ ID N° : 175 et CDR-H3 telle que représentée dans SEQ ID N° : 176.

5. Polypeptide selon l'une quelconque des revendications 1 à 3, dans lequel le premier domaine de liaison comprend une région VL sélectionnée parmi le groupe constitué d'une région VL telle que représentée dans SEQ ID N° : 35, 39, 125, 129, 161 ou 165.

6. Polypeptide selon l'une quelconque des revendications 1 à 4, dans lequel le premier domaine de liaison comprend une région VH sélectionnée parmi le groupe constitué d'une région VH telle que représentée dans SEQ ID N° : 15, 19, 33, 37, 51, 55, 69, 73, 87, 91, 105, 109, 123, 127, 141, 145, 159, 163, 177 ou 181.

7. Polypeptide selon l'une quelconque des revendications 1 à 6, dans lequel le premier domaine de liaison comprend une région VL et une région VH sélectionnées parmi le groupe constitué de :

(a) une région VL telle que représentée dans SEQ ID N° : 17 ou 21 et une région VH telle que représentée dans SEQ ID N° : 15 ou 19 ;
(b) une région VL telle que représentée dans SEQ ID N° : 35 ou 39 et une région VH telle que représentée dans SEQ ID N° : 33 ou 37 ;
(c) une région VL telle que représentée dans SEQ ID N° : 53 ou 57 et une région VH telle que représentée dans SEQ ID N° : 51 ou 55 ;

(d) une région VL telle que représentée dans SEQ ID N° : 71 ou 75 et une région VH telle que représentée dans SEQ ID N° : 69 ou 73 ;

(e) une région VL telle que représentée dans SEQ ID N° : 89 ou 93 et une région VH telle que représentée dans SEQ ID N° : 87 ou 91 ;

(f) une région VL telle que représentée dans SEQ ID N° : 107 ou 111 et une région VH telle que représentée dans SEQ ID N° : 105 ou 109 ;

(g) une région VL telle que représentée dans SEQ ID N° : 125 ou 129 et une région VH telle que représentée dans SEQ ID N° : 123 ou 127 ;

(h) une région VL telle que représentée dans SEQ ID N° : 143 ou 147 et une région VH telle que représentée dans SEQ ID N° : 141 ou 145 ;

(i) une région VL telle que représentée dans SEQ ID N° : 161 ou 165 et une région VH telle que représentée dans SEQ ID N° : 159 ou 163 ; et

(j) une région VL telle que représentée dans SEQ ID N° : 179 ou 183 et une région VH telle que représentée dans SEQ ID N° : 177 ou 181.

**8.** Polypeptide selon la revendication 7, dans lequel le premier domaine de liaison comprend une séquence d'acides aminés sélectionnée parmi le groupe constitué de SEQ ID N° : 23, 25, 41, 43, 59, 61, 77, 79, 95, 97, 113, 115, 131, 133, 149, 151, 167, 169, 185 ou 187.

**9.** Polypeptide selon l'une quelconque des revendications 1 à 8, dans lequel ledit polypeptide est une molécule d'anticorps à chaîne unique bispécifique.

**10.** Polypeptide selon la revendication 9, dans lequel la molécule d'anticorps à chaîne unique bispécifique comprend un groupe des séquences suivantes en tant que CDR H1, CDR H2, CDR H3, CDR L1, CDR L2 et CDR L3 dans le second domaine de liaison sélectionnées parmi les SEQ ID N° : 441 à 446, SEQ ID N° : 453 à 458, SEQ ID N° : 463 à 468, SEQ ID N° : 481 à 486.

**11.** Polypeptide selon la revendication 9, dans lequel la molécule d'anticorps à chaîne unique bispécifique comprend une séquence sélectionnée parmi :

(a) une séquence d'acides aminés telle que représentée dans l'une quelconque des SEQ ID N° : 389, 391, 393, 395, 397, 399, 409, 411, 413, 415, 417, 419, 429, 431, 433, 435, 437, 439, 447, 449, 451, 469, 471, 473, 475, 477, 479, 495, 497, 499, 501, 503 et 505 ; et

(b) une séquence d'acides aminés encodée par une séquence d'acide nucléique telle que représentée dans l'une quelconque des SEQ ID N° : 390, 392, 394, 396, 398, 400, 410, 412, 414, 416, 418, 420, 430, 432, 434, 436, 438, 440, 448, 450, 452, 470, 472, 474, 476, 478, 480, 496, 498, 500, 502, 504 et 506.

**12.** Séquence d'acide nucléique codant pour un polypeptide selon l'une quelconque des revendications 1 à 11.

**13.** Vecteur qui comprend une séquence d'acide nucléique selon la revendication 12.

**14.** Cellule hôte transformée ou transfectée avec un vecteur selon la revendication 13.

**15.** Procédé de production d'un polypeptide selon l'une quelconque des revendications 1 à 11, ledit procédé comprenant la mise en culture d'une cellule hôte selon la revendication 14 dans des conditions permettant l'expression du polypeptide selon l'une quelconque des revendications 1 à 11 et la récupération du polypeptide produit de la culture.

**16.** Composition pharmaceutique comprenant un polypeptide selon l'une quelconque des revendications 1 à 11 ou produite selon le procédé selon la revendication 15.

**17.** Polypeptide selon l'une quelconque des revendications 1 à 11 ou produit selon le procédé selon la revendication 15 destiné à être utilisé dans la prévention, le traitement ou l'amélioration d'une maladie sélectionnée parmi une maladie proliférative, une maladie tumorale ou un trouble immunologique.

**18.** Kit comprenant un polypeptide selon l'une quelconque des revendications 1 à 11, une molécule d'acide nucléique selon la revendication 12, un vecteur selon la revendication 13 ou une cellule hôte selon la revendication 14.

# Figure 1

N-terminal amino acids 1-27 of the primate CD3 epsilon (e.g. human)

Hinge region

Human IgG 1 Fc region

Hexa Histidin tag

Figure 2

Figure 3

EP 2 155 788 B1

# Figure 4

N-terminal amino acids 1-27 of the
primate CD3 epsilon (e.g. human)

Flag tagged full-length cynomolgus
EpCAM (membrane bound)

Cell membrane

Cellular cytoplasm

## Figure 5

Human 27 mer

Marmoset 27 mer

Tamarin 27 mer

Squirrel Monkey 27 mer

Swine 27 mer

Figure 6A

## Figure 6B

Marmoset 27 mer – H2C HLP

Marmoset 27 mer – F12Q HLP

Marmoset 27 mer – E2M HLP

Marmoset 27 mer – G4H HLP

EP 2 155 788 B1

Figure 6C

Tamarin 27 mer – H2C HLP

Tamarin 27 mer – F12Q HLP

Tamarin 27 mer – E2M HLP

Tamarin 27 mer – G4H HLP

EP 2 155 788 B1

Figure 6D

Swine 27 mer – H2C HLP

Swine 27 mer – F12Q HLP

Swine 27 mer – E2M HLP

Swine 27 mer – G4H HLP

## Figure 7

A2J HLP

EP 2 155 788 B1

Figure 8B

**Figure 8C**

H2C HLP

**Figure 8D**

F12Q HLP

Figure 9

**Figure 10**

Figure 11 a

**Figure: 11b**

**Figure: 11 c**

CHO human EGFR  HPB-ALL (human)  CHO cyno EGFR  4119 LnPx (cyno)

EGFR 1 HL x I2C HL

EGFR 1 HL x H2C HL

EGFR 1 HL x F12Q HL

Counts

Fluorescence Intensity

Figure: 11 d

**Figure 12**

**A)**

Effector cells: stimulated CD4/CD56 depleted human PBMC
Target cells: CHO transfected with human EGFR

**B)**

Effector cells: stimulated CD4/CD56 depleted human PBMC
Target cells: CHO transfected with human EGFR

Figure: 12 b

A)    Effector cells: CD56 depleted unstimulated human PBMC
      Target cells: CHO transfected with human EGFR

B)    Effector cells: 4119LnPx
      Target cells: CHO transfected with cyno EGFR

Figure: 12 c

A)  Effector cells: CD56 depleted unstimulated human PBMC
    Target cells: CHO transfected with human EGFR

B)  Effector cells: 4119LnPx
    Target cells: CHO transfected with cyno EGFR

**Figure: 12 d**

**A)** **Effector cells: stimulated CD4/CD56 depleted human PBMC**
**Target cells: CHO transfected with human EGFR**

------△ EGFR2 LH x H2C HL
--- ● EGFR2 LH x F12Q HL
——— ■ EGFR2 LH x I2C HL
------◆ negative control

**B)** **Effector cells: 4119LnPx**
**Target cells: CHO transfected with cyno EGFR**

--- ■ EGFR2 LH x H2C HL
——△ EGFR2 LH x F12Q HL
------● EGFR2 LH x I2C HL
------◆ negative control

**Figure 13**

**A)**

Effector cells: macaque T cell line 4119 LnPx
Target cells: CHO transfected with cynomolgus EGFR

**B)**

Effector cells: macaque T cell line 4119 LnPx
Target cells: CHO transfected with cynomolgus EGFR

Figure 14

Figure 15

Figure 16

**Figure 17**

**A)**

Effector cells: stimulated CD4/CD56 depleted human PBMC
Target cells: CHO transfected with human MCSP D3

**B)**

Effector cells: macaque T cell line 4119 LnPx
Target cells: CHO transfected with cynomolgus MCSP D3

Figure 18

A)

Effector cells: macaque T cell line 4119 LnPx
Target cells: CHO transfected with cynomolgus MCSP D3

B)

Effector cells: macaque T cell line 4119 LnPx
Target cells: CHO transfected with cynomolgus MCSP D3

209

**Figure 19**

**A)**

Effector cells: stimulated CD4/CD56 depleted human PBMC
Target cells: CHO transfected with human MCSP D3

**B)**

Effector cells: stimulated CD4/CD56 depleted human PBMC
Target cells: CHO transfected with human MCSP D3

Figure 20

A)

Effector cells: stimulated CD4/CD56 depleted human PBMC
Target cells: CHO transfected with human MCSP D3

MCSP-G4 HLP x E1L HLP
MCSP-G4 HLP x F12Q HL

B)

Effector cells: macaque T cell line 4119 LnPx
Target cells: CHO transfected with cynomolgus MCSP D

MCSP-G4 HLP x E1L HLP
MCSP-G4 HLP x F12Q HL

Figure 21

A)

Effector cells: stimulated CD4/CD56 depleted human PBMC
Target cells: CHO transfected with human MCSP D3

B)

Effector cells: macaque T cell line 4119 LnPx
Target cells: CHO transfected with cynomolgus MCSP D3

# Figure 22 (1)

- ■ —— G4 VH-VL x I2C VH-VL
- ▼ ..... G4 VH-VL x I2C VH-VL plasma
- ● _ _ _ G4 VH-VL x I2C VH-VL plasma 37°C
- G4 VH-VL x I2C VH-VL plasma 4°C
- ▲ --- Negative control
- ◆ ——

# Figure 22 (2)

specific lysis vs pg/ml

■——— G4 VH-VL x H2C VH-VL
▼····· G4 VH-VL x H2C VH-VL plasma
●--- G4 VH-VL x H2C VH-VL plasma 37°C
      G4 VH-VL x H2C VH-VL plasma 4°C
▲····· Negative control
◆———

# Figure 22 (3)

- ■── G4 VH-VL x F12Q VH-VL
- ▼······ G4 VH-VL x F12Q VH-VL plasma
- ___ G4 VH-VL x F12Q VH-VL plasma 37°C
- ●--- G4 VH-VL x F12Q VH-VL plasma 4°C
- ♦····· Negative control
- ●──

# Figure: 23 a

# Figure: 23 b

EP 2 155 788 B1

## Figure 24 a

**A)**

Effector cells: stimulated CD4/CD56 depleted human PBMC
Target cells: J558L cells transfected with Human IgE

**B)**

Effector cells: 4119 LnPx
Target cells: J558L cells transfected with macaque IgE

# Figure 24 b

## A)

Effector cells: stimulated CD4/CD56 depleted human PBMC
Target cells: J558L cells transfected with Human IgE

## B)

Effector cells: 4119 LnPx
Target cells: J558L cells transfected with macaque IgE

## Figure 25

human CD3 epsilon (aa 1-27) -Fc fusion protein

## Figure 26

human IgG1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007033230 A
- WO 9954440 A
- WO 04106380 A
- US 4946778 A
- WO 2004106380 A
- EP 623679 B1
- WO 9420627 A
- WO 9429469 A
- WO 9700957 A
- US 5580859 A
- US 5589466 A

**Non-patent literature cited in the description**

- **DAVIS ; BJORKMAN.** *Nature,* 1988, vol. 334, 395-402
- **CALL.** *Cell,* 2002, vol. 111, 967-979
- **ALARCON.** *Immunol. Rev.,* 2003, vol. 191, 38-46
- **MALISSEN.** *Immunol. Rev.,* 2003, vol. 191, 7-27
- **DAVIS.** *Cell,* 2002, vol. 110, 285-287
- **DAVIS ; VAN DER MERWE.** *Curr. Biol.,* 2001, vol. 11, R289-R291
- **DAVIS.** *Nat. Immunol.,* 2003, vol. 4, 217-224
- **GIL.** *J. Biol. Chem.,* 2001, vol. 276, 11174-11179
- **GIL.** *Cell,* 2002, vol. 109, 901-912
- **BERKHOUT.** *J. Biol. Chem.,* 1988, vol. 263, 8528-8536
- **WANG.** *J. Exp. Med.,* 1998, vol. 188, 1375-1380
- **KAPPES.** *Curr. Opin. Immunol.,* 1995, vol. 7, 441-447
- **SUN.** *Cell,* 2001, vol. 105, 913-923
- **BORROTO.** *J. Biol. Chem.,* 1998, vol. 273, 12807-12816
- **MANOLIOS.** *Eur. J. Immunol.,* 1994, vol. 24, 84-92
- **MANOLIOS ; LI.** *Immunol. Cell Biol.,* 1995, vol. 73, 532-536
- **KJER-NIELSEN.** *PNAS,* 2004, vol. 101, 7675-7680
- **SGRO.** *Toxicology,* 1995, vol. 105, 23-29
- **CHATENOUD.** *Clin. Transplant,* 1993, vol. 7, 422-430
- **CHATENOUD.** *Nat. Rev. Immunol.,* 2003, vol. 3, 123-132
- **KUMAR.** *Transplant. Proc.,* 1998, vol. 30, 1351-1352
- **UTSET.** *J. Rheumatol.,* 2002, vol. 29, 1907-1913
- **SMITH.** *J. Exp. Med.,* 1997, vol. 185, 1413-1422
- **VAN WAUVE.** *J. Immunol.,* 1980, vol. 124, 2708-18
- **WONG.** *Transplantation,* 1990, vol. 50, 683-9
- **TUNNACLIFFE.** *Int. Immunol.,* 1989, vol. 1, 546-50
- **SALMERON.** *J. Immunol.,* 1991, vol. 147, 3047-52
- **VON WUSSOW.** *J. Immunol.,* 1981, vol. 127, 1197
- **PALACIOUS.** *J. Immunol.,* 1982, vol. 128, 337
- **KUNICKA.** *Lymphocyte Typing II,* 1986, vol. 1, 223
- **LEEWENBERG.** *J. Immunol.,* 1985, vol. 134, 3770
- **PHILLIPS.** *J. Immunol.,* vol. 136, 1579
- **PLATSOUCAS.** *Proc. Natl. Acad. Sci. USA,* 1981, vol. 78, 4500
- **ITOH.** *Cell. Immunol.,* 1987, vol. 108, 283-96
- **MENTZER.** *J. Immunol.,* 1985, vol. 135, 34
- **LANDEGREN.** *J. Exp. Med.,* 1982, vol. 155, 1579
- **CHOI.** *Eur. J. Immunol.,* 2001, vol. 31, 94-106
- **XU.** *Cell Immunol.,* 2000, vol. 200, 16-26
- **KIMBALL.** *Transpl. Immunol.,* 1995, vol. 3, 212-221
- **SELA.** *Science,* 1969, vol. 166, 1365
- **LAVER.** *Cell,* 1990, vol. 61, 553-6
- **LAW.** *Int. Immunol.,* 2002, vol. 14, 389-400
- **COULIE.** *Eur. J. Immunol.,* 1991, vol. 21, 1703-9
- **RISUENO.** *Blood,* 2005, vol. 106, 601-8
- **SANDUSKY et al.** *J. Med. Primatol.,* 1986, vol. 15, 441-451
- **UDA et al.** *J. Med. Primatol.,* 2001, vol. 30, 141-147

- **UDA et al.** *J Med Primatol.,* 2003, vol. 32, 105-10
- **UDA et al.** *J Med Primatol.,* 2004, vol. 33, 34-7
- *Lancet,* 2006, vol. 368, 2206-7
- **WILDMAN et al.** *PNAS,* 2003, vol. 100, 7181
- **HARLOW ; LANE.** Antibodies, A Laboratory Manual. Cold Spring Harbor Laboratory Press, 1988
- **HARLOW ; LANE.** Using Antibodies: A Laboratory Manual. Cold Spring Harbor Laboratory Press, 1999
- **KÖHLER ; MILSTEIN.** *Nature,* 1975, vol. 256, 495-497
- **KOZBOR.** *Immunology Today,* 1983, vol. 4, 72
- **COLE et al.** Monoclonal Antibodies and Cancer Therapy. Alan R. Liss, Inc, 1985, 77-96
- **SCHIER.** *Human Antibodies Hybridomas,* 1996, vol. 7, 97-105
- **MALMBORG.** *J. Immunol. Methods,* 1995, vol. 183, 7-13
- **HARLOW ; LANE.** Antibodies: A Laboratory Manual. Cold Spring Harbor Laboratory Press, 1988
- Using Antibodies: A Laboratory Manual. Cold Spring Harbor Laboratory Press, 1999
- **SAMBROOK et al.** Molecular Cloning: A Laboratory Manual. Cold Spring Harbor Laboratory Press, 2001
- **ROONEY, C.M. et al.** Use of gene-modified virus-specific T lymphocytes to control Epstein-Barr-virus-related lymphoproliferation. *Lancet,* 1995, vol. 345 (8941), 9-13
- **WALTER, E.A. et al.** Reconstitution of cellular immunity against cytomegalovirus in recipients of allogeneic bone marrow by transfer of T-cell clones from the donor. *N Engl J Med,* 1995, vol. 333 (16), 1038-44
- **GORELICK, F.S. ; C. SHUGRUE.** Exiting the endoplasmic reticulum. *Mol Cell Endocrinol,* 2001, vol. 177, 13-8
- **CHU, C.M. ; Y.F. LIAW.** Membrane staining for hepatitis B surface antigen on hepatocytes: a sensitive and specific marker of active viral replication in hepatitis B. *J Clin Pathol,* 1995, vol. 48 (5), 470-3
- **MASIERO, S. et al.** T-cell engineering by a chimeric T-cell receptor with antibody-type specificity for the HIV-1 gp120. *Gene Ther,* 2005, vol. 12 (4), 299-310
- **MILSTEIN ; KÖHLER.** *Nature,* 1975, vol. 256, 495-7
- **LIU.** *Br. J. Cancer,* 2000, vol. 82 (12), 1991-1999
- **BONNER.** *Semin. Radiat. Oncol.,* 2002, vol. 12, 11-20
- **KIYOTA.** *Oncology,* 2002, vol. 63 (1), 92-98
- **KUAN.** *Brain Tumor Pathol.,* 2000, vol. 17 (2), 71-78
- **OLAYIOYE.** *EMBO J.,* 2000, vol. 19, 3159-67
- **MENDELSOHN.** *J. Clin. Oncol.,* 2003, vol. 21, 2787-99
- **MENDELSOHN.** *J. Clin. Oncol.,* 2002, vol. 20 (18), 1S-13S
- **PREWETT.** *Clin. Cancer Res.,* 2002, vol. 8, 994-1003
- **CIARDIELLO.** *Clin. Cancer Res.,* 2001, vol. 7, 2958-70
- **PEREZ-SOLER.** *Oncologist,* 2004, vol. 9, 58-67
- **LASKIN.** *Cancer Treat. Review,* 2004, vol. 30, 1-17
- **AUSUBEL.** Current Protocols in Molecular Biology. Green Publishing Associates and Wiley Interscience, 1989
- **MACK et al.** *PNAS,* 1995, vol. 92, 7021-7025
- **RAUM et al.** *Cancer Immunol Immunother,* 2001, vol. 50 (3), 141-150
- **SMITH.** *J. Virol.,* 1983, vol. 46, 584
- **ENGELHARD.** *Proc. Nat. Acad. Sci. USA,* 1994, vol. 91, 3224-3227
- **REISS.** *Plant Physiol. (Life Sci. Adv.,* 1994, vol. 13, 143-149
- **HERRERA-ESTRELLA.** *EMBO J.,* 1983, vol. 2, 987-995
- **MARSH.** *Gene,* 1984, vol. 32, 481-485
- **HARTMAN.** *Proc. Natl. Acad. Sci. USA,* 1988, vol. 85, 8047
- **MCCONLOGUE.** Current Communications in Molecular Biology. Cold Spring Harbor Laboratory, 1987
- **TAMURA.** *Biosci. Biotechnol. Biochem.,* 1995, vol. 59, 2336-2338
- **GIACOMIN.** *Pl. Sci.,* 1996, vol. 116, 59-72
- **SCIKANTHA.** *J. Bact.,* 1996, vol. 178, 121
- **GERDES.** *FEBS Lett.,* 1996, vol. 389, 44-47
- **JEFFERSON.** *EMBO J.,* 1987, vol. 6, 3901-3907
- **GIORDANO.** *Nature Medicine,* 1996, vol. 2, 534-539
- **SCHAPER.** *Circ. Res.,* 1996, vol. 79, 911-919
- **ANDERSON.** *Science,* 1992, vol. 256, 808-813
- **VERMA.** *Nature,* 1994, vol. 389, 239
- **ISNER.** *Lancet,* 1996, vol. 348, 370-374
- **MUHLHAUSER.** *Circ. Res.,* 1995, vol. 77, 1077-1086
- **ONODERA.** *Blood,* 1998, vol. 91, 30-36
- **VERMA.** *Gene Ther.,* 1998, vol. 5, 692-699
- **NABEL.** *Ann. N.Y. Acad. Sci.,* 1997, vol. 811, 289-292
- **VERZELETTI.** *Hum. Gene Ther.,* 1998, vol. 9, 2243-51
- **WANG.** *Nature Medicine,* 1996, vol. 2, 714-716
- **SCHAPER.** *Current Opinion in Biotechnology,* 1996, vol. 7, 635-640

- **NAGY.** *Proc. Natl. Acad. Sci. USA,* 1993, vol. 90, 8424-8428
- **KROOS.** *Biotechnol. Prog.,* 2003, vol. 19, 163-168
- **SCOPES.** Protein Purification. Springer-Verlag, 1982
- **SCHLERETH et al.** *Cancer Immunol. Immunother.,* 2005, vol. 20, 1-12
- **CHESON BD ; HORNING SJ ; COIFFIER B ; SHIPP MA ; FISHER RI ; CONNORS JM ; LISTER TA ; VOSE J ; GRILLO-LOPEZ A ; HAGENBEEK A.** Report of an international workshop to standardize response criteria for non-Hodgkin's lymphomas. NCI Sponsored International Working Group. *J Clin Oncol.,* April 1999, vol. 17 (4), 1244
- **HERMANSON.** Bioconjugate Techniques. Academic Press, Inc, 1996
- Phage Display: A Laboratory Manual. Cold Spring Harbor Laboratory Press, 2001
- **KNAPPIK et al.** *J Mol. Biol.,* 2000, vol. 296, 57 ff
- **HOLT et al.** *Trends Biotechnol.,* 2003, vol. 21, 484 ff
- **MUYLDERMANS.** *J Biotechnol.,* 2006, vol. 74, 277
- **DE GENST et al.** *Dev Como Immunol.,* 2006, vol. 30, 187 ff
- **GROVES ; OSBOUM.** *Expert Opin Biol Ther.,* 2005, vol. 5, 125 ff
- **LIPOVSEK ; PLUCKTHUN.** *J Immunol Methods,* 2004, vol. 290, 52 ff
- **LEFKOVITS.** Immunology Methods Manual; The Comprehensive Sourcebook of Techniques. Academic Press, 1997
- **GOLEMIS.** Protein-Protein Interactions: A Molecular Cloning Manual. Cold Spring Laboratory Press, 2002
- **MACK et al.** *Proc. Natl. Acad. Sci. USA,* 1995, vol. 92, 7021-7025
- **MACK et al.** *Proc. Natl. Acad. Sci. USA,* 1995, vol. 92, 7021-7025
- **RAUM et al.** *Cancer immunoi immunother.,* 2001, vol. 50 (3), 141-50
- **COLIGAN ; KRUISBEEK ; MARGULIES ; SHEVACH ; STROBER.** Current Protocols in Immunology. Wiley-Interscience, 2002
- **RAUM et al.** *Cancer Immunol. Immunother.,* 2001, vol. 50, 141-150
- **SAMBROOK.** Molecular Cloning; A Laboratory Manual. Cold Spring Harbour Laboratory Press, 2001
- **KAUFMANN R.J.** *Methods Enzymol.,* 1990, vol. 185, 537-566
- **RAUM et al.** *Cancer Immunol Immunother,* 2001, vol. 50, 141-150
- **KNAPPE A ; FICKENSCHER H. et al.** *Blood,* 2000, vol. 95, 3256-61
- **FICKENSCHER H.** *Blood,* 2000, vol. 95, 3256-61